(19) Europäisches Patentamt
European Patent Office
Office européen des brevets

(11) EP 4 316 520 A1

(12) EUROPEAN PATENT APPLICATION
published in accordance with Art. 153(4) EPC

(43) Date of publication:
**07.02.2024 Bulletin 2024/06**

(21) Application number: **22775851.3**

(22) Date of filing: **25.03.2022**

(51) International Patent Classification (IPC):
***A61K 45/00*** *(2006.01)* ***A61P 9/00*** *(2006.01)*
***A61K 9/70*** *(2006.01)* ***A61K 47/10*** *(2017.01)*
***A61K 47/34*** *(2017.01)* ***A61K 47/42*** *(2017.01)*
***A61K 38/46*** *(2006.01)* ***A61K 38/48*** *(2006.01)*
***A61K 31/4406*** *(2006.01)*

(52) Cooperative Patent Classification (CPC):
**A61K 9/70; A61K 31/4406; A61K 38/46; A61K 38/48; A61K 45/00; A61K 47/10; A61K 47/34; A61K 47/42; A61P 9/00**

(86) International application number:
**PCT/JP2022/014632**

(87) International publication number:
**WO 2022/203070 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2021 JP 2021052484**

(71) Applicants:
- **OSAKA UNIVERSITY**
  **Suita-shi**
  **Osaka 565-0871 (JP)**
- **Cuorips Inc.**
  **Tokyo 1030023 (JP)**

(72) Inventors:
- **SAWA, Yoshiki**
  **Suita-shi, Osaka 565-0871 (JP)**
- **MIYAGAWA, Shigeru**
  **Suita-shi, Osaka 565-0871 (JP)**
- **SAKAI, Yoshiki**
  **(deceased) (JP)**
- **YANAGI, Yasuhiro**
  **Kobe-shi, Hyogo 650-0047 (JP)**

(74) Representative: **Studio Torta S.p.A.**
**Via Viotti, 9**
**10121 Torino (IT)**

# (54) PHARMACEUTICAL COMPOSITION FOR IMPROVING CARDIAC FUNCTION

(57) An object is to provide a pharmaceutical composition that improves cardiac function when administered during coronary artery bypass surgery for ischemic cardiomyopathy. A pharmaceutical composition for improving cardiac function comprising: (A) a release formulation comprising at least poly(lactic-co-glycolic acid) (PLGA) and a prostaglandin I2 receptor agonist, the PLGA having an average molecular weight of 10000 to 30000; and (B) a release formulation comprising at least poly(lactic-co-glycolic acid) (PLGA) and a prostaglandin I2 receptor agonist, the PLGA having an average molecular weight of 40000 to 60000.

Fig. 2

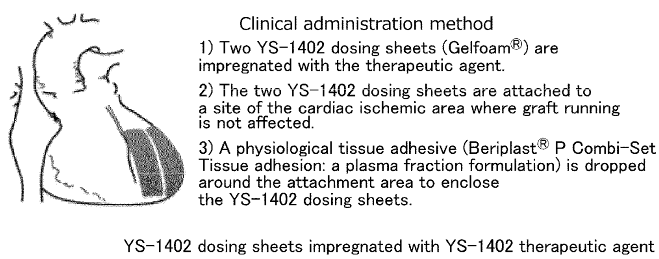


EP 4 316 520 A1

## Description

### Technical Field

The present disclosure relates to a pharmaceutical composition for improving cardiac function.

### Background Art

ONO-1301, which is the active ingredient of the pharmaceutical composition of the present disclosure, is a low-molecular-weight synthetic compound with a non-prostaglandin skeleton, and is a selective prostaglandin 12 receptor (IP receptor) agonist with thromboxane A2 (TXA2) synthase inhibitory activity. Initially, it was examined as an oral platelet aggregation inhibitor; however, its development was suspended because the results of its efficacy (platelet aggregation inhibition) and side effects (epigastric pain, fever, cold sweat, diarrhea, etc.) in Phase I clinical trials indicated that its safety margin was narrow.

ONO-1301 acts on vascular smooth muscle cells, platelets, vascular endothelial cells, and the like to inhibit platelet aggregation and dilate blood vessels; however, subsequent studies have shown that ONO-1301, at concentrations equal to or less than 1/20 of its platelet aggregation inhibitory effect, acts as an in vivo regenerative factor inducer (regeneration inducer) that newly acts on IP receptors on fibroblasts, smooth muscle cells, etc. to increase cyclic adenosine monophosphate (cAMP) and induce the production of various regenerative factors (hepatocyte growth factor (HGF), vascular endothelial growth factor (VEGF), stromal cell-derived factor (SDF-1), high-mobility group box 1 (HMGB1), etc.). These effects have led to the discovery that ONO-1301 exhibits angiogenic, anti-apoptotic, anti-fibrosis, anti-inflammatory, and bone marrow mesenchymal stem cell (MSC) mobilization and accumulation effects (drug repositioning). Furthermore, due to its TXA2 synthase inhibitory effect, ONO-1301 suppresses resistance to IP receptors even after long-term administration, and also promotes the production of endogenous prostaglandin E2 (PGE2) and prostaglandin I2 (PGI2).

### Summary of Invention

### Technical Problem

An object of the present disclosure is to provide a pharmaceutical composition that improves cardiac function when administered during coronary artery bypass surgery for ischemic cardiomyopathy.

More specifically, an object of the present invention is to provide a pharmaceutical composition that can retain the blood concentration of ONO-1301, which is the active ingredient, within a certain concentration range for a certain period of time, that can exhibit a cardiac function improvement effect, and that has confirmed clinical safety and tolerability, by producing a sustained-release formulation of microspheres (MS), such as compound (A), which is a PGI2 receptor agonist.

### Solution to Problem

As a result of various considerations regarding the production of MS sustained-release formulations containing a PGI2 receptor agonist, the present inventors found that by mixing PGI2 receptor agonist-containing release formulations each containing poly(lactic-co-glycolic acid) (PLGA) with a different average molecular weight, the PGI2 receptor agonist is released within a certain concentration range for a certain period of time.

The present inventors found that the approved drugs YS-1402-Gelfoam and YS-1402-Beriplast are optimal for the attachment of the pharmaceutical composition of the present invention to the heart during coronary artery bypass surgery for ischemic cardiomyopathy.

The present invention has been completed through further trial and error based on these findings, and includes the following invention.

Item 1.
A pharmaceutical composition for improving cardiac function, comprising:

(A) a release formulation comprising at least poly(lactic-co-glycolic acid) (PLGA) and a prostaglandin 12 receptor agonist, the PLGA having an average molecular weight of 1000 to 30000; and
(B) a release formulation comprising at least poly(lactic-co-glycolic acid) (PLGA) and a prostaglandin 12 receptor agonist, the PLGA having an average molecular weight of 40000 to 60000.

Item 2.
The pharmaceutical composition for improving cardiac function according to Item 1, wherein the ratio of the release formulation (B) to the release formulation (A) (A:B) is 1:1 to 100:1 or 1:1 to 1:100.
Item 3.
The pharmaceutical composition for improving cardiac function according to Item 1 or 2, wherein the release formulation (A) comprises 0.5 to 50 mg of PGI2 receptor agonist in one vial, and/or the release formulation (B) comprises 0.5 to 50 mg of PGI2 receptor agonist in one vial.
Item 4.
The pharmaceutical composition according to any one of Items 1 to 3, comprising a patch liquid.
Item 5.
The pharmaceutical composition according to Item 4, wherein the patch liquid is a 5 w/v% mannitol aqueous solution comprising 0.2 w/v% of polysorbate.
Item 6.
The pharmaceutical composition according to any one of Items 1 to 5, comprising a gelatin patch.
Item 7.
The pharmaceutical composition according to Item 6, wherein the gelatin patch is a porous sterile formulation comprising 10 g of gelatin per 1000 $cm^3$.
Item 8.
The pharmaceutical composition according to any one of Items 1 to 7, comprising a plasma fraction formulation.
Item 9.
The pharmaceutical composition according to Item 8, wherein the plasma fraction formulation comprises a fibrinogen powder, an aprotinin solution, a thrombin powder, and a calcium chloride solution.
Item 10.
The pharmaceutical composition according to any one of Items 1 to 9, comprising, as the prostaglandin I2 receptor agonist, at least a compound of the following formula (I) or a salt thereof:

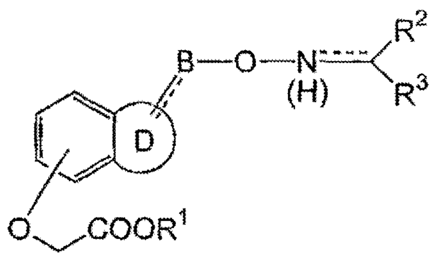 (I)

wherein

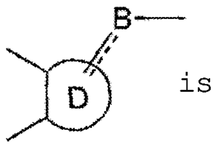 is

(i) 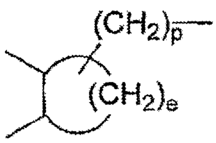 , (ii) 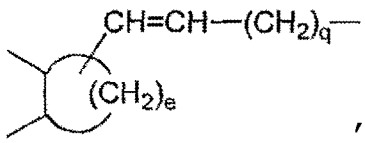 ,

(iii) 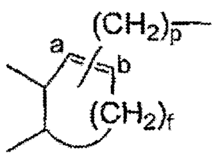 , or (iv) 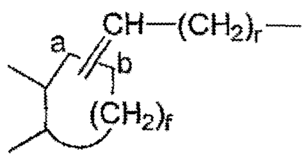 ,

wherein

$R^1$ represents a hydrogen atom or a C1-4 alkyl group,
$R^2$ represents (i) a hydrogen atom, (ii) a C1-8 alkyl group, (iii) a phenyl group or a C4-7 cycloalkyl group, (iv) a 4-to 7-membered monocyclic ring containing one nitrogen atom, (v) a C1-4 alkyl group substituted with a benzene ring or a C4-7 cycloalkyl group, or (vi) a C1-4 alkyl group substituted with a 4- to 7-membered monocyclic ring containing one nitrogen atom,
$R^3$ represents (i) a C1-8 alkyl group, (ii) a phenyl group or a C4-7 cycloalkyl group, (iii) a 4- to 7-membered monocyclic ring containing one nitrogen atom, (iv) a C1-4 alkyl group substituted with a benzene ring or a C4-7 cycloalkyl group, or (v) a C1-4 alkyl group substituted with a 4- to 7-membered monocyclic ring containing one nitrogen atom,
e represents an integer of 3 to 5,
f represents an integer of 1 to 3,
p represents an integer of 1 to 4,
q represents 1 or 2, and
r represents an integer of 1 to 3;

provided that when

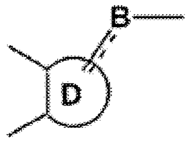

is a group represented by (iii) or (iv) above,

$-(CH_2)p-$ and $=CH-(CH_2)s-$ bind to the position of a or b on the ring, and
ring structures in $R^2$ and $R^3$ are optionally substituted with 1 to 3 C1-4 alkyl groups, C1-4 alkoxy groups, halogen atoms, nitro groups, or trihalomethyl groups.

Item 11.
The pharmaceutical composition according to any one of Items 1 to 10, comprising, as the prostaglandin I2 receptor agonist, at least the following compound (A) or a salt thereof:

(A) ({5-[2-({[(1E)-phenyl(pyridin-3-yl)methylene]amino}oxy)ethyl]-7,8-dihydronaphthalen-1-yl}oxy)acetic acid (ONO-1301) represented by the following formula (II):

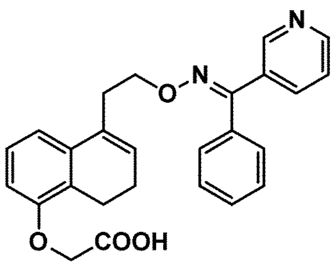
(II)

Item 12.
The pharmaceutical composition according to any one of Items 1 to 11, wherein the pharmaceutical composition is a sheet patch.
Item 13.
The pharmaceutical composition according to any one of Items 1 to 12, wherein the pharmaceutical composition is administered to a patient with ischemic cardiomyopathy who undergoes coronary artery bypass surgery.
Item 14.
The pharmaceutical composition according to any one of Items 1 to 13, wherein the prostaglandin I2 receptor agonist is released over 4 weeks after administration.
Item 15.
The pharmaceutical composition according to any one of Items 1 to 14, which is a sustained-release formulation of microspheres (MS).

Item 16.
The pharmaceutical composition according to Item 15, wherein the sustained-release formulation has an average particle size of 3 to 300 pm.

## Advantageous Effects of Invention

The pharmaceutical composition of the present disclosure is useful to improve cardiac function because the blood concentration of ONO-1301, which is the active ingredient, remains within a certain concentration range for a certain period of time.

## Brief Description of Drawings

Fig. 1 is a schematic view of the preparation of the YS-1402 dosing sheet of the present invention for 30 mg dosing.
Fig. 2 is a schematic view of an administration method of the YS-1402 dosing sheet by heart attachment.
Fig. 3 is a view showing the breakdown of subjects of this trial.
Fig. 4 is a chart of time-dependent transition of blood ONO-1301 concentration when YS-1402-10 mg, 30 mg, or 100 mg was attached once at the time of thoracotomy for coronary artery bypass surgery. [Horizontal axis (time: equally spaced at each blood collection point), vertical axis (blood drug concentration: real number)]
Fig. 5 is a chart of time-dependent transition of blood ONO-1301 concentration when YS-1402-10 mg, 30 mg, or 100 mg was attached once at the time of thoracotomy for coronary artery bypass surgery. [Horizontal axis (time: proportional to the actual time), vertical axis (blood drug concentration: real number)]
Fig. 6 is a chart of time-dependent transition of blood ONO-1301 concentration when YS-1402-10 mg, 30 mg, or 100 mg was attached once at the time of thoracotomy for coronary artery bypass surgery. [Horizontal axis (time: proportional to the actual time), vertical axis (blood drug concentration: logarithm)]
Fig. 7 is a chart of time-dependent transition of measured LVEF values in echocardiography when YS-1402-10 mg, 30 mg, or 100 mg was attached once at the time of thoracotomy for coronary artery bypass surgery.
Fig. 8 is a chart of time-dependent transition of the amount of change in LVEF in echocardiography when YS-1402-10 mg, 30 mg, or 100 mg was attached once at the time of thoracotomy for coronary artery bypass surgery.
Fig. 9 is a view showing the amount of change in LVEF from the baseline at 26 weeks after administration.
Fig. 10 is a view showing the correlation between the rate of change in total myocardial blood flow and the amount of change in LVEF from the baseline at 26 weeks after administration of the investigational drug.
Fig. 11 is a chart of time-dependent transition of measured CI values in cardiac-gated CT.
Fig. 12 is a chart of time-dependent transition of the rate of change in CI in cardiac-gated CT.
Fig. 13 is a view showing the rate of change in CI in cardiac-gated CT from the baseline at 26 weeks after administration.
Fig. 14 is a view showing the correlation between the rate of change in total myocardial blood flow and the rate of change in CI from the baseline at 26 weeks after administration of the investigational drug.
Fig. 15 is a chart of time-dependent transition of measured LVESVI values in cardiac-gated CT.
Fig. 16 is a chart of time-dependent transition of the rate of change in LVESVI in cardiac-gated CT.
Fig. 17 is a chart of time-dependent transition of measured LVEDVI values in cardiac-gated CT.
Fig. 18 is a chart of time-dependent transition of the rate of change in LVEDVI in cardiac-gated CT.
Fig. 19 is a chart of time-dependent transition of measured LVDs values in echocardiography.
Fig. 20 is a chart of time-dependent transition of the rate of change in LVDs in echocardiography.
Fig. 21 is a chart of time-dependent transition of measured LVDd values in echocardiography.
Fig. 22 is a chart of time-dependent transition of the rate of change in LVDd in echocardiography.
Fig. 23 is a chart of time-dependent transition of measured CTR values in chest X-ray.
Fig. 24 is a chart of time-dependent transition of the amount of change in CTR in chest X-ray.
Fig. 25 shows charts of time-dependent transition of the NYHA classification. Percentages were calculated using a population of six cases. The basic number of subjects to be evaluated was six; however, the number of subjects was five at 26 weeks after administration in the placebo group and the YS-1402 100 mg group because one case in each group discontinued during the course of treatment, and one case in the 30 mg group had right hemiparesis and could not be measured. Thus, there were five cases at all measurement points.
Fig. 26 is a chart of time-dependent transition of measured values of 6-minute walking distance.
Fig. 27 is a chart of time-dependent transition of the rate of change in 6-minute walking distance.
Fig. 28 is a view showing the rate of change in 6-minute walking distance from the baseline at 26 weeks after administration.

Fig. 29 is a view showing the rate of change in 6-minute walking distance from the baseline when excluding a case of congestive heart failure due to poor compliance in the YS-1402 30 mg group.

Fig. 30A is a chart of time-dependent transition of measured values of RCA resting myocardial blood flow by ammonia PET.

Fig. 30B is a chart of time-dependent transition of measured values of LAD resting myocardial blood flow by ammonia PET.

Fig. 30C is a chart of time-dependent transition of measured values of LCX resting myocardial blood flow by ammonia PET.

Fig. 30D is a chart of time-dependent transition of measured values of total myocardial blood flow by ammonia PET.

Fig. 31A is a chart of time-dependent transition of the rate of change in RCA resting myocardial blood flow from the baseline by ammonia PET.

Fig. 31B is a chart of time-dependent transition of the rate of change in LAD resting myocardial blood flow from the baseline by ammonia PET.

Fig. 31C is a chart of time-dependent transition of the rate of change in LCX resting myocardial blood flow from the baseline by ammonia PET.

Fig. 31D is a chart of time-dependent transition of the rate of change in total myocardial blood flow from the baseline by ammonia PET.

Fig. 32 is a view showing the rate of change in total myocardial blood flow from the baseline at 26 weeks after administration.

Fig. 33 is a view showing the rate of change in LAD resting myocardial blood flow from the baseline at 26 weeks after administration.

Fig. 34 is a view showing the correlation between the blood concentration (AUC0-t) and the rate of change in total myocardial blood flow from the baseline at 26 weeks after administration of the investigational drug.

Fig. 35 is a view showing the correlation between the blood concentration (Cmax) and the rate of change in total myocardial blood flow from the baseline at 26 weeks after administration of the investigational drug.

Fig. 36 is a view showing the correlation between AUC0-t and the rate of change in LAD resting myocardial blood flow from the baseline at 26 weeks after administration of the investigational drug.

Fig. 37 is a chart of time-dependent transition of measured blood BNP concentration values.

Fig. 38 is a chart of time-dependent transition of the rate of change in blood BNP concentration.

Fig. 39A is a chart of time-dependent transition of measured score values of the SF-36 subscale [physical functioning] set for QOL assessment.

Fig. 39B is a chart of time-dependent transition of measured score values of the SF-36 subscale [role physical] set for QOL assessment.

Fig. 39C is a chart of time-dependent transition of measured score values of the SF-36 subscale [bodily pain] set for QOL assessment.

Fig. 39D is a chart of time-dependent transition of measured score values of the SF-36 subscale [general health] set for QOL assessment.

Fig. 39E is a chart of time-dependent transition of measured score values of the SF-36 subscale [vitality] set for QOL assessment.

Fig. 39F is a chart of time-dependent transition of measured score values of the SF-36 subscale [social functioning] set for QOL assessment.

Fig. 39G is a chart of time-dependent transition of measured score values of the SF-36 subscale [role emotional] set for QOL assessment.

Fig. 39H is a chart of time-dependent transition of measured score values of the SF-36 subscale [mental health] set for QOL assessment.

Fig. 40A is a chart of time-dependent transition of the amount of change in the scores of the SF-36 subscale [physical functioning] set for QOL assessment.

Fig. 40B is a chart of time-dependent transition of the amount of change in the scores of the SF-36 subscale [role physical] set for QOL assessment.

Fig. 40C is a chart of time-dependent transition of the amount of change in the scores of the SF-36 subscale [bodily pain] set for QOL assessment.

Fig. 40D is a chart of time-dependent transition of the amount of change in the scores of the SF-36 subscale [general health] set for QOL assessment.

Fig. 40E is a chart of time-dependent transition of the amount of change in the scores of the SF-36 subscale [vitality] set for QOL assessment.

Fig. 40F is a chart of time-dependent transition of the amount of change in the scores of the SF-36 subscale [social functioning] set for QOL assessment.

Fig. 40G is a chart of time-dependent transition of the amount of change in the scores of the SF-36 subscale [role

emotional] set for QOL assessment.

Fig. 40H is a chart of time-dependent transition of the amount of change in the scores of the SF-36 subscale [mental health] set for QOL assessment.

Fig. 41 show views showing the evaluation of the relationship (FAS) between echocardiography (LVEF) and cardiac-gated CT (LVESVI).

Description of Embodiments

1. Pharmaceutical composition for improving cardiac function

The pharmaceutical composition for improving cardiac function of the present invention comprises:

a release formulation (A) comprising poly(lactic-co-glycolic acid) (PLGA) having an average molecular weight of 10000 to 30000 and a prostaglandin I2 (PGI2) receptor agonist;
a release formulation (B) comprising poly(lactic-co-glycolic acid) (PLGA) having an average molecular weight of 40000 to 60000 and a prostaglandin I2 (PGI2) receptor agonist;
a patch liquid;
a gelatin patch; and
a plasma fraction formulation.

1.1. Release formulation comprising poly(lactic-co-glycolic acid) (PLGA) and prostaglandin I2 (PGI2) receptor agonist

The pharmaceutical composition for improving cardiac function of the present invention comprises release formulations (A) and (B) comprising two types of PLGA having different average molecular weights, and a PGI2 receptor agonist.

The pharmaceutical composition for improving cardiac function of the present invention is prepared (suspended) before use using two types of release formulations (A) and (B). The release formulation (A) is a sterile formulation produced by using PLGA having an average molecular weight of 10000 to 30000, and is a two-week release formulation containing 0.5 to 50 mg of PGI2 receptor agonist in one vial. The release formulation (B) is a sterile formulation produced by using PLGA having an average molecular weight of 40000 to 60000, and is a four-week release formulation containing 0.5 to 50 mg of PGI2 receptor agonist in one vial.

The "average molecular weight" may be any molecular weight, including weight average molecular weight and number average molecular weight.

In the pharmaceutical composition for improving cardiac function of the present invention, the content ratio of the two types of release formulations (A) and (B) (A:B) is not particularly limited, but is preferably 1:1 to 100:1 or 1:1 to 1:100, and more preferably 1:1.

The contents of PLGA and the PGI2 receptor agonist are not particularly limited; however, it is preferable to contain 1 to 100%, preferably 5 to 90%, of the PGI2 receptor agonist relative to PLGA1 in the total amount of the sustained-release formulation of the present invention.

The PGI2 receptor agonist used in the pharmaceutical composition for improving cardiac function of the present invention is not particularly limited, and known PGI2 receptor agonists can be preferably used. Examples of known PGI2 receptor agonists include pharmaceutical compositions, PGI2 derivatives, and PGE derivatives, which are compounds represented by formula (I) or salts thereof:

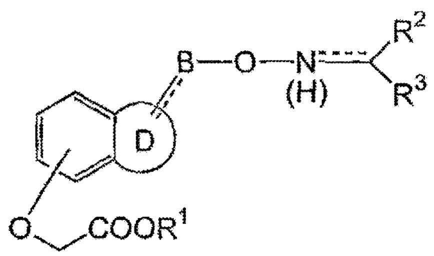

(I)

wherein

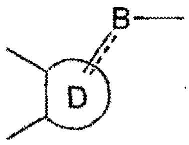

is

(i) 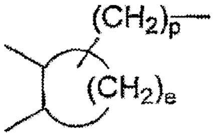 , (ii) 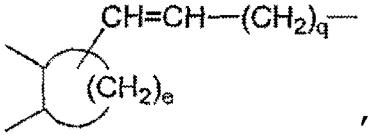 ,

(iii) 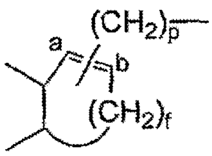 , or (iv) 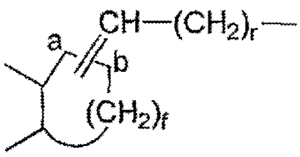 ,

wherein

$R^1$ represents a hydrogen atom or a C1-4 alkyl group,
$R^2$ represents (i) a hydrogen atom, (ii) a C1-8 alkyl group, (iii) a phenyl group or a C4-7 cycloalkyl group, (iv) a 4-to 7-membered monocyclic ring containing one nitrogen atom, (v) a C1-4 alkyl group substituted with a benzene ring or a C4-7 cycloalkyl group, or (vi) a C1-4 alkyl group substituted with a 4- to 7-membered monocyclic ring containing one nitrogen atom,
$R^3$ represents (i) a C1-8 alkyl group, (ii) a phenyl group or a C4-7 cycloalkyl group, (iii) a 4- to 7-membered monocyclic ring containing one nitrogen atom, (iv) a C1-4 alkyl group substituted with a benzene ring or a C4-7 cycloalkyl group, or (v) a C1-4 alkyl group substituted with a 4- to 7-membered monocyclic ring containing one nitrogen atom,
e represents an integer of 3 to 5,
f represents an integer of 1 to 3,
p represents an integer of 1 to 4,
q represents 1 or 2, and
r represents an integer of 1 to 3;

provided that when

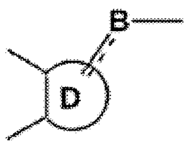

is a group represented by (iii) or (iv) above,

$-(CH_2)p-$ and $=CH-(CH_2)s-$ bind to the position of a or b on the ring, and
ring structures in $R^2$ and $R^3$ are optionally substituted with 1 to 3 C1-4 alkyl groups, C1-4 alkoxy groups, halogen atoms, nitro groups, or trihalomethyl groups.

Preferably, the PGI2 receptor agonist is:

(A) ({5-[2-({[(IE)-phenyl(pyridin-3-yl)methylene]amino}oxy)ethyl]-7,8-dihydronaphthalen-1-yl}oxy)acetic acid represented by the following formula (II):

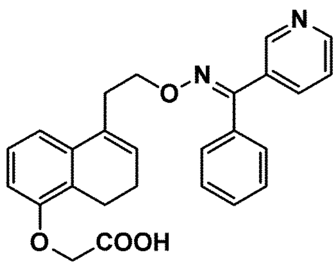

(II)

(CAS 176391-41-6; compound (A) (ONO-1301));

(B) a carbacyclin-based PGI2 derivative, including (±)-(1R,2R,3aS,8bS)-2,3,3a,8b-tetrahydro-2-hydroxy-1-[(E)-(3S,4RS)-3-hydroxy-4-methyl-1-octen-6-ynyl]-1H-cyclopenta[b]benzofuran-5-butanoic acid sodium salt (CAS: 88475-69-8; beraprost) etc. (compound (B));

(C) [4-(5,6-diphenylpyrazinyl)(1-methylethyl)amino]butoxy]-acetic acid (CAS: 475085-57-5; MRE-269; compound (C));

(D) a PGE derivative, including (2E)-7{-(1R,2R,3R)-3-hydroxy-2[-(1E,3S,5S)-3-hydroxy-5-methylnon-1-en-1-yl]-5-oxocyclopentyl}-hept-2-enoic acid (CAS: 74397-12-9; limaprost), ornoprostyl; 17S,20-dimethyl-6-oxo-PGE1 methyl ester, enprostyl, misoprostol, etc. (compound (D)); or

(E) 2-{4-[(5,6-diphenylpyrazin-2-)yl)(propan-2-yl)amino]butoxy}-N-(methanesulfonyl)acetamide (CAS: 475086-01-2; selexipag; NS-304 (compound (E)).

1.2. Patch liquid

The pharmaceutical composition for improving cardiac function of the present invention comprises a patch liquid for use in preparation before use of the two types of release formulations (A) and (B).

The patch liquid is preferably a 5 w/v% mannitol aqueous solution containing 0.2 w/v% of polysorbate.

1.3. Gelatin patch

The pharmaceutical composition for improving cardiac function of the present invention comprises a gelatin patch for use in the production of a dosing sheet of the pharmaceutical composition for improving cardiac function of the present invention.

As the gelatin patch, it is preferable to use the commercial product YS-1402-Gelfoam (registered trademark, Pfizer Japan Inc.). YS-1402-Gelfoam is a spongy sheet agent obtained by blowing bubbles into Japanese Pharmacopoeia gelatin, and is a white porous sterile formulation containing 10 g of Japanese Pharmacopoeia gelatin per 1000 $cm^3$. Although YS-1402-Gelfoam is an approved drug, the use thereof in the present invention is off-label use.

1.4. Plasma fraction formulation

The pharmaceutical composition for improving cardiac function of the present invention comprises a plasma fraction formulation as the physiological tissue adhesive of the present invention in order to prevent the dosing sheet of the pharmaceutical composition for improving cardiac function of the present invention applied to the heart from detaching from the heart.

As the plasma fraction formulation, it is preferable to use YS-1402-Beriplast ((registered trademark) P Combi-Set Tissue adhesion: a plasma fraction formulation, CSL Behring). YS-1402-Beriplast contains a fibrinogen powder, an aprotinin solution, a thrombin powder, and a calcium chloride solution. YS-1402-Beriplast is used by dropping around the attachment site of the dosing sheet of the sustained-release formulation of the present invention. Although YS-1402-Beriplast is an approved drug, the use thereof in the present invention is off-label use.

YS-1402-Beriplast is 1 set for 3 ml formulation, and there are vials 1 to 4. Vial 1 contains 240 mg of fibrinogen and human blood coagulation factor XIII (180 international units), vial 2 contains an aprotinin solution (3000 KIE), vial 3 contains Japanese Pharmacopoeia thrombin (900 units), and vial 4 contains Japanese Pharmacopoeia calcium chloride hydrate (17.64 mg). Vials 1 and 2 are mixed to form liquid A, and vials 3 and 4 are mixed to form liquid B. Liquids A and B are mixed to form a fibrin glue.

1.5. Other components

The pharmaceutical composition for improving cardiac function of the present invention may contain components

other those mentioned above as long as the effects of the present invention are not impaired.

2. Form, preparation method, and clinical administration method of pharmaceutical composition for improving cardiac function

The pharmaceutical composition for improving cardiac function of the present invention is a sustained-release formulation of microspheres (MS).

Although it is not particularly limited, the sustained-release formulation of the present invention preferably has an average particle size of about 3 to 300 μm, more preferably about 5 to 200, and even more preferably about 10 to 100. In the present specification, the "particle size" refers to the diameter of particles measured by any method including a laser diffraction method. The method for adjusting the particle size is not particularly limited.

The form of the pharmaceutical composition for improving cardiac function of the present invention is not particularly limited, but is preferably a sheet-like MS sustained-release formulation.

The method for preparing the dosing sheet of the pharmaceutical composition for improving cardiac function of the present invention comprises the following steps:

(a) suspending two types of release formulations (A) and (B) using a patch liquid; and
(b) dropping the suspension obtained in step (a) on YS-1402-Gelfoam to form a dosing sheet.

In step (a), hydrolysis starts when the patch liquid is added, and the release of a PGI2 receptor agonist, which is the active ingredient, starts. Therefore, administration by heart attachment is performed within, as a guideline, 6 hours after the start of the preparation of the dosing sheet. In order to prevent hydrolysis, the dosing sheet is kept refrigerated in a sterile petri dish.

The method for clinically administering the dosing sheet of the pharmaceutical composition for improving cardiac function of the present invention to the heart comprises the following steps:

(c) attaching the dosing sheet obtained in step (b) to a site of the cardiac ischemic area where graft running is not affected; and
(d) dropping YS-1402-Beriplast around the attachment area to enclose the dosing sheet obtained in step (b).

It is preferable that the pharmaceutical composition for improving cardiac function of the present invention is administered at the end of coronary artery bypass surgery, and that the PGI2 receptor agonist is released over 4 weeks after administration.

In the present disclosure, the phrase "released over 4 weeks" means that the blood concentration of the PGI2 receptor agonist, which is the active ingredient, remains within a certain concentration range over 4 weeks after administration of the pharmaceutical composition for improving cardiac function of the present invention.

The dose of the pharmaceutical composition for improving cardiac function of the present invention depends on the severity of the symptom to be treated, but is preferably an amount in which the dose of the PGI2 receptor agonist, which is the active ingredient contained therein, is 1000 mg or less.

Examples

The present invention is described below with reference to Examples; however, the present invention is not limited to these Examples and the like.

Preparation of YS-1402 dosing sheets and placebo formulation

Dosing sheets of the cardiac function-improving composition of the present invention (hereinafter referred to as "YS-1402") with an ONO-1301 content of 10 mg, 30 mg, or 100 mg, and a placebo formulation not containing ONO-1301 were prepared (Groups 1 to 3).

Group 1: YS-1402 (ONO-1301 content: 10 mg) and placebo groups
Group 2: YS-1402 (ONO-1301 content: 30 mg) and placebo groups
Group 3: YS-1402 (ONO-1301 content: 100 mg) and placebo groups

The YS-1402 dosing sheets with an ONO-1301 content of 10 mg, 30 mg, or 100 mg, and the placebo formulation can be produced, for example, in the following manner. The formulations shown in Table 1 were used for the preparation.

Table 1

| Investigational drug name | Content, dosage form | Production code/ number |
|---|---|---|
| YS-1402 | A sustained-release formulation of microspheres (MS) containing about 15% of ONO-1301 relative to two different molecular weights (20,000 and 50,000) of poly(lactic-co-glycolic acid). This is prepared (suspended) before use using YS-4102-1 and YS-4102-2. | 10 mg (active, placebo) groups: J561P<br>30 mg (active, placebo) groups: J562P<br>100 mg (active, placebo) groups: J791P |
| YS-1402-1 | A sterile formulation produced by using poly(lactic-co-glycolic acid) having a molecular weight of about 20,000 and containing 5 mg of ONO-1301 in one vial. YS-1402-1 is a two-week release formulation | |
| YS-1402-2 | A sterile formulation produced by using poly(lactic-co-glycolic acid) having a molecular weight of about 50,000 and containing 5 mg of ONO-1301 in one vial. YS-1402-2 is a four-week release formulation. | |
| Patch liquid | A sterile formulation containing a solution for suspension before use of YS-1402-1 and YS-1402-2 (component: a 5 w/v% mannitol aqueous solution containing 0.2 w/v% of polysorbate) | J563P<br>J792P<br>J861P |
| YS-1402-Gelfoam | A white porous sterile formulation containing 10 g of Japanese Pharmacopoeia gelatin per 1,000 $cm^3$ | HE479<br>JU497<br>KK507 |
| YS-1402-Beriplast | Fibrinogen powder: white lump lyophilizer<br>Aprotinin solution: colorless transparent liquid<br>Thrombin powder: white to pale yellow amorphous substance<br>Calcium chloride solution: colorless transparent liquid | 609205A<br>609226A<br>609230A<br>609291A<br>609331A<br>609346A<br>609400A<br>609437A<br>609459A<br>P100042737 |

Preparation of YS-1402 dosing sheet containing 10 mg of ONO-1301

An patch liquid is collected using a syringe fitted with a 20G or larger needle. The needle is removed from the syringe collecting the patch liquid, and a needle of the same size is attached. This is used to add the patch liquid to a vial of YS-1402-2. After stirring thoroughly and collecting the liquid inside the vial, the syringe is removed from the needle. The same liquid is placed in a vial of YS-1402-1, after stirring thoroughly, the liquid inside the vial is collected, and the syringe is removed from the needle. The syringe is connected to a three-way stopcock. A new syringe is taken out to collect the patch liquid via the needle inserted into the vial of the patch liquid, each vial is then washed in turn, and the second syringe is attached to the three-way stopcock to which the first syringe is connected. The liquids collected in the syringes are mixed without foaming via the three-way stopcock. The mixture is collected in one syringe and evenly added to two sheets of YS-1402-Gelfoam. After adding the suspension, YS-1402-Beriplast is dropped to fix and hide MS powder. After dropping, the sheets are attached to the heart as soon as possible.

Preparation of YS-1402 dosing sheet containing 30 mg of ONO-1301

Fig. 1 shows a schematic view of the preparation of a YS-1402 dosing sheet containing 30 mg of ONO-1301. An patch liquid is collected using a syringe fitted with a 20G or larger needle. The needle is removed from the syringe collecting the patch liquid, and a needle of the same size is attached. This is used to add the patch liquid to a vial of YS-1402-2. After stirring thoroughly and collecting the liquid inside the vial, the syringe is removed from the needle. A new needle is fitted thereto, and the same liquid is placed in a vial of YS-1402-2, after stirring thoroughly, the liquid inside the vial is collected, and the syringe is removed from the needle. This operation is performed for 3 vials of YS-1402-2

and 3 vials of YS-1402-1, and the syringe is connected to a three-way stopcock. A new syringe is taken out to collect the patch liquid via the needle inserted into the vial of the patch liquid, each vial is then washed in turn, and the second syringe is attached to the three-way stopcock to which the first syringe is connected. The liquids collected in the syringes are mixed without foaming via the three-way stopcock. The mixture is collected in one syringe and evenly added to two sheets of YS-1402-Gelfoam. After adding the suspension, a physiological tissue adhesive (YS-1402-Beriplast) is dropped to fix and hide MS powder. After dropping, the sheets are attached to the heart as soon as possible.

Preparation of YS-1402 dosing sheet containing 100 mg of ONO-1301

An patch liquid is collected using a syringe fitted with a 20G or larger needle. The needle is removed from the syringe collecting the patch liquid, and a needle of the same size is attached. This is used to add the patch liquid to a vial of YS-1402-2. After stirring thoroughly and collecting the liquid inside the vial, the syringe is removed from the needle. A new needle is fitted thereto, and the same liquid is placed in a vial of YS-1402-2, after stirring thoroughly, the liquid inside the vial is collected, and the syringe is removed from the needle. This operation is performed for 10 vials of YS-1402-2 and 10 vials of YS-1402-1, and the syringe is connected to a three-way stopcock. A new syringe is taken out to collect the patch liquid via the needle inserted into the vial of the patch liquid, each vial is then washed in turn, and the second syringe is attached to the three-way stopcock to which the first syringe is connected. The liquids collected in the syringes are mixed without foaming via the three-way stopcock. The mixture is collected in one syringe and evenly added to two sheets of YS-1402-Gelfoam. After adding the suspension, a physiological tissue adhesive (YS-1402-Beriplast) is dropped to fix and hide MS powder. After dropping, the sheets are attached to the heart as soon as possible.

Preparation of placebo formulation

After adding the patch liquid evenly to two similar sheets of YS-1402-Gelfoam, a physiological tissue adhesive (YS-1402-Beriplast) is dropped to hide the addition part. After dropping, the sheets are attached to the heart as soon as possible.

Selection of administration site and administration method

YS-1402 dosing sheets were attached to the left ventricle at the end of coronary artery bypass surgery through thoracotomy (Fig. 2).

For the site of attachment, the area of reduced myocardial blood flow was identified in advance by preoperative ammonia positron emission tomography (ammonia PET). Lesion sites such as highly fibrotic regions and poor contraction regions in complex lesions and multivessel lesions where complete blood flow recovery to cardiac ischemia was difficult were also identified visually and tactilely in the coronary artery bypass surgery, and two YS-1402 dosing sheets were attached to the site, including the peripheral part, where graft running was not affected.

Dosing frequency

Since the investigational drug was administered at the time of thoracotomy for coronary artery bypass surgery, the dosing frequency was once.

Post-dose treatment

In order to prevent the two YS-1402 dosing sheets attached to the heart from detaching from the heart, YS-1402-Beriplast was dropped around the area where the YS-1402 dosing sheets were attached, to enclose the YS-1402 dosing sheets, and the chest was closed.

Identification of therapeutic agents

YS-1402 is a sustained-release formulation of MS with a particle size of about 30 pm (average) containing about 15% of ONO-1301 relative to two different molecular weights (20000 and 50000) of poly(lactic-co-glycolic acid) (1:1). The formulation is designed so that the blood concentration of ONO-1301 remains within a certain concentration range over about 4 weeks after administration.

Evaluation of safety and tolerability

The safety and tolerability were comprehensively evaluated for the following items 1) to 5).

1) Adverse events

This item was set to evaluate safety and tolerability when YS-1402 was attached to the heart. The type, severity, seriousness, occurrence frequency, and occurrence period of adverse events were checked.

2) General clinical examination

This item was set to evaluate the overall safety when YS-1402 was attached to the heart (Table 2).

(1) Hematology test
(2) Blood biochemistry test
(3) Blood coagulation system test

3) Clinical symptoms

This item was set to evaluate the overall safety when YS-1402 was attached to the heart.

(1) Vital signs: blood pressure (diastolic, systolic), heart rate, respiratory rate, body temperature (armpit)
(2) Subjective symptoms: In particular, the presence or absence of diarrhea and heaviness of the head was checked.
(3) Physical findings: The presence or absence of wet rales, edema, and extra heart sounds was checked.

4) Resting standard 12-lead electrocardiography and Holter electrocardiography

This item was set to check the presence or absence of arrhythmia and myocardial ischemia. The presence or absence of arrhythmia, abnormal Q waves (12-lead electrocardiography only), and abnormal findings was checked.

5) Presence or absence and degree of bleeding after attachment (post-operation)

The presence or absence and degree of postoperative bleeding after heart attachment were evaluated according to the BARC definition for bleeding, which is often used to evaluate bleeding after coronary artery bypass surgery. The BARC definition for bleeding is shown in Table 3.

Table 2

| Test item | Measurement item | Basis for setting |
|---|---|---|
| Hematology test | White blood cells, red blood cells, hemoglobin, hematocrit, platelets, differential white blood cells (Neu, Ly, Mo, Eo, Ba) | Indicators of blood disorders and anemia |
| Blood biochemistry test | AST(GOT), ALT(GPT), LDH, ALP<br>BUN, creatinine<br>Blood sugar<br><br>Electrolytes (Na, K, Cl)<br><br>CK, CK einzyme<br>Uric acid<br><br>TG, T-Cho, LDL-Cho,<br><br>Albumin<br>CRP | Indicators of liver damage<br>Indicators of renal damage<br>Indicator of abnormal glucose metabolism<br>Indicators of electrolyte abnormality<br>Indicators of myocardial necrosis<br>Indicator of abnormal nucleic acid metabolism<br>Indicators of abnormal lipid metabolism<br>Indicator of nutritional status<br>Indicator of inflammation |
| Blood coagulation system test | Prothrombin time (PT)<br>Activated partial thromboplastin time (APTT) | Indicators of bleeding tendency |

Table 3

| Type | | Definition |
|---|---|---|
| 0 | | No bleeding |
| 1 | | Bleeding that is not actionable and does not cause the patient to seek unscheduled performance of studies, hospitalization, or treatment by a healthcare professional; may include episodes leading to self-discontinuation of medical therapy by the patient without consulting a healthcare professional |
| 2 | | Any overt, actionable sign of hemorrhage (e.g., more bleeding than would be expected for a clinical circumstance, including bleeding found by imaging alone) that does not fit the criteria for type 3, 4, or 5 but does meet at least one of the following criteria:<br>(1) requiring nonsurgical, medical intervention by a healthcare professional,<br>(2) leading to hospitalization or increased level of care, or<br>(3) prompting evaluation |
| 3 | 3a | - Overt bleeding plus hemoglobin drop of 3 to <5 g/dL* (provided hemoglobin drop is related to bleed)<br>- Any transfusion with overt bleeding |
| | 3b | - Overt bleeding plus hemoglobin drop ≥5 g/dL* (provided hemoglobin drop is related to bleed)<br>- Cardiac tamponade<br>- Bleeding requiring surgical intervention for control (excluding dental/nasal/skin/hemorrhoid)<br>- Bleeding requiring intravenous vasoactive agents |
| | 3c | - Intracranial hemorrhage (does not include microbleeds or hemorrhagic transformation, does include intraspinal)<br>- Subcategories confirmed by autopsy or imaging or lumbar puncture<br>- Intraocular bleed compromising vision |
| 4*1 | | - Perioperative intracranial bleeding within 48 h<br>- Reoperation after closure of sternotomy for the purpose of controlling bleeding<br>- Transfusion of ≥5 U whole blood or packed red blood cells within a 48-h period<br>- Chest tube output ≥2 L within a 24-h period |
| 5*2 | 5a | Probable fatal bleeding; no autopsy or imaging confirmation but clinically suspicious |
| | 5b | Definite fatal bleeding; overt bleeding or autopsy or imaging confirmation |
| *1: Bleeding associated with coronary artery bypass surgery, *2: fatal bleeding | | |

Evaluation of pharmacokinetics

1) Secondary endpoint

(1) Plasma drug concentration

This was set to investigate the pharmacokinetics of active ONO-1301 in blood. It was evaluated by the pharmacokinetic parameter (Cmax) after attachment. Blood concentration measurement points were 1, 3, 6, and 24 hours, 7 days, 10 days, 14 days, 28 days (4 weeks), and 6 weeks after administration, and the measurement was also performed 8 weeks after administration to confirm the disappearance of blood concentrations. 5 ml of each blood sample was collected in a blood collection tube with sodium heparin, kept ice-cold until centrifugation, and centrifuged (3000 rpm × 10 min) immediately. Plasma fractions were collected and then stored frozen (-20°C).

2) Exploratory endpoints

(1) Amount of change in left ventricular ejection fraction (LVEF) at 26 weeks after attachment

This was set to evaluate the contractility of the entire left ventricle. The improvement in the contractility of the

entire left ventricle was evaluated by the amount of change in LVEF obtained by echocardiography (0, 26 weeks).

(2) Changes in left ventricular pump function (cardiac index [CT]) before and after attachment

In order to evaluate the left heart pump function, CT measurement by CT scan was set. CT (ml/min/m2) was calculated by the following equations.

CT (ml/min/m2) = ((LVEDV (ml))−(LVESV (ml))) × (heart rate (beats/min))/(body surface area (m2))

Body surface area (m2) = weight (kg) 0.425 × height (cm) 0.725 × 0.007

184 (Dubois' formula)

For the height and weight, values measured at the following time points were used.

Height: a value measured at the time of screening
Weight: a value measured on the day of cardiac-gated CT scan

(3) Changes in left ventricular remodeling before and after attachment

In order to multilaterally evaluate the inhibition of left ventricular remodeling, the following items were set.

[1] LVESVI
LVESVI is an index of ventricular remodeling for indicating the progression of heart failure. LVESVI has been reported as a life prognostic factor in many documents. Changes in LVESVI are correlated with changes in prognosis, and the direction and magnitude of the changes in LVESVI are considered to be proportional to changes in survivability. For this reason, LVESVI was set as a cardiac function index. Regarding the degree of improvement in LVESVI, considering that a reduction of 10% or more is reportedly used as a criterion for cardiac resynchronization therapy responder, and in consideration of measurement errors, fluctuations within 10% were defined as "constant."
[2] LVEDVI
[3] LVDs
[4] LVDd
[5] Changes in CTR

(4) Changes in heart failure symptoms before and after attachment

The following items were set to evaluate the severity of heart failure and the improvement of symptoms.

[1] NYHA classification
The NYHA classification was set as an endpoint to examine the improvement of the severity of heart failure.
[2] 6-Minute walking distance
The 6-minute walking distance was set as an index of QOL because it is often used as a simple method to measure exercise tolerance.

(5) Changes in myocardial blood flow; ammonia PET study

This is a useful evaluation method for confirming the recovery of blood flow in the ischemic local area (where the test drug is attached).

13NH3: By intravenously administering ammonia, the ammonia gathers in the heart in response to blood flow, and blood flow and movement in the local area of the heart can be known by examining the degree of ammonia gathering by PET/CT.

(6) Changes in brain natriuretic peptide (BNP)

BNP was set because it is very useful and widely used as a clinical index of heart failure. Changes in blood

BNP from before attachment to 26 weeks after attachment were evaluated.

(7) QOL assessment

This was set to assess the QOL of subjects after heart attachment. The patient's QOL status was assessed using the Japanese version of the SF-36 (Version 2) questionnaire.

Table 4 outlines the secondary and exploratory endpoints.

Table 4

| | Secondary endpoint | Summary of endpoint | Measurement method |
|---|---|---|---|
| (1) | Plasma drug concentration | Pharmacokinetic parameter ($C_{max}$) after attachment | |
| | Exploratory endpoints | Summary of endpoints | Measurement method |
| (1) | Amount of change in left ventricular ejection fraction (LVEF) | Changes in left ventricular ejection fraction (LVEF%) before and after heart attachment | Echocardiography |
| (2) | Changes in left ventricular pump function | Changes in cardiac index (CI) before and after heart attachment | CT |
| (3) | Changes in left ventricular remodeling | (i) Changes in left ventricular end-systolic volume index (LVESVI) before and after heart attachment and degree of improvement [increase/constant/decrease] | CT |
| | | (ii) Changes in left ventricular end-diastolic volume index (LVEDVI) before and after heart attachment | CT |
| | | (iii) Changes in left ventricular end-systolic internal diameter (LVDs) before and after heart attachment | Echocardiography |
| | | (iv) Changes in left ventricular end-diastolic internal diameter (LVDd) before and after heart attachment | Echocardiography |
| | | (v) Changes in cardiothoracic ratio (CTR) before and after heart attachment | Chest X-ray |
| (4) | Changes in heart failure symptoms | (i) Changes in NYHA classification before and after heart attachment | |
| | | (ii) Changes in 6-minute walking distance before and after heart attachment | |
| (5) | Changes in myocardial blood flow | Changes in myocardial blood flow before and after heart attachment | Ammonia PET |
| (6) | Changes in BNP | Changes in blood BNP concentration | |
| (7) | QOL assessment | Change in SF-36 | |

Table 5 shows the schedule of observations and examinations of this trial.

Table 5

| Date of observation/examination/evaluation | | Consent acquisition/ screening | Administration | After administration | | | | | | | At trial discontinuation |
|---|---|---|---|---|---|---|---|---|---|---|---|
| | | Within 4 weeks before administration of investigational drug | 0 days | 1 day | 1 week | 2 weeks | 4 weeks | 6 weeks | 8 weeks | 26 weeks | |
| Allowable period | | -32 to -1 days | | - | ± 2 days | ± 6 days | ± 1 week | ±2 weeks | ± 2 weeks | ± 4 weeks | |
| Consent acquisition | | ○ | | | | | | | | | |
| Registration | | ○ | | | | | | | | | |
| Subject background | | ○ | | | | | | | | | |
| Fundus examination | | ○*3 | | | | | | | | | |
| Observation of clinical symptoms | Vital signs | ○ | | ○ | ○ | ○ | ○ | ○ | ○ | ○ | ○ |
| | Subjective symptoms | ○ | | | ○ | ○ | ○ | ○ | ○ | ○ | ○ |
| | Physical findings | ○ | | ○ | ○ | ○ | ○ | ○ | ○ | ○ | ○ |
| Blood test | Hematology test | ○ | | ○ | ○ | ○ | ○ | ○ | ○ | ○ | ○ |
| | Blood biochemistry test | ○ | | ○ | ○ | ○ | ○ | ○ | ○ | ○ | ○ |
| | Blood coagulation system test | ○ | | ○ | ○ | ○ | ○ | ○ | ○ | ○ | ○ |
| 12-Lead electrocardiography | | ○ | | ○ | ○ | ○ | ○ | ○ | | ○ | ○ |
| Holter electrocardiography | | ○*3 | | | | | ○ | ○ | ○ | ○ | ○ |
| Ediocatdiography*1 | | ○*3 | | | | ○ | | ○ | | ○ | ○ |
| ChestX-ray | Cardiothoracic ratio (CTR) | ○*3 | | ○ | ○ | ○ | ○ | ○ | | ○ | ○ |
| Cardiac-gated CT2 | | ○*3 | | | | ○ | | | | ○ | ○ |

(continued)

| Date of observation/examination/ evaluation | | Consent acquisition/ screening | Administration | After administration | | | | | | | At trial discontinuation |
|---|---|---|---|---|---|---|---|---|---|---|---|
| | | Within 4 weeks before administration of investigational drug | 0 days | 1 day | 1 week | 2 weeks | 4 weeks | 6 weeks | 8 weeks | 26 weeks | |
| Ammonia PET | Evaluation of myocardial blood flow | ○*3 | | | | | | ○ | | ○ | ○ |
| BNP | | ○ | | ○ | ○ | ○ | ○ | ○ | ○ | ○ | ○ |
| Blood drug concentration | | | | 1, 3, 6, and 24 hours, 7 days, 10 days, 14 days, 28 days (4 weeks), 6 weeks, and 8 weeks after administration | | | | | | | 0*4 |
| Changes in heart failure symptoms | Changes in NYHA classification | ○ | | | | | | ○ | | ○ | ○ |
| | 6-Minute walking distance | ○ | | | | | | ○ | | ○ | ○ |
| QOL assessment | SF-36 | ○ | | | | | | ○ | | ○ | ○ |
| Presence or absence and degree of bleeding after attachment (post-operation) | | | | ○ | ○ | ○ | ○ | ○ | | | |
| Combination therapy | | → | | | | | | | | | |
| Adverse events | | | → | | | | | | | | |

*1 Left ventricular ejection fraction (LVEF), left ventricular end-diastolic internal diameter (LVDd), left ventricular end-systolic internal diameter (LVDs)
*2 Left ventricular end-systolic volume index (LUES VI), left ventricular end-diastolic volume index (LVEDVI), cardiac index (CI)
*3 If there is test data within 4 weeks before consent acquisition, it is possible to use that data
*4 Conducted only if the trial is discontinued within 8 weeks after administration of the investigational drug.

Fig. 3 shows the breakdown of the subjects of this trial. A total of 24 cases were assigned to this trial, with 8 cases each in Group 1, Group 2, and Group 3 (6 cases in the YS-1402 groups and 2 cases in the placebo group). All subjects in Group 1 and Group 2 completed the trial; however, in Group 3, one subject in the YS-1402-100 mg group and one subject in the placebo group were discontinued. The reasons for discontinuation were that the one subject in the YS-1402-100 mg group had "undergone treatment, which was determined to have a significant impact on the results of this trial, by surgical treatment etc. during the trial period," and that the one subject in the placebo group "stopped coming to the hospital, and examinations and observations could not be performed."

Table 6 shows the breakdown of the population to be analyzed. Among the enrolled cases, a population excluding unattached cases and subjects with no observed safety data after the investigational drug was attached was defined FAS. Analysis was performed on the FAS.

Table 6

| | 10 mg | 30 mg | 100 mg | Placebo |
|---|---|---|---|---|
| Number of cases assigned | 6 | 6 | 6 | 6 |
| FAS* | 6 (100.0) | 6 (100.0) | 6 (100.0) | 6 (100.0) |

():%
*:Largest population to be analyzed

YS-1402 dosing sheets were attached to the left ventricle at the end of coronary artery bypass surgery through thoracotomy. Because of the administration at the time of thoracotomy, the attachment frequency was once. At the end of coronary artery bypass surgery, the investigator or sub-investigator confirmed that the investigational drug was properly attached.

Pharmacokinetic analysis

Of the FAS, subjects whose blood drug concentration was measured were evaluated by dose group except for the placebo group. ONO-1301, the active ingredient of the investigational drug YS-1402, was the subject of blood concentration measurement.

Fig. 4 shows a chart of time-dependent transition of blood ONO-1301 concentration when YS-1402-10 mg, 30 mg, or 100 mg was attached once at the time of thoracotomy for coronary artery bypass surgery [horizontal axis (time: equally spaced at each blood collection point), vertical axis (blood drug concentration: real number, logarithm)]. For reference, Figs. 5 and 6 show charts of time-dependent transition on the horizontal axis (time: proportional to the actual time) and the vertical axis (blood drug concentration: real number, logarithm). Table 7 shows the summary statistics of the blood ONO-1301 concentration, and Table 8 summarizes the pharmacokinetic parameters of ONO-1301 in blood. In addition, a chart of time-dependent transition of blood ONO-1301 concentration for each subject (vertical axis: real number, logarithm), a list of changes in blood ONO-1301 concentration, and a list of pharmacokinetic parameters were attached in [Appendix 16.2.5].

Regarding the summary statistics of the pharmacokinetic parameters of ONO-1301 in blood, in the order of YS-1402-10 mg group, 30 mg group, and 100 mg group, Cmax (mean $\pm$ standard deviation, hereinafter the same) was 2.0788 $\pm$ 1.1579, 4.2967 $\pm$ 1.5310, and 8.8383 $\pm$ 2.1971 ng/ml, Tmax was 230.486 $\pm$ 87.933, 184.097 $\pm$ 143.597, and 419.250 $\pm$ 121.598 hours, MRT0-t was 341.856 $\pm$ 30.693, 400.176 $\pm$ 35.353, and 397.548 $\pm$ 34.640 hours, and AUC0-t was 1059.9076 $\pm$ 522.3988, 2640.5036 $\pm$ 730.4192, and 5572.9516 $\pm$ 1190.7685 ng·h/ml. t1/2 (0-4w) of the YS-1402-100 mg group and t1/2 (4w-8w) of all YS-1402 dosing groups could not be calculated.

The blood ONO-1301 concentration increased over time in all of the YS-1402 groups, reached a plateau 7 days after administration in the YS-1402-10 mg group and 30 mg group, and remained high until 28 days after administration. In the 100 mg group, the concentration peaked at 14 days after administration and remained high until 28 days after administration (continually changed from about 4 ng/ml to 9 ng/ml from 24 hours after administration to 28 days after administration).

Cmax and AUC0-t of blood ONO-1301 increased depending on the YS-1402 dose. When Cmax and AUC0-t in the YS-1402-10 mg group were each set to 1, Cmax and AUC0-t in the 30 mg group were 2.07 and 2.49 times, respectively, and similarly 4.25 and 5.26 times in the 100 mg group, which were less than the common ratio. On the other hand, MRT0-t was almost constant. The blood ONO-1301 concentration decreased gradually from day 14 after administration, further decreased sharply from day 28 after administration, and almost disappeared 8 weeks after administration in all dosing groups.

The maximum Cmax value of ONO-1301 in the YS-1402-100 mg group, which was the maximum dose group, was 11.900 ng/ml. At any dose, none exceeded the no-observed-effect level 15.61 ng/ml and the no-observed-adverse-effect level 23.69 ng/ml obtained in oral dosing phase I study.

Table 7

Unit: [ng/ml]

| Dosing group | Summary statistics | After administration [time] | | | | 7 days after administration | 10 days after administration | 14 days after administration | 28 days after administration (4 weeks after administration) | 6 weeks after administration | 8 weeks after administration |
|---|---|---|---|---|---|---|---|---|---|---|---|
| | | 1 | 3 | 6 | 24 | | | | | | |
| 10 mg | Number of subjects | 6 | 6 | 6 | 6 | 6 | 6 | 6 | 6 | 6 | 6 |
| | Average value | 0.07298 | 0.26867 | 0.68350 | 1.14600 | 1.76333 | 1.83200 | 1.75483 | 0.78167 | 0.12522 | 0.00000 |
| | Standard deviation | 0.04176 | 0.16808 | 0.39874 | 0.44653 | 1.13891 | 1.26721 | 0.90063 | 0.38571 | 0.07395 | 0.00000 |
| | Minimum value | 0.0468 | 0.1290 | 0.2880 | 0.7240 | 0.7900 | 0.7710 | 0.9530 | 0.2220 | 0.0521 | 0.0000 |
| | Median value | 0.05185 | 0.21450 | 0.60900 | 1.04500 | 1.43000 | 1.41000 | 1.56500 | 0.86950 | 0.11750 | 0.00000 |
| | Maximum value | 0.1540 | 0.5840 | 1.4000 | 2.0000 | 3.9100 | 4.1300 | 3.2600 | 1.2700 | 0.2520 | 0.0000 |
| | Geometric mean | 0.06583 | 0.23463 | 0.59856 | 1.08674 | 1.52463 | 1.53579 | 1.57940 | 0.67583 | 0.10811 | |
| | Geometric coefficient of variation | 48.9 | 59.0 | 60.8 | 35.3 | 61.8 | 70.3 | 53.1 | 72.7 | 65.9 | |
| 30 mg | Number of subjects | 6 | 6 | 6 | 6 | 6 | 6 | 6 | 6 | 6 | 6 |
| | Average value | 0.24023 | 0.74533 | 1.51200 | 3.12833 | 3.70500 | 3.33333 | 3.87500 | 2.23000 | 0.81817 | 0.25078 |
| | Standard deviation | 0.13876 | 0.27000 | 0.40491 | 0.94003 | 1.77246 | 1.09220 | 1.27626 | 0.58378 | 0.36484 | 0.20495 |
| | Minimum value | 0.0714 | 0.4050 | 0.8820 | 1.5800 | 1.9200 | 2.2200 | 2.2200 | 1.4600 | 0.1910 | 0.0000 |
| | Median value | 0.24500 | 0.78400 | 1.54000 | 3.18500 | 3.26500 | 2.98500 | 3.70000 | 2.25000 | 0.90650 | 0.26700 |
| | Maximum value | 0.4350 | 1.0200 | 2.0300 | 4.1900 | 6.6700 | 5.2100 | 5.4100 | 2.9800 | 1.1800 | 0.4720 |

(continued)

Unit: [ng/ml]

| Dosing group | Summary statistics | After administration [time] | | | | 7 days after administration | 10 days after administration | 14 days after administration | 28 days after administration | 6 weeks after administration | 8 weeks after administration |
|---|---|---|---|---|---|---|---|---|---|---|---|
| | | 1 | 3 | 6 | 24 | | | | (4 weeks after administration) | | |
| | Geometric mean | 0.20163 | 0.69999 | 1.46162 | 2.98685 | 3.38275 | 3.19956 | 3.69388 | 2.16368 | 0.70897 | 0.21439 |
| | Geometric coefficient of variation | 78.3 | 41.8 | 30.2 | 36.4 | 48.9 | 31.5 | 35.5 | 27.8 | 76.6 | 168.5 |
| 100 mg | Number of subjects | 6 | 6 | 6 | 6 | 6 | 6 | 6 | 6 | 6 | 6 |
| | Average value | 0.43650 | 1.98667 | 4.10000 | 4.57833 | 6.37500 | 7.24167 | 8.48333 | 7.02167 | 1.67333 | 0.08302 |
| | Standard deviation | 0.27822 | 1.03820 | 1.37662 | 1.48535 | 1.53892 | 1.82316 | 1.76189 | 3.28993 | 0.83354 | 0.16680 |
| | Minimum value | 0.2220 | 1.0700 | 2.3400 | 3.2800 | 4.6600 | 5.4200 | 5.8000 | 2.6500 | 1.1400 | 0.0000 |
| | Median value | 0.32200 | 1.68000 | 4.43000 | 4.21000 | 5.84500 | 6.80000 | 8.65500 | 6.80000 | 1.41000 | 0.00000 |
| | Maximum value | 0.9320 | 3.9600 | 5.4500 | 7.3600 | 8.6500 | 10.0000 | 10.5000 | 11.9000 | 3.3400 | 0.4170 |
| | Geometric mean | 0.37729 | 1.80899 | 3.88124 | 4.40854 | 6.22779 | 7.05932 | 8.31932 | 6.31134 | 1.55019 | 0.18390 |
| | Geometric coefficient of variation | 61.9 | 47.8 | 39.0 | 29.7 | 23.8 | 24.9 | 22.4 | 57.1 | 41.2 | 168.0 |

Table 8

| Dosing group | Summary statistics | $C_{max}$ [ng/ml] | $T_{max}$ [hr] | $k_{el}$ [libr] | $t_{1/2}$ (0-4W) [hr] | $t_{1/2}$ (4-8W) [hr] | $MRT_{0-t}$ [hr] | $MRT_{0-\infty}$ [hr] | $AUC_{0-t}$ [ng-hr/ml] | $AUC_{0-\infty}$ [ng-hr/ml] | $Cl_{tot}$ [L/hr] | $V_d$ [L] |
|---|---|---|---|---|---|---|---|---|---|---|---|---|
| 10mg | Number of subjects | 6 | 6 | 4 | 2 | | 6 | 4 | 6 | 4 | 4 | 4 |
| | Average value | 2.0788 | 230.486 | 0.00391 | 5006.597 | | 341.856 | 403.229 | 1059.9076 | 1279.1084 | 8.8949 | 2939.1644 |
| | Standard deviation | 1.1579 | 87.933 | 0.00172 | 6433.561 | | 30.693 | 57.380 | 522.3988 | 545.7913 | 3.5517 | 2186.3127 |
| | Minimum value | 0.953 | 112.97 | 0.0024 | 457.38 | | 287.86 | 343.26 | 561.701 | 743.231 | 4.928 | 967.085 |
| | Median value | 1.9850 | 223.158 | 0.00379 | 5006.597 | | 347.461 | 405.922 | 925.7088 | 1172.0908 | 8.5982 | 2569.0512 |
| | Maximum value | 4.130 | 331.00 | 0.0057 | 9555.81 | | 369.61 | 457.81 | 2002.923 | 2029.021 | 13.455 | 5651.470 |
| 30mg | Number of subjects | 6 | 6 | 5 | 3 | | 6 | 5 | 6 | 5 | 5 | |
| | Average value | 4.2967 | 184.097 | 0.00452 | 1299.195 | | 400.176 | 437.660 | 2640.5036 | 2829.6461 | 11.3668 | 2780.0106 |
| | Standard deviation | 1.5310 | 143.597 | 0.00139 | 951.379 | | 35.353 | 34.198 | 730.4192 | 752.1549 | 3.7009 | 1558.5734 |
| | Minimum value | 2.220 | 23.80 | 0.0032 | 513.39 | | 353.93 | 401.18 | 1626.454 | 1705.351 | 8.050 | 1525.077 |
| | Median value | 4.0200 | 185.067 | 0.00432 | 1027.257 | | 397.455 | 441.823 | 2692.7158 | 2873.2132 | 10.4413 | 2475.1558 |
| Dosing group | Summary statistics | $C_{max}$ [ng/ml] | $T_{max}$ [hr] | $k_{el}$ [libr] | $t_{1/2}$ (0-4W) [hr] | $t_{1/2}$ (4-8W) [hr] | $MRT_{0-t}$ [hr] | $MRT_{0-\infty}$, [hr] | $AUC_{0-t}$ [ng-hr/ml] | $AUC_{0-\infty}$ [ng-hr/ml] | $Cl_{tot}$ [L/hr] | $V_d$ [L] |
| | Maximum value | 6.670 | 353.87 | 0.0068 | 2356.93 | | 440.26 | 487.87 | 3663.908 | 3726.649 | 17.592 | 5442.842 |
| 100 mg | Number of subjects | 6 | 6 | 1 | | | 6 | 1 | 6 | 1 | 1 | 1 |
| | Average value | 8.8383 | 419.250 | 0.00600 | | | 397.548 | 436.323 | 5572.9516 | 6165.1201 | 16.2203 | 2702.6758 |
| | Standard deviation | 2.1971 | 121.598 | | | | 34.640 | | 1190.7685 | | | |
| | Minimum value | 5.800 | 312.00 | 0.0060 | | | 350.81 | 436.32 | 4267.141 | 6165.120 | 16.220 | 2702.676 |
| | Median value | 8.7500 | 366.683 | 0.00600 | | | 403.067 | 436.323 | 5631.3118 | 6165.1201 | 16.2203 | 2702.6758 |
| | Maximum value | 11.900 | 591.72 | 0.0060 | | | 433.98 | 436.32 | 7435.915 | 6165.120 | 16.220 | 2702.676 |

Analysis of exploratory endpoints

(1) Amount of change in left ventricular ejection fraction (LVEF) at 26 weeks after attachment

Fig. 7 shows a chart of time-dependent transition of measured LVEF values in echocardiography when YS-1402-10 mg, 30 mg, or 100 mg was attached once at the time of thoracotomy for coronary artery bypass surgery, Fig. 8 shows a chart of time-dependent transition of the amount of change, Table 9 shows the summary statistics of the measured values, Table 10 shows the summary statistics of the amount of change, and Table 11 shows the results of the analysis of variance of repeated measurements. For reference, Fig. 9 shows the amount of change in LVEF from the baseline at 26 weeks after administration, and Fig. 10 shows the correlation between the rate of change in total myocardial blood flow and the amount of change in LVEF from the baseline at 26 weeks after administration of the investigational drug. In addition, a chart of time-dependent transition of the measured LVEF values and the amount of change in LVEF for each subject, and a list of the measured values and the amount of change were attached in [Appendix 16.2.6].

The amount of change in LVEF (mean ± standard deviation) was as follows, in the order of the YS-1402-10 mg group, 30 mg group, 100 mg group, and placebo group:

2 weeks after administration of the investigational drug: 3.5 ± 4.7, 1.4 ± 5.4, 1.4 ± 4.0, 0.0 ± 4.5%;
6 weeks: 3.3 ± 4.6, 5.4 ± 8.3, 2.7 ± 4.4, 3.3 ± 4.7%;
26 weeks: 10.8 ± 9.5, 3.6 ± 11.0, 6.8 ± 7.7, 5.0 ± 4.4%.

As a result of analysis of variance of repeated measurements with factors of dose group, time of measurement, and dose group × time of measurement, variations in dose group and dose group × time of measurement were not significant; however, only variation in time of measurement was significant. For the two groups, i.e., the active groups combined with three doses of YS-1402 and the placebo group, variations in dose group and dose group × time of measurement were not significant; however, only variation in time of measurement was significant.

The placebo group showed a slight increase over time. The YS-1402 dosing groups showed improvement at 26 weeks after administration; however, there was no dose-related improvement. At 26 weeks, the 10 mg dosing group showed improvement with an amount of change of 5.8% compared to the placebo group. In addition, cardiac function is expected to improve with increased myocardial blood flow. Therefore, the rate of change in total myocardial blood flow and the amount of change in LVEF from the baseline at 26 weeks after administration of the investigational drug were examined. As a result, a positive correlation was observed, but was not significant (p-value: 0.340).

One case (CV-B003) in the YS-1402 30 mg group developed a serious adverse event (congestive heart failure) due to poor medication compliance 26 weeks after administration and 1 week before the test, and LVEF decreased significantly. Therefore, this case was excluded. As a result, the amount of change in LVEF at 26 weeks after administration was from 3.6 ± 110% to 6.3 ± 10.7%.

Table 9

| Test item | Dosing group | Summary statistics | IC/S* | 2 weeks after administration | 6 weeks after administration | 26 weeks after administration |
|---|---|---|---|---|---|---|
| LVEF (left ventricular ejection fraction) [%] | 10 mg | Number of subjects | 6 | 6 | 6 | 6 |
| | | Average value | 28.8 | 32.3 | 32.2 | 39.7 |
| | | Standard deviation | 8.7 | 9.5 | 7.5 | 13.4 |
| | | Minimum value | 16 | 14 | 19 | 22 |
| | | Median value | 32.5 | 34.5 | 33.5 | 38.0 |
| | | Maximum value | 38 | 41 | 41 | 61 |
| | 30 mg | Number of subjects | 5 | 5 | 5 | 5 |
| | | Average value | 31.8 | 33.2 | 37.2 | 35.4 |
| | | Standard deviation | 5.3 | 7.2 | 8.8 | 13.4 |
| | | Minimum value | 27 | 23 | 23 | 20 |
| | | Median value | 30.0 | 35.0 | 40.0 | 35.0 |
| | | Maximum value | 40 | 40 | 44 | 55 |
| | 100 mg | Number of subjects | 6 | 5 | 6 | 5 |
| | | Average value | 30.8 | 31.0 | 33.5 | 39.0 |
| | | Standard deviation | 9.1 | 11.9 | 11.9 | 10.8 |
| | | Minimum value | 17 | 17 | 20 | 30 |
| | | Median value | 33.0 | 27.0 | 33.0 | 38.0 |
| | | Maximum value | 40 | 48 | 50 | 57 |
| | Placebo | Number of subjects | 6 | 6 | 6 | 5 |
| | | Average value | 28.7 | 28.7 | 32.0 | 31.4 |
| | | Standard deviation | 7.2 | 9.2 | 6.6 | 4.9 |
| | | Minimum value | 20 | 18 | 23 | 24 |
| | | Median value | 27.5 | 26.5 | 33.0 | 32.0 |
| | | Maximum value | 40 | 44 | 40 | 37 |

*: Consent acquisition/screening

Table 10

| Test item | Dosing group | Summary statistics | 2 weeks after administration | 6 weeks after administration | 26 weeks after administration |
|---|---|---|---|---|---|
| Amount of change in LVEF (left ventricular ejection fraction) [%] | 10 mg | Number of subjects | 6 | 6 | 6 |
| | | Average value | 3.5 | 3.3 | 10.8 |
| | | Standard deviation | 4.7 | 4.6 | 9.5 |
| | | Minimum value | -2 | -2 | 0 |
| | | Median value | 3.0 | 2.5 | 8.0 |
| | | Maximum value | 12 | 12 | 27 |
| | 30 mg | Number of subjects | 5 | 5 | 5 |
| | | Average value | 1.4 | 5.4 | 3.6 |
| | | Standard deviation | 5.4 | 8.3 | 11.0 |
| | | Minimum value | -7 | -7 | -7 |
| | | Median value | 2.0 | 6.0 | 0.0 |
| | | Maximum value | 7 | 16 | 21 |
| | 100 mg | Number of subjects | 5 | 6 | 5 |
| | | Average value | 1.4 | 2.7 | 6.8 |
| | | Standard deviation | 4.0 | 4.4 | 7.7 |
| | | Minimum value | -2 | -2 | 0 |
| | | Median value | 0.0 | 1.5 | 2.0 |
| | | Maximum value | 8 | 10 | 17 |
| | Placebo | Number of subjects | 6 | 6 | 5 |
| | | Average value | 0.0 | 3.3 | 5.0 |
| | | Standard deviation | 4.5 | 4.7 | 4.4 |
| | | Minimum value | -6 | -1 | 0 |
| | | Median value | -0.5 | 1.5 | 4.0 |
| | | Maximum value | 6 | 11 | 12 |

Table 11

| Test item | Dosing group | Number of subjects | Analysis of variance table | | | |
|---|---|---|---|---|---|---|
| | | | Factor | Degrees of freedom (numerator) | F-value | p-value |
| Amount of change in LVEF (left ventricular ejection fraction) [%] | 10 mg | 6 | Dosing group | 3 | 0.33 | 0.8012 |
| | 30 mg | 5 | Time of measurement | 2 | 11.01 | 0.0011 |
| | 100 mg | 6 | Dosing group × time of measurement | 6 | 0.78 | 0.5955 |
| | Placebo | 6 | | | | |
| | Active group* | 17 | Dosing group | 1 | 0.21 | 0.6541 |
| | Placebo | 6 | Time of measurement | 2 | 11.36 | 0.0007 |
| | | | Dosing group × time of measurement | 2 | 0.48 | 0.6292 |
| *: Combined with 10 mg, 30 mg, and 100 mg dosing groups | | | | | | |

(2) Changes in left ventricular pump function (cardiac index [CI]) before and after attachment

Fig. 11 shows a chart of time-dependent transition of measured CI values in cardiac-gated CT, Fig. 12 shows a chart of time-dependent transition of the rate of change, Table 12 shows the summary statistics of the measured values, Table 13 shows the summary statistics of the rate of change, and Table 14 shows the results of the analysis of variance of repeated measurements. For reference, Fig. 13 shows the rate of change in CI from the baseline at 26 weeks after administration, and Fig. 14 shows the correlation between the rate of change in total myocardial blood flow and the rate of change in CI from the baseline at 26 weeks after administration of the investigational drug. In addition, a chart of time-dependent transition of the measured CI values and the rate of change for each subject, and a list of the measured values and the amount of change were attached in [Appendix 16.2.6].

The rate of change in CI (mean ± standard deviation) was as follows, in the order of the YS-1402-10 mg group, 30 mg group, 100 mg group, and placebo group:

2 weeks after administration of the investigational drug: 16.03 ± 16.34, 8.22 ± 25.27, 6.99 ± 22.64, -4.56 ± 14.84%;
26 weeks: 12.82 ± 25.10, 18.14 ± 25.39, 20.78 ± 28.83, 10.62 ± 23.01%.

The analysis of variance of repeated measurements with factors of dose group, time of measurement, and dose group × time of measurement did not show any significance. The results were similar for the two groups: the active groups combined with three doses of YS-1402 and the placebo group.

The placebo group showed a decrease at 2 weeks after administration, but showed an increase at 26 weeks. The YS-1402 dosing groups generally showed an increase over time, and showed improvement at 26 weeks compared to the placebo group, confirming a dose-related increase. At 26 weeks after administration, the 100 mg group showed improvement with a rate of change of 10.16% compared to the placebo group. From the above, CI increased in a dose-related manner in the order of the placebo group, YS-1402-10 mg group, 30 mg group, and 100 mg group at 26 weeks after administration. In addition, a positive correlation was observed between the rate of change in total myocardial blood flow and the rate of change in CI from the baseline at 26 weeks after administration of the investigational drug; however, the correlation was not significant (p-value: 0.102).

Table 12

| Test item | Dosing group | Summary statistics | IC/S* | 2 weeks after administration | 26 weeks after administration |
|---|---|---|---|---|---|
| CI (cardiac index) [mL/min/$m^2$] | 10 mg | Number of subjects | 6 | 5 | 6 |
| | | Average value | 2769.100 | 3060.968 | 3049.405 |
| | | Standard deviation | 383.107 | 443.657 | 380.462 |
| | | Minimum value | 2338.27 | 2522.89 | 2412.16 |
| | | Median value | 2752.330 | 3003.220 | 3031.520 |
| | | Maximum value | 3353.74 | 3760.26 | 3464.11 |
| | 30 mg | Number of subjects | 6 | 5 | 6 |
| | | Average value | 2826.928 | 3047.230 | 3338.473 |
| | | Standard deviation | 623.652 | 435.135 | 941.260 |
| | | Minimum value | 2320.81 | 2320.81 | 2169.87 |
| | | Median value | 2496.425 | 3142.460 | 3594.830 |
| | | Maximum value | 3637.25 | 3477.96 | 4264.81 |
| | 100 mg | Number of subjects | 6 | 6 | 5 |
| | | Average value | 2514.588 | 2629.082 | 2818.050 |
| | | Standard deviation | 563.446 | 584.668 | 327.177 |
| | | Minimum value | 1824.04 | 1860.60 | 2397.65 |
| | | Median value | 2595.050 | 2687.455 | 2812.150 |
| | | Maximum value | 3200.23 | 3259.18 | 3259.64 |
| | Placebo | Number of subjects | 6 | 6 | 5 |
| | | Average value | 2907.997 | 2761.382 | 3278.670 |
| | | Standard deviation | 226.640 | 370.014 | 852.573 |
| | | Minimum value | 2697.24 | 2323.51 | 2313.91 |
| | | Median value | 2822.345 | 2826.155 | 3000.680 |
| | | Maximum value | 3236.49 | 3322.00 | 4607.95 |

*: Consent acquisition/screening

Table 13

| Test item | Dosing group | Summary statistics | 2 weeks after administration | 26 weeks after administration |
|---|---|---|---|---|
| Rate of change in CI (cardiac index) [%] | 10 mg | Number of subjects | 5 | 6 |
| | | Average value | 16.03 | 12.82 |
| | | Standard deviation | 16.34 | 25.10 |
| | | Minimum value | -11.2 | -28.1 |
| | | Median value | 24.86 | 19.16 |
| | | Maximum value | 28.4 | 45.6 |
| | 30 mg | Number of subjects | 5 | 6 |
| | | Average value | 8.22 | 18.14 |
| | | Standard deviation | 25.27 | 25.39 |
| | | Minimum value | -15.3 | -8.4 |
| | | Median value | 0.00 | 16.53 |
| | | Maximum value | 45.1 | 59.9 |
| | 100 mg | Number of subjects | 6 | 5 |
| | | Average value | 6.99 | 20.78 |
| | | Standard deviation | 22.64 | 28.83 |
| | | Minimum value | -35.1 | -16.4 |
| | | Median value | 11.99 | 21.05 |
| | | Maximum value | 26.5 | 52.5 |
| | Placebo | Number of subjects | 6 | 5 |
| | | Average value | -4.56 | 10.62 |
| | | Standard deviation | 14.84 | 23.01 |
| | | Minimum value | -24.4 | -17.6 |
| | | Median value | -5.88 | 7.81 |
| | | Maximum value | 17.1 | 46.5 |

Table 14

| Test item | Dosing group | Number of subjects | Analysis of variance table | | | |
|---|---|---|---|---|---|---|
| | | | Factor | Degrees of freedom (numerator) | F-value | p-value |
| Rate of change in CI (cardiac index) [%] | 10 mg | 6 | Dosing group | 3 | 0.33 | 0.8017 |
| | 30 mg | 6 | Time of measurement | 1 | 3.89 | 0.0655 |
| | 100 mg | 6 | Dosing group × time of measurement | 3 | 0.48 | 0.6981 |
| | Placebo | 6 | | | | |
| | Active group* | 18 | Dosing group | 1 | 1.01 | 0.3250 |
| | Placebo | 6 | Time of measurement | 1 | 4.30 | 0.0527 |
| | | | Factor | Degrees of freedom (numerator) | F-value | p-value |
| | | | Dosing group × time of measurement | 1 | 0.43 | 0.5205 |

*: Combined with 10 mg, 30 mg, and 100 mg dosing groups

(3) Changes in left ventricular remodeling before and after attachment

1) Changes in left ventricular end-systolic volume index (LVESVI) before and after heart attachment and degree of improvement [increase/constant/decrease]

Fig. 15 shows a chart of time-dependent transition of measured LVESVI values in cardiac-gated CT, Fig. 16 shows a chart of time-dependent transition of the rate of change, Table 15 shows the summary statistics of the measured values, Table 16 shows the summary statistics of the rate of change, and Table 17 shows the results of the analysis of variance of repeated measurements. In addition, a chart of time-dependent transition of the measured LVESVI values and the amount of change for each subject, and a list of the measured values and the amount of change were attached in [Appendix 16.2.6].

The rate of change in LVESVI (mean ± standard deviation) was as follows, in the order of the YS-1402-10 mg group, 30 mg group, 100 mg group, and placebo group:

2 weeks after administration of the investigational drug: -18.68 ± 20.22, -10.09 ± 12.32, -21.18 ± 18.11, -5.70 ± 7.60%;
26 weeks: -38.49 ± 14.79, -10.48 ± 35.42, -35.51 ± 30.81, -18.03 ± 27.42%.

The analysis of variance of repeated measurements with factors of dose group, time of measurement, and dose group × time of measurement did not show any significance. The results were similar for the two groups: the active groups combined with three doses of YS-1402 and the placebo group.

The placebo group showed a decrease over time. The YS-1402 groups also showed a decrease over time. In particular, the degree of decrease in the YS-1402 groups at 2 weeks was greater than in the placebo group, but was not in response to the dose. The degree of improvement was examined in 11.4.7.2 conclusion of exploratory endpoints.

Table 15

| Test item | Dosing group | Summary statistics | IC/S* | 2 weeks after administration | 26 weeks after administration |
|---|---|---|---|---|---|
| LVESVI (left ventricular end-systolic volume index) [mL/$m^2$] | 10 mg | Number of subjects | 6 | 5 | 6 |
| | | Average value | 111.5 | 104.2 | 69.2 |
| | | Standard deviation | 54.5 | 64.8 | 38.8 |
| | | Minimum value | 56 | 50 | 34 |
| | | Median value | 96.5 | 100.0 | 56.0 |
| | | Maximum value | 207 | 212 | 127 |
| | 30 mg | Number of subjects | 6 | 5 | 6 |
| | | Average value | 83.5 | 81.2 | 74.7 |
| | | Standard deviation | 29.8 | 29.9 | 43.2 |
| | | Minimum value | 51 | 59 | 35 |
| | | Median value | 73.0 | 69.0 | 66.0 |
| | | Maximum value | 130 | 130 | 158 |
| | 100 mg | Number of subjects | 6 | 6 | 5 |
| | | Average value | 82.2 | 65.5 | 43.2 |
| | | Standard deviation | 45.2 | 41.9 | 19.3 |
| | | Minimum value | 36 | 29 | 14 |
| | | Median value | 66.0 | 45.5 | 52.0 |
| | | Maximum value | 151 | 133 | 62 |
| | Placebo | Number of subjects | 6 | 6 | 5 |
| | | Average value | 96.3 | 90.0 | 81.8 |
| | | Standard deviation | 31.1 | 27.1 | 25.1 |
| | | Minimum value | 65 | 66 | 46 |
| | | Median value | 86.5 | 79.5 | 97.0 |
| | | Maximum value | 140 | 127 | 104 |

*: Consent acquisition/screening

Table 16

| Test item | Dosing group | Summary statistics | 2 weeks after administration | 26 weeks after administration |
|---|---|---|---|---|
| Rate of change in LVESVI (left ventricular end-systolic volume index) [%] | 10 mg | Number of subjects | 5 | 6 |
| | | Average value | -18.68 | -38.49 |
| | | Standard deviation | 20.22 | 14.79 |
| | | Minimum value | -46.2 | -63.4 |
| | | Median value | -22.97 | -38.07 |
| | | Maximum value | 2.4 | -23.0 |
| | 30 mg | Number of subjects | 5 | 6 |
| | | Average value | -10.09 | -10.48 |
| | | Standard deviation | 12.32 | 35.42 |
| | | Minimum value | -21.3 | -50.0 |
| | | Median value | -16.90 | -18.50 |
| | | Maximum value | 6.2 | 45.0 |
| | 100 mg | Number of subjects | 6 | 5 |
| | | Average value | -21.18 | -35.51 |
| | | Standard deviation | 18.11 | 30.81 |
| | | Minimum value | -39.7 | -64.2 |
| | | Median value | -25.95 | 46.03 |
| | | Maximum value | 7.3 | 4.0 |
| | Placebo | Number of subjects | 6 | 5 |
| | | Average value | -5.70 | -18.03 |
| | | Standard deviation | 7.60 | 27.42 |
| | | Minimum value | -16.3 | -37.0 |
| | | Median value | -7.01 | -30.71 |
| | | Maximum value | 3.1 | 29.3 |

Table 17

| Test item | Dosing group | Number of subjects | Analysis of variance table | | | |
|---|---|---|---|---|---|---|
| | | | Factor | Degrees of freedom (numerator) | F-value | p-value |
| Rate of change in LVESVI (left ventricular end-systolic volume index) [%] | 10 mg | 6 | Dosing group | 3 | 1.71 | 0.2001 |
| | 30 mg | 6 | Time of measurement | 1 | 2.99 | 0.1009 |
| | 100 mg | 6 | Dosing group × time of measurement | 3 | 0.47 | 0.7093 |
| | Placebo | 6 | | | | |
| | Active group* | 18 | Dosing group | 1 | 1.35 | 0.2584 |
| | Placebo | 6 | Time of measurement | 1 | 2.11 | 0.1620 |
| | | | Dosing group × time of measurement | 1 | 0.01 | 0.9182 |

*: Combined with 10 mg, 30 mg, and 100 mg dosing groups

2) Changes in left ventricular end-diastolic volume index (LVEDVI) before and after heart attachment

Fig. 17 shows a chart of time-dependent transition of measured LVESVI values in cardiac-gated CT, Fig. 18 shows a chart of time-dependent transition of the rate of change, Table 18 shows the summary statistics of the measured values, Table 19 shows the summary statistics of the rate of change, and Table 20 shows the results of the analysis of variance of repeated measurements. In addition, a chart of time-dependent transition of the measured LVEDVI values and the amount of change for each subject, and a list of the measured values and the amount of change were attached in [Appendix 16.2.6].

The rate of change in LVEDVI (mean ± standard deviation) was as follows, in the order of the YS-1402-10 mg group, 30 mg group, 100 mg group, and placebo group:

2 weeks after administration of the investigational drug: -14.00 ± 10.99, -8.98 ± 9.16, -19.30 ± 18.78, -9.30 ± 8.52%;
26 weeks: -22.56 ± 10.73, -5.98 ± 27.50, -19.30 ± 25.82, -12.41 ± 15.73%.

The analysis of variance of repeated measurements with factors of dose group, time of measurement, and dose group × time of measurement did not show any significance. The results were similar for the two groups: the active groups combined with three doses of YS-1402 and the placebo group.

The placebo group showed a decrease over time. The YS-1402 groups generally showed a similar decrease over time; however, the decrease was not in response to the dose.

Table 18

| Test item | Dosing group | Summary statistics | IC/S* | 2 weeks after administration | 26 weeks after administration |
|---|---|---|---|---|---|
| LVEDVI (left ventricular end- diastolic volume index) [mL/m$^2$] | 10 mg | Number of subjects | 6 | 5 | 6 |
| | | Average value | 159.0 | 147.0 | 123.7 |
| | | Standard deviation | 50.5 | 53.2 | 43.4 |
| | | Minimum value | 107 | 103 | 72 |
| | | Median value | 144.5 | 135.0 | 109.5 |
| | | Maximum value | 246 | 235 | 188 |
| | 30 mg | Number of subjects | 6 | 5 | 6 |
| | | Average value | 130.3 | 126.4 | 121.8 |
| | | Standard deviation | 37.9 | 35.3 | 50.4 |
| | | Minimum value | 87 | 102 | 62 |
| | | Median value | 118.0 | 107.0 | 113.0 |
| | | Maximum value | 184 | 184 | 213 |
| | 100 mg | Number of subjects | 6 | 6 | 5 |
| | | Average value | 122.2 | 97.8 | 85.2 |
| | | Standard deviation | 46.1 | 44.3 | 21.3 |
| | | Minimum value | 68 | 63 | 55 |
| | | Median value | 108.5 | 75.0 | 95.0 |
| | | Maximum value | 190 | 170 | 105 |
| | Placebo | Number of subjects | 6 | 6 | 5 |
| | | Average value | 141.8 | 128.3 | 127.6 |
| | | Standard deviation | 36.3 | 32.4 | 27.5 |
| | | Minimum value | 105 | 85 | 96 |
| | | Median value | 135.5 | 124.5 | 141.0 |
| | | Maximum value | 201 | 175 | 158 |

*: Consent acquisition/screening

Table 19

| Test item | Dosing group | Summary statistics | 2 weeks after administration | 26 weeks after administration |
|---|---|---|---|---|
| Rate of change in LVEDVI (left ventricular end-diastolic volume index) [%] | 10 mg | Number of subjects | 5 | 6 |
| | | Average value | -14.00 | -22.56 |
| | | Standard deviation | 10.99 | 10.73 |
| | | Minimum value | -28.3 | -34.2 |
| | | Median value | -17.60 | -24.20 |
| | | Maximum value | -1.5 | -7.2 |
| | 30 mg | Number of subjects | 5 | 6 |
| | | Average value | -8.98 | -5.98 |
| | | Standard deviation | 9.16 | 27.50 |
| | | Minimum value | -18.9 | -43.5 |
| | | Median value | -10.53 | -5.70 |
| | | Maximum value | 0.9 | 26.0 |
| | 100 mg | Number of subjects | 6 | 5 |
| | | Average value | -19.30 | -19.30 |
| | | Standard deviation | 18.78 | 25.82 |
| | | Minimum value | -46.6 | -50.0 |
| | | Median value | -22.20 | -19.12 |
| | | Maximum value | 3.7 | 6.4 |
| | Placebo | Number of subjects | 6 | 5 |
| | | Average value | -9.30 | -12.41 |
| | | Standard deviation | 8.52 | 15.73 |
| | | Minimum value | -19.0 | -29.9 |
| | | Median value | -9.52 | -14.02 |
| | | Maximum value | 2.4 | 11.8 |

Table 20

| Test item | Dosing group | Number of subjects | Analysis of variance table | | | |
|---|---|---|---|---|---|---|
| | | | Factor | Degrees of freedom (numerator) | F-value | p-value |
| Rate of change in LVEDVI (left ventricular end-diastolic volume, index) [%] | 10 mg | 6 | Dosing group | 3 | 0.94 | 0.4420 |
| | 30 mg | 6 | Time of measurement | 1 | 0.10 | 0.7606 |
| | 100 mg | 6 | Dosing group × time of measurement | 3 | 0.30 | 0.8269 |
| | Placebo | 6 | | | | |
| | Active group* | 18 | Dosing group | 1 | 0.43 | 0.5209 |
| | Placebo | 6 | Time of measurement | 1 | 0.08 | 0.7820 |
| | | | Dosing group × time of measurement | 1 | 0.03 | 0.8601 |
| *: Combined with 10 mg, 30 mg, and 100 mg dosing groups | | | | | | |

3) Changes in left ventricular end-systolic internal diameter (LVDs) before and after heart attachment

Fig. 19 shows a chart of time-dependent transition of measured LVDs values in echocardiography, Fig. 20 shows a chart of time-dependent transition of the rate of change, Table 21 shows the summary statistics of the measured values, Table 22 shows the summary statistics of the rate of change, and Table 23 shows the results of the analysis of variance of repeated measurements. In addition, a chart of time-dependent transition of the measured LVDs values and the rate of change for each subject, and a list of the measured values and the rate of change were attached in [Appendix 16.2.6].

The rate of change in LVDs (mean ± standard deviation) was as follows, in the order of the YS-1402-10 mg group, 30 mg group, 100 mg group, and placebo group:

2 weeks after administration of the investigational drug: -8.33 ± 9.16, -16.56 ± 7.86, -15.51 ± 6.29, -3.70 ± 6.89%;
6 weeks: -10.67 ± 11.43, -16.41 ± 8.95, -14.13 ± 11.60, -10.74 ± 7.06%;
26 weeks: -8.33 ± 14.92, -7.12 ± 13.65, -15.34 ± 19.89, -12.15 ± 3.76%.

The analysis of variance of repeated measurements with factors of dose group, time of measurement, and dose group × time of measurement did not show any significance. Regarding the two groups, i.e., the active groups combined with three doses of YS-1402 and the placebo group, variations in dose group and dose group × time of measurement were not significant; however, only variation in time of measurement was significant.

The placebo group showed a decrease over time. The YS-1402 groups showed a greater decrease than the placebo group at 2 weeks after administration; however, the decrease was not in response to the dose. The decrease was not temporal or dose-dependent at 6 weeks and 26 weeks after administration.

Table 21

| Test item | Dosing group | Summary statistics | IC/S* | 2 weeks after administration | 6 weeks after administration | 26 weeks after administration |
|---|---|---|---|---|---|---|
| LVDs (left ventricular end-systolic internal diameter) [mm] | 10 mg | Number of subjects | 6 | 6 | 6 | 6 |
| | | Average value | 54.2 | 49.7 | 48.5 | 49.3 |
| | | Standard deviation | 8.4 | 9.2 | 10.3 | 9.7 |
| | | Minimum value | 40 | 36 | 34 | 40 |
| | | Median value | 55.0 | 49.0 | 47.0 | 47.5 |
| | | Maximum value | 64 | 61 | 62 | 65 |
| | 30 mg | Number of subjects | 5 | 5 | 5 | 5 |
| | | Average value | 51.4 | 42.4 | 42.6 | 47.4 |
| | | Standard deviation | 8.6 | 3.8 | 5.6 | 8.1 |
| | | Minimum value | 41 | 38 | 36 | 35 |
| | | Median value | 51.0 | 44.0 | 40.0 | 48.0 |
| | | Maximum value | 63 | 47 | 49 | 57 |
| | 100 mg | Number of subjects | 6 | 5 | 6 | 5 |
| | | Average value | 53.0 | 46.0 | 45.5 | 41.2 |
| | | Standard deviation | 11.3 | 11.9 | 11.4 | 9.3 |
| | | Minimum value | 40 | 32 | 30 | 29 |
| | | Median value | 49.5 | 48.0 | 44.0 | 44.0 |
| | | Maximum value | 72 | 63 | 65 | 50 |
| | Placebo | Number of subjects | 6 | 6 | 6 | 5 |
| | | Average value | 53.2 | 51.0 | 47.2 | 48.6 |
| | | Standard deviation | 7.3 | 6.4 | 5.0 | 4.5 |
| | | Minimum value | 42 | 42 | 40 | 42 |
| | | Median value | 56.5 | 52.5 | 48.0 | 49.0 |
| | | Maximum value | 59 | 59 | 53 | 54 |

*: Consent acquisition/screening

Table 22

| Test item | Dosing group | Summary statistics | 2 weeks after administration | 6 weeks after administration | 26 weeks after administration |
|---|---|---|---|---|---|
| Rate of change in LVDs (left ventricular end-systolic internal diameter) [%] | 10 mg | Number of subjects | 6 | 6 | 6 |
| | | Average value | -8.33 | -10.67 | -8.33 |
| | | Standard deviation | 9.16 | 11.43 | 14.92 |
| | | Minimum value | -18.8 | -21.9 | -25.0 |
| | | Median value | -9.90 | -14.36 | -725 |
| | | Maximum value | 7.0 | 8.8 | 14.0 |
| | 30 mg | Number of subjects | 5 | 5 | 5 |
| | | Average value | -1656 | -16.41 | -7.12 |
| | | Standard deviation | 7.86 | 8.95 | 13.65 |
| | | Minimum value | -25.4 | -23.8 | -23.8 |
| | | Median value | -17.39 | -21.57 | -9.80 |
| | | Maximum value | 4.9 | -2.4 | 10.9 |
| | 100 mg | Number of subjects | 5 | 6 | 5 |
| | | Average value | -15.51 | -14.13 | -15.34 |
| | | Standard deviation | 629 | 11.60 | 19.89 |
| | | Minimum value | -20.8 | -30.0 | 43.3 |
| | | Median value | -18.33 | -11.09 | -8.33 |
| | | Maximum value | -5.9 | 2.1 | 6.4 |
| | Placebo | Number of subjects | 6 | 6 | 5 |
| | | Average value | -3.70 | -10.74 | -12.15 |
| | | Standard deviation | 6.89 | 7.06 | 3.76 |
| | | Minimum value | -11.9 | -17.9 | -17.5 |
| | | Median value | -4.40 | -11.78 | -12.50 |
| | | Maximum value | 7.1 | 2.4 | -85 |

Table 23

| Test item | Dosing group | Number of subjects | Analysis of variance table | | | |
|---|---|---|---|---|---|---|
| | | | Factor | Degrees of freedom (numerator) | F-value | p-value |
| Rate of change in LVDs (left ventricular end-systolic internal diameter) [%] | 10 mg | 6 | Dosing group | 3 | 0.56 | 0.6497 |
| | 30 mg | 5 | Time of measurement | 2 | 2.58 | 0.1053 |
| | 100 mg | 6 | Dosing group × time of measurement | 6 | 1.16 | 0.3628 |
| | Placebo | 6 | | | | |
| | Active group* | 17 | Dosing group | 1 | 0.77 | 0.3914 |
| | Placebo | 6 | Time of measurement | 2 | 4.54 | 0.0243 |
| | | | Dosing group × time of measurement | 2 | 2.33 | 0.1238 |

*: Combined with 10 mg, 30 mg, and 100 mg dosing groups

4) Changes in left ventricular end-diastolic internal diameter (LVDd) before and after heart attachment

Fig. 21 shows a chart of time-dependent transition of measured LVDd values in echocardiography, Fig. 22 shows a chart of time-dependent transition of the rate of change, Table 24 shows the summary statistics of the measured values, Table 25 shows the summary statistics of the rate of change, and Table 26 shows the results of the analysis of variance of repeated measurements. In addition, a chart of time-dependent transition of the measured LVDd values and the rate of change for each subject, and a list of the measured values and the rate of change were attached in [Appendix 16.2.6].

The rate of change in LVDd (mean ± standard deviation) was as follows, in the order of the YS-1402-10 mg group, 30 mg group, 100 mg group, and placebo group:

2 weeks after administration of the investigational drug: -7.46 ± 9.12, -15.14 ± 4.40, -13.84 ± 3.80, -4.06 ± 8.29;
6 weeks: -8.03 ± 10.36, -14.41 ± 3.71, -13.26 ± 9.94, -8.96 ± 7.15%;
26 weeks: -4.14 ± 12.91, -2.40 ± 8.22, -9.82 ± 9.69, -9.21 ± 2.20%.

As a result of analysis of variance of repeated measurements with factors of dose group, time of measurement, and dose group × time of measurement, variations in dose group and dose group × time of measurement were not significant; however, only variation in time of measurement was significant. For the two groups, i.e., the active groups combined with three doses of YS-1402 and the placebo group, variations in dose group and dose group × time of measurement were not significant; however, only variation in time of measurement was significant.

The placebo group showed a decrease over time. As with LDVs, the YS-1402 groups showed a greater decrease than the placebo group at 2 weeks after administration; however, the decrease was not in response to the dose. The decrease was not temporal or dose-dependent at 6 weeks and 26 weeks after administration.

Table 24

| Test item | Dosing group | Summary statistics | IC/S* | 2 weeks after administration | 6 weeks after administration | 26 weeks after administration |
|---|---|---|---|---|---|---|
| LVDd (left ventricular end-diastolic internal diameter) [mm] | 10 mg | Number of subjects | 6 | 6 | 6 | 6 |
| | | Average value | 62.8 | 58.2 | 57.8 | 60.0 |
| | | Standard deviation | 5.3 | 7.5 | 82 | 7.5 |
| | | Minimum value | 56 | 47 | 45 | 52 |
| | | Median value | 63.5 | 59.5 | 59.0 | 57.5 |
| | | Maximum value | 69 | 66 | 66 | 73 |
| | 30 mg | Number of subjects | 5 | 5 | 5 | 5 |
| | | Average value | 59.4 | 50.2 | 50.8 | 57.6 |
| | | Standard deviation | 8.0 | 5.1 | 6.7 | 5.5 |
| | | Minimum value | 52 | 44 | 42 | 52 |
| | | Median value | 57.0 | 50.0 | 50.0 | 55.0 |
| | | Maximum value | 71 | 58 | 60 | 66 |
| | 100 mg | Number of subjects | 6 | 5 | 6 | 5 |
| | | Average value | 60.2 | 52.2 | 52.5 | 51.6 |
| | | Standard deviation | 8.3 | 9.5 | 11.1 | 7.0 |
| | | Minimum value | 50 | 42 | 37 | 42 |
| | | Median value | 58.5 | 52.0 | 50.5 | 52.0 |
| | | Maximum value | 75 | 67 | 70 | 60 |
| | Placebo | Number of subjects | 6 | 6 | 6 | 5 |
| | | Average value | 62.5 | 59.7 | 56.7 | 59.0 |
| | | Standard deviation | 7.5 | 6.1 | 6.0 | 4.4 |
| | | Minimum value | 50 | 50 | 47 | 52 |
| | | Median value | 65.5 | 61.5 | 59.0 | 59.0 |
| | | Maximum value | 70 | 65 | 63 | 64 |

*: Consent acquisition/screening

Table 25

| Test item | Dosing group | Summary statistics | 2 weeks after administration | 6 weeks after administration | 26 weeks after administration |
|---|---|---|---|---|---|
| Rate of change in LVDd (left ventricular end-diastolic internal diameter) [%] | 10 mg | Number of subjects | 6 | 6 | 6 |
| | | Average value | -7.46 | -8.03 | 4.14 |
| | | Standard deviation | 9.12 | 10.36 | 12.91 |
| | | Minimum value | -16.1 | -19.6 | -15.9 |
| | | Median value | -10.97 | -10.40 | -5.30 |
| | | Maximum value | 82 | 82 | 19.7 |
| | 30 mg | Number of subjects | 5 | 5 | 5 |
| | | Average value | -15.14 | -14.41 | -2.40 |
| | | Standard deviation | 4.40 | 3.71 | 8.22 |
| | | Minimum value | -20.3 | -19.2 | -15.5 |
| | | Median value | -15.38 | -15.49 | -1.89 |
| | | Maximum value | -9.4 | -9.4 | 5.8 |
| | 100 mg | Number of subjects | 5 | 6 | 5 |
| | | Average value | -13.84 | -13.26 | -9.82 |
| | | Standard deviation | 3.80 | 9.94 | 9.69 |
| | | Minimum value | -19.3 | -26.0 | -22.6 |
| | | Median value | -12.90 | -12.14 | -8.77 |
| | | Maximum value | -10.3 | 0.0 | 1.7 |
| | Placebo | Number of subjects | 6 | 6 | 5 |
| | | Average value | -4.06 | -8.96 | -9.21 |
| | | Standard deviation | 829 | 7.15 | 2.20 |
| | | Minimum value | -12.3 | -17.5 | -11.9 |
| | | Median value | -6.04 | -10.30 | -8.77 |
| | | Maximum value | 10.0 | 4.0 | -6.2 |

Table 26

| Test item | Dosing group | Number of subjects | Analysis of variance table | | | |
|---|---|---|---|---|---|---|
| | | | Factor | Degrees of freedom (numerator) | F-value | p-value |
| Rate of change in LVDd (left ventricular end-diastolic internal diameter) [%] | 10 mg | 6 | Dosing group | 3 | 0.67 | 0.5828 |
| | 30 mg | 5 | Time of measurement | 2 | 6.21 | 0.0095 |
| | 100 mg | 6 | Dosing group × time of measurement | 6 | 1.96 | 0.1193 |
| | Placebo | 6 | | | | |
| | Active group* | 17 | Dosing group | 1 | 0.56 | 0.4618 |
| | Placebo | 6 | Time of measurement | 2 | 3.91 | 0.0376 |
| | | | Dosing group × time of measurement | 2 | 2.94 | 0.0768 |
| *: Combined with 10 mg, 30 mg, and 100 mg dosing groups | | | | | | |

5) Changes in cardiothoracic ratio (CTR) before and after heart attachment

Fig. 23 shows a chart of time-dependent transition of measured CTR values in chest X-ray, Fig. 24 shows a chart of time-dependent transition of the amount of change, Table 27 shows the summary statistics of the measured values, Table 28 shows the summary statistics of the amount of change, and Table 29 shows the results of the analysis of variance of repeated measurements. In addition, a chart of time-dependent transition of the measured CTR values and the rate of change for each subject, and a list of the measured values and the amount of change were attached in [Appendix 16.2.6].

The amount of change in CTR (mean ± standard deviation) was as follows, in the order of the YS-1402-10 mg group, 30 mg group, 100 mg group, and placebo group:

1 day after administration of the investigational drug: 12.08 ± 4.10, 4.63 ± 3.92, 11.58 ± 4.09, 8.63 ± 3.93;
2 weeks: 6.17 ± 4.93, 4.58 ± 4.31, 9.22 ± 3.54, 2.87 ± 3.92;
6 weeks: 0.27 ± 4.56, -1.00 ± 5.20, 2.92 ± 2.79, 0.58 ± 4.17%;
26 weeks: -0.75 ± 3.62, -1.48 ± 4.41, -2.80 ± 4.08, -0.82 ± 4.86%.

As a result of analysis of variance of repeated measurements with factors of dose group, time of measurement, and dose group × time of measurement, variations in dose group and dose group × time of measurement were not significant; however, only variation in time of measurement was significant. For the two groups, i.e., the active groups combined with three doses of YS-1402 and the placebo group, variations in dose group and dose group × time of measurement were not significant; however, only variation in time of measurement was significant.

The placebo group showed a maximum value on the first day after administration and then showed a decrease over time. Similarly, the YS-1402 groups showed an approximate maximum value on the first day after administration, and then showed a decrease over time. The maximum values and midway values were not dose-dependent.

Table 27

| Test item | Dosing group | Summary statistics | IC/S* | 1 day after administration | 1 week after administration | 2 weeks after administration | 4 weeks after administration | 6 weeks after administration | 26 weeks after administration |
|---|---|---|---|---|---|---|---|---|---|
| CTR | 10 mg | Number of subjects | 6 | 6 | 6 | 6 | 6 | 6 | 6 |
| (cardiothoracic ratio) | | Average value | 50.58 | 62.67 | 58.62 | 56.75 | 52.55 | 50.85 | 49.83 |
| | | Standard deviation | 3.66 | 3.08 | 2.47 | 4.85 | 4.31 | 4.09 | 4.33 |
| | | Minimum value | 45.1 | 59.1 | 55.8 | 50.0 | 47.7 | 46.8 | 44.3 |
| | | Median value | 50.70 | 63.00 | 58.15 | 56.20 | 51.35 | 49.75 | 50.10 |
| | | Maximum value | 55.0 | 65.9 | 62.0 | 62.9 | 59.8 | 58.5 | 54.9 |
| | 30 mg | Number of subjects | 6 | 6 | 5 | 6 | 6 | 6 | 6 |
| | | Average value | 53.63 | 58.27 | 61.50 | 58.22 | 52.68 | 52.63 | 52.15 |
| | | Standard deviation | 5.60 | 4.75 | 4.31 | 4.49 | 5.99 | 5.95 | 5.84 |
| | | Minimum value | 43.7 | 51.2 | 54.9 | 53.2 | 43.6 | 43.1 | 45.3 |
| | | Median value | 53.75 | 59.55 | 64.30 | 58.25 | 53.85 | 53.50 | 51.35 |
| | | Maximum value | 59.4 | 64.1 | 64.6 | 64.7 | 58.6 | 60.1 | 59.8 |
| | 100 mg | Number of subjects | 6 | 6 | 6 | 6 | 6 | 6 | 5 |
| | | Average value | 49.03 | 60.62 | 59.07 | 58.25 | 54.22 | 51.95 | 46.20 |
| | | Standard deviation | 6.00 | 3.91 | 4.85 | 5.45 | 6.42 | 7.68 | 3.49 |
| | | Minimum value | 41.7 | 56.9 | 52.5 | 51.3 | 43.3 | 41.2 | 40.7 |

(continued)

| Test item | Dosing group | Summary statistics | IC/S* | 1 day after administration | 1 week after administration | 2 weeks after administration | 4 weeks after administration | 6 weeks after administration | 26 weeks after administration |
|---|---|---|---|---|---|---|---|---|---|
| | | Median value | 48.40 | 59.50 | 59.50 | 58.80 | 55.30 | 51.15 | 46.20 |
| | | Maximum value | 59.7 | 66.7 | 65.3 | 64.8 | 63.1 | 63.7 | 50.2 |
| | Placebo | Number of subjects | 6 | 6 | 6 | 6 | 6 | 6 | 5 |
| | | Average value | 52.10 | 60.73 | 57.77 | 54.97 | 54.10 | 52.68 | 51.32 |
| | | Standard deviation | 5.51 | 3.85 | 5.48 | 6.36 | 6.51 | 7.05 | 6.86 |
| | | Minimum value | 45.8 | 56.5 | 51.7 | 46.8 | 45.9 | 44.1 | 41.0 |
| | | Median value | 50.95 | 60.05 | 57.15 | 54.70 | 54.75 | 52.60 | 52.00 |
| | | Maximum value | 60.5 | 67.4 | 65.7 | 63.8 | 61.6 | 62.1 | 59.9 |

*: Consent acquisition/screening

Table 28

| Test item | Dosing group | Summary statistics | 1 day after administration | 1 week after administration | 2 weeks after administration | 4 weeks after administration | 6 weeks after administration | 26 weeks after administration |
|---|---|---|---|---|---|---|---|---|
| Amount of change in CTR (cardiothoracic ratio) [%] | 10 mg | Number of subjects | 6 | 6 | 6 | 6 | 6 | 6 |
| | | Average value | 12.08 | 8.03 | 6.17 | 1.97 | 0.27 | -0.75 |
| | | Standard deviation | 4.10 | 3.50 | 4.93 | 4.17 | 4.56 | 3.62 |
| | | Minimum value | 6.7 | 3.0 | -1.4 | -4.3 | -8.2 | -5.6 |
| | | Median value | 12.20 | 7.65 | 5.50 | 1.75 | 1.10 | 0.10 |
| | | Maximum value | 17.8 | 13.0 | 13.5 | 7.0 | 4.6 | 3.8 |
| | 30 mg | Number of subjects | 6 | 5 | 6 | 6 | 6 | 6 |
| | | Average value | 4.63 | 5.88 | 4.58 | -0.95 | -1.00 | -1.48 |
| | | Standard deviation | 3.92 | 4.91 | 4.31 | 5.45 | 5.20 | 4.41 |
| | | Minimum value | -0.2 | -0.1 | 0.0 | -11.1 | -9.7 | -7.6 |
| | | Median value | 4.90 | 6.20 | 3.55 | -0.30 | -1.05 | -1.15 |
| | | Maximum value | 9.5 | 11.5 | 10.1 | 4.0 | 5.5 | 4.6 |
| | 100 mg | Number of subjects | 6 | 6 | 6 | 6 | 6 | 5 |
| | | Average value | 11.58 | 10.03 | 9.22 | 5.18 | 2.92 | -2.80 |
| | | Standard deviation | 4.09 | 2.63 | 3.54 | 3.13 | 2.79 | 4.08 |
| | | Minimum value | 7.0 | 5.6 | 5.1 | 1.6 | -0.5 | -9.5 |

(continued)

| Test item | Dosing group | Summary statistics | 1 day after administration | 1 week after administration | 2 weeks after administration | 4 weeks after administration | 6 weeks after administration | 26 weeks after administration |
|---|---|---|---|---|---|---|---|---|
| | | Median value | 11.65 | 10.55 | 9.80 | 5.00 | 2.70 | -1.00 |
| | | Maximum value | 17.3 | 13.0 | 14.0 | 9.4 | 7.5 | 0.2 |
| | Placebo | Number of subjects | 6 | 6 | 6 | 6 | 6 | 5 |
| | | Average value | 8.63 | 5.67 | 2.87 | 2.00 | 0.58 | -0.82 |
| | | Standard deviation | 3.93 | 2.86 | 3.92 | 3.68 | 4.17 | 4.86 |
| | | Minimum value | 1.9 | 1.7 | -3.2 | -1.8 | -4.1 | -6.8 |
| | | Median value | 9.75 | 5.50 | 4.20 | 1.50 | -1.10 | 0.00 |
| | | Maximum value | 12.2 | 10.3 | 7.3 | 7.2 | 5.9 | 4.1 |

Table 29

| Test item | Dosing group | Number of subjects | Analysis of variance table | | | |
|---|---|---|---|---|---|---|
| | | | Factor | Degrees of freedom (numerator) | F-value | p-value |
| Amount of change in CTR (cardiothoracic ratio) [%] | 10 mg | 6 | Dosing group | 3 | 1.86 | 0.1681 |
| | 30 mg | 6 | Time of measurement | 5 | 32.98 | p<0.0001 |
| | 100 mg | 6 | Dosing group × time of measurement | 15 | 1.58 | 0.1529 |
| | Placebo | 6 | | | | |
| | Active group * | 18 | Dosing group | 1 | 0.44 | 0.5146 |
| | Placebo | 6 | Time of measurement | 5 | 20.18 | p<0.0001 |
| | | | Dosing group × time of measurement | 5 | 1.86 | 0.1530 |
| *: Combined with 10 mg, 30 mg, and 100 mg dosing groups | | | | | | |

(4) Changes in symptoms of heart failure before and after attachment

1) Changes in NYHA classification before and after heart attachment

Fig. 25 shows charts of time-dependent transition of the NYHA classification, Table 30 shows a cross-tabulation of the baseline and the NYHA classification at each examination time point, Table 31 shows a dose-response relationship for the rate of improvement in the NYHA classification from the baseline at each examination time point, and Table 32 shows the results of the analysis of variance of repeated measurements. In addition, a list of changes in heart failure symptoms, including measured values of the NYHA classification and the amount of change for each subject, was attached in [Appendix 16.2.6].

The degree of improvement was regarded as the amount of change from the baseline value and defined as follows:

improvement of Level II or more, improvement of Level I, constant, and worsening. The distribution of the percentage of the degree of improvement in each YS-1402 dose group was compared to that of the placebo group by applying the Wilcoxon rank sum test; however, there was no significant difference in any YS-1402 dose group from the placebo group. The Cochran-Armitage test was used to evaluate the dose-response relationship for two patterns, i.e., the percentage of improvement of Level II or more and the percentage of improvement of Level I or more; however, none of them was significant. As a result of analysis of variance of repeated measurements with factors of dose group, time of measurement, and dose group × time of measurement, variations in dose group and dose group × time of measurement were not significant; however, only variation in time of measurement was significant.

For the two groups, i.e., the active groups combined with three doses of YS-1402 and the placebo group, variations in dose group and dose group × time of measurement were not significant; however, only variation in time of measurement was significant.

The placebo group showed improvement over time. The YS-1402 groups also showed improvement over time. Particularly in the 100 mg group, all cases were improved to Level I at 26 weeks after administration.

Table 30

| Test item | Dosing group | IC/S* | 6 weeks after administration | | | | | 26 weeks after administration | | | | |
|---|---|---|---|---|---|---|---|---|---|---|---|---|
| | | | Level I | Level II | Level III | Level IV | Total | Level I | Level II | Level III | Level IV | Total |
| NYHA classification | 10 mg (N=6) | LevelI | 0 | 0 | 0 | 0 | 0 | 0 | 0 | 0 | 0 | 0 |
| | | LevelII | 1 | 3 | 0 | 0 | 4 | 4 | 0 | 0 | 0 | 4 |
| | | LevelIII | 1 | 1 | 0 | 0 | 2 | 1 | 1 | 0 | 0 | 2 |
| | | LevelIV | 0 | 0 | 0 | 0 | 0 | 0 | 0 | 0 | 0 | 0 |
| | | Total | 2 | 4 | 0 | 0 | 6 | 5 | 1 | 0 | 0 | 6 |
| | 30 mg (N--5) | LevelI | 0 | 0 | 0 | 0 | 0 | 0 | 0 | 0 | 0 | 0 |
| | | LevelII | 2 | 2 | 0 | 0 | 4 | 3 | 1 | 0 | 0 | 4 |
| | | LevelIII | 0 | 1 | 0 | 0 | 1 | 1 | 0 | 0 | 0 | 1 |
| | | LevelIV | 0 | 0 | 0 | 0 | 0 | 0 | 0 | 0 | 0 | 0 |
| | | Total | 2 | 3 | 0 | 0 | 5 | 4 | 1 | 0 | 0 | 5 |
| Test item | Dosing group | IC/S* | 6 weeks after administration | | | | | 26 weeks after administration | | | | |
| | | | Level I | Level II | Level III | Level IV | Total | Level I | Level II | Level III | Level IV | Total |
| | 100 mg (N=6) | LevelI | 0 | 0 | 0 | 0 | 0 | 0 | 0 | 0 | 0 | 0 |
| | | LevelII | 3 | 1 | 0 | 1 | 5 | 4 | 0 | 0 | 0 | 4 |
| | | LevelIII | 0 | 1 | 0 | 0 | 1 | 1 | 0 | 0 | 0 | 1 |
| | | LevelIV | 0 | 0 | 0 | 0 | 0 | 0 | 0 | 0 | 0 | 0 |
| | | Total | 3 | 2 | 0 | 1 | 6 | 5 | 0 | 0 | 0 | 5 |
| | Placebo (N=6) | LevelI | 0 | 0 | 0 | 0 | 0 | 0 | 0 | 0 | 0 | 0 |
| | | LevelII | 2 | 3 | 0 | 0 | 5 | 4 | 0 | 0 | 0 | 4 |
| | | LevelIII | 0 | 1 | 0 | 0 | 1 | 0 | 1 | 0 | 0 | 1 |
| | | LevelIV | 0 | 0 | 0 | 0 | 0 | 0 | 0 | 0 | 0 | 0 |
| | | Total | 2 | 4 | 0 | 0 | 6 | 4 | 1 | 0 | 0 | 5 |

*: Consent acquisition/screening

Table 31

| Test item | Dosing group | 6 weeks after administration | | | | | Evaluation of dose-response relationship | | | |
|---|---|---|---|---|---|---|---|---|---|---|
| | | Improvement of Level II or more | Improvement of Level I | Constant | Worsening | p-value*1 | Improvement of Level II or more | p-value*2 | Improvement of Level I or more | p-value*2 |
| NYHA classification | Placebo (N=6) | 0 (0.0) | 3 (50.0) | 3 (50.0) | 0 (0.0) | - | 0 (0.0) | 0.7826 | 3 (50.0) | 0.5308 |
| | 10 mg (N=6) | 1 (16.7) | 2 (33.3) | 3 (50.0) | 0 (0.0) | 1.0000 | 1 (16.7) | | 3 (50.0) | |
| | 30 mg (N=5) | 0 (0.0) | 3 (60.0) | 2 (40.0) | 0 (0.0) | 1.0000 | 0 (0.0) | | 3 (60.0) | |
| | 100 mg (N=6) | 0 (0.0) | 4 (66.7) | 1 (16.7) | 1 (16.7) | 1.0000 | 0 (0.0) | | 4 (66.7) | |

():%
*1: Wilcoxon rank sum test
*2: Cochran-Armitage test

| Test item | Dosing group | 26 weeks after administration | | | | | Evaluation of dose-response relationship | | | |
|---|---|---|---|---|---|---|---|---|---|---|
| | | Improvement of Level II or more | Improvement of Level I | Constant | Worsening | p-value*1 | Improvement of Level II or more | p-value*2 | Improvement of Level I or more | p-value*2 |
| NYHA classification | Placebo (N=5) | 0 (0.0) | 5 (100.0) | 0 (0.0) | 0 (0.0) | - | 0 (0.0) | 0.6466 | 5 (100.0) | 1.0000 |
| | 10 mg (N=6) | 1 (16.7) | 5 (83.3) | 0 (0.0) | 0 (0.0) | 1.0000 | 1 (16.7) | | 6 (100.0) | |
| | 30 mg (N=5) | 1 (20.0) | 3 (60.0) | 1 (20.0) | 0 (0.0) | 1.0000 | 1 (20.0) | | 4 (80.0) | |
| | 100 mg (N=5) | 1 (20.0) | 4 (80.0) | 0 (0.0) | 0 (0.0) | 1.0000 | 1 (20.0) | | 5 (100.0) | |

():%
*1: Wilcoxon rank sum test
*2: Cochran-Armitage test

Table 32

| Test item | Dosing group | Number of subjects | Analysis of variance table | | | |
|---|---|---|---|---|---|---|
| | | | Factor | Degrees of freedom (numerator) | F-value | p-value |
| Rate of change in NYHA classification | 10 mg | 6 | Dosing group | 3 | 0.17 | 0.9158 |
| | 30 mg | 5 | Time of measurement | 1 | 13.68 | 0.0026 |
| | 100 mg | 6 | Dosing group × time of measurement | 3 | 0.19 | 0.8989 |
| | Placebo | 6 | | | | |
| | Active group* | 17 | Dosing group | 1 | 0.05 | 0.8204 |
| | Placebo | 6 | Time of measurement | 1 | 10.68 | 0.0052 |
| | | | Dosing group × time of measurement | 1 | 0.06 | 0.8144 |
| *: Combined with 10 mg, 30 mg, and 100 mg dosing groups | | | | | | |

2) Changes in 6-minute walking distance before and after heart attachment

Fig. 26 shows a chart of time-dependent transition of measured values of 6-minute walking distance, Fig. 27 shows a chart of time-dependent transition of the rate of change, Table 33 shows the summary statistics of the measured values, Table 34 shows the summary statistics of the rate of change, Table 35 shows a summary table of the rate of change in 6-minute walking distance at 26 weeks after administration, and Table 36 shows the results of the analysis of variance of repeated measurements. For reference, Fig. 28 shows the rate of change in 6-minute walking distance from the baseline at 26 weeks after administration. In addition, a chart of time-dependent transition of the measured values of 6-minute walking distance and the rate of change for each subject, and a list of changes in heart failure symptoms, including the measured values of 6-minute walking distance and the rate of change, were attached in [Appendix 16.2.6].

The rate of change in 6-minute walking distance (mean ± standard deviation) was as follows, in the order of the YS-1402-10 mg group, 30 mg group, 100 mg group, and placebo group:

6 weeks after administration of the investigational drug: 12.07 ± 10.91, 10.67 ± 13.06, 6.07 ± 11.88, 3.57 ± 9.13%;
26 weeks: 14.33 ± 20.14, -4.83 ± 26.07, 20.77 ± 20.69, 14.28 ± 7.24%.

The analysis of variance of repeated measurements with factors of dose group, time of measurement, and dose group × time of measurement did not show any significance. The results were similar for the two groups: the active groups combined with three doses of YS-1402 and the placebo group.

In both the placebo group and the YS-1402 groups, the walking distance generally increased over time. At 6 weeks after administration, the YS-1402 groups showed a greater degree of increase than the placebo group; however, there was no dose-related increase. The 6-minute walking distance at 26 weeks after administration was similar in the 10 mg group to the placebo group and greater in the 100 mg group than in the placebo group, but rather decreased in the 30 mg group. The increase was not dose-related. At 26 weeks after administration, the 100 mg group showed an increase with a rate of change of 6.49% compared to the placebo group.

One case (CV-B003) in the YS-1402 30 mg group developed a serious adverse event (congestive heart failure) due to poor medication compliance 26 weeks after administration and 1 week before the test, and the 6-minute walking distance greatly decreased. Therefore, the results excluding this case were shown in Fig. 29 for reference. The rate of change in 6-minute walking distance at 26 weeks after administration was from -4.83 ± 26.07% to 6.23 ± 9.54; however, there was no dose-related increase.

Table 33

| Test item | Dosing group | Summary statistics | IC1S * | 6 weeks after administration | 26 weeks after administration |
|---|---|---|---|---|---|
| 6-Minute walking distance [m] | 10 mg | Number of subjects | 6 | 6 | 6 |
| | | Average value | 435.7 | 487.3 | 491.0 |
| | | Standard deviation | 92.1 | 102.8 | 101.9 |
| | | Minimum value | 311 | 317 | 339 |
| | | Median value | 428.0 | 500.5 | 506.0 |
| | | Maximum value | 561 | 581 | 598 |
| | 30 mg | Number of subjects | 5 | 5 | 5 |
| | | Average value | 348.4 | 379.2 | 356.0 |
| | | Standard deviation | 128.6 | 132.7 | 187.4 |
| | | Minimum value | 159 | 192 | 81 |
| | | Median value | 329.0 | 410.0 | 349.0 |
| | | Maximum value | 499 | 554 | 593 |
| | 100 mg | Number of subjects | 6 | 5 | 5 |
| | | Average value | 380.8 | 397.4 | 444.8 |
| | | Standard deviation | 116.5 | 109.9 | 99.2 |
| | | Minimum value | 205 | 258 | 280 |
| | | Median value | 403.5 | 474.0 | 485.0 |
| | | Maximum value | 492 | 481 | 534 |
| | Placebo | Number of subjects | 5 | 5 | 5 |
| | | Average value | 375.6 | 394.2 | 431.8 |
| | | Standard deviation | 115.0 | 137.4 | 140.7 |
| | | Minimum value | 200 | 186 | 217 |
| | | Median value | 376.0 | 391.0 | 430.0 |
| | | Maximum value | 512 | 538 | 580 |
| *: Consent acquisition/screening | | | | | |

Table 34

| Test item | Dosing group | Summary statistics | 6 weeks after administration | 26 weeks after administration |
|---|---|---|---|---|
| Rate of change in 6-Minute walking distance [%] | 10 mg | Number of subjects | 6 | 6 |
| | | Average value | 12.07 | 14.33 |
| | | Standard deviation | 10.91 | 20.14 |
| | | Minimum value | 1.9 | -21.0 |
| | | Median value | 8.75 | 20.95 |
| | | Maximum value | 28.8 | 32.8 |

(continued)

| Test item | Dosing group | Summary statistics | 6 weeks after administration | 26 weeks after administration |
|---|---|---|---|---|
| | 30 mg | Number of subjects | 5 | 5 |
| | | Average value | 10.67 | -4.83 |
| | | Standard deviation | 13.06 | 26.07 |
| | | Minimum value | -4.0 | -49.1 |
| | | Median value | 11.02 | 2.33 |
| | | Maximum value | 26.2 | 18.8 |
| | 100 mg | Number of subjects | 5 | 5 |
| | | Average value | 6.07 | 20.77 |
| | | Standard deviation | 11.88 | 20.69 |
| | | Minimum value | -3.5 | 0.4 |
| | | Median value | 2.76 | 9.48 |
| | | Maximum value | 25.9 | 48.6 |
| | Placebo | Number of subjects | 5 | 5 |
| | | Average value | 3.57 | 14.28 |
| | | Standard deviation | 9.13 | 7.24 |
| | | Minimum value | -7.0 | 6.4 |
| | | Median value | 5.08 | 13.28 |
| | | Maximum value | 14.8 | 23.1 |

Table 35

| | 6-Minute walking distance |
|---|---|
| Placebo group (N=5)* | 14.28±7.24% |
| 10 mg group (N=6) | 14.33±20.14% |
| 30 mg group (N=5) | -4.83±26.07% |
| 100 mg group (N=5)* | 22.77±20.69% |

* One case in each of the placebo group and 100 mg group was excluded from analysis at 26 weeks after administration due to discontinuation. One case in the 30 mg group was excluded from analysis because of impossibility of measurement due to complications of right hemiparesis.
Rate of change in 6-minute walking distance: (6-minute walking distance at 26 weeks after administration - 6-minute walking distance at pre-administration)/6-minute walking distance at pre-administration x 100%

Table 36

| Test item | Dosing group | Number of subjects | Analysis of variance table | | | |
|---|---|---|---|---|---|---|
| | | | Factor | Degrees of freedom (numerator) | F-value | p-value |
| Rate of change in 6-Minute walking distance [%] | 10 mg | 6 | Dosing group | 3 | 0.81 | 0.5074 |
| | 30 mg | 5 | Time of measurement | 1 | 0.46 | 0.5067 |
| | 100 mg | 6 | Dosing group × time measurementof | 3 | 2.14 | 0.1328 |
| | Placebo | 5 | | | | |
| | Active group* | 17 | Dosing group | 1 | 0.03 | 0.8628 |
| | Placebo | 5 | Time of measurement | 1 | 0.98 | 0.3346 |
| | | | Dosing group × time of measurement | 1 | 0.78 | 0.3869 |

*: Combined with 10 mg, 30 mg, and 100 mg dosing groups

(5) Changes in myocardial blood flow

Fig. 30 shows a chart of time-dependent transition of measured values of RCA resting myocardial blood flow, LAD resting myocardial blood flow, LCX resting myocardial blood flow, and total myocardial blood flow by ammonia PET, Fig. 31 shows a chart of time-dependent transition of the rate of change from the baseline, Table 37 shows the summary statistics of the measured values, Table 38 shows the summary statistics of the rate of change from the baseline, and Table 39 shows the results of the analysis of variance of repeated measurements. In addition, Table 40 shows a summary table of the rate of change in myocardial blood flow from the baseline at 26 weeks after administration. For reference, Fig. 32 shows the rate of change in total myocardial blood flow, Fig. 33 shows the rate of change in LAD resting myocardial blood flow, Figs. 34 and 35 show the correlation between blood concentrations (AUC0-t and Cmax) and the rate of change in total myocardial blood flow from the baseline at 26 weeks after administration of the investigational drug, and Fig. 36 shows the correlation between AUC0-t and the rate of change in LAD resting myocardial blood flow from the baseline at 26 weeks after administration of the investigational drug. In addition, a chart of time-dependent transition of the measured values of the ammonia PET items and the rate of change for each subject, and a list of the measured values and the rate of change [including the name of the site of the left ventricular myocardium (classified into 17 segments) where the investigational drug was attached] were attached in [Appendix 16.2.6]. For reference, blood flow by 17 segments in the left ventricular myocardium for each subject was attached in [Appendix 16.2.9].

The rate of change in RCA resting myocardial blood flow (mean ± standard deviation) was as follows, in the order of the YS-1402-10 mg group, 30 mg group, 100 mg group, and placebo group:

6 weeks after administration of the investigational drug: -3.01 ± 23.11, 26.78 ± 30.22, 0.82 ± 18.49, 0.21 ± 23.17%; 26 weeks: -1.73 ± 17.50, 11.76 ± 14.33, 16.13 ± 24.20, -0.91 ± 23.85%.

Similarly, the rate of change in LAD resting myocardial blood flow was as follows:

6 weeks after administration of the investigational drug: 13.31 ± 29.16, 19.77 ± 34.62, 13.86 ± 23.11, 5.32 ± 24.69%; 26 weeks: 13.38 ± 19.48, 14.59 ± 16.85, 27.59 ± 32.47, 6.41 ± 32.39%.

The rate of change in LCX resting myocardial blood flow was as follows:

6 weeks after administration of the investigational drug: 9.12 ± 30.95, 16.05 ± 32.75, 8.77 ± 20.53, 6.27 ± 3.11%; 26 weeks: 8.32 ± 20.96, 6.58 ± 15.81, 4.98 ± 18.23, 0.71 ± 14.00%.

The rate of change in total myocardial blood flow was as follows:

6 weeks after administration of the investigational drug: 6.64 ± 26.28, 20.07 ± 31.94, 7.53 ± 13.54, 3.67 ± 15.38%; 26 weeks: 6.78 ± 15.98, 11.15 ± 13.34, 16.66 ± 21.04, 1.89 ± 20.80%.

The analysis of variance of repeated measurements with factors of dose group, time of measurement, and dose group × time of measurement did not show any significance. The results were similar for the two groups: the active groups combined with three doses of YS-1402 and the placebo group.

The RCA resting myocardial blood flow did not change over time in the placebo group. At 6 weeks after administration, the YS-1402 groups generally showed a greater increase in blood flow than the placebo group; however, there was no dose correlation. On the other hand, at 26 weeks after administration, the blood flow increased in a dose-related manner compared to 6 weeks, although it was maintained, decreased, or increased depending on the dosing group.

The LAD resting myocardial blood flow slightly increased over time in the placebo group. The YS-1402 groups showed a greater increase in blood flow than the placebo group at 6 weeks after administration; however, there was no dose correlation. On the other hand, at 26 weeks after administration, the blood flow increased in a dose-related manner compared to 6 weeks, although it was maintained, decreased, or increased depending on the dosing group. The 100 mg group showed an increase in blood flow with a rate of change of 21.18% compared to the placebo group. From the above, at 26 weeks after administration, the LAD myocardial blood flow increased in a dose-related manner in the placebo group, YS-1402-10 mg group, 30 mg group, and 100 mg group. A positive correlation was observed between the blood concentration (AUC0-t) and the rate of change in LAD resting myocardial blood flow from the baseline at 26 weeks after administration of the investigational drug; however, the correlation was not significant (p-value: 0.149).

The LCX resting myocardial blood flow increased 6 weeks after administration in the placebo group. At 26 weeks after administration, the blood flow in the placebo group returned to near the baseline. In the YS-1402 groups, the blood flow increased 6 weeks after administration; however, there was no dose-related increase. At 26 weeks after administration, the increase in blood flow continued from 6 weeks after administration; however, its degree was lowered. The blood flow was higher than the placebo group; however, there was no dose-related increase. From the above, at 26 weeks after administration, compared to the YS-1402 dosing groups, the placebo group showed the lowest LCX myocardial blood flow. On the other hand, no dose-related increase was observed in the YS-1402 dosing groups.

The total myocardial blood flow increased 6 weeks after administration in the placebo group. At 26 weeks after administration, the blood flow in the placebo group returned to near the baseline. The YS-1402 groups outperformed the placebo group at 6 weeks after administration; however, no dose-related increase was observed. On the other hand, at 26 weeks after administration, the blood flow increased in a dose-related manner compared to 6 weeks, although it was maintained, decreased, or increased depending on the dosing group. The 100 mg group showed an increase in blood flow with a rate of change of 14.77% compared to the placebo group. From the above, at 26 weeks after administration, the total myocardial blood flow increased in a dose-related manner in the order of the placebo group, YS-1402-10 mg group, 30 mg group, and 100 mg group. A positive correlation was observed between the blood concentrations (AUC0-t and Cmax) and the rate of change in total myocardial blood flow from the baseline at 26 weeks after administration of the investigational drug; however, the correlation was not significant (p-value, AUC0-t: 0.160, Cmax: 0.258).

Table 37

| Test item | Dosing group | Summary statistics | IC/S* | 6 weeks after administration | 26 weeks after administration |
|---|---|---|---|---|---|
| RCA (right coronary artery) resting myocardial blood flow [mL/min/g] | 10 mg | Number of subjects | 6 | 6 | 6 |
| | | Average value | 0.7087 | 0.6672 | 0.6830 |
| | | Standard deviation | 0.1590 | 0.1231 | 0.1124 |
| | | Minimum value | 0.561 | 0.450 | 0.518 |
| | | Median value | 0.6890 | 0.7140 | 0.6750 |
| | | Maximum value | 0.991 | 0.761 | 0.850 |

(continued)

| Test item | Dosing group | Summary statistics | IC/S* | 6 weeks after administration | 26 weeks after administration |
|---|---|---|---|---|---|
| | 30 mg | Number of subjects | 6 | 6 | 6 |
| | | Average value | 0.6675 | 0.8060 | 0.7415 |
| | | Standard deviation | 0.2600 | 0.2140 | 0.2802 |
| | | Minimum value | 0.430 | 0.560 | 0.500 |
| | | Median value | 0.6120 | 0.7960 | 0.6660 |
| | | Maximum value | 1.166 | 1.055 | 1.240 |
| | 100 mg | Number of subjects | 6 | 6 | 5 |
| | | Average value | 0.6648 | 0.6707 | 0.7672 |
| | | Standard deviation | 0.0448 | 0.1308 | 0.1592 |
| | | Minimum value | 0.612 | 0.504 | 0.566 |
| | | Median value | 0.6575 | 0.7075 | 0.7530 |
| | | Maximum value | 0.728 | 0.810 | 1.009 |
| | Placebo | Number of subjects | 6 | 6 | 5 |
| | | Average value | 0.6740 | 0.6597 | 0.6466 |
| | | Standard deviation | 0.1343 | 0.1283 | 0.0330 |
| | | Minimum value | 0.441 | 0.446 | 0.612 |
| | | Median value | 0.6895 | 0.6750 | 0.6430 |
| | | Maximum value | 0.847 | 0.794 | 0.692 |

*: Consent acquisition/screening

| Test item | Dosing group | Summary statistics | IC/S* | 6 weeks after administration | 26 weeks after administration |
|---|---|---|---|---|---|
| LAD (left anterior descending coronary artery) resting myocardial blood flow [mL/min/g] | 10 mg | Number of subjects | 6 | 6 | 6 |
| | | Average value | 0.6450 | 0.6967 | 0.7135 |
| | | Standard deviation | 0.2003 | 0.1672 | 0.1623 |
| | | Minimum value | 0.451 | 0.493 | 0.466 |
| | | Median value | 0.5930 | 0.6880 | 0.7765 |
| | | Maximum value | 1.002 | 0.931 | 0.874 |
| | 30 mg | Number of subjects | 6 | 6 | 6 |
| | | Average value | 0.6477 | 0.7557 | 0.7555 |
| | | Standard deviation | 0.1403 | 0.1966 | 0.2474 |
| | | Minimum value | 0.514 | 0.561 | 0.464 |
| | | Median value | 0.5910 | 0.7275 | 0.6935 |
| | | Maximum value | 0.870 | 1.056 | 1.055 |
| | 100 mg | Number of subjects | 6 | 6 | 5 |
| | | Average value | 0.5812 | 0.6505 | 0.6998 |
| | | Standard deviation | 0.0895 | 0.1187 | 0.1047 |
| | | Minimum value | 0.440 | 0.471 | 0.578 |
| | | Median value | 0.5980 | 0.6290 | 0.7700 |
| | | Maximum value | 0.671 | 0.792 | 0.787 |
| | Placebo | Number of subjects | 6 | 6 | 5 |
| | | Average value | 0.6015 | 0.6208 | 0.6240 |
| | | Standard deviation | 0.1329 | 0.1343 | 0.1341 |
| | | Minimum value | 0.437 | 0.382 | 0.403 |
| | | Median value | 0.5835 | 0.6450 | 0.6610 |
| | | Maximum value | 0.823 | 0.786 | 0.754 |

*: Consent acquisition/screening

| Test item | Dosing group | Summary | IC/S* | 6 weeks after administration | 26 weeks after administration |
|---|---|---|---|---|---|
| LCX (left circumflex coronary artery) resting myocardial blood flow [mL/min/g] | 10 mg | Number of subjects | 6 | 6 | 6 |
| | | Average value | 0.6220 | 0.6502 | 0.6575 |
| | | Standard deviation | 0.1512 | 0.1272 | 0.1188 |
| | | Minimum value | 0.504 | 0.524 | 0.486 |
| | | Median value | 0.5665 | 0.6260 | 0.6665 |
| | | Maximum value | 0.888 | 0.814 | 0.795 |
| | 30 mg | Number of subjects | 6 | 6 | 6 |
| | | Average value | 0.7110 | 0.7938 | 0.7750 |
| | | Standard deviation | 0.1839 | 0.1879 | 0.3024 |
| | | Minimum value | 0.537 | 0.572 | 0.520 |
| | | Median value | 0.6680 | 0.8085 | 0.6235 |
| | | Maximum value | 1.001 | 1.001 | 1.215 |
| | 100 mg | Number of subjects | 6 | 6 | 5 |
| | | Average value | 0.6042 | 0.6477 | 0.6534 |
| | | Standard deviation | 0.0728 | 0.0904 | 0.1390 |
| | | Minimum value | 0.516 | 0.511 | 0.477 |
| | | Median value | 0.5850 | 0.6455 | 0.6430 |
| | | Maximum value | 0.701 | 0.768 | 0.866 |
| | Placebo | Number of subjects | 6 | 6 | 5 |
| | | Average value | 0.6212 | 0.6602 | 0.6350 |
| | | Standard deviation | 0.0512 | 0.0588 | 0.0638 |
| | | Minimum value | 0.557 | 0.596 | 0.591 |
| | | Median value | 0.6075 | 0.6455 | 0.6120 |
| | | Maximum value | 0.694 | 0.748 | 0.748 |

*: Consent acquisition/screening

| Test item | Dosing group | Summary statistics | IC/S* | 6 weeks after administration | 26 weeks after administration |
|---|---|---|---|---|---|
| Total myocardial blood flow [mL/min/g] | 10 mg | Number of subjects | 6 | 6 | 6 |
| | | Average value | 0.6575 | 0.6752 | 0.6885 |
| | | Standard deviation | 0.1701 | 0.1376 | 0.1253 |
| | | Minimum value | 0.500 | 0.490 | 0.487 |
| | | Median value | 0.5985 | 0.6695 | 0.7050 |
| | | Maximum value | 0.967 | 0.848 | 0.845 |
| | 30 mg | Number of subjects | 6 | 6 | 6 |
| | | Average value | 0.6712 | 0.7805 | 0.7575 |
| | | Standard deviation | 0.1811 | 0.1957 | 0.2644 |
| | | Minimum value | 0.496 | 0.564 | 0.492 |
| | | Median value | 0.6185 | 0.7685 | 0.6615 |
| | | Maximum value | 0.997 | 1.039 | 1.152 |
| | 100 mg | Number of subjects | 6 | 6 | 5 |
| | | Average value | 0.6105 | 0.6567 | 0.7056 |
| | | Standard deviation | 0.0388 | 0.0953 | 0.1207 |
| | | Minimum value | 0.558 | 0.528 | 0.546 |
| | | Median value | 0.6220 | 0.6815 | 0.7330 |
| | | Maximum value | 0.657 | 0.782 | 0.867 |
| | Placebo | Number of subjects | 6 | 6 | 5 |
| | | Average value | 0.6285 | 0.6477 | 0.6354 |
| | | Standard deviation | 0.0971 | 0.1113 | 0.0669 |
| | | Minimum value | 0.487 | 0.456 | 0.535 |
| | | Median value | 0.6320 | 0.6595 | 0.6440 |
| | | Maximum value | 0.764 | 0.771 | 0.708 |

*: Consent acquisition/screening

Table 38

| Test item | Dosing group | Summary statistics | 6 weeks after administration | 26 weeks after administration |
|---|---|---|---|---|
| Rate of change in RCA (right coronary artery) resting myocardial blood flow [%] | 10 mg | Number of subjects | 6 | 6 |
| | | Average value | -3.01 | -1.73 |
| | | Standard deviation | 23.11 | 17.50 |
| | | Minimum value | -38.7 | -14.2 |
| | | Median value | 1.96 | -7.43 |
| | | Maximum value | 19.6 | 32.3 |
| | 30 mg | Number of subjects | 6 | 6 |
| | | Average value | 26.78 | 11.76 |
| | | Standard deviation | 30.22 | 14.33 |
| | | Minimum value | -11.3 | -1.2 |
| | | Median value | 36.74 | 6.63 |
| | | Maximum value | 55.4 | 38.0 |
| | 100 mg | Number of subjects | 6 | 5 |
| | | Average value | 0.82 | 16.13 |
| | | Standard deviation | 18.49 | 24.20 |
| | | Minimum value | -24.7 | -22.3 |
| | | Median value | 3.50 | 23.04 |
| | | Maximum value | 25.4 | 43.1 |
| | Placebo | Number of subjects | 6 | 5 |
| | | Average value | 0.21 | -0.91 |
| | | Standard deviation | 23.17 | 23.85 |
| | | Minimum value | -30.4 | -21.4 |
| | | Median value | -0.65 | -12.31 |
| | | Maximum value | 36.3 | 38.8 |

(continued)

| Test item | Dosing group | Summary statistics | 6 weeks after administration | 26 weeks after administration |
|---|---|---|---|---|
| Rate of change in LAD (left anterior descending coronary artery) resting myocardial blood flow [%] | 10 mg | Number of subjects | 6 | 6 |
| | | Average value | 13.31 | 13.38 |
| | | Standard deviation | 29.16 | 19.48 |
| | | Minimum value | -39.9 | -12.8 |
| | | Median value | 26.90 | 18.64 |
| | | Maximum value | 36.3 | 36.8 |
| | 30 mg | Number of subjects | 6 | 6 |
| | | Average value | 19.77 | 14.59 |
| | | Standard deviation | 34.62 | 16.85 |
| | | Minimum value | -27.1 | -9.7 |
| | | Median value | 15.51 | 13.81 |
| | | Maximum value | 76.0 | 35.3 |
| | 100 mg | Number of subjects | 6 | 5 |
| | | Average value | 13.86 | 27.59 |
| | | Standard deviation | 23.11 | 32.47 |
| | | Minimum value | -29.8 | -11.8 |
| | | Median value | 18.46 | 21.23 |
| | | Maximum value | 39.3 | 75.0 |
| | Placebo | Number of subjects | 6 | 5 |
| | | Average value | 5.32 | 6.41 |
| | | Standard deviation | 24.69 | 32.39 |
| | | Minimum value | -30.7 | -34.6 |
| | | Median value | 4.68 | 14.42 |
| | | Maximum value | 45.3 | 39.6 |

(continued)

| Test item | Dosing group | Summary statistics | 6 weeks after administration | 26 weeks after administration |
|---|---|---|---|---|
| Rate of change in LCX (left circumflex coronary artery) resting myocardial blood flow [%] | 10 mg | Number of subjects | 6 | 6 |
| | | Average value | 9.12 | 8.32 |
| | | Standard deviation | 30.95 | 20.96 |
| | | Minimum value | -34.5 | -20.6 |
| | | Median value | 9.82 | 10.52 |
| | | Maximum value | 50.5 | 35.3 |
| | 30 mg | Number of subjects | 6 | 6 |
| | | Average value | 16.05 | 6.58 |
| | | Standard deviation | 32.75 | 15.81 |
| | | Minimum value | -32.8 | -11.8 |
| | | Median value | 12.29 | 1.65 |
| | | Maximum value | 56.8 | 29.7 |
| | 100 mg | Number of subjects | 6 | 5 |
| | | Average value | 8.77 | 4.98 |
| | | Standard deviation | 20.53 | 18.23 |
| | | Minimum value | -27.1 | -18.7 |
| | | Median value | 12.13 | 12.43 |
| | | Maximum value | 30.8 | 26.8 |
| | Placebo | Number of subjects | 6 | 5 |
| | | Average value | 6.27 | 0.71 |
| | | Standard deviation | 3.11 | 14.00 |
| | | Minimum value | 0.2 | -11.5 |
| | | Median value | 7.15 | -0.33 |
| | | Maximum value | 9.0 | 24.0 |

(continued)

| Test item | Dosing group | Summary statistics | 6 weeks after administration | 26 weeks after administration |
|---|---|---|---|---|
| Rate of change in total myocardial blood flow [%] | 10 mg | Number of subjects | 6 | 6 |
| | | Average value | 6.64 | 6.78 |
| | | Standard deviation | 26.28 | 15.98 |
| | | Minimum value | -38.2 | -12.6 |
| | | Median value | 17.02 | 10.26 |
| | | Maximum value | 29.8 | 23.0 |
| | 30 mg | Number of subjects | 6 | 6 |
| | | Average value | 20.07 | 11.15 |
| | | Standard deviation | 31.94 | 13.34 |
| | | Minimum value | -24.7 | -0.8 |
| | | Median value | 19.88 | 8.23 |
| | | Maximum value | 64.4 | 34.8 |

| Test item | Dosing group | Summary statistics | 6 weeks after administration | 26 weeks after administration |
|---|---|---|---|---|
| | 100 mg | Number of subjects | 6 | 5 |
| | | Average value | 7.53 | 16.66 |
| | | Standard deviation | 13.54 | 21.04 |
| | | Minimum value | -15.5 | -16.9 |
| | | Median value | 10.65 | 20.19 |
| | | Maximum value | 20.0 | 36.5 |
| | Placebo | Number of subjects | 6 | 5 |
| | | Average value | 3.67 | 1.89 |
| | | Standard deviation | 15.38 | 20.80 |
| | | Minimum value | -21.1 | -21.1 |
| | | Median value | 4.08 | 0.59 |
| | | Maximum value | 27.1 | 25.5 |

Table 39

| Test item | Dosing group | Number of subjects | Analysis of variance table | | | |
|---|---|---|---|---|---|---|
| | | | Factor | Degrees of freedom (numerator) | F-value | p-value |
| Rate of change in RCA (right coronary artery) resting myocardial blood flow [%] | 10 mg | 6 | Dosing group | 3 | 2.10 | 0.1342 |
| | 30 mg | 6 | Time of measurement | 1 | 0.00 | 0.9730 |
| | 100 mg | 6 | Dosing group × of measurement time | 3 | 1.00 | 0.4133 |
| | Placebo | 6 | | | | |
| | Active group* | 18 | Dosing group | 1 | 1.05 | 0.3179 |
| | Placebo | 6 | Time of measurement | 1 | 0.03 | 0.8702 |
| | | | Dosing group × of measurement time | 1 | 0.02 | 0.8956 |
| Rate of change in LAD (left anterior descending coronary artery) resting myocardial blood flow [%] | 10 mg | 6 | Dosing group | 3 | 0.45 | 0.7189 |
| | 30 mg | 6 | Time of measurement | 1 | 0.02 | 0.8959 |
| | 100 mg | 6 | Dosing group × of measurement time | 3 | 0.29 | 0.8325 |
| | Placebo | 6 | | | | |
| | Active group* | 18 | Dosing group | 1 | 1.22 | 0.2815 |
| | Placebo | 6 | Time of measurement | 1 | 0.00 | 0.9560 |
| | | | Dosing group × time of measurement | 1 | 0.05 | 0.8189 |
| Rate of change in LCX (left circumflex coronary artery) resting myocardial blood flow [%] | 10 mg | 6 | Dosing group | 3 | 0.24 | 0.8688 |
| | 30 mg | 6 | Time of measurement | 1 | 0.67 | 0.4214 |
| | 100 mg | 6 | Dosing group × time of measurement | 3 | 0.10 | 0.9595 |
| | Placebo | 6 | | | | |
| | Active group* | 18 | Dosing group | 1 | 0.55 | 0.4660 |
| | Placebo | 6 | Time of measurement | 1 | 0.58 | 0.4529 |
| | | | Dosing group × time of measurement | 1 | 0.01 | 0.9391 |

(continued)

| Test item | Dosing group | Number of subjects | Analysis of variance table | | | |
|---|---|---|---|---|---|---|
| | | | Factor | Degrees of freedom (numerator) | F-value | p-value |
| Rate of change in total myocardial blood flow [%] | 10 mg | 6 | Dosing group | 3 | 0.82 | 0.5007 |
| | 30 mg | 6 | Time of measurement | 1 | 0.01 | 0.9264 |
| | 100 mg | 6 | Dosing group × time of measurement | 3 | 0.40 | 0.7555 |
| | Placebo | 6 | | | | |
| | Active group* | 18 | Dosing group | 1 | 1.42 | 0.2458 |
| | Placebo | 6 | Time of measurement | 1 | 0.04 | 0.8487 |
| | | | Dosing group × time of measurement | 1 | 0.02 | 0.8868 |
| *: Combined with 10 mg, 30 mg, and 100 mg dosing groups | | | | | | |

Table 40

| | Whole myocardium | Left anterior descending artery (LAD) | Right coronary artery (RCA) | Left circumflex artery (LCX) |
|---|---|---|---|---|
| Placebo group (N=5)* | 1.89±20.80% | 6.41±32.39% | -0.91±23.85% | 0.71±14.00% |
| 10 mg group (N=6) | 6.78±15.98% | 13.38±19.48% | -1.73±17.50% | 8.32±20.96% |
| 30 mg group (N=6) | 11.15±13.34% | 14.59±16.85% | 11.76±14.33% | 6.58±15.81% |
| 100 mg group (N=5)* | 16.66±21.04% | 27.59±32.47% | 16.13±24.20% | 4.98±8.23% |
| *: One case in each of the placebo group and 100 mg group was excluded from analysis at 26 weeks after administration due to discontinuation. | | | | |

Reasons for discontinuation

Placebo group: Discontinued because the subject underwent mitral valvuloplasty due to exacerbation of mitral regurgitation.

100 mg group: Discontinued because the subject with putaminal hemorrhage stopped coming to the hospital after discharge

Rate of change in myocardial blood flow: (blood flow at 26 weeks after administration - blood flow at pre-administration)/ blood flow at pre-administration x 100%

(6) Changes in brain natriuretic peptide (BNP)

Fig. 37 shows a chart of time-dependent transition of measured blood BNP concentration values, Fig. 38 shows

a chart of time-dependent transition of the rate of change, Table 41 shows the summary statistics of the measured values, Table 42 shows the summary statistics of the rate of change, and Table 43 shows the results of the analysis of variance of repeated measurements. In addition, a chart of time-dependent transition of the measured BNP concentration values and the rate of change for each subject, and a list of the measured values and the amount of change were attached in [Appendix 16.2.6].

The rate of change in blood BNP concentration (mean ± standard deviation) was as follows, in the order of the YS-1402-10 mg group, 30 mg group, 100 mg group, and placebo group:

1 day after administration of the investigational drug: 148.41 ± 156.07, 184.81 ± 207.24, 255.29 ± 247.65, 77.53 ± 66.69;
after 1 week: 104.10 ± 132.51, 268.73 ± 283.06, 356.63 ± 381.21, 131.60 ± 101.04;
after 2 weeks: 150.38 ± 250.68, 150.47 ± 232.24, 237.35 ± 212.48, 73.67 ± 78.76;
after 6 weeks: 13.57 ± 78.60, 66.24 ± 128.44, 162.51 ± 228.08, - 12.21 ± 25.15%;
after 26 weeks: -8.84 ± 57.09, 49.44 ± 161.03, 28.00 ± 90.06, - 26.84 ± 26.45%.

As a result of analysis of variance of repeated measurements with factors of dose group, time of measurement, and dose group × time of measurement, variations in dose group and dose group × time of measurement were not significant; however, only variation in time of measurement was significant. There was no significance in the active groups combined with three doses of YS-1402 and the placebo group.

The rate of change in blood BNP concentration increased after administration and then decreased in both the placebo group and the YS-1402 groups; however, no definite tendency was observed even at 26 weeks after administration.

Table 41

| Test item | Dosing group | Summary statistics | IC/S* | 1 day after administration | 1 week after administration | 2 weeks after administration | 4 weeks after administration | 6 weeks after administration | 8 weeks after administration | 26 weeks after administration |
|---|---|---|---|---|---|---|---|---|---|---|
| Blood BNP concentration [pg/mL] | 10 mg | Number of subjects | 6 | 5 | 6 | 6 | 6 | 6 | 6 | 6 |
| | | Average value | 271.60 | 935.90 | 520.80 | 773.52 | 282.15 | 277.13 | 257.00 | 183.88 |
| | | Standard deviation | 169.20 | 819.78 | 520.96 | 1304.17 | 302.24 | 289.99 | 180.44 | 136.86 |
| | | Minimum value | 32.2 | 81.6 | 84.0 | 116.5 | 63.0 | 62.6 | 36.7 | 54.3 |
| | | Median value | 333.85 | 594.50 | 393.10 | 290.15 | 182.55 | 181.65 | 212.25 | 160.80 |
| | | Maximum value | 479.5 | 2193.2 | 1471.5 | 3426.5 | 882.9 | 835.2 | 517.3 | 424.5 |
| | 30 mg | Number of subjects | 6 | 6 | 6 | 6 | 6 | 6 | 6 | 6 |
| | | Average value | 116.50 | 280.87 | 363.60 | 237.90 | 137.35 | 168.50 | 154.00 | 179.90 |
| | | Standard deviation | 33.68 | 122.76 | 211.82 | 152.21 | 72.65 | 81.60 | 84.53 | 178.60 |
| | | Minimum value | 75.5 | 163.0 | 137.0 | 111.5 | 40.2 | 94.3 | 75.6 | 19.9 |
| | | Median value | 120.30 | 241.05 | 329.90 | 201.90 | 142.10 | 158.50 | 120.30 | 139.25 |
| | | Maximum value | 152.2 | 478.6 | 640.1 | 512.3 | 232.9 | 315.6 | 288.2 | 511.8 |
| | 100 mg | Number of subjects | 6 | 6 | 6 | 6 | 6 | 6 | 6 | 5 |
| | | Average value | 124.50 | 259.98 | 287.55 | 301.53 | 290.62 | 226.48 | 212.10 | 61.24 |
| | | Standard deviation | 132.63 | 251.86 | 177.77 | 408.76 | 478.11 | 353.54 | 254.02 | 46.05 |
| | | Minimum value | 10.0 | 81.3 | 116.5 | 56.0 | 50.2 | 20.0 | 88.9 | 192 |

(continued)

| Test item | Dosing group | Summary statistics | IC/S* | 1 day after administration | 1 week after administration | 2 weeks after administration | 4 weeks after administration | 6 weeks after administration | 8 weeks after administration | 26 weeks after administration |
|---|---|---|---|---|---|---|---|---|---|---|
| | | Median value | 68.80 | 193.40 | 201.15 | 161.35 | 96.70 | 105.90 | 105.50 | 50.30 |
| | | Maximum value | 352.4 | 759.6 | 519.3 | 1127.8 | 1262.9 | 943.7 | 728.5 | 134.2 |
| | Placebo | Number of subjects | 6 | 6 | 6 | 6 | 6 | 6 | 6 | 5 |
| | | Average value | 241.23 | 362.73 | 473.18 | 350.38 | 299.57 | 216.07 | 262.50 | 224.40 |
| | | Standard deviation | 236.05 | 269.22 | 371.52 | 290.90 | 333.75 | 217.36 | 328.69 | 218.47 |
| | | Minimum value | 33.3 | 69.9 | 99.3 | 106.5 | 94.8 | 26.0 | 44.7 | 50.7 |
| | | Median value | 186.80 | 321.60 | 366.40 | 268.55 | 182.60 | 144.40 | 130.30 | 122.00 |
| | | Maximum value | 702.6 | 827.9 | 1122.9 | 884.5 | 968.4 | 627.6 | 914.6 | 595.3 |

*: Consent acquisition/screening

Table 42

| Test item | Dosing group | Summary statistics | 1 day after administration | 1 week after administration | 2 weeks after administration | 4 weeks after administration | 6 weeks after administration | 8 weeks after administration | 26 weeks after administration |
|---|---|---|---|---|---|---|---|---|---|
| Rate of change in blood BNP concentration [%] | 10mg | Number of subjects | 5 | 6 | 6 | 6 | 6 | 6 | 6 |
| | | Average value | 148.41 | 104.10 | 150.38 | 14.18 | 13.57 | 3.12 | -8.84 |
| | | Standard deviation | 156.07 | 132.51 | 250.68 | 63.92 | 78.60 | 36.41 | 57.09 |
| | | Minimum value | -20.2 | -36.7 | -38.7 | -56.9 | -58.9 | -54.4 | -79.0 |
| | | Median value | 71.52 | 83.01 | 28.93 | -2.49 | -19.12 | 10.93 | -8.52 |
| | | Maximum value | 357.4 | 306.2 | 614.6 | 95.7 | 143.2 | 45.5 | 68.6 |
| | 30 mg | Number of subjects | 6 | 6 | 6 | 6 | 6 | 6 | 6 |
| | | Average value | 184.81 | 268.73 | 150.47 | 35.41 | 66.24 | 44.37 | 49.44 |
| | | Standard deviation | 207.24 | 283.06 | 232.24 | 98.07 | 128.44 | 95.31 | 161.03 |
| | | Minimum value | 21.9 | -8.9 | -25.9 | -73.3 | -35.4 | -38.9 | -75.2 |
| | | Median value | 70.67 | 198.11 | 56.07 | 22.20 | 25.40 | 3.00 | -0.80 |
| | | Maximumvalue | 496.0 | 697.1 | 578.5 | 208.5 | 318.0 | 201.6 | 367.0 |
| | 100 mg | Number of subjects | 6 | 6 | 6 | 6 | 6 | 6 | 5 |
| | | Average value | 255.29 | 356.63 | 237.35 | 180.31 | 162.51 | 236.01 | 28.00 |
| | | Standard deviation | 247.65 | 381.21 | 212.48 | 225.53 | 228.08 | 333.43 | 90.06 |
| | | Minimum value | -39.3 | 44.0 | -39.6 | -51.6 | -69.2 | -74.8 | -85.7 |
| | | Median value | 214.96 | 215.48 | 284.55 | 116.45 | 150.75 | 144.72 | 58.63 |
| | | Maximum value | 713.0 | 1065.0 | 460.0 | 491.5 | 463.0 | 881.0 | 122.1 |
| | Placebo | Number of subjects | 6 | 6 | 6 | 6 | 6 | 6 | 5 |
| | | Average value | 77.53 | 131.60 | 73.67 | 39.66 | -12.21 | 0.09 | -26.84 |
| | | Standard deviation | 66.69 | 101.04 | 78.76 | 75.73 | 25.15 | 33.31 | 26.45 |
| | | Minimum value | -5.1 | 59.8 | 14.0 | -35.2 | -48.0 | -465 | -57.3 |
| | | Median value | 81.78 | 79.24 | 44.45 | 25.30 | -16.30 | 3.01 | -25.38 |
| | | Maximum value | 169.7 | 308.9 | 219.8 | 184.7 | 22.8 | 34.2 | 10.0 |

Table 43

| Test item | Dosing group | Number of subjects | Analysis of variance table | | | |
|---|---|---|---|---|---|---|
| | | | Factor | Degrees of freedom (numerator) | F-value | p-value |
| Rate of change in blood BNP concentration [%] | 10 mg | 6 | Dosing group | 3 | 1.95 | 0.1545 |
| | 30 mg | 6 | Time of measurement | 6 | 3.27 | 0.0454 |
| | 100 mg | 6 | Dosing group × time of measurement | 18 | 0.83 | 0.6513 |
| | Placebo | 6 | | | | |
| | Active group * | 18 | Dosing group | 1 | 1.64 | 0.2132 |
| | Placebo | 6 | Time of measurement | 6 | 2.68 | 0.0671 |
| | | | Dosing group × time of measurement | 6 | 0.88 | 0.5371 |

*: Combined with 10 mg, 30 mg, and 100 mg dosing groups

(7) Assessment of QOL

Fig. 39 shows a chart of time-dependent transition of measured score values of 8 subscale items of SF-36 [physical functioning, role physical, bodily pain, general health, vitality, social functioning, role emotional, and mental health] set for QOL assessment, Fig. 40 shows a chart of time-dependent transition of the amount of change, Table 44 shows the summary statistics of the measured values, Table 45 shows the summary statistics of the amount of change, and Table 46 shows the results of the analysis of variance of repeated measurements. In addition, a chart of time-dependent transition of the measured values of SF-36 subscale scores and the amount of change for each subject, and a list of QOL assessment in the measured values and the amount of change were attached in [Appendix 16.2.6].

As a result of analysis of variance, regarding 7 items except bodily pain, the analysis of variance of repeated measurements with factors of dose group, time of measurement, and dose group × time of measurement did not show any significance. The results were similar for the two groups: the active groups combined with three doses of YS-1402 and the placebo group. Regarding bodily pain, as a result of analysis of variance of repeated measurements with factors of dose group, time of measurement, and dose group × time of measurement, variations in dose group and dose group × time of measurement were not significant; however, only variation in time of measurement was significant. For the two groups, i.e., the active groups combined with three doses of YS-1402 and the placebo group, variations in dose group and dose group × time of measurement were not significant; however, only variation in time of measurement was significant.

The placebo group showed improvement in physical functioning, body pain, general health, vitality, social functioning, and mental health at 26 weeks after administration. On the other hand, no improvement tendency was observed in role physical and role emotional.

Physical functioning in the YS-1402 groups was improved 6 weeks after administration compared to the placebo group; however, there was no difference from the placebo group at 26 weeks after administration. Other items did not change compared to the placebo group.

Table 44

| Subscale item | Dosing group | Summary statistics | IC/S* | 6 weeks after administration | 26 weeks after administration |
|---|---|---|---|---|---|
| Physical functioning (PF) | 10 mg | Number of subjects | 6 | 6 | 6 |
| | | Average value | 70.00 | 76.67 | 84.17 |
| | | Standard deviation | 15.81 | 11.69 | 8.61 |
| | | Minimum value | 45.0 | 65.0 | 70.0 |
| | | Median value | 72.50 | 75.00 | 85.00 |
| | | Maximum value | 90.0 | 90.0 | 95.0 |
| | 30 mg | Number of subjects | 6 | 6 | 6 |
| | | Average value | 44.17 | 50.83 | 50.00 |
| | | Standard deviation | 32.00 | 31.21 | 36.06 |
| | | Minimum value | 0.0 | 0.0 | 0.0 |
| | | Median value | 45.00 | 52.50 | 50.00 |
| | | Maximum value | 90.0 | 95.0 | 95.0 |
| | 100 mg | Number of subjects | 6 | 5 | 5 |
| | | Average value | 72.50 | 80.00 | 81.00 |
| | | Standard deviation | 27.88 | 10.61 | 22.75 |
| | | Minimum value | 30.0 | 65.0 | 45.0 |
| | | Median value | 80.00 | 80.00 | 85.00 |
| | | Maximum value | 100.0 | 95.0 | 100.0 |
| | Placebo | Number of subjects | 6 | 5 | 5 |
| | | Average value | 60.83 | 65.00 | 77.00 |
| | | Standard deviation | 25.77 | 31.02 | 22.53 |
| | | Minimum value | 20.0 | 15.0 | 40.0 |
| | | Median value | 65.00 | 80.00 | 80.00 |
| | | Maximum value | 95.0 | 90.0 | 95.0 |
| Subscale item (RP) | Dosing group | Summary statistics | IC/S* | 6 weeks after administration | 26 weeks after administration |
| Role physical | 10 mg | Number of subjects | 6 | 6 | 6 |

(continued)

| Subscale item (RP) | Dosing group | Summary statistics | IC/S* | 6 weeks after administration | 26 weeks after administration |
|---|---|---|---|---|---|
| | | Average value | 60.43 | 42.73 | 77.10 |
| | | Standard deviation | 35.07 | 23.54 | 29.22 |
| | | Minimum value | 25.0 | 0.0 | 31.3 |
| | | Median value | 59.40 | 46.90 | 90.65 |
| | | Maximum value | 100.0 | 68.8 | 100.0 |
| | 30 mg | Number of subjects | 6 | 6 | 6 |
| | | Average value | 41.68 | 51.07 | 50.00 |
| | | Standard deviation | 24.90 | 21.42 | 46.77 |
| | | Minimum value | 0.0 | 31.3 | 0.0 |
| | | Median value | 50.05 | 46.90 | 56.25 |
| | | Maximum value | 62.5 | 87.5 | 100.0 |
| | 100 mg | Number of subjects | 6 | 5 | 5 |
| | | Average value | 86.47 | 52.52 | 73.76 |
| | | Standard deviation | 30.21 | 27.44 | 28.78 |
| | | Minimum value | 25.0 | 31.3 | 37.5 |
| | | Median value | 100.00 | 43.80 | 81.30 |
| | | Maximum value | 100.0 | 100.0 | 100.0 |
| | Placebo | Number of subjects | 6 | 5 | 5 |
| | | Average value | 67.73 | 65.02 | 67.54 |
| | | Standard deviation | 13.35 | 32.66 | 11.20 |
| | | Minimum value | 50.0 | 12.5 | 50.0 |
| | | Median value | 68.80 | 68.80 | 68.80 |
| Subscale item | Dosing group | Summary statistics | IC/S* | 6 weeks after administration | 26 weeks after administration |
| | | Maximum value | 87.5 | 100.0 | 81.3 |

(continued)

| Subscale item | Dosing group | Summary statistics | IC/S* | 6 weeks after administration | 26 weeks after administration |
|---|---|---|---|---|---|
| Bodily pain (BP) | 10 mg | Number of subjects | 6 | 6 | 6 |
| | | Average value | 85.18 | 65.18 | 94.07 |
| | | Standard deviation | 21.93 | 24.18 | 9.19 |
| | | Minimum value | 46.7 | 34.4 | 82.2 |
| | | Median value | 96.65 | 64.45 | 100.00 |
| | | Maximum value | 100.0 | 100.0 | 100.0 |
| | 30 mg | Number of subjects | 6 | 6 | 6 |
| | | Average value | 71.87 | 48.33 | 74.63 |
| | | Standard deviation | 25.56 | 22.19 | 29.80 |
| | | Minimum value | 45.6 | 24.4 | 34.4 |
| | | Median value | 70.00 | 40.60 | 83.90 |
| | | Maximum value | 100.0 | 82.2 | 100.0 |
| | 100 mg | Number of subjects | 6 | 5 | 5 |
| | | Average value | 90.00 | 77.34 | 81.56 |
| | | Standard deviation | 21.38 | 29.18 | 19.10 |
| | | Minimum value | 46.7 | 35.6 | 56.7 |
| | | Median value | 100.00 | 93.30 | 82.20 |
| | | Maximum value | 100.0 | 100.0 | 100.0 |
| | Placebo | Number of subjects | 6 | 5 | 5 |
| | | Average value | 75.20 | 70.46 | 93.78 |
| | | Standard deviation | 22.30 | 19.19 | 13.91 |
| | | Minimum value | 45.6 | 56.7 | 68.9 |
| | | Median value | 73.90 | 57.80 | 100.00 |
| | | Maximum value | 100.0 | 100.0 | 100.0 |

(continued)

| Subscale item | Dosing group | Summary statistics | IC/S* | 6 weeks after administration | 26 weeks after administration |
|---|---|---|---|---|---|
| General health (GH) | 10 mg | Number of subjects | 6 | 6 | 6 |
| | | Average value | 43.17 | 53.67 | 53.33 |
| | | Standard deviation | 15.12 | 14.72 | 16.51 |
| | | Minimum value | 27.0 | 32.0 | 32.0 |
| | | Median value | 40.00 | 57.00 | 52.00 |
| | | Maximum value | 62.0 | 72.0 | 82.0 |
| | 30 mg | Number of subjects | 6 | 6 | 6 |
| | | Average value | 48.50 | 56.83 | 58.33 |
| | | Standard deviation | 14.52 | 15.60 | 28.30 |
| | | Minimum value | 30.0 | 35.0 | 15.0 |
| | | Median value | 43.50 | 58.50 | 64.50 |
| | | Maximum value | 67.0 | 77.0 | 92.0 |
| | 100 mg | Number of subjects | 6 | 5 | 5 |
| | | Average value | 59.17 | 67.00 | 68.00 |
| | | Standard deviation | 25.30 | 9.35 | 17.10 |
| | | Minimum value | 30.0 | 52.0 | 47.0 |
| | | Median value | 62.00 | 67.00 | 77.00 |
| | | Maximum value | 92.0 | 77.0 | 82.0 |
| | Placebo | Number of subjects | 6 | 5 | 5 |
| | | Average value | 52.17 | 68.20 | 62.00 |
| | | Standard deviation | 18.72 | 8.07 | 17.68 |
| | | Minimum value | 30.0 | 57.0 | 47.0 |
| | | Median value | 49.50 | 67.00 | 57.00 |
| | | Maximum value | 82.0 | 77.0 | 92.0 |

(continued)

| Subscale item | Dosing group | Summary statistics | IC/S* | 6 weeks after administration | 26 weeks after administration |
|---|---|---|---|---|---|
| Vitality (VT) | 10 mg | Number of subjects | 6 | 6 | 6 |
| | | Average value | 53.15 | 52.10 | 63.57 |
| | | Standard deviation | 21.21 | 20.42 | 17.41 |
| | | Minimum value | 25.0 | 12.5 | 31.3 |
| | | Median value | 53.15 | 59.40 | 65.65 |
| | | Maximum value | 81.3 | 68.8 | 81.3 |
| | 30 mg | Number of subjects | 6 | 6 | 6 |
| | | Average value | 49.00 | 44.82 | 50.05 |
| | | Standard deviation | 28.06 | 22.15 | 39.33 |
| | | Minimum value | 6.3 | 18.8 | 6.3 |
| | | Median value | 50.05 | 46.90 | 50.05 |
| | | Maximum value | 81.3 | 68.8 | 93.8 |
| | 100 mg | Number of subjects | 6 | 5 | 5 |
| | | Average value | 61.48 | 73.78 | 75.04 |
| | | Standard deviation | 20.71 | 12.01 | 12.49 |
| | | Minimum value | 37.5 | 56.3 | 56.3 |
| | | Median value | 65.65 | 75.00 | 81.30 |
| | | Maximum value | 81.3 | 87.5 | 87.5 |
| | Placebo | Number of subjects | 6 | 5 | 5 |
| | | Average value | 60.43 | 71.28 | 80.04 |
| | | Standard deviation | 11.63 | 14.39 | 12.81 |
| | | Minimum value | 50.0 | 56.3 | 68.8 |
| | | Median value | 56.30 | 68.80 | 75.00 |
| | | Maximum value | 75.0 | 93.8 | 93.8 |

(continued)

| Subscale item | Dosing group | Summary statistics | IC/S* | 6 weeks after administration | 26 weeks after administration |
|---|---|---|---|---|---|
| Social functioning (SF) | 10 mg | Number of subjects | 6 | 6 | 6 |
| | | Average value | 72.92 | 72.92 | 75.00 |
| | | Standard deviation | 32.03 | 25.52 | 23.72 |
| | | Minimum value | 12.5 | 37.5 | 50.0 |
| | | Median value | 75.00 | 75.00 | 75.00 |
| | | Maximum value | 100.0 | 100.0 | 100.0 |
| | 30 mg | Number of subjects | 6 | 6 | 6 |
| | | Average value | 58.33 | 68.75 | 79.17 |
| | | Standard deviation | 38.46 | 29.32 | 24.58 |
| | | Minimum value | 0.0 | 25.0 | 50.0 |
| | | Median value | 68.75 | 68.75 | 87.50 |
| | | Maximum value | 100.0 | 100.0 | 100.0 |
| | 100 mg | Number of subjects | 6 | 5 | 5 |
| | | Average value | 91.67 | 67.50 | 80.00 |
| | | Standard deviation | 12.91 | 25.92 | 25.92 |
| | | Minimum value | 75.0 | 37.5 | 37.5 |
| | | Median value | 100.00 | 62.50 | 87.50 |
| | | Maximum value | 100.0 | 100.0 | 100.0 |
| | Placebo | Number of subjects | 6 | 5 | 5 |
| | | Average value | 75.00 | 82.50 | 87.50 |
| | | Standard deviation | 32.60 | 16.77 | 21.65 |
| | | Minimum value | 12.5 | 62.5 | 50.0 |
| | | Median value | 81.25 | 75.00 | 100.00 |
| | | Maximum value | 100.0 | 100.0 | 100.0 |
| Subscale item | Dosing group | Summary statistics | IC/S* | 6 weeks after administration | 26 weeks after administration |
| Role emotional (RF) | 10 mg | Number of subjects | 6 | 6 | 6 |
| | | Average value | 55.55 | 51.38 | 77.78 |
| | | Standard deviation | 34.82 | 34.73 | 25.09 |

(continued)

| Subscale item | Dosing group | Summary statistics | IC/S* | 6 weeks after administration | 26 weeks after administration |
|---|---|---|---|---|---|
| | | Minimum value | 16.7 | 0.0 | 50.0 |
| | | Median value | 54.15 | 50.00 | 83.35 |
| | | Maximum value | 100.0 | 100.0 | 100.0 |
| | 30 mg | Number of subjects | 6 | 6 | 6 |
| | | Average value | 58.33 | 56.95 | 65.27 |
| | | Standard deviation | 34.95 | 30.46 | 47.85 |
| | | Minimum value | 16.7 | 16.7 | 0.0 |
| | | Median value | 50.00 | 58.35 | 91.65 |
| | | Maximum value | 100.0 | 91.7 | 100.0 |
| | 100 mg | Number of subjects | 6 | 5 | 5 |
| | | Average value | 88.90 | 76.66 | 83.34 |
| | | Standard deviation | 14.58 | 25.95 | 24.28 |
| | | Minimum value | 66.7 | 41.7 | 41.7 |
| | | Median value | 95.85 | 83.30 | 91.70 |
| | | Maximum value | 100.0 | 100.0 | 100.0 |
| | Placebo | Number of subjects | 6 | 5 | 5 |
| | | Average value | 74.98 | 74.98 | 66.68 |
| | | Standard deviation | 22.37 | 28.86 | 15.61 |
| | | Minimum value | 50.0 | 25.0 | 50.0 |
| | | Median value | 70.80 | 83.30 | 66.70 |
| | | Maximum value | 100.0 | 100.0 | 91.7 |

(continued)

| Subscale item | Dosing group | Summary statistics | IC/S* | 6 weeks after administration | 26 weeks after administration |
|---|---|---|---|---|---|
| Mental health (MH) | 10 mg | Number of subjects | 6 | 6 | 6 |
| | | Average value | 58.33 | 58.33 | 69.17 |
| | | Standard deviation | 23.80 | 28.40 | 14.29 |
| | | Minimum value | 30.0 | 5.0 | 50.0 |
| | | Median value | 55.00 | 67.50 | 67.50 |
| | | Maximum value | 90.0 | 80.0 | 90.0 |
| | 30 mg | Number of subjects | 6 | 6 | 6 |
| | | Average value | 48.33 | 45.83 | 64.17 |
| | | Standard deviation | 24.22 | 27.10 | 34.41 |
| | | Minimum value | 20.0 | 5.0 | 10.0 |
| | | Median value | 52.50 | 42.50 | 72.50 |
| | | Maximum value | 75.0 | 80.0 | 100.0 |
| | 100 mg | Number of subjects | 6 | 5 | 5 |
| | | Average value | 65.00 | 67.00 | 74.00 |
| | | Standard deviation | 23.87 | 15.25 | 19.49 |
| | | Minimum value | 30.0 | 40.0 | 40.0 |
| | | Median value | 65.00 | 75.00 | 85.00 |
| | | Maximum value | 100.0 | 75.0 | 85.0 |
| | Placebo | Number of subjects | 6 | 5 | 5 |
| | | Average value | 67.50 | 75.00 | 83.00 |
| | | Standard deviation | 23.18 | 28.28 | 19.56 |
| | | Minimum value | 25.0 | 40.0 | 55.0 |
| | | Median value | 72.50 | 85.00 | 95.00 |
| | | Maximum value | 95.0 | 100.0 | 100.0 |

*: Consent acquisition/screening

Table 45

| Subscale item | Dosing group | Summary statistics | 6 weeks after administration | 26 weeks after administration |
|---|---|---|---|---|
| Amount of change in physical functioning (PF) | 10 mg ° | Number of subjects | 6 | 6 |
| | | Average value | 6.67 | 14.17 |
| | | Standard deviation | 10.80 | 12.81 |
| | | Minimum value | -10.0 | -5.0 |
| | | Median value | 7.50 | 17.50 |
| | | Maximum value | 20.0 | 25.0 |
| | 30 mg | Number of subjects | 6 | 6 |
| | | Average value | 6.67 | 5.83 |
| | | Standard deviation | 10.80 | 12.42 |
| | | Minimum value | -10.0 | -5.0 |
| | | Median value | 7.50 | 2.50 |
| | | Maximum value | 20.0 | 30.0 |
| | 100 mg | Number of subjects | 5 | 5 |
| | | Average value | 7.00 | 8.00 |
| | | Standard deviation | 24.39 | 21.10 |
| | | Minimum value | -20.0 | -5.0 |
| | | Median value | 0.00 | 0.00 |
| | | Maximum value | 35.0 | 45.0 |
| | Placebo | Number of subjects | 5 | 5 |
| | | Average value | 1.00 | 13.00 |
| | | Standard deviation | 11.94 | 12.04 |
| | | Minimum value | -10.0 | 0.0 |
| | | Median value | -5.00 | 10.00 |
| | | Maximum value | 20.0 | 30.0 |

(continued)

| Subscale item | Dosing group | Summary statistics | 6 weeks after administration | 26 weeks after administration |
|---|---|---|---|---|
| Amount of change in role physical (RP) | 10 mg | Number of subjects | 6 | 6 |
| | | Average value | -17.70 | 16.67 |
| | | Standard deviation | 46.69 | 41.79 |
| | | Minimum value | -81.3 | -50.0 |
| | | Median value | -15.60 | 15.60 |
| | | Maximum value | 31.3 | 68.8 |
| | 30 mg | Number of subjects | 6 | 6 |
| | | Average value | 9.38 | 8.32 |
| | | Standard deviation | 40.83 | 33.94 |
| | | Minimum value | -31.2 | -43.8 |
| | | Median value | 3.10 | 25.00 |
| | | Maximum value | 87.5 | 37.5 |
| | 100 mg | Number of subjects | 5 | 5 |
| | | Average value | -31.24 | -10.00 |
| | | Standard deviation | 34.79 | 33.24 |
| | | Minimum value | -62.5 | -62.5 |
| | | Median value | -50.00 | 0.00 |
| | | Maximum value | 12.5 | 25.0 |
| | Placebo | Number of subjects | 5 | 5 |
| | | Average value | 1.24 | 3.76 |
| | | Standard deviation | 28.08 | 17.47 |
| | | Minimum value | -37.5 | -25.0 |
| | | Median value | 0.00 | 12.50 |
| | | Maximum value | 31.2 | 18.8 |

(continued)

| Subscale item | Dosing group | Summary statistics | 6 weeks after administration | 26 weeks after administration |
|---|---|---|---|---|
| Amount of change in bodily pain (BP) | 10 mg | Number of subjects | 6 | 6 |
| | | Average value | -20.00 | 8.88 |
| | | Standard deviation | 26.44 | 23.88 |
| | | Minimum value | -54.4 | -17.8 |
| | | Median value | -20.60 | 3.35 |
| | | Maximum value | 11.1 | 53.3 |
| | 30 mg | Number of subjects | 6 | 6 |
| | | Average value | -23.53 | 2.77 |
| | | Standard deviation | 36.45 | 31.03 |
| | | Minimum value | -65.6 | -32.2 |
| | | Median value | -32.80 | 0.00 |
| | | Maximum value | 36.6 | 54.4 |
| | 100 mg | Number of subjects | 5 | 5 |
| | | Average value | -10.66 | -6.44 |
| | | Standard deviation | 30.42 | 17.48 |
| | | Minimum value | -64.4 | -31.1 |
| | | Median value | 0.00 | 0.00 |
| | | Maximum value | 11.1 | 10.0 |
| | Placebo | Number of subjects | 5 | 5 |
| | | Average value | 0.22 | 23.54 |
| | | Standard deviation | 27.11 | 20.91 |
| | | Minimum value | -42.2 | 0.0 |
| | | Median value | 0.00 | 20.00 |
| | | Maximum value | 32.2 | 54.4 |

(continued)

| Subscale item | Dosing group | Summary statistics | 6 weeks after administration | 26 weeks after administration |
|---|---|---|---|---|
| Amount of change in general health (GH) | 10 mg | Number of subjects | 6 | 6 |
| | | Average value | 10.50 | 10.17 |
| | | Standard deviation | 10.86 | 9.45 |
| | | Minimum value | 2.0 | -3.0 |
| | | Median value | 7.00 | 8.50 |
| | | Maximum value | 32.0 | 22.0 |
| | 30 mg | Number of subjects | 6 | 6 |
| | | Average value | 8.33 | 9.83 |
| | | Standard deviation | 9.61 | 21.32 |
| | | Minimum value | -7.0 | -15.0 |
| | | Median value | 10.00 | 8.50 |
| | | Maximum value | 20.0 | 47.0 |
| | 100 mg | Number of subjects | 5 | 5 |
| | | Average value | 2.40 | 3.40 |
| | | Standard deviation | 21.59 | 30.09 |
| | | Minimum value | -20.0 | -25.0 |
| | | Median value | 0.00 | -5.00 |
| | | Maximum value | 37.0 | 52.0 |
| | Placebo | Number of subjects | 5 | 5 |
| | | Average value | 22.00 | 15.80 |
| | | Standard deviation | 9.33 | 16.92 |
| | | Minimum value | 10.0 | -10.0 |
| | | Median value | 25.00 | 15.00 |
| | | Maximum value | 33.0 | 32.0 |

(continued)

| Subscale item | Dosing group | Summary statistics | 6 weeks after administration | 26 weeks after administration |
|---|---|---|---|---|
| Amount of change in vitality (V1) | 10 mg | Number of subjects | 6 | 6 |
| | | Average value | -1.05 | 10.42 |
| | | Standard deviation | 29.97 | 17.07 |
| | | Minimum value | -50.0 | -6.2 |
| | | Median value | 6.25 | 3.10 |
| | | Maximum value | 25.0 | 37.5 |
| | 30 mg | Number of subjects | 6 | 6 |
| | | Average value | -4.18 | 1.05 |
| | | Standard deviation | 15.62 | 17.86 |
| | | Minimum value | -25.0 | -25.0 |
| | | Median value | -6.25 | 3.15 |
| | | Maximum value | 18.7 | 25.0 |
| | 100 mg | Number of subjects | 5 | 5 |
| | | Average value | 7.50 | 8.76 |
| | | Standard deviation | 11.17 | 13.71 |
| | | Minimum value | -6.2 | 0.0 |
| | | Median value | 6.20 | 0.00 |
| | | Maximum value | 18.8 | 31.3 |
| | Placebo | Number of subjects | 5 | 5 |
| | | Average value | 8.76 | 17.52 |
| | | Standard deviation | 17.44 | 22.27 |
| | | Minimum value | -6.2 | -6.2 |
| Subscale item | Dosing group | Summary statistics Median value Maximum value | 6 weeks after administration 0.00 37.5 | 26 weeks after administration 12.50 43.8 |

(continued)

| Subscale item | Dosing group | Summary statistics | 6 weeks after administration | 26 weeks after administration |
|---|---|---|---|---|
| Amount of change in social functioning (SF) | 10 mg | Number of subjects | 6 | 6 |
| | | Average value | 0.00 | 2.08 |
| | | Standard deviation | 41.08 | 35.72 |
| | | Minimum value | -50.0 | -50.0 |
| | | Median value | 0.00 | 6.25 |
| | | Maximum value | 62.5 | 50.0 |
| | 30 mg | Number of subjects | 6 | 6 |
| | | Average value | 10.42 | 20.83 |
| | | Standard deviation | 39.86 | 34.16 |
| | | Minimum value | -62.5 | -37.5 |
| | | Median value | 18.75 | 31.25 |
| | | Maximum value | 50.0 | 50.0 |
| | 100 mg | Number of subjects | 5 | 5 |
| | | Average value | -22.50 | -10.00 |
| | | Standard deviation | 29.84 | 16.30 |
| | | Minimum value | -50.0 | -37.5 |
| | | Median value | -37.50 | 0.00 |
| | | Maximum value | 25.0 | 0.0 |
| | Placebo | Number of subjects | 5 | 5 |
| | | Average value | 12.50 | 17.50 |
| | | Standard deviation | 21.65 | 37.08 |
| | | Minimum value | 0.0 | -25.0 |
| | | Median value | 0.00 | 12.50 |
| | | Maximum value | 50.0 | 75.0 |

(continued)

| Subscale item | Dosing group | Summary statistics | 6 weeks after administration | 26 weeks after administration |
|---|---|---|---|---|
| Amount of change in role emotional (RE) | 10 mg | Number of subjects | 6 | 6 |
| | | Average value | -4.17 | 22.23 |
| | | Standard deviation | 31.97 | 32.36 |
| | | Minimum value | -41.7 | -25.0 |
| | | Median value | -8.35 | 25.00 |
| | | Maximum value | 50.0 | 66.7 |
| | 30 mg | Number of subjects | 6 | 6 |
| | | Average value | -1.38 | 6.93 |
| | | Standard deviation | 28.11 | 50.67 |
| | | Minimum value | -25.0 | -50.0 |
| | | Median value | -12.50 | 0.00 |
| | | Maximum value | 41.7 | 66.7 |
| | 100 mg | Number of subjects | 5 | 5 |
| | | Average value | -16.68 | -10.00 |
| | | Standard deviation | 28.25 | 27.86 |
| | | Minimum value | -58.3 | -58.3 |
| | | Median value | 0.00 | 0.00 |
| | | Maximum value | 8.3 | 8.3 |
| | Placebo | Number of subjects | 5 | 5 |
| | | Average value | 5.00 | -3.30 |
| | | Standard deviation | 28.03 | 32.08 |
| | | Minimum value | -25.0 | -50.0 |
| | | Median value | 0.00 | 0.00 |
| | | Maximum value | 41.7 | 33.4 |

(continued)

| Subscale item | Dosing group | Summary statistics | 6 weeks after administration | 26 weeks after administration |
|---|---|---|---|---|
| Amount of change in mental health (MH) | 10 mg | Number of subjects | 6 | 6 |
| | | Average value | 0.00 | 10.83 |
| | | Standard deviation | 30.82 | 13.20 |
| | | Minimum value | -35.0 | 0.0 |
| | | Median value | -7.50 | 5.00 |
| | | Maximum value | 50.0 | 30.0 |
| | 30 mg | Number of subjects | 6 | 6 |
| | | Average value | -2.50 | 15.83 |
| | | Standard deviation | 14.75 | 34.56 |
| | | Minimum value | -25.0 | -10.0 |
| | | Median value | 2.50 | 5.00 |
| | | Maximum value | 15.0 | 80.0 |
| | 100 mg | Number of subjects | 5 | 5 |
| | | Average value | -5.00 | 2.00 |
| | | Standard deviation | 15.41 | 21.97 |
| | | Minimum value | -25.0 | -25.0 |
| | | Median value | 0.00 | 10.00 |
| | | Maximum value | 15.0 | 30.0 |
| | Placebo | Number of subjects | 5 | 5 |
| | | Average value | 9.00 | 17.00 |
| | | Standard deviation | 15.17 | 21.97 |
| | | Minimum value | -15.0 | -10.0 |
| | | Median value | 15.00 | 25.00 |
| | | Maximum value | 25.0 | 45.0 |

Table 46

| Subscale item | Dosing group | Number of subjects | Analysis of variance table | | | |
|---|---|---|---|---|---|---|
| | | | Factor | Degrees of freedom (numerator) | F-value | p-value |
| Amount of change in physical functioning (PF) | 10 mg | 6 | Dosing group | 3 | 0.12 | 0.9501 |
| | 30 mg | 6 | Time of measurement | 1 | 2.49 | 0.1318 |
| | 100 mg | 6 | Dosing group × time of measurement | 3 | 0.89 | 0.4647 |
| | Placebo | 6 | | | | |
| | Active | 18 | Dosing group | 1 | 0.03 | 0.8661 |
| | Placebo | 6 | Time of measurement | 1 | 4.11 | 0.0562 |
| | | | Dosing group × time of measurement | 1 | 1.67 | 0.2104 |

(continued)

| Subscale item | Dosing group | Number of subjects | Analysis of variance table | | | |
|---|---|---|---|---|---|---|
| | | | Factor | Degrees of freedom (numerator) | F-value | p-value |
| Amount of change in role physical (RP) | 10 mg | 6 | Dosing group | 3 | 0.86 | 0.4796 |
| | 30 mg | 6 | Time of measurement | 1 | 3.41 | 0.0814 |
| | 100 mg<br>Placebo | 6 | Dosing group × time of measurement | 3 | 1.22 | 0.3315 |
| | Active group * | 18 | Dosing group | 1 | 0.12 | 0.7319 |
| | Placebo | 6 | Time of measurement | 1 | 1.19 | 0.2879 |
| | | | Dosing group × time of measurement | 1 | 0.68 | 0.4199 |
| Amount of change in bodily pain (BP) | 10 mg | 6 | Dosing group | 3 | 0.97 | 0.4279 |
| | 30 mg | 6 | Time of measurement | 1 | 11.33 | 0.0034 |
| | 100 mg | 6 | Dosing group × time of measurement | 3 | 0.80 | 0.5103 |
| | Placebo | 6 | | | | |
| | Active group* | 18 | Dosing group | 1 | 3.07 | 0.0949 |
| | Placebo | 6 | Time of measurement | 1 | 8.94 | 0.0072 |
| | | | Dosing group × time of measurement | 1 | 0.03 | 0.8615 |
| Amount of change in general health (GH) | 10 mg | 6 | Dosing group | 3 | 0.87 | 0.4755 |
| | 30 mg | 6 | Time of measurement | 1 | 0.11 | 0.7385 |
| | 100 mg | 6 | Dosing group × time of measurement | 3 | 0.34 | 0.7990 |
| | Placebo | 6 | | | | |
| | Active group* | 18 | Dosing | 1 | 2.07 | 0.1656 |
| | Placebo | 6 | Time of measurement | 1 | 0.67 | 0.4229 |
| | | | Dosing group × time of measurement | 1 | 1.06 | 0.3160 |

| Subscale item | Dosing group | Number of subjects | Analysis of variance table | | | |
|---|---|---|---|---|---|---|
| | | | Factor | Degrees of freedom (numerator) | F-value | p-value |
| Amount of change in vitality (VT) | 10 mg | 6 | Dosing group | 3 | 0.75 | 0.5345 |
| | 30 mg | 6 | Time of measurement | 1 | 2.74 | 0.1151 |
| | 100 mg | 6 | Dosing group × time of measurement | 3 | 0.30 | 0.8260 |
| | Placebo | 6 | | | | |
| | Active group* | 18 | Dosing group | 1 | 1.36 | 0.2568 |
| | Placebo | 6 | Time of measurement | 1 | 2.61 | 0.1219 |
| | | | Dosing group × time of measurement | 1 | 0.07 | 0.7912 |

(continued)

| Subscale item | Dosing group | Number of subjects | Analysis of variance table | | | |
|---|---|---|---|---|---|---|
| | | | Factor | Degrees of freedom (numerator) | F-value | p-value |
| Amount of change in social functioning (SF) | 10 mg | 6 | Dosing group | 3 | 1.19 | 0.3423 |
| | 30 mg | 6 | Time of measurement | 1 | 2.18 | 0.1571 |
| | 100 mg | 6 | Dosing group × time of measurement | 3 | 0.23 | 0.8765 |
| | Placebo | 6 | | | | |
| | Active group* | 18 | Dosing group | 1 | 0.73 | 0.4040 |
| | Placebo | 6 | Time of measurement | 1 | 1.26 | 0.2744 |
| | | | Dosing group × time of measurement | 1 | 0.07 | 0.7936 |
| Subscale item | Dosing group | Number of subjects | Analysis of variance table | | | |
| | | | Factor | Degrees of freedom (numerator) | F-value | p-value |
| Amount of change in role emotional (RE) | 10 mg | 6 | Dosing group | 3 | 0.54 | 0.6616 |
| | 30 mg | 6 | Time of measurement | 1 | 1.50 | 0.2359 |
| | 100 mg | 6 | Dosing group × time of measurement | 3 | 1.12 | 0.3666 |
| | Placebo | 6 | | | | |
| | Active group* | 18 | Dosing group | 1 | 0.00 | 0.9681 |
| | Placebo | 6 | Time of measurement | 1 | 0.14 | 0.7118 |
| | | | Dosing group × time of measurement | 1 | 2.03 | 0.1693 |
| Amount of change in mental health (MH) | 10 mg | 6 | Dosing group | 3 | 0.60 | 0.6219 |
| | 30 mg | 6 | Time of measurement | 1 | 3.06 | 0.0974 |
| | 100 mg | 6 | Dosing group × time of measurement | 3 | 0.17 | 0.9150 |
| | Placebo | 6 | | | | |
| | Active group* | 18 | Dosing group | 1 | 1.18 | 0.2894 |
| | Placebo | 6 | Time of measurement | 1 | 2.00 | 0.1731 |
| | | | Dosing group × time of measurement | 1 | 0.09 | 0.7656 |

*: Combined with 10 mg, 30 mg, and 100 mg dosing groups

Relationship between LVEF and LVESVI

In order to examine the correlation between the improvement of left ventricular remodeling and the improvement of cardiac function, i.e., the correlation between the decrease in LVESVI in cardiac-gated CT and the increase in LVEF in echocardiography, LVEF values were plotted on the x-axis and LVESVI values on the y-axis, and the movement of each subject between two time points of the baseline and 26 weeks after administration was plotted for each dose group, and shown in Fig. 41. In addition, the percentage of cases with a right downward straight line (the coefficient of the slope

of the straight line was negative and the LVESVI value at 26 weeks after administration was lower than the baseline value) was calculated for each dose group, and shown in Table 47.

The number of cases with a right downward straight line was 5/6 cases (83.3%), 2/5 cases (40.0%), 3/6 cases (50.0%), and 3/6 cases (50.0%) in the order of the YS-1402-10 mg group, 30 mg group, 100 mg group, and placebo group. There was no dose-dependent increase.

Table 47

| Dosing group | Number of subjects | Number of subjects with negative slope of straight line |
|---|---|---|
| 10 mg | 6 | 5 (83.3) |
| 30 mg | 5 | 2(40.0) |
| 100 mg | 6 | 3 (50.0) |
| Placebo | 6 | 3 (50.0) |

( ):%

*: When LVEF (left ventricular ejection fraction) is plotted on the x-axis, and LVESVI (left ventricular end-systolic volume index) on the y-axis, the slope between IC/S and 26 weeks after administration is negative, and the LVESVI value at 26 weeks after administration is lower than the value at IOS.

Conclusion of the pharmacokinetic analysis of ONO-1301 when YS-1402 was attached to the left ventricle during coronary artery bypass surgery for ischemic cardiomyopathy

The pharmacokinetics of active ONO-1301 in blood when YS-1402 was attached to the left ventricle during coronary artery bypass surgery for ischemic cardiomyopathy were checked.

Regarding the summary statistics of the pharmacokinetic parameters of ONO-1301 in blood, in the order of the YS-1402-10 mg group, 30 mg group, and 100 mg group, Cmax (mean ± standard deviation, hereinafter the same) was 2.0788 ± 1.1579, 4.2967 ± 1.5310, 8.8383 ± 2.1971 ng/ml, and AUCO-t was 1059.9076 ± 522.3988, 2640.5036 ± 730.4192, 5572.9516 ± 1190.7685 ng·h/ml. Cmax and AUCO-t increased with dose. On the other hand, when Cmax and AUCO-t in the YS-1402-10 mg group were each set to 1, Cmax and AUCO-t in the 30 mg group were 2.07 and 2.49 times, respectively, and similarly 4.25 and 5.26 times in the 100 mg group, which were less than the common ratio. MRT0-t was almost constant.

The blood concentration of ONO-1301 increased after administration and reached a plateau 7 to 14 days after administration in the YS-1402-10 mg group and 30 mg group. The blood concentration in the 100 mg group continually changed from about 4 ng/ml to 9 ng/ml from 24 hours after administration to 28 days, and then decreased rapidly. In all groups, drug concentrations in the blood almost disappeared 8 weeks after administration. The maximum Cmax for ONO-1301 in the YS-1402-100 mg group, which was the maximum dose group, was 11.900 ng/ml, which did not exceed the no-observed-effect level 15.61 ng/ml and the maximum no-observed-adverse-effect level 23.69 ng/ml, satisfying the secondary hypothesis that Cmax was 23.7 ng/ml or less.

Conclusion of the analysis of changes in indicators related to the improvement of cardiac function when YS-1402 was attached to the left ventricle during coronary artery bypass surgery for ischemic cardiomyopathy

Changes in indicators related to the improvement of cardiac function when YS-1402 was attached to the left ventricle during coronary artery bypass surgery for ischemic cardiomyopathy were searched. For the site of attachment, the area of reduced myocardial blood flow was identified in advance by preoperative ammonia PET. Lesion sites such as highly fibrotic regions and poor contraction regions in complex lesions and multivessel lesions where complete blood flow recovery to cardiac ischemia was difficult were also identified visually and tactilely in the coronary artery bypass surgery, and sheets were attached to the left ventricle site, including the peripheral part, where graft running was not affected.

For each evaluation item, as a result of analysis of variance of repeated measurements with factors of dose group, time of measurement, and dose group × time of measurement, variations in dose group and dose group × time of measurement were not significant. The results were similar for the two groups: the active groups combined with three doses of YS-1402 and the placebo group. On the other hand, variation in time of measurement was significant for various evaluation items, suggesting that coronary artery bypass surgery had a significant effect.

Regarding the amount of change in LVEF in echocardiography, the placebo group showed a slight increase over time. Compared to the placebo group, all the YS-1402 dosing groups showed improvement at 26 weeks after administration; however, there was no dose-related improvement. At 26 weeks after administration, the 10 mg dosing group showed improvement with an amount of change of 5.8% compared to the placebo group. A positive correlation was observed between the rate of change in total myocardial blood flow and the amount of change in LVEF from the

baseline at 26 weeks after administration of the investigational drug; however, the correlation was not significant (p-value: 0.340). One case in the YS-1402 30 mg group developed a severe adverse event (congestive heart failure) due to poor medication compliance 26 weeks after administration and 1 week before the test, which significantly decreased LVEF and thus also affected the average value of LVEF.

The rate of change in CT in cardiac-gated CT decreased in the placebo group at 2 weeks after administration, but increased at 26 weeks more than before administration. The YS-1402 groups generally showed an increase over time, surpassing the placebo group at 26 weeks, and a dose-related increase was observed. Compared to the placebo group, the 100 mg dosing group showed improvement with a rate of change of 10.16%. A positive correlation was observed between the rate of change in total myocardial blood flow and the rate of change in CT from the baseline at 26 weeks after administration of the investigational drug; however, the correlation was not significant (p-value: 0.102).

Regarding changes in left ventricular remodeling, the rate of change in LVESVI in cardiac-gated CT decreased over time in the placebo group. The YS-1402 groups also showed a decrease over time. Particularly at 2 weeks, the degree of decrease in the YS-1402 groups was greater than in the placebo group; however, the decrease was not dose-dependent. Considering that a reduction of 10% or more in the rate of change in LVESVI is reportedly used as a criterion for cardiac resynchronization therapy responder, and in consideration of measurement errors, fluctuations within 10% were defined as "constant." Then, aggregation was performed for each subject. As a result, decrease/constant/increase were 1/5/0 and 4/0/1 in the placebo group, in the order of 2 weeks and 26 weeks after administration. Similarly, these were 3/2/0 and 6/0/0 in the YS-1402-10 mg group, 3/2/0 and 3/1/2 in the 30 mg group, and 4/2/0 and 4/1/0 in the 100 mg group. The placebo group generally showed improvement over time. Compared to the placebo group, the YS-1402 groups showed a better degree of improvement at 2 weeks after administration, and was dose-dependent. At week 26, there was an increase in subjects in the YS-1402 30 mg group and placebo group, while the degree of improvement in the 10 mg group and 100 mg group was good, but was not dose-dependent. The rate of change in LVEDVI in cardiac-gated CT decreased over time in the placebo group. Similarly, the YS-1402 groups generally showed a decrease over time; however, it was not dose-dependent. The rate of change in LVDs and LVDd in echocardiography decreased over time in the placebo group. The YS-1402 groups showed a greater decrease than the placebo group at the second week of administration; however, it was not dose-dependent. The decrease was not temporal or dose-dependent at 6 weeks and 26 weeks after administration. The amount of change in CTR in chest X-ray reached its maximum on the first day after administration, and then decreased over time in the placebo group. Similarly, the YS-1402 groups showed an approximate maximum value on the first day after administration, and then showed a decrease over time. The maximum values and midway values were not dose-dependent.

Regarding changes in symptoms of heart failure, both the placebo group and the YS-1402 groups showed a similar degree of improvement over time for changes in the NYHA classification. Particularly in the 100 mg dosing group, all cases were improved to Level I at 26 weeks after administration. Regarding the rate of change in 6-minute walking distance, both the placebo group and the YS-1402 groups generally showed an increase in the walking distance over time, and at 6 weeks after administration, the YS-1402 groups showed a greater degree of increase than the placebo group. On the other hand, the 6-minute walking distance at 26 weeks after administration was similar to the placebo group in the 10 mg group, and exceeded the placebo group in the 100 mg group, showing an increase with a rate of change of 6.49% compared to the placebo group. The 30 mg group was lower than the placebo group. The reason for this was thought to be that one case in the 30 mg group developed a severe adverse event (congestive heart failure) due to poor medication compliance 26 weeks after administration and 1 week before the test, resulting in a significant decrease in distance from before administration. Therefore, for reference, the 26-week data of this case was excluded; however, there was no dose-related increase.

Regarding changes in myocardial blood flow by ammonia PET, in the placebo group, the LAD resting myocardial blood flow, LCX resting myocardial blood flow, and total myocardial blood flow at 26 weeks after administration slightly increased compared to the baseline. At 26 weeks after administration, a dose-related increase in blood flow was observed in the LAD resting myocardial blood flow, RCA resting myocardial blood flow, and total myocardial blood flow in the YS-1402-10 mg group, 30 mg group, and 100 mg group. In the 100 mg group, the LAD resting myocardial blood flow, RCA resting myocardial blood flow, and total myocardial blood flow at 26 weeks after administration increased compared to the placebo group as shown below: the rate of change in LAD resting myocardial blood flow was 21.18%, the rate of change in RCA resting myocardial blood flow was 17.04%, and the rate of change in total myocardial blood flow was 14.77%. On the other hand, the LCX resting myocardial blood flow did not show a dose-dependent increase. A positive correlation was observed between AUC0-t and the rate of change in total myocardial blood flow from the baseline at 26 weeks after administration of the investigational drug; however, the correlation was not significant (p-value: 0.160).

No definite tendency was observed in blood BNP at 26 weeks after administration.

Regarding SF-36, which was set for QOL assessment, transition was different for each subscale, and no definite tendency was observed.

There was no dose-response relationship between LVEF and LVESVI.

As a result of investigating the relationship over time between myocardial blood flow, cardiac function (CT,

LVEF), and 6-minute walking distance in the placebo group and YS-1402 dosing groups, the myocardial blood flow increased at 6 weeks after administration. At 26 weeks, even if it was similar or lower than that at 6 weeks, the cardiac function (CT, LVEF) was higher at 26 weeks than at 6 weeks (2 weeks for CT), and in some cases, the 6-minute walking distance increased. This suggested that the cardiac function and the symptoms of heart failure are improved with the increase in myocardial blood flow, and that the improvement in the cardiac function and the symptoms of heart failure continues even after the increase in myocardial blood flow has ceased. For example, in the YS-1402-100 mg group, the myocardial blood flow at 26 weeks after administration generally increased compared to 6 weeks, and the improvement in cardiac function (CT, LVEF) and the increase in 6-minute walking distance were recognized, suggesting the need for follow-up after 26 weeks.

Evaluation of safety

1) Adverse events

Table 48 shows the frequency of occurrence of adverse events and side effects.

Adverse events were observed in all 6 cases in any dosing group. Of these, those that were determined to be side effects were 2 cases (33.3%) in the YS-1402-10 mg group, 1 case (16.7%) in the 30 mg group, and 1 case (16.7%) in the placebo group. No cases of side effects were observed in the 100 mg group.

Serious adverse events were observed in 2 cases (33.3%) in the YS-1402-10 mg group and 30 mg group, 3 cases (50.0%) in the 100 mg group, and 2 cases (33.3%) in the placebo group. Of these, those that were determined to be serious side effects were pneumonia in 1 case (16.7%) in the YS-1402-10 mg group and lung abscess in 1 case (16.7%) in the placebo group.

There were no adverse events leading to discontinuation or deaths.

Table 48

| Endpoints | Dosing group | Number of subjects | Number of occurrences | Incidence |
|---|---|---|---|---|
| Adverse events | 10 mg | 6 | 6 | 100.0 |
| | 30 mg | 6 | 6 | 100.0 |
| | 100 mg | 6 | 6 | 100.0 |
| | Placebo | 6 | 6 | 100.0 |
| Serious adverse events | 10 mg | 6 | 2 | 33.3 |
| | 30 mg | 6 | 2 | 33.3 |
| | 100 mg | 6 | 3 | 50.0 |
| | Placebo | 6 | 2 | 33.3 |
| Side effects | 10 mg | 6 | 2 | 33.3 |
| | 30 mg | 6 | 1 | 16.7 |
| | 100 mg | 6 | 0 | 0.0 |
| | Placebo | 6 | 1 | 16.7 |
| Serious side effects | 10 mg | 6 | 1 | 16.7 |
| | 30 mg | 6 | 0 | 0.0 |
| | 100 mg | 6 | 0 | 0.0 |
| | Placebo | 6 | 1 | 16.7 |

Tables 49 and 50 show the frequency of occurrence of adverse events and side effects by SOC/PT, Tables 51 and 52 show the frequency of occurrence by degree, and Tables 53 and 54 show the frequency of occurrence by period of occurrence. Further, Table 55 shows the frequency of occurrence of adverse events by causal relationship, and Table 56 shows the frequency of occurrence by outcome.

By PT, abnormal clinical test values were frequently observed due to coronary artery bypass surgery; thus, the adverse events were classified into those of clinical test and those other than clinical test. That is, examination was performed on adverse events other than clinical test that occurred in at least 2 cases (33.3%) in any of the dosing groups by PT, and adverse events of clinical test that occurred in at least 5 cases (83.3%) in any of the dosing groups in the same way.

The adverse events other than clinical test were atrial fibrillation, tachycardia, diarrhea, edema, fever, dehydration, restlessness, sleep disturbance, and pleural effusion. The adverse events of clinical test were increased alanine

aminotransferase, increased aspartate aminotransferase, decreased blood albumin, increased blood creatine phosphokinase, increased blood lactate dehydrogenase, increased C-reactive protein, decreased hematocrit, decreased hemoglobin, decreased lymphocyte count, increased neutrophil count, decreased platelet count, decreased erythrocyte count, increased platelet count, and increased brain natriuretic peptide. Of these, pleural effusion was suspected to be related to the dose of YS-1402. Other than the above, there were no adverse events of clinical test with a suspected relationship, including four cases or less of adverse events in any of the dosing groups.

Pleural effusion was observed in 4 cases (66.7%) in the YS-1402-10 mg group, 6 cases (100.0%) in each of the 30 mg group and 100 mg group, and 2 cases (33.3%) in the placebo group. The severity of the disease was mild in 4 cases (66.7%) in the YS-1402-10 mg group, mild in 5 cases (83.3%) and moderate in 1 case (16.7%) in the 30 mg group, mild in 1 case (16.7%) and moderate in 5 cases (83.3%) in the 100 mg group, and mild in 2 cases (33.3%) in the placebo group. The time of onset was within 1 week after administration, and the outcome was recovery in all cases. The cause of pleural effusion was attributed to coronary artery bypass surgery and heart failure, and it was not considered to be a side effect. Pleural effusion could be treated by administration of diuretics or puncture, and was determined not to be a serious adverse event.

Among the adverse events, those that were determined to be side effects were 2 cases (33.3%) in the YS-1402-10 mg group, 1 case (16.7%) in the 30 mg group, and 1 case (16.7%) in the placebo group.

The aggregation by PT showed 1 case of pneumonia (16.7%) and 1 case of increased blood triglyceride and increased blood uric acid (16.7%) in the YS-1402-10 mg group, 1 case of increased alanine aminotransferase and increased aspartate aminotransferase (16.7%) in the 30 mg group, and 1 case of lung abscess (16.7%) in the placebo group. Of these, those determined to be serious were pneumonia in the YS-1402-10 mg group and lung abscess in the placebo group, and the severity was high in pneumonia and moderate in lung abscess. All four items in the clinical test were non-serious and mild.

Table 49

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 6 | 100.0 | 125 | 6 | 100.0 | 119 | 6 | 100.0 | 107 | 6 | 100.0 | 110 |
| Heart failure | | 2 | 33.3 | 2 | 1 | 16.7 | 2 | 5 | 83.3 | 8 | 3 | 50.0 | 4 |
| | Atrial fibrillation | 0 | 0.0 | 0 | 1 | 16.7 | 1 | 3 | 50.0 | 3 | 1 | 16.7 | 1 |
| | First-degree atrioventricular block | 0 | 0.0 | 0 | 0 | 0.0 | 0 | 1 | 16.7 | 1 | 0 | 0.0 | 0 |
| | Right bundle branch block | 0 | 0.0 | 0 | 0 | 0.0 | 0 | 0 | 0.0 | 0 | 1 | 16.7 | 1 |
| | Cardiac tamponade | 0 | 0.0 | 0 | 0 | 0.0 | 0 | 1 | 16.7 | 1 | 0 | 0.0 | 0 |
| | Mitral regurgitation | 0 | 0.0 | 0 | 0 | 0.0 | 0 | 1 | 16.7 | 1 | 0 | 0.0 | 0 |
| | Pericardial effusion | 0 | 0.0 | 0 | 0 | 0.0 | 0 | 1 | 16.7 | 1 | 0 | 0.0 | 0 |
| | Sinus tachycardia | 0 | 0.0 | 0 | 0 | 0.0 | 0 | 0 | 0.0 | 0 | 1 | 16.7 | 1 |
| | Tachycardia | 2 | 33.3 | 2 | 1 | 16.7 | 1 | 0 | 0.0 | 0 | 1 | 16.7 | 1 |
| | Ventricular fibrillation | 0 | 0.0 | 0 | 0 | 0.0 | 0 | 1 | 16.7 | 1 | 0 | 0.0 | 0 |
| Gastrointestinal disorders | | 3 | 50.0 | 5 | 2 | 33.3 | 2 | 1 | 16.7 | 3 | 3 | 50.0 | 3 |
| | Constipation | 1 | 16.7 | 1 | 0 | 0.0 | 0 | 0 | 0.0 | 0 | 1 | 16.7 | 1 |
| | Dianhea | 0 | 0.0 | 0 | 2 | 33.3 | 2 | 0 | 0.0 | 0 | 2 | 33.3 | 2 |
| | Dry lips | 1 | 16.7 | 1 | 0 | 0.0 | 0 | 0 | 0.0 | 0 | 0 | 0.0 | 0 |
| | Lip ulcer | 1 | 16.7 | 1 | 0 | 0.0 | 0 | 0 | 0.0 | 0 | 0 | 0.0 | 0 |
| | Vomiting | 1 | 16.7 | 1 | 0 | 0.0 | 0 | 1 | 16.7 | 2 | 0 | 0.0 | 0 |
| | Anal bleeding | 0 | 0.0 | 0 | 0 | 0.0 | 0 | 1 | 16.7 | 1 | 0 | 0.0 | 0 |
| | Loose stool | 1 | 16.7 | 1 | 0 | 0.0 | 0 | 0 | 0.0 | 0 | 0 | 0.0 | 0 |
| General/systemic disorders and administration site conditions | | 3 | 50.0 | 4 | 5 | 83.3 | 8 | 5 | 83.3 | 6 | 4 | 66.7 | 4 |
| | Chills | 0 | 0.0 | 0 | 0 | 0.0 | 0 | 1 | 16.7 | 1 | 0 | 0.0 | 0 |
| | Poor healing | 0 | 0.0 | 0 | 1 | 16.7 | 1 | 0 | 0.0 | 0 | 0 | 0.0 | 0 |
| | Edema | 1 | 16.7 | 1 | 0 | 0.0 | 0 | 2 | 33.3 | 2 | 0 | 0.0 | 0 |
| | Fever | 2 | 33.3 | 3 | 5 | 83.3 | 7 | 3 | 50.0 | 3 | 4 | 66.7 | 4 |

(continued)

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 | 1 | 16.7 | 1 |
| | Acute cholangitis | 0 | 0.0 | 0 | 0 | 0.0 | 0 | 0 | 0.0 | 0 | 1 | 16.7 | 1 |
| MedDRA/J Version 18.1 | | | | | | | | | | | | | |
| Infectious and parasitic diseases | | 2 | 33.3 | 2 | 2 | 33.3 | 2 | 1 | 16.7 | 1 | 1 | 16.7 | 2 |
| | Bronchitis | 1 | 16.7 | 1 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 1 | 16.7 | 1 |
| | Mediastinitis | 0 | 0.0 | 0 | 1 | 16.7 | 1 | 0 | 0.0 | 0 | 0 | 0.0 | 0 |
| | Pneumonia | 1 | 16.7 | 1 | 1 | 16.7 | 1 | 0 | 0.0 | 0 | 1 | 16.7 | 1 |
| | Tracheostomy site infection | 0 | 0.0 | 0 | 0 | 0.0 | 0 | 1 | 16.7 | 1 | 0 | 0.0 | 0 |
| Injury, poisoning and procedural complications | | 0 | 0.0 | 0 | 1 | 16.7 | 1 | 0 | 0.0 | 0 | 1 | 16.7 | 1 |
| | Wound dehiscence | 0 | 0.0 | 0 | 1 | 16.7 | 1 | 0 | 0.0 | 0 | 1 | 16.7 | 1 |
| Clinical test | | 6 | 100.0 | 97 | 6 | 100.0 | 94 | 6 | 100.0 | 77 | 6 | 100.0 | 86 |
| | Increased alanine aminotransferase | 5 | 83.3 | 5 | 2 | 33.3 | 3 | 1 | 16.7 | 2 | 2 | 33.3 | 2 |
| | Increased amylase | 1 | 16.7 | 1 | 0 | 0.0 | 0 | 0 | 0.0 | 0 | 0 | 0.0 | 0 |
| | Increased aspartate aminotransferase | 6 | 100.0 | 6 | 3 | 50.0 | 3 | 1 | 16.7 | 1 | 3 | 50.0 | 3 |
| | Decreased blood albumin | 6 | 100.0 | 6 | 4 | 66.7 | 5 | 1 | 16.7 | 1 | 2 | 33.3 | 2 |
| | Decreased blood chloride | 0 | 0.0 | 0 | 1 | 16.7 | 1 | 0 | 0.0 | 0 | 1 | 16.7 | 1 |
| | Decreased blood cholesterol | 0 | 0.0 | 0 | 3 | 50.0 | 3 | 0 | 0.0 | 0 | 1 | 16.7 | 1 |
| | Increased blood creatine phosphokinase | 6 | 100.0 | 6 | 4 | 66.7 | 4 | 4 | 66.7 | 4 | 5 | 83.3 | 5 |

(continued)

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 2 | 33.3 | 2 | 1 | 16.7 | 1 | 1 | 16.7 | 1 |
| | Increased blood glucose | 4 | 66.7 | 4 | 2 | 33.3 | 2 | 0 | 0.0 | 0 | 3 | 50.0 | 3 |
| | Increased blood lactate dehydrogenase | 6 | 100.0 | 6 | 5 | 83.3 | 5 | 3 | 50.0 | 3 | 6 | 100.0 | 6 |
| | Increased blood potassium | 0 | 0.0 | 0 | 1 | 16.7 | 1 | 0 | 0.0 | 0 | 2 | 33.3 | 2 |
| | Decreased blood pressure | 1 | 16.7 | 1 | 0 | 0.0 | 0 | 0 | 0.0 | 0 | 0 | 0.0 | 0 |
| | Increased blood pressure | 0 | 0.0 | 0 | 1 | 16.7 | 1 | 0 | 0.0 | 0 | 0 | 0.0 | 0 |
| | Decreased blood sodium | 0 | 0.0 | 0 | 1 | 16.7 | 1 | 0 | 0.0 | 0 | 1 | 16.7 | 1 |
| | Increased blood triglyceride | 1 | 16.7 | 1 | 1 | 16.7 | 1 | 0 | 0.0 | 0 | 0 | 0.0 | 0 |
| | Increased blood urea | 0 | 0.0 | 0 | 2 | 33.3 | 2 | 1 | 16.7 | 1 | 0 | 0.0 | 0 |
| | Decreased blood uric acid | 0 | 0.0 | 0 | 0 | 0.0 | 0 | 1 | 16.7 | 1 | 0 | 0.0 | 0 |
| | Increased blood uric acid | 1 | 16.7 | 1 | 2 | 33.3 | 2 | 0 | 0.0 | 0 | 0 | 0.0 | 0 |
| MedDRA/J Version 18.1 | | | | | | | | | | | | | |
| Clinical test | Increased C-reactive protein | 6 | 100.0 | 6 | 6 | 100.0 | 6 | 6 | 100.0 | 6 | 6 | 100.0 | 6 |
| | Electrocardiographic T-wave inversion | 0 | 0.0 | 0 | 3 | 50.0 | 3 | 2 | 33.3 | 2 | 2 | 33.3 | 2 |
| | Increased eosinophil count | 1 | 16.7 | 1 | 0 | 0.0 | 0 | 1 | 16.7 | 1 | 0 | 0.0 | 0 |

(continued)

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased hematocrit | 5 | 83.3 | 5 | 4 | 66.7 | 4 | 6 | 100.0 | 6 | 6 | 100.0 | 6 |
| | Decreased hemoglobin | 5 | 83.3 | 5 | 4 | 66.7 | 4 | 6 | 100.0 | 6 | 6 | 100.0 | 6 |
| | Decreased heart rate | 1 | 16.7 | 1 | 0 | 0.0 | 0 | 0 | 0.0 | 0 | 0 | 0.0 | 0 |
| | Decreased lymphocyte count | 6 | 100.0 | 6 | 6 | 100.0 | 6 | 6 | 100.0 | 6 | 6 | 100.0 | 6 |
| | Decreased monocyte count | 1 | 16.7 | 1 | 0 | 0.0 | 0 | 1 | 16.7 | 1 | 0 | 0.0 | 0 |
| | Increased monocyte count | 1 | 16.7 | 1 | 0 | 0.0 | 0 | 0 | 0.0 | 0 | 0 | 0.0 | 0 |
| | Increased neutrophil count | 6 | 100.0 | 6 | 6 | 100.0 | 6 | 6 | 100.0 | 6 | 6 | 100.0 | 6 |
| | Oxygen desaturation | 0 | 0.0 | 0 | 0 | 0.0 | 0 | 0 | 0.0 | 0 | 1 | 16.7 | 1 |
| | Decreased platelet count | 4 | 66.7 | 4 | 5 | 83.3 | 6 | 4 | 66.7 | 5 | 4 | 66.7 | 4 |
| | Increased prothrombin time | 2 | 33.3 | 2 | 2 | 33.3 | 2 | 1 | 16.7 | 2 | 2 | 33.3 | 2 |
| | Decreased erythrocyte count | 5 | 83.3 | 5 | 4 | 66.7 | 4 | 6 | 100.0 | 6 | 6 | 100.0 | 6 |
| | Increased leukocyte count | 4 | 66.7 | 4 | 4 | 66.7 | 4 | 3 | 50.0 | 4 | 2 | 33.3 | 2 |
| | Increased platelet count | 4 | 66.7 | 4 | 6 | 100.0 | 7 | 4 | 66.7 | 4 | 6 | 100.0 | 6 |
| | Increased brain natriuretic peptide | 5 | 83.3 | 5 | 5 | 83.3 | 5 | 6 | 100.0 | 7 | 5 | 83.3 | 5 |
| | Increased blood alkaline phosphatase | 4 | 66.7 | 4 | 1 | 16.7 | 1 | 1 | 16.7 | 1 | 1 | 16.7 | 1 |

(continued)

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Metabolic and nutritional disorders | | 1 | 16.7 | 1 | 2 | 33.3 | 2 | 0 | 0.0 | 0 | 2 | 33.3 | 2 |
| | Acidosis | 0 | 0.0 | 0 | 0 | 0.0 | 0 | 0 | 0.0 | 0 | 1 | 16.7 | 1 |
| | Dehydration | 0 | 0.0 | 0 | 2 | 33.3 | 2 | 0 | 0.0 | 0 | 0 | 0.0 | 0 |
| | Hypovolemia | 1 | 16.7 | 1 | 0 | 0.0 | 0 | 0 | 0.0 | 0 | 1 | 16.7 | 1 |
| Musculoskeletal and connective tissue disorders | | 1 | 16.7 | 1 | 1 | 16.7 | 1 | 0 | 0.0 | 0 | 0 | 0.0 | 0 |
| | Muscle pain | 1 | 16.7 | 1 | 0 | 0.0 | 0 | 0 | 0.0 | 0 | 0 | 0.0 | 0 |
| | Musculoskeletal stiffness | 0 | 0.0 | 0 | 1 | 16.7 | 1 | 0 | 0.0 | 0 | 0 | 0.0 | 0 |
| MedDRA/J Version 18.1 | | | | | | | | | | | | | |
| Nervous system disorders | | 0 | 0.0 | 0 | 0 | 0.0 | 0 | 0 | 0.0 | 0 | 1 | 16.7 | 1 |
| | Pitaminal hemorrhage | 0 | 0.0 | 0 | 0 | 0.0 | 0 | 0 | 0.0 | 0 | 1 | 16.7 | 1 |
| Mental disorders | | 5 | 83.3 | 6 | 0 | 0.0 | 0 | 0 | 0.0 | 0 | 0 | 0.0 | 0 |
| | Delirium | 1 | 16.7 | 1 | 0 | 0.0 | 0 | 0 | 0.0 | 0 | 0 | 0.0 | 0 |
| | Restlessness | 2 | 33.3 | 2 | 0 | 0.0 | 0 | 0 | 0.0 | 0 | 0 | 0.0 | 0 |
| | Sleeping disorder | 3 | 50.0 | 3 | 0 | 0.0 | 0 | 0 | 0.0 | 0 | 0 | 0.0 | 0 |
| Nenal and urinary tract disorders | | 0 | 0.0 | 0 | 0 | 0.0 | 0 | 1 | 16.7 | 1 | 0 | 0.0 | 0 |
| | Kidney failure | 0 | 0.0 | 0 | 0 | 0.0 | 0 | 1 | 16.7 | 1 | 0 | 0.0 | 0 |
| Reproductive system and breast disorders | | 0 | 0.0 | 0 | 1 | 16.7 | 1 | 0 | 0.0 | 0 | 0 | 0.0 | 0 |
| | Gynecomastia | 0 | 0.0 | 0 | 1 | 16.7 | 1 | 0 | 0.0 | 0 | 0 | 0.0 | 0 |

(continued)

| System organ classification | Preferred term | 10mg(N=6) Number of examples | 10mg(N=6) Incidence [%] | 10mg(N=6) Number of occurrences | 30 mg (N=6) Number of examples | 30 mg (N=6) Incidence [%] | 30 mg (N=6) Number of occurrences | 100 mg (N=6) Number of examples | 100 mg (N=6) Incidence [%] | 100 mg (N=6) Number of occurrences | Placebo (N=6) Number of examples | Placebo (N=6) Incidence [%] | Placebo (N=6) Number of occurrences |
|---|---|---|---|---|---|---|---|---|---|---|---|---|---|
| Respiratory, thoracic and mediastinal disorders | | 4 | 66.7 | 6 | 6 | 100.0 | 6 | 6 | 100.0 | 9 | 4 | 66.7 | 4 |
| | Atelectasis | 1 | 16.7 | 1 | 0 | 0.0 | 0 | 1 | 16.7 | 1 | 1 | 16.7 | 1 |
| | Difficulty breathing | 0 | 0.0 | 0 | 0 | 0.0 | 0 | 1 | 16.7 | 1 | 0 | 0.0 | 0 |
| | Pleural effusion | 4 | 66.7 | 4 | 6 | 100.0 | 6 | 6 | 100.0 | 6 | 2 | 33.3 | 2 |
| | Aspiration pneumonia | 0 | 0.0 | 0 | 0 | 0.0 | 0 | 0 | 0.0 | 0 | 1 | 16.7 | 1 |
| | Pulmonary congestion | 0 | 0.0 | 0 | 0 | 0.0 | 0 | 1 | 16.7 | 1 | 0 | 0.0 | 0 |
| | Respiratory failure | 1 | 16.7 | 1 | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 1 | 16.7 | 1 | 2 | 33.3 | 2 |
| | Decubitus ulcer | 0 | 0.0 | 0 | 0 | 0.0 | 0 | 1 | 16.7 | 1 | 0 | 0.0 | 0 |
| | Drug eruption | 1 | 16.7 | 1 | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 | 1 | 16.7 | 1 |
| | Hives | 0 | 0.0 | 0 | 0 | 0.0 | 0 | 0 | 0.0 | 0 | 1 | 16.7 | 1 |
| Vascular disorder | | 0 | 0.0 | 0 | 0 | 0.0 | 0 | 1 | 16.7 | 1 | 0 | 0.0 | 0 |
| | Thrombosis | 0 | 0.0 | 0 | 0 | 0.0 | 0 | 1 | 16.7 | 1 | 0 | 0.0 | 0 |

MedDRA/J Version 18.1

Table 50

| System organ classification | Preferred term | 10mg(N=6) | | | 30 mg (N=6) | | | 100 mg (N=6) | | | Placebo (N=6) | | |
|---|---|---|---|---|---|---|---|---|---|---|---|---|---|
| | | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 2 | 33.3 | 3 | 1 | 16.7 | 2 | 0 | 0.0 | 0 | 1 | 16.7 | 1 |
| Infectious and parasitic diseases | | 1 | 16.7 | 1 | 0 | 0.0 | 0 | 0 | 0.0 | 0 | 1 | 16.7 | 1 |
| | Lung abscess | 0 | 0.0 | 0 | 0 | 0.0 | 0 | 0 | 0.0 | 0 | 1 | 16.7 | 1 |
| | Pneumonia | 1 | 16.7 | 1 | 0 | 0.0 | 0 | 0 | 0.0 | 0 | 0 | 0.0 | 0 |
| Clinical test | | 1 | 16.7 | 2 | 1 | 16.7 | 2 | 0 | 0.0 | 0 | 0 | 0.0 | 0 |
| | Increased alanine aminotransferase | 0 | 0.0 | 0 | 1 | 16.7 | 1 | 0 | 0.0 | 0 | 0 | 0.0 | 0 |
| | Increased aspartate aminotransferase | 0 | 0.0 | 0 | 1 | 16.7 | 1 | 0 | 0.0 | 0 | 0 | 0.0 | 0 |
| | Increased blood triglyceride | 1 | 16.7 | 1 | 0 | 0.0 | 0 | 0 | 0.0 | 0 | 0 | 0.0 | 0 |
| | Increased blood uric acid | 1 | 16.7 | 1 | 0 | 0.0 | 0 | 0 | 0.0 | 0 | 0 | 0.0 | 0 |

MedDRA/J Version 18.1

Table 51

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 99 | 4 | 66.7 | 24 | 2 | 33.3 | 2 |
| Heart failure | | 2 | 33.3 | 2 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 2 | 33.3 | 2 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 3 | 50.0 | 5 | 0 | 0.0 | 0 | 0 | 0.0 | 0 |
| | Constipation | 1 | 16.7 | 1 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 1 | 16.7 | 1 | 0 | 0.0 | 0 | 0 | 0.0 | 0 |
| | Lip ulcer | 1 | 16.7 | 1 | 0 | 0.0 | 0 | 0 | 0.0 | 0 |
| | Vomiting | 1 | 16.7 | 1 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 1 | 16.7 | 1 | 0 | 0.0 | 0 | 0 | 0.0 | 0 |
| General/systemic disorders and administration site conditions | | 2 | 33.3 | 3 | 1 | 16.7 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 1 | 16.7 | 1 | 0 | 0.0 | 0 | 0 | 0.0 | 0 |
| | Fever | 1 | 16.7 | 2 | 1 | 16.7 | 1 | 0 | 0.0 | 0 |

(continued)

| | | Whole period | | | | | | | | |
|---|---|---|---|---|---|---|---|---|---|---|
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 99 | 4 | 66.7 | 19 | 1 | 16.7 | 1 |
| Heart failure | | 0 | 0.0 | 1 | 1 | 16.7 | 1 | 0 | 0.0 | 0 |
| | Atrial fibrillation | 1 | 16.7 | 1 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 2 | 33.3 | 2 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 2 | 33.3 | 2 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 2 | 33.3 | 5 | 3 | 50.0 | 3 | 0 | 0.0 | 0 |
| | Chills | 0 | 0.0 | 0 | 0 | 0.0 | 0 | 0 | 0.0 | 0 |
| | Poor healing | 1 | 16.7 | 1 | 0 | 0.0 | 0 | 0 | 0.0 | 0 |
| | Edema | 0 | 0.0 | 0 | 0 | 0.0 | 0 | 0 | 0.0 | 0 |
| | Fever | 2 | 33.3 | 4 | 3 | 50.0 | 3 | 0 | 0.0 | 0 |

(continued)

| Severity | | Whole period | | | | | | | | |
|---|---|---|---|---|---|---|---|---|---|---|
| | | 100 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 78 | 4 | 66.7 | 28 | 1 | 16.7 | 1 |
| Heart failure | | 1 | 16.7 | 2 | 3 | 50.0 | 5 | 1 | 16.7 | 1 |
| | Atrial fibrillation | 1 | 16.7 | 1 | 2 | 33.3 | 2 | 0 | 0.0 | 0 |
| | First-degree atrioventricular block | 1 | 16.7 | 1 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 1 | 16.7 | 1 | 0 | 0.0 | 0 |
| | Mitral regurgitation | 0 | 0.0 | 0 | 1 | 16.7 | 1 | 0 | 0.0 | 0 |
| | Pericardial effusion | 0 | 0.0 | 0 | 1 | 16.7 | 1 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 1 | 16.7 | 1 |
| Gastrointestinal disorders | | 1 | 16.7 | 3 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 1 | 16.7 | 2 | 0 | 0.0 | 0 | 0 | 0.0 | 0 |
| | Anal bleeding | 1 | 16.7 | 1 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 3 | 50.0 | 4 | 2 | 33.3 | 2 | 0 | 0.0 | 0 |
| | Chills | 1 | 16.7 | 1 | 0 | 0.0 | 0 | 0 | 0.0 | 0 |
| | Poor healing | 0 | 0.0 | 0 | 0 | 0.0 | 0 | 0 | 0.0 | 0 |
| | Edema | 2 | 33.3 | 2 | 0 | 0.0 | 0 | 0 | 0.0 | 0 |
| | Fever | 1 | 16.7 | 1 | 2 | 33.3 | 2 | 0 | 0.0 | 0 |

(continued)

| | | Whole period | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 4 | 66.7 | 99 | 1 | 16.7 | 10 | 1 | 16.7 | 1 |
| Heart failure | | 2 | 33.3 | 3 | 1 | 16.7 | 1 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 1 | 16.7 | 1 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 1 | 16.7 | 1 | 0 | 0.0 | 0 | 0 | 0.0 | 0 |
| | Tachycardia | 1 | 16.7 | 1 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 3 | 50.0 | 3 | 0 | 0.0 | 0 | 0 | 0.0 | 0 |
| | Constipation | 1 | 16.7 | 1 | 0 | 0.0 | 0 | 0 | 0.0 | 0 |
| | Dianhea | 2 | 33.3 | 2 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 4 | 66.7 | 4 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 4 | 66.7 | 4 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 99 | 4 | 66.7 | 24 | 2 | 33.3 | 2 |
| Heart failure | | 2 | 33.3 | 2 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 2 | 33.3 | 2 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 3 | 50.0 | 5 | 0 | 0.0 | 0 | 0 | 0.0 | 0 |
| | Constipation | 1 | 16.7 | 1 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 1 | 16.7 | 1 | 0 | 0.0 | 0 | 0 | 0.0 | 0 |
| | Lip ulcer | 1 | 16.7 | 1 | 0 | 0.0 | 0 | 0 | 0.0 | 0 |
| | Vomiting | 1 | 16.7 | 1 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 1 | 16.7 | 1 | 0 | 0.0 | 0 | 0 | 0.0 | 0 |
| General/systemic disorders and administration site conditions | | 2 | 33.3 | 3 | 1 | 16.7 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 1 | 16.7 | 1 | 0 | 0.0 | 0 | 0 | 0.0 | 0 |
| | Fever | 1 | 16.7 | 2 | 1 | 16.7 | 1 | 0 | 0.0 | 0 |

(continued)

| Severity | | Safety evaluation period (within 6 weeks after administration) | | | | | | | | |
|---|---|---|---|---|---|---|---|---|---|---|
| | | 30 mg (N=6) | | | | | | | | |
| | | Mild | | | Moderate | | | High | | |
| System organ classification | Preferred term | Number of examples | Incidence 1%1 | Number of occurrences | Number of examples | Incidence [%\| | Number of occurrences | Number of examples | Incidence [%] | Number of occurrences |
| Whole | | 2 | 33.3 | 93 | 3 | 50.0 | 15 | 1 | 16.7 | 1 |
| Heart failure | | 0 | 0.0 | 1 | 1 | 16.7 | 1 | 0 | 0.0 | 0 |
| | Atrial fibrillation | 1 | 16.7 | 1 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 2 | 33.3 | 2 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 2 | 33.3 | 2 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 3 | 50.0 | 5 | 2 | 33.3 | 2 | 0 | 0.0 | 0 |
| | Chills | 0 | 0.0 | 0 | 0 | 0.0 | 0 | 0 | 0.0 | 0 |
| | Poor healing | 1 | 16.7 | 1 | 0 | 0.0 | 0 | 0 | 0.0 | 0 |
| | Edema | 0 | 0.0 | 0 | 0 | 0.0 | 0 | 0 | 0.0 | 0 |
| | Fever | 3 | 50.0 | 4 | 2 | 33.3 | 2 | 0 | 0.0 | 0 |

(continued)

| | | Safety evaluation period (within 6 weeks after administration) | | | | | | | | |
|---|---|---|---|---|---|---|---|---|---|---|
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 74 | 4 | 66.7 | 27 | 1 | 16.7 | 1 |
| Heart failure | | 1 | 16.7 | 2 | 3 | 50.0 | 4 | 1 | 16.7 | 1 |
| | Atrial fibrillation | 1 | 16.7 | 1 | 2 | 33.3 | 2 | 0 | 0.0 | 0 |
| | First-degree atrioventricular block | 1 | 16.7 | 1 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 1 | 16.7 | 1 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 1 | 16.7 | 1 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 1 | 16.7 | 1 |
| Gastrointestinal disorders | | 1 | 16.7 | 1 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 1 | 16.7 | 1 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 3 | 50.0 | 4 | 2 | 33.3 | 2 | 0 | 0.0 | 0 |
| | Chills | 1 | 16.7 | 1 | 0 | 0.0 | 0 | 0 | 0.0 | 0 |
| | Poor healing | 0 | 0.0 | 0 | 0 | 0.0 | 0 | 0 | 0.0 | 0 |
| | Edema | 2 | 33.3 | 2 | 0 | 0.0 | 0 | 0 | 0.0 | 0 |
| | Fever | 1 | 16.7 | 1 | 2 | 33.3 | 2 | 0 | 0.0 | 0 |

(continued)

| Severity | | Placebo (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 4 | 66.7 | 99 | 1 | 16.7 | 10 | 1 | 16.7 | 1 |
| Heart failure | | 2 | 33.3 | 3 | 1 | 16.7 | 1 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 1 | 16.7 | 1 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 1 | 16.7 | 1 | 0 | 0.0 | 0 | 0 | 0.0 | 0 |
| | Tachycardia | 1 | 16.7 | 1 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 3 | 50.0 | 3 | 0 | 0.0 | 0 | 0 | 0.0 | 0 |
| | Constipation | 1 | 16.7 | 1 | 0 | 0.0 | 0 | 0 | 0.0 | 0 |
| | Dianhea | 2 | 33.3 | 2 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 4 | 66.7 | 4 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 4 | 66.7 | 4 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 2 | 33.3 | 6 | 3 | 50.0 | 4 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 1 | 16.7 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 1 | 16.7 | 1 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 2 | 33.3 | 4 | 1 | 16.7 | 1 | 0 | 0.0 | 0 |
| Heart failure | | 0 | 0.0 | 0 | 1 | 16.7 | 1 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 1 | 16.7 | 1 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 1 | 16.7 | 2 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 1 | 16.7 | 2 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 1 | 16.7 | 1 | 0 | 0.0 | 0 | 1 | 16.7 | 1 |
| | Bronchitis | 1 | 16.7 | 1 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 75 | 6 | 100.0 | 22 | 0 | 0.0 | 0 |
| | Increased alanine aminotransferase | 3 | 50.0 | 3 | 2 | 33.3 | 2 | 0 | 0.0 | 0 |
| | Increased amylase | 1 | 16.7 | 1 | 0 | 0.0 | 0 | 0 | 0.0 | 0 |
| | Increased aspartate aminotransferase | 4 | 66.7 | 4 | 2 | 33.3 | 2 | 0 | 0.0 | 0 |
| | Decreased blood albumin | 6 | 100.0 | 6 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 6 | 100.0 | 6 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 4 | 66.7 | 4 | 0 | 0.0 | 0 | 0 | 0.0 | 0 |
| | Increased blood lactate dehydrogenase | 5 | 83.3 | 5 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 1 | 16.7 | 1 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 1 | 16.7 | 1 | 0 | 0.0 | 0 | 0 | 0.0 | 0 |

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | Acute cholangitis | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 1 | 16.7 | 1 | 1 | 16.7 | 1 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 1 | 16.7 | 1 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 2 | 33.3 | 82 | 4 | 66.7 | 12 | 0 | 0.0 | 0 |
| | Increased alanine aminotransferase | 1 | 16.7 | 2 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 2 | 33.3 | 2 | 1 | 16.7 | 1 | 0 | 0.0 | 0 |
| | Decreased blood albumin | 4 | 66.7 | 5 | 0 | 0.0 | 0 | 0 | 0.0 | 0 |
| | Decreased blood chloride | 1 | 16.7 | 1 | 0 | 0.0 | 0 | 0 | 0.0 | 0 |
| | Decreased blood cholesterol | 3 | 50.0 | 3 | 0 | 0.0 | 0 | 0 | 0.0 | 0 |
| | Increased blood creatine phosphokinase | 4 | 66.7 | 4 | 0 | 0.0 | 0 | 0 | 0.0 | 0 |

(continued)

| Severity | | Mild | | | Moderate | | | High | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 2 | 33.3 | 2 | 0 | 0.0 | 0 | 0 | 0.0 | 0 |
| | Increased blood glucose | 2 | 33.3 | 2 | 0 | 0.0 | 0 | 0 | 0.0 | 0 |
| | Increased blood lactate dehydrogenase | 4 | 66.7 | 4 | 1 | 16.7 | 1 | 0 | 0.0 | 0 |
| | Increased blood potassium | 1 | 16.7 | 1 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 1 | 16.7 | 1 | 0 | 0.0 | 0 |
| | Decreased blood sodium | 1 | 16.7 | 1 | 0 | 0.0 | 0 | 0 | 0.0 | 0 |
| | Increased blood triglyceride | 0 | 0.0 | 0 | 1 | 16.7 | 1 | 0 | 0.0 | 0 |

| | | Whole period | | | | | | | | |
|---|---|---|---|---|---|---|---|---|---|---|
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| Severity | | Mild | | | Moderate | | | High | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 1 | 16.7 | 1 | 0 | 0.0 | 0 |
| Injury poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 5 | 83.3 | 65 | 1 | 16.7 | 12 | 0 | 0.0 | 0 |
| | Increased alanine aminotransferase | 1 | 16.7 | 2 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 1 | 16.7 | 1 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 3 | 50.0 | 3 | 1 | 16.7 | 1 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 2 | 33.3 | 2 | 1 | 16.7 | 1 | 0 | 0.0 | 0 |

(continued)

| Severity | | Mild | | | Moderate | | | High | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 1 | 16.7 | 1 | 0 | 0.0 | 0 |
| | Acute cholangitis | 0 | 0.0 | 0 | 1 | 16.7 | 1 | 0 | 0.0 | 0 |
| Infectious and parasitic diseases | | 0 | 0.0 | 0 | 1 | 16.7 | 2 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 1 | 16.7 | 1 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| injury, poisoning and procedural complications | | 1 | 16.7 | 1 | 0 | 0.0 | 0 | 0 | 0.0 | 0 |
| | Wound dehiscence | 1 | 16.7 | 1 | 0 | 0.0 | 0 | 0 | 0.0 | 0 |
| Clinical test | | 5 | 83.3 | 81 | 1 | 16.7 | 5 | 0 | 0.0 | 0 |
| | Increased alanine aminotransferase | 2 | 33.3 | 2 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased amylase | 0 | 0.0 | 0 | 0 | 0.0 | 0 | 0 | 0.0 | 0 |
| | Increased aspartate aminotransferase | 3 | 50.0 | 3 | 0 | 0.0 | 0 | 0 | 0.0 | 0 |
| | Decreased blood albumin | 2 | 33.3 | 2 | 0 | 0.0 | 0 | 0 | 0.0 | 0 |
| | Decreased blood chloride | 1 | 16.7 | 1 | 0 | 0.0 | 0 | 0 | 0.0 | 0 |
| | Decreased blood cholesterol | 1 | 16.7 | 1 | 0 | 0.0 | 0 | 0 | 0.0 | 0 |
| | Increased blood creatine phosphokinase | 5 | 83.3 | 5 | 0 | 0.0 | 0 | 0 | 0.0 | 0 |
| | Increased blood creatinine | 1 | 16.7 | 1 | 0 | 0.0 | 0 | 0 | 0.0 | 0 |
| | Increased blood glucose | 3 | 50.0 | 3 | 0 | 0.0 | 0 | 0 | 0.0 | 0 |
| | Increased blood lactate dehydrogenase | 6 | 100.0 | 6 | 0 | 0.0 | 0 | 0 | 0.0 | 0 |
| | Increased blood potassium | 2 | 33.3 | 2 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 1 | 16.7 | 1 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 1 | 16.7 | 1 | 0 | 0.0 | 0 | 1 | 16.7 | 1 |
| | Bronchitis | 1 | 16.7 | 1 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 0 | 0.0 | 75 | 6 | 100.0 | 22 | 0 | 0.0 | 0 |
| | Increased alanine aminotransferase | 3 | 50.0 | 3 | 2 | 33.3 | 2 | 0 | 0.0 | 0 |
| | Increased amylase | 1 | 16.7 | 1 | 0 | 0.0 | 0 | 0 | 0.0 | 0 |
| | Increased aspartate aminotransferase | 4 | 66.7 | 4 | 2 | 33.3 | 2 | 0 | 0.0 | 0 |
| | Decreased blood albumin | 6 | 100.0 | 6 | 0 | 0.0 | 0 | 0 | 0.0 | 0 |
| | Decreased blood chloride | 0 | 0.0 | 0 | 0 | 0.0 | 0 | 0 | 0.0 | 0 |

(continued)

| Severity | | Mild | | | Moderate | | | High | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased blood cholesterol | 0 | 0.0 | 0 | 0 | 0.0 | 0 | 0 | 0.0 | 0 |
| | Increased blood creatine phosphokinase | 6 | 100.0 | 6 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 4 | 66.7 | 4 | 0 | 0.0 | 0 | 0 | 0.0 | 0 |
| | Increased blood lactate dehydrogenase | 5 | 83.3 | 5 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 1 | 16.7 | 1 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 1 | 16.7 | 1 | 0 | 0.0 | 0 | 0 | 0.0 | 0 |

| | | Safety evaluation period (within 6 weeks after administration) | | | | | | | | |
|---|---|---|---|---|---|---|---|---|---|---|
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 1 | 16.7 | 1 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 1 | 16.7 | 1 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Clinical test | | 4 | 66.7 | 78 | 2 | 33.3 | 10 | 0 | 0.0 | 0 |
| | Increased alanine aminotransferase | 0 | 0.0 | 0 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 1 | 16.7 | 1 | 1 | 16.7 | 1 | 0 | 0.0 | 0 |
| | Decreased blood albumin | 4 | 66.7 | 5 | 0 | 0.0 | 0 | 0 | 0.0 | 0 |
| | Decreased blood chloride | 1 | 16.7 | 1 | 0 | 0.0 | 0 | 0 | 0.0 | 0 |
| | Decreased blood cholesterol | 3 | 50.0 | 3 | 0 | 0.0 | 0 | 0 | 0.0 | 0 |
| | Increased blood creatine phosphokinase | 4 | 66.7 | 4 | 0 | 0.0 | 0 | 0 | 0.0 | 0 |
| | Increased blood creatinine | 2 | 33.3 | 2 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood glucose | 2 | 33.3 | 2 | 0 | 0.0 | 0 | 0 | 0.0 | 0 |
| | Increased blood lactate dehydrogenase | 4 | 66.7 | 4 | 1 | 16.7 | 1 | 0 | 0.0 | 0 |
| | Increased blood potassium | 1 | 16.7 | 1 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 1 | 16.7 | 1 | 0 | 0.0 | 0 | 0 | 0.0 | 0 |
| | Increased blood triglyceride | 0 | 0.0 | 0 | 0 | 0.0 | 0 | 0 | 0.0 | 0 |
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 1 | 16.7 | 1 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 5 | 83.3 | 63 | 1 | 16.7 | 12 | 0 | 0.0 | 0 |
| | Increased alanine aminotransferase | 1 | 16.7 | 2 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 1 | 16.7 | 1 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 3 | 50.0 | 3 | 1 | 16.7 | 1 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 2 | 33.3 | 2 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 1 | 16.7 | 1 | 0 | 0.0 | 0 |
| | Acute cholangitis | 0 | 0.0 | 0 | 1 | 16.7 | 1 | 0 | 0.0 | 0 |
| Infectious and parasitic diseases | | 0 | 0.0 | 0 | 1 | 16.7 | 2 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 1 | 16.7 | 1 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 1 | 16.7 | 1 | 0 | 0.0 | 0 | 0 | 0.0 | 0 |
| | Wound dehiscence | 1 | 16.7 | 1 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 5 | 83.3 | 81 | 1 | 16.7 | 5 | 0 | 0.0 | 0 |
| | Increased alanine aminotransferase | 2 | 33.3 | 2 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 3 | 50.0 | 3 | 0 | 0.0 | 0 | 0 | 0.0 | 0 |
| | Decreased blood albumin | 2 | 33.3 | 2 | 0 | 0.0 | 0 | 0 | 0.0 | 0 |
| | Decreased blood chloride | 1 | 16.7 | 1 | 0 | 0.0 | 0 | 0 | 0.0 | 0 |
| | Decreased blood cholesterol | 1 | 16.7 | 1 | 0 | 0.0 | 0 | 0 | 0.0 | 0 |
| | Increased blood creatine phosphokinase | 5 | 83.3 | 5 | 0 | 0.0 | 0 | 0 | 0.0 | 0 |
| | Increased blood creatinine | 1 | 16.7 | 1 | 0 | 0.0 | 0 | 0 | 0.0 | 0 |
| | Increased blood glucose | 3 | 50.0 | 3 | 0 | 0.0 | 0 | 0 | 0.0 | 0 |
| | Increased blood lactate dehydrogenase | 6 | 100.0 | 6 | 0 | 0.0 | 0 | 0 | 0.0 | 0 |
| | Increased blood potassium | 2 | 33.3 | 2 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 1 | 16.7 | 1 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood triglyceride | 0 | 0.0 | 0 | 0 | 0.0 | 0 | 0 | 0.0 | 0 |

MedDRA/J Version 18.1

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 2 | 33.3 | 4 | 2 | 33.3 | 2 | 0 | 0.0 | 0 |
| | Increased alanine aminotransferase | 2 | 33.3 | 2 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 1 | 16.7 | 1 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |

(continued)

| Severity | | 30 mg (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 1 | 16.7 | 1 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |

| Severity | | 100 mg (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 2 | 33.3 | 2 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |

(continued)

| Severity | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence % | Number of occurrences | Number of examples | Incidence % | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 1 | 16.7 | 1 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 1 | 16.7 | 1 | 0 | 0.0 | 0 | 0 | 0.0 | 0 |
| | Decreased hematocrit | 2 | 33.3 | 2 | 3 | 50.0 | 3 | 0 | 0.0 | 0 |
| | Decreased hemoglobin | 2 | 33.3 | 2 | 3 | 50.0 | 3 | 0 | 0.0 | 0 |
| | Decreased heart rate | 0 | 0.0 | 0 | 1 | 16.7 | 1 | 0 | 0.0 | 0 |
| | Decreased lymphocyte count | 6 | 100.0 | 6 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence % | Number of occurrences | Number of examples | Incidence % | Number of occurrences | Number of examples | Incidence [%] | Number of occurrences |
| | Decreased monocyte count | 1 | 16.7 | 1 | 0 | 0.0 | 0 | 0 | 0.0 | 0 |
| | Increased monocyte count | 1 | 16.7 | 1 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 2 | 33.3 | 2 | 3 | 50.0 | 3 | 0 | 0.0 | 0 |
| | Increased leukocyte count | 4 | 66.7 | 4 | 0 | 0.0 | 0 | 0 | 0.0 | 0 |
| | Increased platelet count | 4 | 66.7 | 4 | 0 | 0.0 | 0 | 0 | 0.0 | 0 |
| | Increased brain natriuretic peptide | 3 | 50.0 | 3 | 2 | 33.3 | 2 | 0 | 0.0 | 0 |
| | Increased blood alkaline phosphatase | 1 | 16.7 | 1 | 3 | 50.0 | 3 | 0 | 0.0 | 0 |
| Metabolic and nutritional disorders | | 1 | 16.7 | 1 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |

(continued)

| Severity | | 30 mg (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 2 | 33.3 | 2 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 2 | 33.3 | 2 | 0 | 0.0 | 0 | 0 | 0.0 | 0 |
| | Increased C-reactive protein | 6 | 100.0 | 6 | 0 | 0.0 | 0 | 0 | 0.0 | 0 |
| | Electrocardiographic T-wave inversion | 3 | 50.0 | 3 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 2 | 33.3 | 2 | 2 | 33.3 | 2 | 0 | 0.0 | 0 |
| | Decreased hemoglobin | 2 | 33.3 | 2 | 2 | 33.3 | 2 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 5 | 1 | 16.7 | 1 | 0 | 0.0 | 0 |
| | Increased prothrombin time | 2 | 33.3 | 2 | 0 | 0.0 | 0 | 0 | 0.0 | 0 |
| | Decreased erythrocyte count | 2 | 33.3 | 2 | 2 | 33.3 | 2 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased leukocyte count | 4 | 66.7 | 4 | 0 | 0.0 | 0 | 0 | 0.0 | 0 |
| | Increased platelet count | 6 | 100.0 | 7 | 0 | 0.0 | 0 | 0 | 0.0 | 0 |
| | Increased brain natriuretic peptide | 5 | 83.3 | 5 | 0 | 0.0 | 0 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 2 | 33.3 | 2 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 2 | 33.3 | 2 | 0 | 0.0 | 0 | 0 | 0.0 | 0 |
| | Hypovolemia | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 1 | 16.7 | 1 | 0 | 0.0 | 0 |
| | Decreased blood uric acid | 1 | 16.7 | 1 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 2 | 33.3 | 2 | 0 | 0.0 | 0 | 0 | 0.0 | 0 |
| | Increased eosinophil count | 1 | 16.7 | 1 | 0 | 0.0 | 0 | 0 | 0.0 | 0 |
| | Decreased hematocrit | 5 | 83.3 | 5 | 1 | 16.7 | 1 | 0 | 0.0 | 0 |
| | Decreased hemoglobin | 5 | 83.3 | 5 | 1 | 16.7 | 1 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 1 | 16.7 | 1 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 5 | 0 | 0.0 | 0 | 0 | 0.0 | 0 |
| | Increased prothrombin time | 1 | 16.7 | 2 | 0 | 0.0 | 0 | 0 | 0.0 | 0 |
| | Decreased erythrocyte count | 5 | 83.3 | 5 | 1 | 16.7 | 1 | 0 | 0.0 | 0 |
| | Increased leukocyte count | 2 | 33.3 | 3 | 1 | 16.7 | 1 | 0 | 0.0 | 0 |
| | Increased platelet count | 4 | 66.7 | 4 | 0 | 0.0 | 0 | 0 | 0.0 | 0 |
| | Increased brain natriuretic peptide | 5 | 83.3 | 6 | 1 | 16.7 | 1 | 0 | 0.0 | 0 |
| | Increased blood alkaline phosphatase | 0 | 0.0 | 0 | 1 | 16.7 | 1 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |

(continued)

| Severity | | Placebo (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 2 | 33.3 | 2 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 5 | 83.3 | 5 | 1 | 16.7 | 1 | 0 | 0.0 | 0 |
| | Decreased hemoglobin | 5 | 83.3 | 5 | 1 | 16.7 | 1 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 1 | 16.7 | 1 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 5 | 83.3 | 5 | 1 | 16.7 | 1 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased leukocyte count | 2 | 33.3 | 2 | 0 | 0.0 | 0 | 0 | 0.0 | 0 |
| | Increased platelet count | 6 | 100.0 | 6 | 0 | 0.0 | 0 | 0 | 0.0 | 0 |
| | Increased brain natriuretic peptide | 4 | 66.7 | 4 | 1 | 16.7 | 1 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 2 | 33.3 | 2 | 0 | 0.0 | 0 | 0 | 0.0 | 0 |
| | Acidosis | 1 | 16.7 | 1 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 1 | 16.7 | 1 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 1 | 16.7 | 1 | 0 | 0.0 | 0 | 0 | 0.0 | 0 |

(continued)

| Severity | | Safety evaluation period (within 6 weeks after administration) | | | | | | | | |
|---|---|---|---|---|---|---|---|---|---|---|
| | | 10 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased hematocrit | 2 | 33.3 | 2 | 3 | 50.0 | 3 | 0 | 0.0 | 0 |
| | Decreased hemoglobin | 2 | 33.3 | 2 | 3 | 50.0 | 3 | 0 | 0.0 | 0 |
| | Decreased heart rate | 0 | 0.0 | 0 | 1 | 16.7 | 1 | 0 | 0.0 | 0 |
| | Decreased lymphocyte count | 6 | 100.0 | 6 | 0 | 0.0 | 0 | 0 | 0.0 | 0 |
| | Decreased monocyte count | 1 | 16.7 | 1 | 0 | 0.0 | 0 | 0 | 0.0 | 0 |
| | Increased monocyte count | 1 | 16.7 | 1 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 2 | 33.3 | 2 | 3 | 50.0 | 3 | 0 | 0.0 | 0 |
| | Increased leukocyte count | 4 | 66.7 | 4 | 0 | 0.0 | 0 | 0 | 0.0 | 0 |
| | Increased platelet count | 4 | 66.7 | 4 | 0 | 0.0 | 0 | 0 | 0.0 | 0 |
| | Increased brain natriuretic peptide | 3 | 50.0 | 3 | 2 | 33.3 | 2 | 0 | 0.0 | 0 |
| | Increased blood alkaline phosphatase | 1 | 16.7 | 1 | 3 | 50.0 | 3 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Metabolic and nutritional disorders | | 1 | 16.7 | 1 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 2 | 33.3 | 2 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 2 | 33.3 | 2 | 0 | 0.0 | 0 | 0 | 0.0 | 0 |
| | Increased C-reactive protein | 6 | 100.0 | 6 | 0 | 0.0 | 0 | 0 | 0.0 | 0 |
| | Electrocardiographic T-wave inversion | 3 | 50.0 | 3 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 2 | 33.3 | 2 | 2 | 33.3 | 2 | 0 | 0.0 | 0 |
| | Decreased hemoglobin | 2 | 33.3 | 2 | 2 | 33.3 | 2 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 5 | 1 | 16.7 | 1 | 0 | 0.0 | 0 |
| | Increased prothrombin time | 2 | 33.3 | 2 | 0 | 0.0 | 0 | 0 | 0.0 | 0 |
| | Decreased erythrocyte count | 2 | 33.3 | 2 | 2 | 33.3 | 2 | 0 | 0.0 | 0 |
| | Increased leukocyte count | 4 | 66.7 | 4 | 0 | 0.0 | 0 | 0 | 0.0 | 0 |
| | Increased platelet count | 6 | 100.0 | 6 | 0 | 0.0 | 0 | 0 | 0.0 | 0 |
| | Increased brain natriuretic peptide | 5 | 83.3 | 5 | 0 | 0.0 | 0 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 2 | 33.3 | 2 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 2 | 33.3 | 2 | 0 | 0.0 | 0 | 0 | 0.0 | 0 |
| | Hypovolemia | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 1 | 16.7 | 1 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood uric acid | 1 | 16.7 | 1 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 2 | 33.3 | 2 | 0 | 0.0 | 0 | 0 | 0.0 | 0 |
| | Increased eosinophil count | 1 | 16.7 | 1 | 0 | 0.0 | 0 | 0 | 0.0 | 0 |
| | Decreased hematocrit | 5 | 83.3 | 5 | 1 | 16.7 | 1 | 0 | 0.0 | 0 |
| | Decreased hemoglobin | 5 | 83.3 | 5 | 1 | 16.7 | 1 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 1 | 16.7 | 1 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 5 | 0 | 0.0 | 0 | 0 | 0.0 | 0 |
| | Increased prothrombin time | 1 | 16.7 | 1 | 0 | 0.0 | 0 | 0 | 0.0 | 0 |
| | Decreased erythrocyte count | 5 | 83.3 | 5 | 1 | 16.7 | 1 | 0 | 0.0 | 0 |
| | Increased leukocyte count | 2 | 33.3 | 3 | 1 | 16.7 | 1 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased platelet count | 4 | 66.7 | 4 | 0 | 0.0 | 0 | 0 | 0.0 | 0 |
| | Increased brain natriuretic peptide | 5 | 83.3 | 5 | 1 | 16.7 | 1 | 0 | 0.0 | 0 |
| | Increased blood alkaline phosphatase | 0 | 0.0 | 0 | 1 | 16.7 | 1 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 5 | 83.3 | 5 | 1 | 16.7 | 1 | 0 | 0.0 | 0 |
| | Electrocardiographic T-wave inversion | 2 | 33.3 | 2 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 5 | 83.3 | 5 | 1 | 16.7 | 1 | 0 | 0.0 | 0 |
| | Decreased hemoglobin | 5 | 83.3 | 5 | 1 | 16.7 | 1 | 0 | 0.0 | 0 |
| | Decreased heart rate | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 1 | 16.7 | 1 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 5 | 83.3 | 5 | 1 | 16.7 | 1 | 0 | 0.0 | 0 |
| | Increased leukocyte count | 2 | 33.3 | 2 | 0 | 0.0 | 0 | 0 | 0.0 | 0 |
| | Increased platelet count | 6 | 100.0 | 6 | 0 | 0.0 | 0 | 0 | 0.0 | 0 |
| | Increased brain natriuretic peptide | 4 | 66.7 | 4 | 1 | 16.7 | 1 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 2 | 33.3 | 2 | 0 | 0.0 | 0 | 0 | 0.0 | 0 |
| | Acidosis | 1 | 16.7 | 1 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |

(continued)

| Severity | | 30 mg (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 |
| | Increased platelet count | 1 | 16.7 | 1 | 0 | 0.0 | 0 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 1 | 16.7 | 1 | 0 | 0.0 | 0 | 0 | 0.0 | 0 |
| | Decreased erythrocyte count | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 1 | 16.7 | 1 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |

(continued)

| Severity | | Placebo (N=6) | | | | | | | | |
|---|---|---|---|---|---|---|---|---|---|---|
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 0 | 0.0 | 0 |
| | Muscle pain | 1 | 16.7 | 1 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 5 | 83.3 | 6 | 0 | 0.0 | 0 | 0 | 0.0 | 0 |
| | Delirium | 1 | 16.7 | 1 | 0 | 0.0 | 0 | 0 | 0.0 | 0 |
| | Restlessness | 2 | 33.3 | 2 | 0 | 0.0 | 0 | 0 | 0.0 | 0 |
| | Sleeping disorder | 3 | 50.0 | 3 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 3 | 50.0 | 5 | 0 | 0.0 | 0 | 1 | 16.7 | 1 |
| | Atelectasis | 1 | 16.7 | 1 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 4 | 66.7 | 4 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 1 | 16.7 | 1 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Skin and subcutaneous tissue disorders | | 0 | 0.0 | 0 | 1 | 16.7 | 1 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 1 | 16.7 | 1 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 1 | 16.7 | 1 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 1 | 16.7 | 1 | 0 | 0.0 | 0 | 0 | 0.0 | 0 |
| | Gynecomastia | 1 | 16.7 | 1 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Respiratory, thoracic and mediastinal disorders | | 5 | 83.3 | 5 | 1 | 16.7 | 1 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 5 | 83.3 | 5 | 1 | 16.7 | 1 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Nenal and urinary tract disorders | | 0 | 0.0 | 0 | 1 | 16.7 | 1 | 0 | 0.0 | 0 |
| | Kidney failure | 0 | 0.0 | 0 | 1 | 16.7 | 1 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 1 | 16.7 | 3 | 5 | 83.3 | 6 | 0 | 0.0 | 0 |
| | Atelectasis | 1 | 16.7 | 1 | 0 | 0.0 | 0 | 0 | 0.0 | 0 |
| | Difficulty breathing | 0 | 0.0 | 0 | 1 | 16.7 | 1 | 0 | 0.0 | 0 |
| | Pleural effusion | 1 | 16.7 | 1 | 5 | 83.3 | 5 | 0 | 0.0 | 0 |
| | Aspiration pneumonia | 0 | 0.0 | 0 | 0 | 0.0 | 0 | 0 | 0.0 | 0 |
| | Pulmonary congestion | 1 | 16.7 | 1 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 0 | 0.0 | 0 |
| | Decubitus ulcer | 1 | 16.7 | 1 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 1 | 16.7 | 1 |
| | Pitaminal hemorrhage | 0 | 0.0 | 0 | 0 | 0.0 | 0 | 1 | 16.7 | 1 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 3 | 50.0 | 3 | 1 | 16.7 | 1 | 0 | 0.0 | 0 |
| | Atelectasis | 1 | 16.7 | 1 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 2 | 33.3 | 2 | 0 | 0.0 | 0 | 0 | 0.0 | 0 |
| | Aspiration pneumonia | 0 | 0.0 | 0 | 1 | 16.7 | 1 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 2 | 33.3 | 2 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 1 | 16.7 | 1 | 0 | 0.0 | 0 | 0 | 0.0 | 0 |
| | Hives | 1 | 16.7 | 1 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 0 | 0.0 | 0 |
| | Muscle pain | 1 | 16.7 | 1 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 5 | 83.3 | 6 | 0 | 0.0 | 0 | 0 | 0.0 | 0 |
| | Delirium | 1 | 16.7 | 1 | 0 | 0.0 | 0 | 0 | 0.0 | 0 |
| | Restlessness | 2 | 33.3 | 2 | 0 | 0.0 | 0 | 0 | 0.0 | 0 |
| | Sleeping disorder | 3 | 50.0 | 3 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 3 | 50.0 | 5 | 0 | 0.0 | 0 | 1 | 16.7 | 1 |
| | Atelectasis | 1 | 16.7 | 1 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 4 | 66.7 | 4 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 1 | 16.7 | 1 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Skin and subcutaneous tissue disorders | | 0 | 0.0 | 0 | 1 | 16.7 | 1 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 1 | 16.7 | 1 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Respiratory, thoracic and mediastinal disorders | | 5 | 83.3 | 5 | 1 | 16.7 | 1 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 5 | 83.3 | 5 | 1 | 16.7 | 1 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Nenal and urinary tract disorders | | 0 | 0.0 | 0 | 1 | 16.7 | 1 | 0 | 0.0 | 0 |
| | Kidney failure | 0 | 0.0 | 0 | 1 | 16.7 | 1 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 1 | 16.7 | 3 | 5 | 83.3 | 6 | 0 | 0.0 | 0 |
| | Atelectasis | 1 | 16.7 | 1 | 0 | 0.0 | 0 | 0 | 0.0 | 0 |
| | Difficulty breathing | 0 | 0.0 | 0 | 1 | 16.7 | 1 | 0 | 0.0 | 0 |
| | Pleural effusion | 1 | 16.7 | 1 | 5 | 83.3 | 5 | 0 | 0.0 | 0 |
| | Aspiration pneumonia | 0 | 0.0 | 0 | 0 | 0.0 | 0 | 0 | 0.0 | 0 |
| | Pulmonary congestion | 1 | 16.7 | 1 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 0 | 0.0 | 0 |
| | Decubitus ulcer | 1 | 16.7 | 1 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system | | 0 | 0.0 | 0 | 0 | 0.0 | 0 | 1 | 16.7 | 1 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| disorders | Putaminal hemorrhage | 0 | 0.0 | 0 | 0 | 0.0 | 0 | 1 | 16.7 | 1 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract | | 0 | 0.0 | 0 | 0 | 0.0 | 0 | 0 | 0.0 | 0 |
| disorders | Kidney failure | 0 | 0.0 | 0 | 0 | 0.0 | 0 | 0 | 0.0 | 0 |
| Reproductive system | | 0 | 0.0 | 0 | 0 | 0.0 | 0 | 0 | 0.0 | 0 |
| and breast disorders | Gynecomastia | 0 | 0.0 | 0 | 0 | 0.0 | 0 | 0 | 0.0 | 0 |
| Respiratory, thoracic | | 3 | 50.0 | 3 | 1 | 16.7 | 1 | 0 | 0.0 | 0 |
| and mediastinal | Atelectasis | 1 | 16.7 | 1 | 0 | 0.0 | 0 | 0 | 0.0 | 0 |
| disorders | Difficulty breathing | 0 | 0.0 | 0 | 0 | 0.0 | 0 | 0 | 0.0 | 0 |
| | Pleural effusion | 2 | 33.3 | 2 | 0 | 0.0 | 0 | 0 | 0.0 | 0 |
| | Aspiration pneumonia | 0 | 0.0 | 0 | 1 | 16.7 | 1 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous | | 2 | 33.3 | 2 | 0 | 0.0 | 0 | 0 | 0.0 | 0 |
| tissue disorders | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 1 | 16.7 | 1 | 0 | 0.0 | 0 | 0 | 0.0 | 0 |
| | Hives | 1 | 16.7 | 1 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 1 | 16.7 | 1 | 0 | 0.0 | 0 | 0 | 0.0 | 0 |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system | | 1 | 16.7 | 1 | 0 | 0.0 | 0 | 0 | 0.0 | 0 |

(continued)

| | | 30 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| and breast disorders | Gynecomastia | 1 | 16.7 | 1 | 0 | 0.0 | 0 | 0 | 0.0 | 0 |
| Respiratory, thoracic | | 0 | 0.0 | 0 | 0 | 0.0 | 0 | 0 | 0.0 | 0 |
| and mediastinal | Atelectasis | 0 | 0.0 | 0 | 0 | 0.0 | 0 | 0 | 0.0 | 0 |
| disorders | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous | | 0 | 0.0 | 0 | 0 | 0.0 | 0 | 0 | 0.0 | 0 |
| tissue disorders | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| | | Whole period | | | | | | | | |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| | | Whole period | | | | | | | | |
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 1 | 16.7 | 1 | 0 | 0.0 | 0 |
| | Thrombosis | 0 | 0.0 | 0 | 1 | 16.7 | 1 | 0 | 0.0 | 0 |
| | | Whole period | | | | | | | | |
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| | | Safety evaluation period (within 6 weeks after administration) | | | | | | | | |
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| | | Safety evaluation period (within 6 weeks after administration) | | | | | | | | |
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 1 | 16.7 | 1 | 0 | 0.0 | 0 |
| | Thrombosis | 0 | 0.0 | 0 | 1 | 16.7 | 1 | 0 | 0.0 | 0 |
| | | Safety evaluation period (within 6 weeks after administration) | | | | | | | | |
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

| | | Safety and efficacy evaluation period (more than 6 weeks after administration to 26 weeks after administration) | | | | | | | | |
|---|---|---|---|---|---|---|---|---|---|---|
| | | 30 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| MedDRA/J Version 18.1 | | | | | | | | | | |

| | | Safety and efficacy evaluation period (more than 6 weeks after administration to 26 weeks after administration) | | | | | | | | |
|---|---|---|---|---|---|---|---|---|---|---|
| | | 100 mg (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| MedDRA/J Version 18.1 | | | | | | | | | | |

(continued)

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| MedDRA/J Version 18.1 | | | | | | | | | | |

Table 52

| Severity System organ classification | Preferred term | Whole period | | | | | | | | |
|---|---|---|---|---|---|---|---|---|---|---|
| | | 10 mg (N=6) | | | | | | | | |
| | | Mild | | | Moderate | | | High | | |
| | | Number of examples | Incidence [%] | Number of occurrences | Number of examples | Incidence [%] | Number of occurrences | Number of examples | Incidence [%] | Number of occurrences |
| Whole | | 1 | 16.7 | 2 | 0 | 0.0 | 0 | 1 | 16.7 | 1 |
| Infectious and parasitic diseases | | 0 | 0.0 | 0 | 0 | 0.0 | 0 | 1 | 16.7 | 1 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 |
| Clinical test | | 1 | 16.7 | 2 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 1 | 16.7 | 1 | 0 | 0.0 | 0 | 0 | 0.0 | 0 |
| | Increased blood uric acid | 1 | 16.7 | 1 | 0 | 0.0 | 0 | 0 | 0.0 | 0 |

MedDRA/J Version 18.1

| Severity System organ classification | Preferred term | Whole period | | | | | | | | |
|---|---|---|---|---|---|---|---|---|---|---|
| | | 30 mg (N=6) | | | | | | | | |
| | | Mild | | | Moderate | | | High | | |
| | | Number of examples | Incidence [%] | Number of occurrences | Number of examples | Incidence [%] | Number of occurrences | Number of examples | Incidence [%] | Number of occurrences |
| Whole | | 1 | 16.7 | 2 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 1 | 16.7 | 2 | 0 | 0.0 | 0 | 0 | 0.0 | 0 |
| | Increased alanine aminotransferase | 1 | 16.7 | 1 | 0 | 0.0 | 0 | 0 | 0.0 | 0 |

(continued)

| Severity | | Whole period | | | | | | | | |
|---|---|---|---|---|---|---|---|---|---|---|
| | | 30 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 1 | 16.7 | 1 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

| | | 100 mg (N=6) | | | | | | | | |
|---|---|---|---|---|---|---|---|---|---|---|
| | | Mild | | | Moderate | | | High | | |
| Number of examples | Incidence [%] | Number of occurrences | Number of examples | Incidence [%] | Number of occurrences | Number of examples | Incidence [%] | Number of occurrences | | |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

(continued)

| Severity | | Whole period | | | | | | | | |
|---|---|---|---|---|---|---|---|---|---|---|
| | | Placebo (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 1 | 0 | 0.0 | 0 |
| Infectious and parasitic diseases | | 0 | 0.0 | 0 | 1 | 16.7 | 1 | 0 | 0.0 | 0 |
| | Lung abscess | 0 | 0.0 | 0 | 1 | 16.7 | 1 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

| Severity | | Safety evaluation period (within 6 weeks after administration) | | | | | | | | |
|---|---|---|---|---|---|---|---|---|---|---|
| | | 10 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 2 | 0 | 0.0 | 0 | 1 | 16.7 | 1 |
| Infectious and parasitic diseases | | 0 | 0.0 | 0 | 0 | 0.0 | 0 | 1 | 16.7 | 1 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 |
| Clinical test | | 1 | 16.7 | 2 | 0 | 0.0 | 0 | 0 | 0.0 | 0 |

(continued)

| Severity | | Safety evaluation period (within 6 weeks after administration) | | | | | | | | |
|---|---|---|---|---|---|---|---|---|---|---|
| | | 10 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 1 | 16.7 | 1 | 0 | 0.0 | 0 | 0 | 0.0 | 0 |
| | Increased blood uric acid | 1 | 16.7 | 1 | 0 | 0.0 | 0 | 0 | 0.0 | 0 |

MedDRA/J Version 18.1

| Severity | | Safety evaluation period (within 6 weeks after administration) | | | | | | | | |
|---|---|---|---|---|---|---|---|---|---|---|
| | | 30 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| Severity | | Safety evaluation period (within 6 weeks after administration) | | | | | | | | |
|---|---|---|---|---|---|---|---|---|---|---|
| | | 30 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood uric acid | 0 | 0.0 | 0 | 0 | 0.0 | 0 | 0 | 0.0 | 0 |

MedDRA/J Version 18.1

| | | 100 mg (N=6) | | | | | | | | |
|---|---|---|---|---|---|---|---|---|---|---|
| | | Mild | | | Moderate | | | High | | |
| Number of examples | Incidence [%] | Number of occurrences | Number of examples | Incidence [%] | Number of occurrences | Number of examples | Incidence [%] | Number of occurrences | | |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

(continued)

| Severity | | Safety evaluation period (within 6 weeks after administration) | | | | | | | | |
|---|---|---|---|---|---|---|---|---|---|---|
| | | Placebo (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 1 | 0 | 0.0 | 0 |
| Infectious and parasitic diseases | | 0 | 0.0 | 0 | 1 | 16.7 | 1 | 0 | 0.0 | 0 |
| | Lung abscess | 0 | 0.0 | 0 | 1 | 16.7 | 1 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

| Severity | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |

(continued)

| Severity | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

| Severity | | Safety and efficacy evaluation period (more than 6 weeks after administration to 26 weeks after administration) | | | | | | | | |
|---|---|---|---|---|---|---|---|---|---|---|
| | | 30 mg (N=6) | | | | | | | | |
| | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 2 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 1 | 16.7 | 2 | 0 | 0.0 | 0 | 0 | 0.0 | 0 |
| | Increased alanine aminotransferase | 1 | 16.7 | 1 | 0 | 0.0 | 0 | 0 | 0.0 | 0 |
| | Increased aspartate aminotransferase | 1 | 16.7 | 1 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

(continued)

| | | 100 mg (N=6) | | | | | | | | |
|---|---|---|---|---|---|---|---|---|---|---|
| | | Mild | | | Moderate | | | High | | |
| Number of examples | Incidence [%] | Number of occurrences | Number of examples | Incidence [%] | Number of occurrences | Number of examples | Incidence [%] | Number of occurrences | | |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| MedDRA/J Version 18.1 | | | | | | | | | | |

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Severity | | Mild | | | Moderate | | | High | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

Table 53

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 6 | 100.0 | 108 | 0 | 0.0 | 12 | 0 | 0.0 | 5 |
| Heart failure | | 2 | 33.3 | 2 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 2 | 33.3 | 2 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 2 | 33.3 | 3 | 1 | 16.7 | 1 | 0 | 0.0 | 1 |
| | Constipation | 1 | 16.7 | 1 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 1 | 16.7 | 1 |
| | Lip ulcer | 1 | 16.7 | 1 | 0 | 0.0 | 0 | 0 | 0.0 | 0 |
| | Vomiting | 1 | 16.7 | 1 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 1 | 16.7 | 1 | 0 | 0.0 | 0 |
| General/systemic disorders and administration site conditions | | 3 | 50.0 | 3 | 0 | 0.0 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 1 | 16.7 | 1 | 0 | 0.0 | 0 | 0 | 0.0 | 0 |
| | Fever | 2 | 33.3 | 2 | 0 | 0.0 | 1 | 0 | 0.0 | 0 |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| MedDRA/J Version 18.1 | | | | | | | | | | |

| | | 10 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | 10 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| MedDRA/J Version 18.1 | | | | | | | | | | |

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 6 | 100.0 | 88 | 0 | 0.0 | 11 | 0 | 0.0 | 8 |
| Heart failure | | 1 | 16.7 | 2 | 0 | 0.0 | 0 | 0 | 0.0 | 0 |
| | Atrial fibrillation | 1 | 16.7 | 1 | 0 | 0.0 | 0 | 0 | 0.0 | 0 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 1 | 16.7 | 1 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 1 | 16.7 | 1 | 1 | 16.7 | 1 | 0 | 0.0 | 0 |
| | Constipation | 0 | 0.0 | 0 | 0 | 0.0 | 0 | 0 | 0.0 | 0 |
| | Diarrhea | 1 | 16.7 | 1 | 1 | 16.7 | 1 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 5 | 83.3 | 5 | 0 | 0.0 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 1 | 16.7 | 1 | 0 | 0.0 | 0 |
| | Edema | 0 | 0.0 | 0 | 0 | 0.0 | 0 | 0 | 0.0 | 0 |
| | Fever | 5 | 83.3 | 5 | 0 | 0.0 | 0 | 0 | 0.0 | 0 |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 2 | 0 | 0.0 | 4 | 0 | 0.0 | 6 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 1 | 0 | 0.0 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 1 | 0 | 0.0 | 1 | 0 | 0.0 | 0 |
| Hepatobiliary disorders | | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Acute cholangitis | 0 | 0.0 | 0 | 0 | 0.0 | 0 | 0 | 0.0 | 0 |
| MedDRA/J Version 18.1 | | | | | | | | | | |

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 6 | 100.0 | 82 | 0 | 0.0 | 10 | 0 | 0.0 | 4 |
| Heart failure | | 4 | 66.7 | 5 | 1 | 16.7 | 1 | 0 | 0.0 | 1 |
| | Atrial fibrillation | 3 | 50.0 | 3 | 0 | 0.0 | 0 | 0 | 0.0 | 0 |
| | First-degree atrioventricular block | 1 | 16.7 | 1 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 1 | 16.7 | 1 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 1 | 16.7 | 1 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 1 | 16.7 | 1 | 0 | 0.0 | 0 |

(continued)

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 1 | 16.7 | 1 | 0 | 0.0 | 0 |
| | Constipation | 0 | 0.0 | 0 | 0 | 0.0 | 0 | 0 | 0.0 | 0 |
| | Dianhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 1 | 16.7 | 1 | 0 | 0.0 | 0 |
| | Loose stool | 0 | 0.0 | 0 | 0 | 0.0 | 0 | 0 | 0.0 | 0 |
| General/systemic disorders and administration site conditions | | 5 | 83.3 | 6 | 0 | 0.0 | 0 | 0 | 0.0 | 0 |
| | Chills | 1 | 16.7 | 1 | 0 | 0.0 | 0 | 0 | 0.0 | 0 |
| | Poor healing | 0 | 0.0 | 0 | 0 | 0.0 | 0 | 0 | 0.0 | 0 |
| | Edema | 2 | 33.3 | 2 | 0 | 0.0 | 0 | 0 | 0.0 | 0 |
| | Fever | 3 | 50.0 | 3 | 0 | 0.0 | 0 | 0 | 0.0 | 0 |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

MedDRA/J Version 18.1

| | | 100 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 6 | 0 | 0.0 | 0 | 0 | 0.0 | 5 |
| Heart failure | | 0 | 0.0 | 0 | 0 | 0.0 | 0 | 0 | 0.0 | 1 |
| | Atrial fibrillation | 0 | 0.0 | 0 | 0 | 0.0 | 0 | 0 | 0.0 | 0 |

(continued)

| | | 100 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 1 | 16.7 | 1 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 2 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 1 | 16.7 | 2 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 6 | 100.0 | 93 | 0 | 0.0 | 8 | 0 | 0.0 | 5 |
| Heart failure | | 3 | 50.0 | 4 | 0 | 0.0 | 0 | 0 | 0.0 | 0 |
| | Atrial fibrillation | 1 | 16.7 | 1 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 1 | 16.7 | 1 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 1 | 16.7 | 1 | 0 | 0.0 | 0 | 0 | 0.0 | 0 |
| | Tachycardia | 1 | 16.7 | 1 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 1 | 16.7 | 1 | 0 | 0.0 | 0 | 1 | 16.7 | 1 |
| | Constipation | 0 | 0.0 | 0 | 0 | 0.0 | 0 | 1 | 16.7 | 1 |
| | Dianhea | 1 | 16.7 | 1 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 4 | 66.7 | 4 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 4 | 66.7 | 4 | 0 | 0.0 | 0 | 0 | 0.0 | 0 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

MedDRA/J Version 18.1

| | | Placebo | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 4 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| Expression period | | Placebo | | | | | | | | |
|---|---|---|---|---|---|---|---|---|---|---|
| | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 1 | 16.7 | 1 | 0 | 0.0 | 0 | 0 | 0.0 | 0 |
| | Constipation | 0 | 0.0 | 0 | 0 | 0.0 | 0 | 0 | 0.0 | 0 |
| | Dianhea | 1 | 16.7 | 1 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Hepatobiliary disorders | | 1 | 16.7 | 1 | 0 | 0.0 | 0 | 0 | 0.0 | 0 |
| | Acute cholangitis | 1 | 16.7 | 1 | 0 | 0.0 | 0 | 0 | 0.0 | 0 |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 2 | 33.3 | 2 | 0 | 0.0 | 0 |
| | Bronchitis | 0 | 0.0 | 0 | 1 | 16.7 | 1 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 6 | 100.0 | 87 | 0 | 0.0 | 7 | 0 | 0.0 | 3 |
| | Increased alanine aminotransferase | 4 | 66.7 | 4 | 1 | 16.7 | 1 | 0 | 0.0 | 0 |
| | Increased amylase | 0 | 0.0 | 0 | 1 | 16.7 | 1 | 0 | 0.0 | 0 |
| | Increased aspartate aminotransferase | 6 | 100.0 | 6 | 0 | 0.0 | 0 | 0 | 0.0 | 0 |
| | Decreased blood albumin | 6 | 100.0 | 6 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 6 | 100.0 | 6 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 4 | 66.7 | 4 | 0 | 0.0 | 0 | 0 | 0.0 | 0 |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 6 | 100.0 | 6 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 1 | 16.7 | 1 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 1 | 16.7 | 1 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 1 | 16.7 | 1 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 1 | 16.7 | 1 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Clinical test | | 6 | 100.0 | 72 | 0 | 0.0 | 8 | 0 | 0.0 | 7 |
| | Increased alanine aminotransferase | 1 | 16.7 | 1 | 0 | 0.0 | 0 | 0 | 0.0 | 0 |
| | Increased amylase | 0 | 0.0 | 0 | 0 | 0.0 | 0 | 0 | 0.0 | 0 |

(continued)

| Expression period | | 30 mg | | | | | | | | |
|---|---|---|---|---|---|---|---|---|---|---|
| | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 2 | 33.3 | 2 | 0 | 0.0 | 0 | 0 | 0.0 | 0 |
| | Decreased blood albumin | 3 | 50.0 | 3 | 1 | 16.7 | 1 | 0 | 0.0 | 1 |
| | Decreased blood chloride | 0 | 0.0 | 0 | 0 | 0.0 | 0 | 1 | 16.7 | 1 |
| | Decreased blood cholesterol | 3 | 50.0 | 3 | 0 | 0.0 | 0 | 0 | 0.0 | 0 |
| | Increased blood creatine phosphokinase | 4 | 66.7 | 4 | 0 | 0.0 | 0 | 0 | 0.0 | 0 |
| | Increased blood creatinine | 2 | 33.3 | 2 | 0 | 0.0 | 0 | 0 | 0.0 | 0 |
| | Increased blood glucose | 2 | 33.3 | 2 | 0 | 0.0 | 0 | 0 | 0.0 | 0 |
| | Increased blood lactate dehydrogenase | 5 | 83.3 | 5 | 0 | 0.0 | 0 | 0 | 0.0 | 0 |
| | Increased blood potassium | 1 | 16.7 | 1 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 1 | 16.7 | 1 |
| | Increased blood triglyceride | 0 | 0.0 | 0 | 0 | 0.0 | 0 | 0 | 0.0 | 0 |
| | Increased blood urea | 2 | 33.3 | 2 | 0 | 0.0 | 0 | 0 | 0.0 | 0 |
| | Decreased blood uric acid | 0 | 0.0 | 0 | 0 | 0.0 | 0 | 0 | 0.0 | 0 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood uric acid | 1 | 16.7 | 1 | 1 | 16.7 | 1 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 1 | 16.7 | 1 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 1 | 0 | 0.0 | 3 | 0 | 0.0 | 3 |
| | Increased alanine aminotransferase | 0 | 0.0 | 0 | 0 | 0.0 | 1 | 1 | 16.7 | 1 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 1 | 16.7 | 1 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 1 | 16.7 | 1 |
| | Decreased blood sodium | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

(continued)

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 1 | 16.7 | 1 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 1 | 16.7 | 1 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 6 | 100.0 | 62 | 0 | 0.0 | 7 | 0 | 0.0 | 1 |
| | Increased alanine aminotransferase | 1 | 16.7 | 1 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 1 | 16.7 | 1 | 0 | 0.0 | 0 | 0 | 0.0 | 0 |
| | Decreased blood albumin | 1 | 16.7 | 1 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 4 | 66.7 | 4 | 0 | 0.0 | 0 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 0 | 0.0 | 0 | 0 | 0.0 | 0 |

(continued)

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 3 | 50.0 | 3 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood urea | 0 | 0.0 | 0 | 1 | 16.7 | 1 | 0 | 0.0 | 0 |
| | Decreased blood uric acid | 1 | 16.7 | 1 | 0 | 0.0 | 0 | 0 | 0.0 | 0 |
| | Increased blood uric acid | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 5 | 0 | 0.0 | 0 | 0 | 0.0 | 2 |
| | Increased alanine aminotransferase | 0 | 0.0 | 1 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 1 | 16.7 | 1 | 0 | 0.0 | 0 | 0 | 0.0 | 1 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 1 | 16.7 | 1 |
| | Mediastinitis | 0 | 0.0 | 0 | 0 | 0.0 | 0 | 0 | 0.0 | 0 |
| | Pneumonia | 1 | 16.7 | 1 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 6 | 100.0 | 76 | 0 | 0.0 | 8 | 0 | 0.0 | 1 |
| | Increased alanine aminotransferase | 2 | 33.3 | 2 | 0 | 0.0 | 0 | 0 | 0.0 | 0 |
| | Increased amylase | 0 | 0.0 | 0 | 0 | 0.0 | 0 | 0 | 0.0 | 0 |

(continued)

| Expression period | | Placebo | | | | | | | | |
|---|---|---|---|---|---|---|---|---|---|---|
| | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 3 | 50.0 | 3 | 0 | 0.0 | 0 | 0 | 0.0 | 0 |
| | Decreased blood albumin | 2 | 33.3 | 2 | 0 | 0.0 | 0 | 0 | 0.0 | 0 |
| | Decreased blood chloride | 1 | 16.7 | 1 | 0 | 0.0 | 0 | 0 | 0.0 | 0 |
| | Decreased blood cholesterol | 1 | 16.7 | 1 | 0 | 0.0 | 0 | 0 | 0.0 | 0 |
| | Increased blood creatine phosphokinase | 5 | 83.3 | 5 | 0 | 0.0 | 0 | 0 | 0.0 | 0 |
| | Increased blood creatinine | 0 | 0.0 | 0 | 1 | 16.7 | 1 | 0 | 0.0 | 0 |
| | Increased blood glucose | 3 | 50.0 | 3 | 0 | 0.0 | 0 | 0 | 0.0 | 0 |
| | Increased blood lactate dehydrogenase | 6 | 100.0 | 6 | 0 | 0.0 | 0 | 0 | 0.0 | 0 |
| | Increased blood potassium | 0 | 0.0 | 0 | 2 | 33.3 | 2 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 1 | 16.7 | 1 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood uric acid | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 1 | 16.7 | 1 | 0 | 0.0 | 0 | 0 | 0.0 | 0 |
| | Wound dehiscence | 1 | 16.7 | 1 | 0 | 0.0 | 0 | 0 | 0.0 | 0 |
| Clinical test | | 0 | 0.0 | 1 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood urea | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 6 | 100.0 | 6 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 1 | 16.7 | 1 |
| | Decreased hematocrit | 5 | 83.3 | 5 | 0 | 0.0 | 0 | 0 | 0.0 | 0 |
| | Decreased hemoglobin | 5 | 83.3 | 5 | 0 | 0.0 | 0 | 0 | 0.0 | 0 |
| | Decreased heart rate | 1 | 16.7 | 1 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 1 | 16.7 | 1 | 0 | 0.0 | 0 |
| | Increased monocyte count | 0 | 0.0 | 0 | 0 | 0.0 | 0 | 1 | 16.7 | 1 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 1 | 16.7 | 1 | 1 | 16.7 | 1 | 0 | 0.0 | 0 |
| | Decreased erythrocyte count | 5 | 83.3 | 5 | 0 | 0.0 | 0 | 0 | 0.0 | 0 |
| | Increased leukocyte count | 4 | 66.7 | 4 | 0 | 0.0 | 0 | 0 | 0.0 | 0 |
| | Increased platelet count | 2 | 33.3 | 2 | 2 | 33.3 | 2 | 0 | 0.0 | 0 |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased brain natriuretic peptide | 5 | 83.3 | 5 | 0 | 0.0 | 0 | 0 | 0.0 | 0 |
| | Increased blood alkaline phosphatase | 4 | 66.7 | 4 | 0 | 0.0 | 0 | 0 | 0.0 | 0 |
| Metabolic and nutritional disorders | | 1 | 16.7 | 1 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |
| Musculoskeletal and connective tissue disorders | | 0 | 0.0 | 0 | 1 | 16.7 | 1 | 0 | 0.0 | 0 |
| | Muscle pain | 0 | 0.0 | 0 | 1 | 16.7 | 1 | 0 | 0.0 | 0 |
| | Musculoskeletal stiffness | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 6 | 100.0 | 6 | 0 | 0.0 | 0 | 0 | 0.0 | 0 |
| | Electrocardiographic T-wave inversion | 1 | 16.7 | 1 | 2 | 33.3 | 2 | 0 | 0.0 | 0 |
| | Increased eosinophil count | 0 | 0.0 | 0 | 0 | 0.0 | 0 | 0 | 0.0 | 0 |
| | Decreased hematocrit | 3 | 50.0 | 3 | 0 | 0.0 | 0 | 1 | 16.7 | 1 |
| | Decreased hemoglobin | 3 | 50.0 | 3 | 0 | 0.0 | 0 | 1 | 16.7 | 1 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 5 | 83.3 | 5 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 3 | 50.0 | 3 | 0 | 0.0 | 0 | 1 | 16.7 | 1 |
| | Increased leukocyte count | 4 | 66.7 | 4 | 0 | 0.0 | 0 | 0 | 0.0 | 0 |
| | Increased platelet count | 1 | 16.7 | 1 | 4 | 66.7 | 4 | 1 | 16.7 | 1 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased brain natriuretic peptide | 5 | 83.3 | 5 | 0 | 0.0 | 0 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 1 | 16.7 | 1 | 0 | 0.0 | 0 | 1 | 16.7 | 1 |
| | Acidosis | 0 | 0.0 | 0 | 0 | 0.0 | 0 | 0 | 0.0 | 0 |
| | Dehydration | 1 | 16.7 | 1 | 0 | 0.0 | 0 | 1 | 16.7 | 1 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 1 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 1 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 1 | 16.7 | 1 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 1 | 16.7 | 1 |

(continued)

| Expression period | | 100 mg | | | | | | | | |
|---|---|---|---|---|---|---|---|---|---|---|
| | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 6 | 100.0 | 6 | 0 | 0.0 | 0 | 0 | 0.0 | 0 |
| | Electrocardiographic T-wave inversion | 0 | 0.0 | 0 | 2 | 33.3 | 2 | 0 | 0.0 | 0 |
| | Increased eosinophil count | 0 | 0.0 | 0 | 0 | 0.0 | 0 | 1 | 16.7 | 1 |
| | Decreased hematocrit | 6 | 100.0 | 6 | 0 | 0.0 | 0 | 0 | 0.0 | 0 |
| | Decreased hemoglobin | 6 | 100.0 | 6 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 1 | 16.7 | 1 | 0 | 0.0 | 0 | 0 | 0.0 | 0 |
| | Decreased erythrocyte count | 6 | 100.0 | 6 | 0 | 0.0 | 0 | 0 | 0.0 | 0 |
| | Increased leukocyte count | 3 | 50.0 | 3 | 0 | 0.0 | 0 | 0 | 0.0 | 0 |
| | Increased platelet count | 1 | 16.7 | 1 | 3 | 50.0 | 3 | 0 | 0.0 | 0 |

(continued)

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased brain natriuretic peptide | 6 | 100.0 | 6 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased monocyte count | 1 | 16.7 | 1 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 1 | 0 | 0.0 | 0 | 0 | 0.0 | 0 |
| | Increased prothrombin time | 0 | 0.0 | 0 | 0 | 0.0 | 0 | 0 | 0.0 | 1 |
| | Decreased erythrocyte count | 0 | 0.0 | 0 | 0 | 0.0 | 0 | 0 | 0.0 | 0 |
| | Increased leukocyte count | 0 | 0.0 | 1 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 0 | 0.0 | 0 | 0 | 0.0 | 0 | 0 | 0.0 | 1 |
| | Increased blood alkaline phosphatase | 1 | 16.7 | 1 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 6 | 100.0 | 6 | 0 | 0.0 | 0 | 0 | 0.0 | 0 |
| | Electrocardiographic T-wave inversion | 0 | 0.0 | 0 | 1 | 16.7 | 1 | 0 | 0.0 | 0 |
| | Increased eosinophil count | 0 | 0.0 | 0 | 0 | 0.0 | 0 | 0 | 0.0 | 0 |
| | Decreased hematocrit | 6 | 100.0 | 6 | 0 | 0.0 | 0 | 0 | 0.0 | 0 |
| | Decreased hemoglobin | 6 | 100.0 | 6 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 6 | 100.0 | 6 | 0 | 0.0 | 0 | 0 | 0.0 | 0 |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 6 | 100.0 | 6 | 0 | 0.0 | 0 | 0 | 0.0 | 0 |
| | Oxygen desaturation | 1 | 16.7 | 1 | 0 | 0.0 | 0 | 0 | 0.0 | 0 |
| | Decreased platelet count | 4 | 66.7 | 4 | 0 | 0.0 | 0 | 0 | 0.0 | 0 |
| | Increased prothrombin time | 2 | 33.3 | 2 | 0 | 0.0 | 0 | 0 | 0.0 | 0 |
| | Decreased erythrocyte count | 6 | 100.0 | 6 | 0 | 0.0 | 0 | 0 | 0.0 | 0 |
| | Increased leukocyte count | 2 | 33.3 | 2 | 0 | 0.0 | 0 | 0 | 0.0 | 0 |
| | Increased platelet count | 1 | 16.7 | 1 | 4 | 66.7 | 4 | 1 | 16.7 | 1 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased brain natriuretic peptide | 5 | 83.3 | 5 | 0 | 0.0 | 0 | 0 | 0.0 | 0 |
| | Increased blood alkaline phosphatase | 1 | 16.7 | 1 | 0 | 0.0 | 0 | 0 | 0.0 | 0 |
| Metabolic and nutritional disorders | | 2 | 33.3 | 2 | 0 | 0.0 | 0 | 0 | 0.0 | 0 |
| | Acidosis | 1 | 16.7 | 1 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 1 | 16.7 | 1 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | Increased C-reactive protein | 0 | 0.0 | 0 | 0 | 0.0 | 0 | 0 | 0.0 | 0 |
| | Electrocardiographic T-wave inversion | 1 | 16.7 | 1 | 0 | 0.0 | 0 | 0 | 0.0 | 0 |
| | Increased eosinophil count | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased monocyte count | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 |
| | Increased brain natriuretic peptide | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 |
| Musculoskeletal and connective tissue disorders | | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 |
| MedDRA/J Version 18.1 | | | | | | | | | | |

(continued)

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 5 | 83.3 | 6 | 0 | 0.0 | 0 | 0 | 0.0 | 0 |
| | Delirium | 1 | 16.7 | 1 | 0 | 0.0 | 0 | 0 | 0.0 | 0 |
| | Restlessness | 2 | 33.3 | 2 | 0 | 0.0 | 0 | 0 | 0.0 | 0 |
| | Sleeping disorder | 3 | 50.0 | 3 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 4 | 66.7 | 6 | 0 | 0.0 | 0 | 0 | 0.0 | 0 |
| | Atelectasis | 1 | 16.7 | 1 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 4 | 66.7 | 4 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 1 | 16.7 | 1 | 0 | 0.0 | 0 | 0 | 0.0 | 0 |
| Skin and subcutaneous tissue disorders | | 0 | 0.0 | 0 | 0 | 0.0 | 0 | 1 | 16.7 | 1 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 1 | 16.7 | 1 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |
| MedDRA/J Version 18.1 | | | | | | | | | | |

(continued)

| Expression period | | 10 mg | | | | | | | | |
|---|---|---|---|---|---|---|---|---|---|---|
| | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 6 | 100.0 | 6 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 6 | 100.0 | 6 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 1 | 16.7 | 1 |
| | Gynecomastia | 0 | 0.0 | 0 | 0 | 0.0 | 0 | 1 | 16.7 | 1 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| | | 100 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 1 | 16.7 | 1 |
| | Kidney failure | 0 | 0.0 | 0 | 0 | 0.0 | 0 | 1 | 16.7 | 1 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 6 | 100.0 | 8 | 0 | 0.0 | 1 | 0 | 0.0 | 0 |
| | Atelectasis | 1 | 16.7 | 1 | 0 | 0.0 | 0 | 0 | 0.0 | 0 |
| | Difficulty breathing | 0 | 0.0 | 0 | 1 | 16.7 | 1 | 0 | 0.0 | 0 |
| | Pleural effusion | 6 | 100.0 | 6 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 1 | 16.7 | 1 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 1 | 16.7 | 1 |
| | Decubitus ulcer | 0 | 0.0 | 0 | 0 | 0.0 | 0 | 1 | 16.7 | 1 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 1 | 16.7 | 1 | 0 | 0.0 | 0 | 0 | 0.0 | 0 |
| | Thrombosis | 1 | 16.7 | 1 | 0 | 0.0 | 0 | 0 | 0.0 | 0 |

(continued)

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 1 | 16.7 | 1 |
| | Putaminal hemorrhage | 0 | 0.0 | 0 | 0 | 0.0 | 0 | 1 | 16.7 | 1 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 3 | 50.0 | 3 | 0 | 0.0 | 0 | 1 | 16.7 | 1 |
| | Atelectasis | 1 | 16.7 | 1 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 2 | 33.3 | 2 | 0 | 0.0 | 0 | 0 | 0.0 | 0 |
| | Aspiration pneumonia | 0 | 0.0 | 0 | 0 | 0.0 | 0 | 1 | 16.7 | 1 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 2 | 33.3 | 2 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 1 | 16.7 | 1 | 0 | 0.0 | 0 | 0 | 0.0 | 0 |
| | Hives | 1 | 16.7 | 1 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

(continued)

| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 |
| | Putaminal | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 |
| Respiratory, thoracic and mediastinal disorders | | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 |
| Vascular disorder | | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 |

MedDRA/J Version 18.1

Table 54

| | | 10 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 2 | 33.3 | 2 | 0 | 0.0 | 1 |
| Infectious and parasitic diseases | | 0 | 0.0 | 0 | 1 | 16.7 | 1 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| Clinical test | | 0 | 0.0 | 0 | 1 | 16.7 | 1 | 0 | 0.0 | 1 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |
| | Increased blood uric acid | 0 | 0.0 | 0 | 0 | 0.0 | 0 | 1 | 16.7 | 1 |
| MedDRA/J Version 18.1 | | | | | | | | | | |

| | | 10 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |

(continued)

| | | 10 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

MedDRA/J Version 18.1

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |

(continued)

| | | 30 mg | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 1 | 16.7 | 2 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 1 | 16.7 | 2 |
| | Increased alanine aminotransferase | 0 | 0.0 | 0 | 0 | 0.0 | 0 | 1 | 16.7 | 1 |
| | Increased aspartate aminotransferase | 0 | 0.0 | 0 | 0 | 0.0 | 0 | 1 | 16.7 | 1 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| MedDRA/J Version 18.1 | | | | | | | | | | |

(continued)

| Expression period | | 100 mg | | | | | | | | |
|---|---|---|---|---|---|---|---|---|---|---|
| | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| MedDRA/J Version 18.1 | | | | | | | | | | |

| Expression period | | 100 mg | | | | | | | | |
|---|---|---|---|---|---|---|---|---|---|---|
| | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurences | Number of examples | Incidence [%] | Number of occurences | Number of examples | Incidence [%] | Number of occurences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |

(continued)

| | | 100 mg | | | | | | | | |
|---|---|---|---|---|---|---|---|---|---|---|
| Expression period | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurences | Number of examples | Incidence [%] | Number of occurences | Number of examples | Incidence [%] | Number of occurences |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

| | | Placebo | | | | | | | | |
|---|---|---|---|---|---|---|---|---|---|---|
| Expression period | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 1 | 16.7 | 1 |
| Infectious and parasitic diseases | | 0 | 0.0 | 0 | 0 | 0.0 | 0 | 1 | 16.7 | 1 |
| | Lung abscess | 0 | 0.0 | 0 | 0 | 0.0 | 0 | 1 | 16.7 | 1 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |

(continued)

| Expression period | | Placebo | | | | | | | | |
|---|---|---|---|---|---|---|---|---|---|---|
| | | Wthin 1 week after administration (N=6) | | | More than 1 week after administration to 2 weeks after administration (N=6) | | | More than 2 weeks after administration to 4 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| MedDRA/J Version 18.1 | | | | | | | | | | |

| Expression period | | Placebo | | | | | | | | |
|---|---|---|---|---|---|---|---|---|---|---|
| | | More than 4 weeks after administration to 6 weeks after administration (N=6) | | | More than 6 weeks after administration to 8 weeks after administration (N=6) | | | More than 8 weeks after administration to 26 weeks after administration (N=6) | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 |
| MedDRA/J Version 18.1 | | | | | | | | | | |

Table 55

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 4 | 66.7 | 122 | 2 | 33.3 | 3 | 5 | 83.3 | 117 | 1 | 16.7 | 2 |
| Heart failure | | 2 | 33.3 | 2 | 0 | 0.0 | 0 | 1 | 16.7 | 2 | 0 | 0.0 | 0 |
| | Atrial fibrillation | 0 | 0.0 | 0 | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 | 0 | 0.0 | 0 |
| | Tachycardia | 2 | 33.3 | 2 | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 | 0 | 0.0 | 0 |
| Gastrointestinal disorders | | 3 | 50.0 | 5 | 0 | 0.0 | 0 | 2 | 33.3 | 2 | 0 | 0.0 | 0 |
| | Constipation | 1 | 16.7 | 1 | 0 | 0.0 | 0 | 0 | 0.0 | 0 | 0 | 0.0 | 0 |
| | Dianhea | 0 | 0.0 | 0 | 0 | 0.0 | 0 | 2 | 33.3 | 2 | 0 | 0.0 | 0 |
| | Drylips | 1 | 16.7 | 1 | 0 | 0.0 | 0 | 0 | 0.0 | 0 | 0 | 0.0 | 0 |
| | Lip ulcer | 1 | 16.7 | 1 | 0 | 0.0 | 0 | 0 | 0.0 | 0 | 0 | 0.0 | 0 |
| | Vomiting | 1 | 16.7 | 1 | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 | 0 | 0.0 | 0 |
| | Loose stool | 1 | 16.7 | 1 | 0 | 0.0 | 0 | 0 | 0.0 | 0 | 0 | 0.0 | 0 |

(continued)

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| General/systemic disorders and administration site conditions | | 3 | 50.0 | 4 | 0 | 0.0 | 0 | 5 | 83.3 | 8 | 0 | 0.0 | 0 |
| | Chills | 0 | 0.0 | 0 | 0 | 0.0 | 0 | 0 | 0.0 | 0 | 0 | 0.0 | 0 |
| | Poor healing | 0 | 0.0 | 0 | 0 | 0.0 | 0 | 1 | 16.7 | 1 | 0 | 0.0 | 0 |
| | Edema | 1 | 16.7 | 1 | 0 | 0.0 | 0 | 0 | 0.0 | 0 | 0 | 0.0 | 0 |
| | Fever | 2 | 33.3 | 3 | 0 | 0.0 | 0 | 5 | 83.3 | 7 | 0 | 0.0 | 0 |

MedDRA/J Version 18.1

| | | Whole period | | | | | | | | | | | |
|---|---|---|---|---|---|---|---|---|---|---|---|---|---|
| | | 100 mg (N=6) | | | | | | Placebo (N=6) | | | | | |
| Causal relationship System organ classification | | Unrelated | | | Related | | | Unrelated | | | Related | | |
| | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 6 | 100.0 | 107 | 0 | 0.0 | 0 | 5 | 83.3 | 109 | 1 | 16.7 | 1 |
| Heart failure | | 5 | 83.3 | 8 | 0 | 0.0 | 0 | 3 | 50.0 | 4 | 0 | 0.0 | 0 |
| | Atrial fibrillation | 3 | 50.0 | 3 | 0 | 0.0 | 0 | 1 | 16.7 | 1 | 0 | 0.0 | 0 |
| | First-degree atrioventricular block | 1 | 16.7 | 1 | 0 | 0.0 | 0 | 0 | 0.0 | 0 | 0 | 0.0 | 0 |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Right bundle branch block | 0 | 0.0 | 0 | 0 | 0.0 | 0 | 1 | 16.7 | 1 | 0 | 0.0 | 0 |

(continued)

| Causal relationship | | Unrelated | | | Related | | | Unrelated | | | Related | | |
|---|---|---|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Cardiac tamponade | 1 | 16.7 | 1 | 0 | 0.0 | 0 | 0 | 0.0 | 0 | 0 | 0.0 | 0 |
| | Mitral regurgitation | 1 | 16.7 | 1 | 0 | 0.0 | 0 | 0 | 0.0 | 0 | 0 | 0.0 | 0 |
| | Pericardial effusion | 1 | 16.7 | 1 | 0 | 0.0 | 0 | 0 | 0.0 | 0 | 0 | 0.0 | 0 |
| | Sinus tachycardia | 0 | 0.0 | 0 | 0 | 0.0 | 0 | 1 | 16.7 | 1 | 0 | 0.0 | 0 |
| | Tachycardia | 0 | 0.0 | 0 | 0 | 0.0 | 0 | 1 | 16.7 | 1 | 0 | 0.0 | 0 |
| | Ventricular fibrillation | 1 | 16.7 | 1 | 0 | 0.0 | 0 | 0 | 0.0 | 0 | 0 | 0.0 | 0 |
| Gastrointestinal disorders | | 1 | 16.7 | 3 | 0 | 0.0 | 0 | 3 | 50.0 | 3 | 0 | 0.0 | 0 |
| | Constipation | 0 | 0.0 | 0 | 0 | 0.0 | 0 | 1 | 16.7 | 1 | 0 | 0.0 | 0 |
| | Dianhea | 0 | 0.0 | 0 | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 | 0 | 0.0 | 0 |
| | Vomiting | 1 | 16.7 | 2 | 0 | 0.0 | 0 | 0 | 0.0 | 0 | 0 | 0.0 | 0 |
| | Anal bleeding | 1 | 16.7 | 1 | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 | 0 | 0.0 | 0 |
| General/systemic disorders and administration site conditions | | 5 | 83.3 | 6 | 0 | 0.0 | 0 | 4 | 66.7 | 4 | 0 | 0.0 | 0 |
| | Chills | 1 | 16.7 | 1 | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 | 0 | 0.0 | 0 |
| | Edema | 2 | 33.3 | 2 | 0 | 0.0 | 0 | 0 | 0.0 | 0 | 0 | 0.0 | 0 |
| | Fever | 3 | 50.0 | 3 | 0 | 0.0 | 0 | 4 | 66.7 | 4 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 4 | 66.7 | 122 | 2 | 33.3 | 3 | 6 | 100.0 | 109 | 0 | 0.0 | 0 |
| Heart failure | | 2 | 33.3 | 2 | 0 | 0.0 | 0 | 1 | 16.7 | 2 | 0 | 0.0 | 0 |
| | Atrial fibrillation | 0 | 0.0 | 0 | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 | 0 | 0.0 | 0 |
| | Tachycardia | 2 | 33.3 | 2 | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 | 0 | 0.0 | 0 |
| Gastrointestinal disorders | | 3 | 50.0 | 5 | 0 | 0.0 | 0 | 2 | 33.3 | 2 | 0 | 0.0 | 0 |
| | Constipation | 1 | 16.7 | 1 | 0 | 0.0 | 0 | 0 | 0.0 | 0 | 0 | 0.0 | 0 |
| | Diarrhea | 0 | 0.0 | 0 | 0 | 0.0 | 0 | 2 | 33.3 | 2 | 0 | 0.0 | 0 |
| | Drylips | 1 | 16.7 | 1 | 0 | 0.0 | 0 | 0 | 0.0 | 0 | 0 | 0.0 | 0 |
| | Lip ulcer | 1 | 16.7 | 1 | 0 | 0.0 | 0 | 0 | 0.0 | 0 | 0 | 0.0 | 0 |
| | Vomiting | 1 | 16.7 | 1 | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 | 0 | 0.0 | 0 |
| | Loose stool | 1 | 16.7 | 1 | 0 | 0.0 | 0 | 0 | 0.0 | 0 | 0 | 0.0 | 0 |

(continued)

| | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| General/systemic disorders and administration site conditions | | 3 | 50.0 | 4 | 0 | 0.0 | 0 | 5 | 83.3 | 7 | 0 | 0.0 | 0 |
| | Chills | 0 | 0.0 | 0 | 0 | 0.0 | 0 | 0 | 0.0 | 0 | 0 | 0.0 | 0 |
| | Poor healing | 0 | 0.0 | 0 | 0 | 0.0 | 0 | 1 | 16.7 | 1 | 0 | 0.0 | 0 |
| | Edema | 1 | 16.7 | 1 | 0 | 0.0 | 0 | 0 | 0.0 | 0 | 0 | 0.0 | 0 |
| | Fever | 2 | 33.3 | 3 | 0 | 0.0 | 0 | 5 | 83.3 | 6 | 0 | 0.0 | 0 |
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 6 | 100.0 | 102 | 0 | 0.0 | 0 | 5 | 83.3 | 109 | 1 | 16.7 | 1 |
| Heart failure | | 5 | 83.3 | 7 | 0 | 0.0 | 0 | 3 | 50.0 | 4 | 0 | 0.0 | 0 |
| | Atrial fibrillation | 3 | 50.0 | 3 | 0 | 0.0 | 0 | 1 | 16.7 | 1 | 0 | 0.0 | 0 |
| | First-degree atrioventricular block | 1 | 16.7 | 1 | 0 | 0.0 | 0 | 0 | 0.0 | 0 | 0 | 0.0 | 0 |
| | Right bundle branch block | 0 | 0.0 | 0 | 0 | 0.0 | 0 | 1 | 16.7 | 1 | 0 | 0.0 | 0 |
| | Cardiac tamponade | 1 | 16.7 | 1 | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 | 0 | 0.0 | 0 |
| | Pericardial effusion | 1 | 16.7 | 1 | 0 | 0.0 | 0 | 0 | 0.0 | 0 | 0 | 0.0 | 0 |
| | Sinus tachycardia | 0 | 0.0 | 0 | 0 | 0.0 | 0 | 1 | 16.7 | 1 | 0 | 0.0 | 0 |
| | Tachycardia | 0 | 0.0 | 0 | 0 | 0.0 | 0 | 1 | 16.7 | 1 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Ventricular fibrillation | 1 | 16.7 | 1 | 0 | 0.0 | 0 | 0 | 0.0 | 0 | 0 | 0.0 | 0 |
| Gastrointestinal disorders | | 1 | 16.7 | 1 | 0 | 0.0 | 0 | 3 | 50.0 | 3 | 0 | 0.0 | 0 |
| | Constipation | 0 | 0.0 | 0 | 0 | 0.0 | 0 | 1 | 16.7 | 1 | 0 | 0.0 | 0 |
| | Diarrhea | 0 | 0.0 | 0 | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 | 0 | 0.0 | 0 |
| | Anal bleeding | 1 | 16.7 | 1 | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 | 0 | 0.0 | 0 |
| General/systemic disorders and administration site conditions | | 5 | 83.3 | 6 | 0 | 0.0 | 0 | 4 | 66.7 | 4 | 0 | 0.0 | 0 |
| | Chills | 1 | 16.7 | 1 | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 | 0 | 0.0 | 0 |
| | Edema | 2 | 33.3 | 2 | 0 | 0.0 | 0 | 0 | 0.0 | 0 | 0 | 0.0 | 0 |
| | Fever | 3 | 50.0 | 3 | 0 | 0.0 | 0 | 4 | 66.7 | 4 | 0 | 0.0 | 0 |

| | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 4 | 66.7 | 8 | 1 | 16.7 | 2 |
| Heart failure | | 0 | 0.0 | 0 | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 1 | 16.7 | 1 | 0 | 0.0 | 0 |
| | Chills | 0 | 0.0 | 0 | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 | 0 | 0.0 | 0 |
| | Fever | 0 | 0.0 | 0 | 0 | 0.0 | 0 | 1 | 16.7 | 1 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Whole | | 3 | 50.0 | 5 | 0 | 0.0 | 0 | 0 | 0.0 | 0 | 0 | 0.0 | 0 |
| Heart failure | | 1 | 16.7 | 1 | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 | 0 | 0.0 | 0 |
| | Mitral regurgitation | 1 | 16.7 | 1 | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 | 0 | 0.0 | 0 |
| Gastrointestinal disorders | | 1 | 16.7 | 2 | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 | 0 | 0.0 | 0 |
| | Vomiting | 1 | 16.7 | 2 | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 | 0 | 0.0 | 0 |

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 | 0 | 0.0 | 0 |
| Infectious and parasitic diseases | | 1 | 16.7 | 1 | 1 | 16.7 | 1 | 2 | 33.3 | 2 | 0 | 0.0 | 0 |
| | Bronchitis | 1 | 16.7 | 1 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 0 | 0.0 | 0 |
| | Mediastinitis | 0 | 0.0 | 0 | 0 | 0.0 | 0 | 1 | 16.7 | 1 | 0 | 0.0 | 0 |
| | Pneumonia | 0 | 0.0 | 0 | 1 | 16.7 | 1 | 1 | 16.7 | 1 | 0 | 0.0 | 0 |
| | Tracheostomy site infection | 0 | 0.0 | 0 | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 1 | 16.7 | 1 | 0 | 0.0 | 0 |
| | Wound dehiscence | 0 | 0.0 | 0 | 0 | 0.0 | 0 | 1 | 16.7 | 1 | 0 | 0.0 | 0 |
| Clinical test | | 5 | 83.3 | 95 | 1 | 16.7 | 2 | 5 | 83.3 | 92 | 1 | 16.7 | 2 |

(continued)

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased alanine aminotransferase | 5 | 83.3 | 5 | 0 | 0.0 | 0 | 1 | 16.7 | 2 | 1 | 16.7 | 1 |
| | Increased amylase | 1 | 16.7 | 1 | 0 | 0.0 | 0 | 0 | 0.0 | 0 | 0 | 0.0 | 0 |
| | Increased aspartate aminotransferase | 6 | 100.0 | 6 | 0 | 0.0 | 0 | 2 | 33.3 | 2 | 1 | 16.7 | 1 |
| | Decreased blood albumin | 6 | 100.0 | 6 | 0 | 0.0 | 0 | 4 | 66.7 | 5 | 0 | 0.0 | 0 |
| | Decreased blood chloride | 0 | 0.0 | 0 | 0 | 0.0 | 0 | 1 | 16.7 | 1 | 0 | 0.0 | 0 |
| | Decreased blood cholesterol | 0 | 0.0 | 0 | 0 | 0.0 | 0 | 3 | 50.0 | 3 | 0 | 0.0 | 0 |
| | Increased blood creatine phosphokinase | 6 | 100.0 | 6 | 0 | 0.0 | 0 | 4 | 66.7 | 4 | 0 | 0.0 | 0 |
| | Increased blood creatinine | 0 | 0.0 | 0 | 0 | 0.0 | 0 | 2 | 33.3 | 2 | 0 | 0.0 | 0 |
| | Increased blood glucose | 4 | 66.7 | 4 | 0 | 0.0 | 0 | 2 | 33.3 | 2 | 0 | 0.0 | 0 |
| | Increased blood lactate dehydrogenase | 6 | 100.0 | 6 | 0 | 0.0 | 0 | 5 | 83.3 | 5 | 0 | 0.0 | 0 |
| | Increased blood potassium | 0 | 0.0 | 0 | 0 | 0.0 | 0 | 1 | 16.7 | 1 | 0 | 0.0 | 0 |
| | Decreased blood pressure | 1 | 16.7 | 1 | 0 | 0.0 | 0 | 0 | 0.0 | 0 | 0 | 0.0 | 0 |
| | Increased blood pressure | 0 | 0.0 | 0 | 0 | 0.0 | 0 | 1 | 16.7 | 1 | 0 | 0.0 | 0 |

(continued)

| Causal relationship | | Whole period | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 1 | 16.7 | 1 | 0 | 0.0 | 0 |
| | Increased blood triglyceride | 0 | 0.0 | 0 | 1 | 16.7 | 1 | 1 | 16.7 | 1 | 0 | 0.0 | 0 |

MedDRA/J Version 18.1

| Causal relationship | | Whole period | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 1 | 16.7 | 1 | 0 | 0.0 | 0 |
| | Acute cholangitis | 0 | 0.0 | 0 | 0 | 0.0 | 0 | 1 | 16.7 | 1 | 0 | 0.0 | 0 |
| Infectious and parasitic diseases | | 1 | 16.7 | 1 | 0 | 0.0 | 0 | 0 | 0.0 | 1 | 1 | 16.7 | 1 |
| | Bronchitis | 0 | 0.0 | 0 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 1 | 16.7 | 1 |
| | Mediastinitis | 0 | 0.0 | 0 | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 | 0 | 0.0 | 0 |
| | Tracheostomy site infection | 1 | 16.7 | 1 | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 1 | 16.7 | 1 | 0 | 0.0 | 0 |
| | Wound dehiscence | 0 | 0.0 | 0 | 0 | 0.0 | 0 | 1 | 16.7 | 1 | 0 | 0.0 | 0 |

(continued)

| Causal relationship | | Whole period | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | | 6 | 100.0 | 77 | 0 | 0.0 | 0 | 6 | 100.0 | 86 | 0 | 0.0 | 0 |
| | Increased alanine aminotransferase | 1 | 16.7 | 2 | 0 | 0.0 | 0 | 2 | 33.3 | 2 | 0 | 0.0 | 0 |
| | Increased amylase | 0 | 0.0 | 0 | 0 | 0.0 | 0 | 0 | 0.0 | 0 | 0 | 0.0 | 0 |
| | Increased aspartate aminotransferase | 1 | 16.7 | 1 | 0 | 0.0 | 0 | 3 | 50.0 | 3 | 0 | 0.0 | 0 |
| | Decreased blood albumin | 1 | 16.7 | 1 | 0 | 0.0 | 0 | 2 | 33.3 | 2 | 0 | 0.0 | 0 |
| | Decreased blood chloride | 0 | 0.0 | 0 | 0 | 0.0 | 0 | 1 | 16.7 | 1 | 0 | 0.0 | 0 |
| | Decreased blood cholesterol | 0 | 0.0 | 0 | 0 | 0.0 | 0 | 1 | 16.7 | 1 | 0 | 0.0 | 0 |
| | Increased blood creatine phosphokinase | 4 | 66.7 | 4 | 0 | 0.0 | 0 | 5 | 83.3 | 5 | 0 | 0.0 | 0 |
| | Increased blood creatinine | 1 | 16.7 | 1 | 0 | 0.0 | 0 | 1 | 16.7 | 1 | 0 | 0.0 | 0 |
| | Increased blood glucose | 0 | 0.0 | 0 | 0 | 0.0 | 0 | 3 | 50.0 | 3 | 0 | 0.0 | 0 |
| | Increased blood lactate dehydrogenase | 3 | 50.0 | 3 | 0 | 0.0 | 0 | 6 | 100.0 | 6 | 0 | 0.0 | 0 |
| | Increased blood potassium | 0 | 0.0 | 0 | 0 | 0.0 | 0 | 2 | 33.3 | 2 | 0 | 0.0 | 0 |
| | Decreased blood pressure | 0 | 0.0 | 0 | 0 | 0.0 | 0 | 0 | 0.0 | 0 | 0 | 0.0 | 0 |

(continued)

| | | Whole period | | | | | | | | | | | |
|---|---|---|---|---|---|---|---|---|---|---|---|---|---|
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased blood pressure | 0 | 0.0 | 0 | 0 | 0.0 | 0 | 0 | 0.0 | 0 | 0 | 0.0 | 0 |
| | Decreased blood sodium | 0 | 0.0 | 0 | 0 | 0.0 | 0 | 1 | 16.7 | 1 | 0 | 0.0 | 0 |
| | Increased blood triglyceride | 0 | 0.0 | 0 | 0 | 0.0 | 0 | 0 | 0.0 | 0 | 0 | 0.0 | 0 |

MedDRA/J Version 18.1

| | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 | 0 | 0.0 | 0 |
| Infectious and parasitic diseases | | 1 | 16.7 | 1 | 1 | 16.7 | 1 | 1 | 16.7 | 1 | 0 | 0.0 | 0 |
| | Bronchitis | 1 | 16.7 | 1 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 0 | 0.0 | 0 |
| | Mediastinitis | 0 | 0.0 | 0 | 0 | 0.0 | 0 | 1 | 16.7 | 1 | 0 | 0.0 | 0 |
| | Pneumonia | 0 | 0.0 | 0 | 1 | 16.7 | 1 | 0 | 0.0 | 0 | 0 | 0.0 | 0 |
| | Tracheostomy site infection | 0 | 0.0 | 0 | 0 | 0.0 | 0 | 0 | 0.0 | 0 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 1 | 16.7 | 1 | 0 | 0.0 | 0 |
| | Wound dehiscence | 0 | 0.0 | 0 | 0 | 0.0 | 0 | 1 | 16.7 | 1 | 0 | 0.0 | 0 |
| Clinical test | | 5 | 83.3 | 95 | 1 | 16.7 | 2 | 6 | 100.0 | 88 | 0 | 0.0 | 0 |
| | Increased alanine aminotransferase | 5 | 83.3 | 5 | 0 | 0.0 | 0 | 1 | 16.7 | 1 | 0 | 0.0 | 0 |
| | Increased amylase | 1 | 16.7 | 1 | 0 | 0.0 | 0 | 0 | 0.0 | 0 | 0 | 0.0 | 0 |
| | Increased aspartate aminotransferase | 6 | 100.0 | 6 | 0 | 0.0 | 0 | 2 | 33.3 | 2 | 0 | 0.0 | 0 |
| | Decreased blood albumin | 6 | 100.0 | 6 | 0 | 0.0 | 0 | 4 | 66.7 | 5 | 0 | 0.0 | 0 |
| | Decreased blood chloride | 0 | 0.0 | 0 | 0 | 0.0 | 0 | 1 | 16.7 | 1 | 0 | 0.0 | 0 |
| | Decreased blood cholesterol | 0 | 0.0 | 0 | 0 | 0.0 | 0 | 3 | 50.0 | 3 | 0 | 0.0 | 0 |
| | Increased blood creatine phosphokinase | 6 | 100.0 | 6 | 0 | 0.0 | 0 | 4 | 66.7 | 4 | 0 | 0.0 | 0 |
| | Increased blood creatinine | 0 | 0.0 | 0 | 0 | 0.0 | 0 | 2 | 33.3 | 2 | 0 | 0.0 | 0 |
| | Increased blood glucose | 4 | 66.7 | 4 | 0 | 0.0 | 0 | 2 | 33.3 | 2 | 0 | 0.0 | 0 |
| | Increased blood lactate dehydrogenase | 6 | 100.0 | 6 | 0 | 0.0 | 0 | 5 | 83.3 | 5 | 0 | 0.0 | 0 |

(continued)

| | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased blood potassium | 0 | 0.0 | 0 | 0 | 0.0 | 0 | 1 | 16.7 | 1 | 0 | 0.0 | 0 |
| | Decreased blood pressure | 1 | 16.7 | 1 | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 | 0 | 0.0 | 0 |
| | Decreased blood sodium | 0 | 0.0 | 0 | 0 | 0.0 | 0 | 1 | 16.7 | 1 | 0 | 0.0 | 0 |
| | Increased blood triglyceride | 0 | 0.0 | 0 | 1 | 16.7 | 1 | 0 | 0.0 | 0 | 0 | 0.0 | 0 |

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 1 | 16.7 | 1 | 0 | 0.0 | 0 |
| | Acute cholangitis | 0 | 0.0 | 0 | 0 | 0.0 | 0 | 1 | 16.7 | 1 | 0 | 0.0 | 0 |
| Infectious and parasitic diseases | | 1 | 16.7 | 1 | 0 | 0.0 | 0 | 0 | 0.0 | 1 | 1 | 16.7 | 1 |
| | Bronchitis | 0 | 0.0 | 0 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 1 | 16.7 | 1 |
| | Mediastinitis | 0 | 0.0 | 0 | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 | 0 | 0.0 | 0 |
| | Tracheostomy site infection | 1 | 16.7 | 1 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 1 | 16.7 | 1 | 0 | 0.0 | 0 |
| | Wound dehiscence | 0 | 0.0 | 0 | 0 | 0.0 | 0 | 1 | 16.7 | 1 | 0 | 0.0 | 0 |
| Clinical test | | 6 | 100.0 | 75 | 0 | 0.0 | 0 | 6 | 100.0 | 86 | 0 | 0.0 | 0 |
| | Increased alanine aminotransferase | 1 | 16.7 | 2 | 0 | 0.0 | 0 | 2 | 33.3 | 2 | 0 | 0.0 | 0 |
| | Increased amylase | 0 | 0.0 | 0 | 0 | 0.0 | 0 | 0 | 0.0 | 0 | 0 | 0.0 | 0 |
| | Increased aspartate aminotransferase | 1 | 16.7 | 1 | 0 | 0.0 | 0 | 3 | 50.0 | 3 | 0 | 0.0 | 0 |
| | Decreased blood albumin | 1 | 16.7 | 1 | 0 | 0.0 | 0 | 2 | 33.3 | 2 | 0 | 0.0 | 0 |
| | Decreased blood chloride | 0 | 0.0 | 0 | 0 | 0.0 | 0 | 1 | 16.7 | 1 | 0 | 0.0 | 0 |
| | Decreased blood cholesterol | 0 | 0.0 | 0 | 0 | 0.0 | 0 | 1 | 16.7 | 1 | 0 | 0.0 | 0 |
| | Increased blood creatine phosphokinase | 4 | 66.7 | 4 | 0 | 0.0 | 0 | 5 | 83.3 | 5 | 0 | 0.0 | 0 |
| | Increased blood creatinine | 1 | 16.7 | 1 | 0 | 0.0 | 0 | 1 | 16.7 | 1 | 0 | 0.0 | 0 |
| | Increased blood glucose | 0 | 0.0 | 0 | 0 | 0.0 | 0 | 3 | 50.0 | 3 | 0 | 0.0 | 0 |
| | Increased blood lactate dehydrogenase | 3 | 50.0 | 3 | 0 | 0.0 | 0 | 6 | 100.0 | 6 | 0 | 0.0 | 0 |
| | Increased blood potassium | 0 | 0.0 | 0 | 0 | 0.0 | 0 | 2 | 33.3 | 2 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased blood pressure | 0 | 0.0 | 0 | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 | 0 | 0.0 | 0 |
| | Decreased blood sodium | 0 | 0.0 | 0 | 0 | 0.0 | 0 | 1 | 16.7 | 1 | 0 | 0.0 | 0 |
| | Increased blood triglyceride | 0 | 0.0 | 0 | 0 | 0.0 | 0 | 0 | 0.0 | 0 | 0 | 0.0 | 0 |

MedDRA/J Version 18.1

| | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 | 0 | 0.0 | 0 |
| Infectious and parasitic diseases | | 0 | 0.0 | 0 | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 | 0 | 0.0 | 0 |
| | Pneumonia | 0 | 0.0 | 0 | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 | 0 | 0.0 | 0 |
| Clinical test | | 0 | 0.0 | 0 | 0 | 0.0 | 0 | 3 | 50.0 | 4 | 1 | 16.7 | 2 |
| | Increased alanine aminotransferase | 0 | 0.0 | 0 | 0 | 0.0 | 0 | 1 | 16.7 | 1 | 1 | 16.7 | 1 |
| | Increased amylase | 0 | 0.0 | 0 | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 | 1 | 16.7 | 1 |
| | Decreased blood albumin | 0 | 0.0 | 0 | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased blood potassium | 0 | 0.0 | 0 | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 | 0 | 0.0 | 0 |
| | Increased blood pressure | 0 | 0.0 | 0 | 0 | 0.0 | 0 | 1 | 16.7 | 1 | 0 | 0.0 | 0 |
| | Decreased blood sodium | 0 | 0.0 | 0 | 0 | 0.0 | 0 | 0 | 0.0 | 0 | 0 | 0.0 | 0 |
| | Increased blood triglyceride | 0 | 0.0 | 0 | 0 | 0.0 | 0 | 1 | 16.7 | 1 | 0 | 0.0 | 0 |

MedDRA/J Version 18.1

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 | 0 | 0.0 | 0 |
| Clinical test | | 2 | 33.3 | 2 | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | | | | |
|---|---|---|---|---|---|---|---|---|---|---|---|---|---|
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 | 0 | 0.0 | 0 |

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 2 | 33.3 | 2 | 0 | 0.0 | 0 |

(continued)

| Causal relationship | | Whole period | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased blood uric acid | 0 | 0.0 | 0 | 0 | 0.0 | 0 | 0 | 0.0 | 0 | 0 | 0.0 | 0 |
| | Increased blood uric acid | 0 | 0.0 | 0 | 1 | 16.7 | 1 | 2 | 33.3 | 2 | 0 | 0.0 | 0 |
| | Increased C-reactive protein | 6 | 100.0 | 6 | 0 | 0.0 | 0 | 6 | 100.0 | 6 | 0 | 0.0 | 0 |
| | Electrocardiographic T-wave inversion | 0 | 0.0 | 0 | 0 | 0.0 | 0 | 3 | 50.0 | 3 | 0 | 0.0 | 0 |
| | Increased eosinophil count | 1 | 16.7 | 1 | 0 | 0.0 | 0 | 0 | 0.0 | 0 | 0 | 0.0 | 0 |
| | Decreased hematocrit | 5 | 83.3 | 5 | 0 | 0.0 | 0 | 4 | 66.7 | 4 | 0 | 0.0 | 0 |
| | Decreased hemoglobin | 5 | 83.3 | 5 | 0 | 0.0 | 0 | 4 | 66.7 | 4 | 0 | 0.0 | 0 |
| | Decreased heart rate | 1 | 16.7 | 1 | 0 | 0.0 | 0 | 0 | 0.0 | 0 | 0 | 0.0 | 0 |
| | Decreased lymphocyte count | 6 | 100.0 | 6 | 0 | 0.0 | 0 | 6 | 100.0 | 6 | 0 | 0.0 | 0 |
| | Decreased monocyte count | 1 | 16.7 | 1 | 0 | 0.0 | 0 | 0 | 0.0 | 0 | 0 | 0.0 | 0 |
| | Increased monocyte count | 1 | 16.7 | 1 | 0 | 0.0 | 0 | 0 | 0.0 | 0 | 0 | 0.0 | 0 |
| | Increased neutrophil count | 6 | 100.0 | 6 | 0 | 0.0 | 0 | 6 | 100.0 | 6 | 0 | 0.0 | 0 |
| | Oxygen desaturation | 0 | 0.0 | 0 | 0 | 0.0 | 0 | 0 | 0.0 | 0 | 0 | 0.0 | 0 |

(continued)

| Causal relationship | | Whole period | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased platelet count | 4 | 66.7 | 4 | 0 | 0.0 | 0 | 5 | 83.3 | 6 | 0 | 0.0 | 0 |
| | Increased prothrombin time | 2 | 33.3 | 2 | 0 | 0.0 | 0 | 2 | 33.3 | 2 | 0 | 0.0 | 0 |
| | Decreased erythrocyte count | 5 | 83.3 | 5 | 0 | 0.0 | 0 | 4 | 66.7 | 4 | 0 | 0.0 | 0 |
| | Increased leukocyte count | 4 | 66.7 | 4 | 0 | 0.0 | 0 | 4 | 66.7 | 4 | 0 | 0.0 | 0 |
| | Increased platelet count | 4 | 66.7 | 4 | 0 | 0.0 | 0 | 6 | 100.0 | 7 | 0 | 0.0 | 0 |
| | Increased brain natriuretic peptide | 5 | 83.3 | 5 | 0 | 0.0 | 0 | 5 | 83.3 | 5 | 0 | 0.0 | 0 |
| | Increased blood alkaline phosphatase | 4 | 66.7 | 4 | 0 | 0.0 | 0 | 1 | 16.7 | 1 | 0 | 0.0 | 0 |
| Metabolic and nutritional disorders | | 1 | 16.7 | 1 | 0 | 0.0 | 0 | 2 | 33.3 | 2 | 0 | 0.0 | 0 |
| | Acidosis | 0 | 0.0 | 0 | 0 | 0.0 | 0 | 0 | 0.0 | 0 | 0 | 0.0 | 0 |
| | Dehydration | 0 | 0.0 | 0 | 0 | 0.0 | 0 | 2 | 33.3 | 2 | 0 | 0.0 | 0 |
| | Hypovolemia | 1 | 16.7 | 1 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 1 | 16.7 | 1 | 0 | 0.0 | 0 | 0 | 0.0 | 0 | 0 | 0.0 | 0 |
| | Decreased blood uric acid | 1 | 16.7 | 1 | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 | 0 | 0.0 | 0 |
| | Increased C-reactive protein | 6 | 100.0 | 6 | 0 | 0.0 | 0 | 6 | 100.0 | 6 | 0 | 0.0 | 0 |
| | Electrocardiographic T-wave inversion | 2 | 33.3 | 2 | 0 | 0.0 | 0 | 2 | 33.3 | 2 | 0 | 0.0 | 0 |
| | Increased eosinophil count | 1 | 16.7 | 1 | 0 | 0.0 | 0 | 0 | 0.0 | 0 | 0 | 0.0 | 0 |
| | Decreased hematocrit | 6 | 100.0 | 6 | 0 | 0.0 | 0 | 6 | 100.0 | 6 | 0 | 0.0 | 0 |
| | Decreased hemoglobin | 6 | 100.0 | 6 | 0 | 0.0 | 0 | 6 | 100.0 | 6 | 0 | 0.0 | 0 |
| | Decreased heart rate | 0 | 0.0 | 0 | 0 | 0.0 | 0 | 0 | 0.0 | 0 | 0 | 0.0 | 0 |
| | Decreased lymphocyte count | 6 | 100.0 | 6 | 0 | 0.0 | 0 | 6 | 100.0 | 6 | 0 | 0.0 | 0 |
| | Decreased monocyte count | 1 | 16.7 | 1 | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 | 0 | 0.0 | 0 |
| | Increased neutrophil count | 6 | 100.0 | 6 | 0 | 0.0 | 0 | 6 | 100.0 | 6 | 0 | 0.0 | 0 |
| | Oxygen desaturation | 0 | 0.0 | 0 | 0 | 0.0 | 0 | 1 | 16.7 | 1 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased platelet count | 4 | 66.7 | 5 | 0 | 0.0 | 0 | 4 | 66.7 | 4 | 0 | 0.0 | 0 |
| | Increased prothrombin time | 1 | 16.7 | 2 | 0 | 0.0 | 0 | 2 | 33.3 | 2 | 0 | 0.0 | 0 |
| | Decreased erythrocyte count | 6 | 100.0 | 6 | 0 | 0.0 | 0 | 6 | 100.0 | 6 | 0 | 0.0 | 0 |
| | Increased leukocyte count | 3 | 50.0 | 4 | 0 | 0.0 | 0 | 2 | 33.3 | 2 | 0 | 0.0 | 0 |
| | Increased platelet count | 4 | 66.7 | 4 | 0 | 0.0 | 0 | 6 | 100.0 | 6 | 0 | 0.0 | 0 |
| | Increased brain natriuretic peptide | 6 | 100.0 | 7 | 0 | 0.0 | 0 | 5 | 83.3 | 5 | 0 | 0.0 | 0 |
| | Increased blood alkaline phosphatase | 1 | 16.7 | 1 | 0 | 0.0 | 0 | 1 | 16.7 | 1 | 0 | 0.0 | 0 |
| Metabolic and nutritional disorders | | 0 | 0.0 | 0 | 0 | 0.0 | 0 | 2 | 33.3 | 2 | 0 | 0.0 | 0 |
| | Acidosis | 0 | 0.0 | 0 | 0 | 0.0 | 0 | 1 | 16.7 | 1 | 0 | 0.0 | 0 |
| | Dehydration | 0 | 0.0 | 0 | 0 | 0.0 | 0 | 0 | 0.0 | 0 | 0 | 0.0 | 0 |
| | Hypovolemia | 0 | 0.0 | 0 | 0 | 0.0 | 0 | 1 | 16.7 | 1 | 0 | 0.0 | 0 |

MedDRA/J Version 18.1

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 2 | 33.3 | 2 | 0 | 0.0 | 0 |
| | Decreased blood uric acid | 0 | 0.0 | 0 | 0 | 0.0 | 0 | 0 | 0.0 | 0 | 0 | 0.0 | 0 |
| | Increased blood uric acid | 0 | 0.0 | 0 | 1 | 16.7 | 1 | 2 | 33.3 | 2 | 0 | 0.0 | 0 |
| | Increased C-reactive protein | 6 | 100.0 | 6 | 0 | 0.0 | 0 | 6 | 100.0 | 6 | 0 | 0.0 | 0 |
| | Electrocardiographic T-wave inversion | 0 | 0.0 | 0 | 0 | 0.0 | 0 | 3 | 50.0 | 3 | 0 | 0.0 | 0 |
| | Increased eosinophil count | 1 | 16.7 | 1 | 0 | 0.0 | 0 | 0 | 0.0 | 0 | 0 | 0.0 | 0 |
| | Decreased hematocrit | 5 | 83.3 | 5 | 0 | 0.0 | 0 | 4 | 66.7 | 4 | 0 | 0.0 | 0 |
| | Decreased hemoglobin | 5 | 83.3 | 5 | 0 | 0.0 | 0 | 4 | 66.7 | 4 | 0 | 0.0 | 0 |
| | Decreased heart rate | 1 | 16.7 | 1 | 0 | 0.0 | 0 | 0 | 0.0 | 0 | 0 | 0.0 | 0 |
| | Decreased lymphocyte count | 6 | 100.0 | 6 | 0 | 0.0 | 0 | 6 | 100.0 | 6 | 0 | 0.0 | 0 |
| | Decreased monocyte count | 1 | 16.7 | 1 | 0 | 0.0 | 0 | 0 | 0.0 | 0 | 0 | 0.0 | 0 |
| | Increased monocyte count | 1 | 16.7 | 1 | 0 | 0.0 | 0 | 0 | 0.0 | 0 | 0 | 0.0 | 0 |
| | Increased neutrophil count | 6 | 100.0 | 6 | 0 | 0.0 | 0 | 6 | 100.0 | 6 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Oxygen desaturation | 0 | 0.0 | 0 | 0 | 0.0 | 0 | 0 | 0.0 | 0 | 0 | 0.0 | 0 |
| | Decreased platelet count | 4 | 66.7 | 4 | 0 | 0.0 | 0 | 5 | 83.3 | 6 | 0 | 0.0 | 0 |
| | Increased prothrombin time | 2 | 33.3 | 2 | 0 | 0.0 | 0 | 2 | 33.3 | 2 | 0 | 0.0 | 0 |
| | Decreased erythrocyte count | 5 | 83.3 | 5 | 0 | 0.0 | 0 | 4 | 66.7 | 4 | 0 | 0.0 | 0 |
| | Increased leukocyte count | 4 | 66.7 | 4 | 0 | 0.0 | 0 | 4 | 66.7 | 4 | 0 | 0.0 | 0 |
| | Increased platelet count | 4 | 66.7 | 4 | 0 | 0.0 | 0 | 6 | 100.0 | 6 | 0 | 0.0 | 0 |
| | Increased brain natriuretic peptide | 5 | 83.3 | 5 | 0 | 0.0 | 0 | 5 | 83.3 | 5 | 0 | 0.0 | 0 |
| | Increased blood alkaline phosphatase | 4 | 66.7 | 4 | 0 | 0.0 | 0 | 1 | 16.7 | 1 | 0 | 0.0 | 0 |
| Metabolic and nutritional disorders | | 1 | 16.7 | 1 | 0 | 0.0 | 0 | 2 | 33.3 | 2 | 0 | 0.0 | 0 |
| | Acidosis | 0 | 0.0 | 0 | 0 | 0.0 | 0 | 0 | 0.0 | 0 | 0 | 0.0 | 0 |
| | Dehydration | 0 | 0.0 | 0 | 0 | 0.0 | 0 | 2 | 33.3 | 2 | 0 | 0.0 | 0 |
| | Hypovolemia | 1 | 16.7 | 1 | 0 | 0.0 | 0 | 0 | 0.0 | 0 | 0 | 0.0 | 0 |
| MedDRA/J Version 18.1 | | | | | | | | | | | | | |

(continued)

| Causal relationship | | Safety evaluation period (within 6 weeks after administration) | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 1 | 16.7 | 1 | 0 | 0.0 | 0 | 0 | 0.0 | 0 | 0 | 0.0 | 0 |
| | Decreased blood uric acid | 1 | 16.7 | 1 | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 | 0 | 0.0 | 0 |
| | Increased C-reactive protein | 6 | 100.0 | 6 | 0 | 0.0 | 0 | 6 | 100.0 | 6 | 0 | 0.0 | 0 |
| | Electrocardiographic T-wave inversion | 2 | 33.3 | 2 | 0 | 0.0 | 0 | 2 | 33.3 | 2 | 0 | 0.0 | 0 |
| | Increased eosinophil count | 1 | 16.7 | 1 | 0 | 0.0 | 0 | 0 | 0.0 | 0 | 0 | 0.0 | 0 |
| | Decreased hematocrit | 6 | 100.0 | 6 | 0 | 0.0 | 0 | 6 | 100.0 | 6 | 0 | 0.0 | 0 |
| | Decreased hemoglobin | 6 | 100.0 | 6 | 0 | 0.0 | 0 | 6 | 100.0 | 6 | 0 | 0.0 | 0 |
| | Decreased heart rate | 0 | 0.0 | 0 | 0 | 0.0 | 0 | 0 | 0.0 | 0 | 0 | 0.0 | 0 |
| | Decreased lymphocyte count | 6 | 100.0 | 6 | 0 | 0.0 | 0 | 6 | 100.0 | 6 | 0 | 0.0 | 0 |
| | Decreased monocyte count | 1 | 16.7 | 1 | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 | 0 | 0.0 | 0 |
| | Increased neutrophil count | 6 | 100.0 | 6 | 0 | 0.0 | 0 | 6 | 100.0 | 6 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Oxygen desaturation | 0 | 0.0 | 0 | 0 | 0.0 | 0 | 1 | 16.7 | 1 | 0 | 0.0 | 0 |
| | Decreased platelet count | 4 | 66.7 | 5 | 0 | 0.0 | 0 | 4 | 66.7 | 4 | 0 | 0.0 | 0 |
| | Increased prothrombin time | 1 | 16.7 | 1 | 0 | 0.0 | 0 | 2 | 33.3 | 2 | 0 | 0.0 | 0 |
| | Decreased erythrocyte count | 6 | 100.0 | 6 | 0 | 0.0 | 0 | 6 | 100.0 | 6 | 0 | 0.0 | 0 |
| | Increased leukocyte count | 3 | 50.0 | 4 | 0 | 0.0 | 0 | 2 | 33.3 | 2 | 0 | 0.0 | 0 |
| | Increased platelet count | 4 | 66.7 | 4 | 0 | 0.0 | 0 | 6 | 100.0 | 6 | 0 | 0.0 | 0 |
| | Increased brain natriuretic peptide | 6 | 100.0 | 6 | 0 | 0.0 | 0 | 5 | 83.3 | 5 | 0 | 0.0 | 0 |
| | Increased blood alkaline phosphatase | 1 | 16.7 | 1 | 0 | 0.0 | 0 | 1 | 16.7 | 1 | 0 | 0.0 | 0 |
| Metabolic and nutritional disorders | | 0 | 0.0 | 0 | 0 | 0.0 | 0 | 2 | 33.3 | 2 | 0 | 0.0 | 0 |
| | Acidosis | 0 | 0.0 | 0 | 0 | 0.0 | 0 | 1 | 16.7 | 1 | 0 | 0.0 | 0 |
| | Dehydration | 0 | 0.0 | 0 | 0 | 0.0 | 0 | 0 | 0.0 | 0 | 0 | 0.0 | 0 |
| | Hypovolemia | 0 | 0.0 | 0 | 0 | 0.0 | 0 | 1 | 16.7 | 1 | 0 | 0.0 | 0 |

(continued)

| | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Oxygen desaturation | 0 | 0.0 | 0 | 0 | 0.0 | 0 | 0 | 0.0 | 0 | 0 | 0.0 | 0 |
| | Decreased platelet count | 0 | 0.0 | 0 | 0 | 0.0 | 0 | 0 | 0.0 | 0 | 0 | 0.0 | 0 |
| | Increased prothrombin time | 0 | 0.0 | 0 | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 | 0 | 0.0 | 0 |
| | Increased platelet count | 0 | 0.0 | 0 | 0 | 0.0 | 0 | 1 | 16.7 | 1 | 0 | 0.0 | 0 |
| | Increased brain natriuretic peptide | 0 | 0.0 | 0 | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Clinical test | Increased blood urea | 0 | 0.0 | 0 | 0 | 0.0 | 0 | 0 | 0.0 | 0 | 0 | 0.0 | 0 |
| | Decreased blood uric acid | 0 | 0.0 | 0 | 0 | 0.0 | 0 | 0 | 0.0 | 0 | 0 | 0.0 | 0 |
| | Increased blood uric acid | 0 | 0.0 | 0 | 0 | 0.0 | 0 | 0 | 0.0 | 0 | 0 | 0.0 | 0 |
| | Increased C-reactive protein | 0 | 0.0 | 0 | 0 | 0.0 | 0 | 0 | 0.0 | 0 | 0 | 0.0 | 0 |
| | Electrocardiographic T-wave inversion | 0 | 0.0 | 0 | 0 | 0.0 | 0 | 0 | 0.0 | 0 | 0 | 0.0 | 0 |
| | Increased eosinophil count | 0 | 0.0 | 0 | 0 | 0.0 | 0 | 0 | 0.0 | 0 | 0 | 0.0 | 0 |
| | Decreased hematocrit | 0 | 0.0 | 0 | 0 | 0.0 | 0 | 0 | 0.0 | 0 | 0 | 0.0 | 0 |
| | Decreased hemoglobin | 0 | 0.0 | 0 | 0 | 0.0 | 0 | 0 | 0.0 | 0 | 0 | 0.0 | 0 |
| | Decreased heart rate | 0 | 0.0 | 0 | 0 | 0.0 | 0 | 0 | 0.0 | 0 | 0 | 0.0 | 0 |
| | Decreased lymphocyte count | 0 | 0.0 | 0 | 0 | 0.0 | 0 | 0 | 0.0 | 0 | 0 | 0.0 | 0 |
| | Decreased monocyte count | 0 | 0.0 | 0 | 0 | 0.0 | 0 | 0 | 0.0 | 0 | 0 | 0.0 | 0 |
| | Increased monocyte count | 0 | 0.0 | 0 | 0 | 0.0 | 0 | 0 | 0.0 | 0 | 0 | 0.0 | 0 |
| | Increased neutrophil count | 0 | 0.0 | 0 | 0 | 0.0 | 0 | 0 | 0.0 | 0 | 0 | 0.0 | 0 |
| | Oxygen desaturation | 0 | 0.0 | 0 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| | Decreased platelet count | 0 | 0.0 | 0 | 0 | 0.0 | 0 | 0 | 0.0 | 0 | 0 | 0.0 | 0 |
| | Increased prothrombin time | 1 | 16.7 | 1 | 0 | 0.0 | 0 | 0 | 0.0 | 0 | 0 | 0.0 | 0 |
| | Decreased erythrocyte count | 0 | 0.0 | 0 | 0 | 0.0 | 0 | 0 | 0.0 | 0 | 0 | 0.0 | 0 |
| | Increased leukocyte count | 0 | 0.0 | 0 | 0 | 0.0 | 0 | 0 | 0.0 | 0 | 0 | 0.0 | 0 |
| | Increased platelet count | 0 | 0.0 | 0 | 0 | 0.0 | 0 | 0 | 0.0 | 0 | 0 | 0.0 | 0 |
| | Increased brain natriuretic peptide | 1 | 16.7 | 1 | 0 | 0.0 | 0 | 0 | 0.0 | 0 | 0 | 0.0 | 0 |
| | Increased blood alkaline phosphatase | 0 | 0.0 | 0 | 0 | 0.0 | 0 | 0 | 0.0 | 0 | 0 | 0.0 | 0 |
| Metabolic and nutritional disorders | | 0 | 0.0 | 0 | 0 | 0.0 | 0 | 0 | 0.0 | 0 | 0 | 0.0 | 0 |
| | Acidosis | 0 | 0.0 | 0 | 0 | 0.0 | 0 | 0 | 0.0 | 0 | 0 | 0.0 | 0 |
| | Dehydration | 0 | 0.0 | 0 | 0 | 0.0 | 0 | 0 | 0.0 | 0 | 0 | 0.0 | 0 |
| | Hypovolemia | 0 | 0.0 | 0 | 0 | 0.0 | 0 | 0 | 0.0 | 0 | 0 | 0.0 | 0 |

MedDRA/J Version 18.1

(continued)

| Causal relationship | | Whole period | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 1 | 16.7 | 1 | 0 | 0.0 | 0 |
| | Muscle pain | 1 | 16.7 | 1 | 0 | 0.0 | 0 | 0 | 0.0 | 0 | 0 | 0.0 | 0 |
| | Musculoskeletal stiffness | 0 | 0.0 | 0 | 0 | 0.0 | 0 | 1 | 16.7 | 1 | 0 | 0.0 | 0 |
| Nervous system disorders | | 0 | 0.0 | 0 | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 | 0 | 0.0 | 0 |
| Mental disorders | | 5 | 83.3 | 6 | 0 | 0.0 | 0 | 0 | 0.0 | 0 | 0 | 0.0 | 0 |
| | Delirium | 1 | 16.7 | 1 | 0 | 0.0 | 0 | 0 | 0.0 | 0 | 0 | 0.0 | 0 |
| | Restlessness | 2 | 33.3 | 2 | 0 | 0.0 | 0 | 0 | 0.0 | 0 | 0 | 0.0 | 0 |
| | Sleeping disorder | 3 | 50.0 | 3 | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 | 0 | 0.0 | 0 |
| Reproductive system and breast disorders | | 0 | 0.0 | 0 | 0 | 0.0 | 0 | 1 | 16.7 | 1 | 0 | 0.0 | 0 |
| | Gynecomastia | 0 | 0.0 | 0 | 0 | 0.0 | 0 | 1 | 16.7 | 1 | 0 | 0.0 | 0 |
| Respiratory, thoracic and | | 4 | 66.7 | 6 | 0 | 0.0 | 0 | 6 | 100.0 | 6 | 0 | 0.0 | 0 |
| | Atelectasis | 1 | 16.7 | 1 | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 | 0 | 0.0 | 0 |

(continued)

| Causal relationship | | Whole period | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| mediastinal disorders | Pleural effusion | 4 | 66.7 | 4 | 0 | 0.0 | 0 | 6 | 100.0 | 6 | 0 | 0.0 | 0 |
| | Aspiration pneumonia | 0 | 0.0 | 0 | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 | 0 | 0.0 | 0 |
| | Respiratory failure | 1 | 16.7 | 1 | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 | 0 | 0.0 | 0 |
| | Drug eruption | 1 | 16.7 | 1 | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 | 0 | 0.0 | 0 |

| Causal relationship | | Whole period | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 | 0 | 0.0 | 0 |
| Nervous system disorders | | 0 | 0.0 | 0 | 0 | 0.0 | 0 | 1 | 16.7 | 1 | 0 | 0.0 | 0 |
| | Pitaminal hemorrhage | 0 | 0.0 | 0 | 0 | 0.0 | 0 | 1 | 16.7 | 1 | 0 | 0.0 | 0 |

(continued)

| Causal relationship | | Whole period | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Mental disorders | | 0 | 0.0 | 0 | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 | 0 | 0.0 | 0 |
| Nenal and urinary tract disorders | | 1 | 16.7 | 1 | 0 | 0.0 | 0 | 0 | 0.0 | 0 | 0 | 0.0 | 0 |
| | Kidney failure | 1 | 16.7 | 1 | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 | 0 | 0.0 | 0 |
| Respiratory, thoracic and mediastinal disorders | | 6 | 100.0 | 9 | 0 | 0.0 | 0 | 4 | 66.7 | 4 | 0 | 0.0 | 0 |
| | Atelectasis | 1 | 16.7 | 1 | 0 | 0.0 | 0 | 1 | 16.7 | 1 | 0 | 0.0 | 0 |
| | Difficulty breathing | 1 | 16.7 | 1 | 0 | 0.0 | 0 | 0 | 0.0 | 0 | 0 | 0.0 | 0 |
| | Pleural effusion | 6 | 100.0 | 6 | 0 | 0.0 | 0 | 2 | 33.3 | 2 | 0 | 0.0 | 0 |
| | Aspiration pneumonia | 0 | 0.0 | 0 | 0 | 0.0 | 0 | 1 | 16.7 | 1 | 0 | 0.0 | 0 |
| | Pulmonary congestion | 1 | 16.7 | 1 | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 2 | 33.3 | 2 | 0 | 0.0 | 0 |
| | Decubitus ulcer | 1 | 16.7 | 1 | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 | 0 | 0.0 | 0 |
| | Rash | 0 | 0.0 | 0 | 0 | 0.0 | 0 | 1 | 16.7 | 1 | 0 | 0.0 | 0 |
| | Hives | 0 | 0.0 | 0 | 0 | 0.0 | 0 | 1 | 16.7 | 1 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 1 | 16.7 | 1 | 0 | 0.0 | 0 | 0 | 0.0 | 0 | 0 | 0.0 | 0 |
| | Muscle pain | 1 | 16.7 | 1 | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 | 0 | 0.0 | 0 |
| Mental disorders | | 5 | 83.3 | 6 | 0 | 0.0 | 0 | 0 | 0.0 | 0 | 0 | 0.0 | 0 |
| | Delirium | 1 | 16.7 | 1 | 0 | 0.0 | 0 | 0 | 0.0 | 0 | 0 | 0.0 | 0 |
| | Restlessness | 2 | 33.3 | 2 | 0 | 0.0 | 0 | 0 | 0.0 | 0 | 0 | 0.0 | 0 |
| | Sleeping disorder | 3 | 50.0 | 3 | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 | 0 | 0.0 | 0 |

(continued)

| Causal relationship | | Safety evaluation period (within 6 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Respiratory, thoracic and mediastinal disorders | | 4 | 66.7 | 6 | 0 | 0.0 | 0 | 6 | 100.0 | 6 | 0 | 0.0 | 0 |
| | Atelectasis | 1 | 16.7 | 1 | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 | 0 | 0.0 | 0 |
| | Pleural effusion | 4 | 66.7 | 4 | 0 | 0.0 | 0 | 6 | 100.0 | 6 | 0 | 0.0 | 0 |
| | Aspiration pneumonia | 0 | 0.0 | 0 | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 | 0 | 0.0 | 0 |
| | Respiratory failure | 1 | 16.7 | 1 | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 | 0 | 0.0 | 0 |
| | Drug eruption | 1 | 16.7 | 1 | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 | 0 | 0.0 | 0 |

MedDRA/J Version 18.1

(continued)

| Causal relationship | | Safety evaluation period (within 6 weeks after administration) | | | | | | | | | | | |
|---|---|---|---|---|---|---|---|---|---|---|---|---|---|
| | | 100 mg (N=6) | | | | | | Placebo (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 | 0 | 0.0 | 0 |
| Nervous system disorders | | 0 | 0.0 | 0 | 0 | 0.0 | 0 | 1 | 16.7 | 1 | 0 | 0.0 | 0 |
| | Putaminal hemorrhage | 0 | 0.0 | 0 | 0 | 0.0 | 0 | 1 | 16.7 | 1 | 0 | 0.0 | 0 |
| Mental disorders | | 0 | 0.0 | 0 | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 | 0 | 0.0 | 0 |
| Nenal and urinary tract disorders | | 1 | 16.7 | 1 | 0 | 0.0 | 0 | 0 | 0.0 | 0 | 0 | 0.0 | 0 |
| | Kidney failure | 1 | 16.7 | 1 | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 | 0 | 0.0 | 0 |
| Respiratory, thoracic and | | 6 | 100.0 | 9 | 0 | 0.0 | 0 | 4 | 66.7 | 4 | 0 | 0.0 | 0 |
| | Atelectasis | 1 | 16.7 | 1 | 0 | 0.0 | 0 | 1 | 16.7 | 1 | 0 | 0.0 | 0 |
| | Difficulty breathing | 1 | 16.7 | 1 | 0 | 0.0 | 0 | 0 | 0.0 | 0 | 0 | 0.0 | 0 |

(continued)

| | | Safety evaluation period (within 6 weeks after administration) | | | | | | | | | | | |
|---|---|---|---|---|---|---|---|---|---|---|---|---|---|
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| mediastinal disorders | Pleural effusion | 6 | 100.0 | 6 | 0 | 0.0 | 0 | 2 | 33.3 | 2 | 0 | 0.0 | 0 |
| | Aspiration pneumonia | 0 | 0.0 | 0 | 0 | 0.0 | 0 | 1 | 16.7 | 1 | 0 | 0.0 | 0 |
| | Pulmonary congestion | 1 | 16.7 | 1 | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 | 0 | 0.0 | 0 |
| Skin and subcutaneous tissue disorders | | 1 | 16.7 | 1 | 0 | 0.0 | 0 | 2 | 33.3 | 2 | 0 | 0.0 | 0 |
| | Decubitus ulcer | 1 | 16.7 | 1 | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 | 0 | 0.0 | 0 |
| | Rash | 0 | 0.0 | 0 | 0 | 0.0 | 0 | 1 | 16.7 | 1 | 0 | 0.0 | 0 |
| | Hives | 0 | 0.0 | 0 | 0 | 0.0 | 0 | 1 | 16.7 | 1 | 0 | 0.0 | 0 |

MedDRA/J Version 18.1

| | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 1 | 16.7 | 1 | 0 | 0.0 | 0 |
| | Muscle pain | 0 | 0.0 | 0 | 0 | 0.0 | 0 | 0 | 0.0 | 0 | 0 | 0.0 | 0 |
| | Musculoskeletal stiffness | 0 | 0.0 | 0 | 0 | 0.0 | 0 | 1 | 16.7 | 1 | 0 | 0.0 | 0 |

(continued)

| Causal relationship | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Nervous system disorders | | 0 | 0.0 | 0 | 0 | 0.0 | 0 | 0 | 0.0 | 0 | 0 | 0.0 | 0 |
| | Pitaminal hemorrhage | 0 | 0.0 | 0 | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 | 0 | 0.0 | 0 |
| Nenal and urinary tract disorders | | 0 | 0.0 | 0 | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 | 0 | 0.0 | 0 |
| Reproductive system and breast disorders | | 0 | 0.0 | 0 | 0 | 0.0 | 0 | 1 | 16.7 | 1 | 0 | 0.0 | 0 |
| | Gynecomastia | 0 | 0.0 | 0 | 0 | 0.0 | 0 | 1 | 16.7 | 1 | 0 | 0.0 | 0 |
| Respiratory, thoracic and mediastinal disorders | | 0 | 0.0 | 0 | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Skin and subcutaneous tissue disorders | | 0 | 0.0 | 0 | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 | 0 | 0.0 | 0 |
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Musculoskeletal and connective tissue disorders | | 0 | 0.0 | 0 | 0 | 0.0 | 0 | 0 | 0.0 | 0 | 0 | 0.0 | 0 |
| | Muscle pain | 0 | 0.0 | 0 | 0 | 0.0 | 0 | 0 | 0.0 | 0 | 0 | 0.0 | 0 |
| | Musculoskeletal stiffness | 0 | 0.0 | 0 | 0 | 0.0 | 0 | 0 | 0.0 | 0 | 0 | 0.0 | 0 |
| Nervous system disorders | | 0 | 0.0 | 0 | 0 | 0.0 | 0 | 0 | 0.0 | 0 | 0 | 0.0 | 0 |
| | Putaminal hemorrhage | 0 | 0.0 | 0 | 0 | 0.0 | 0 | 0 | 0.0 | 0 | 0 | 0.0 | 0 |
| Mental disorders | | 0 | 0.0 | 0 | 0 | 0.0 | 0 | 0 | 0.0 | 0 | 0 | 0.0 | 0 |
| | Delirium | 0 | 0.0 | 0 | 0 | 0.0 | 0 | 0 | 0.0 | 0 | 0 | 0.0 | 0 |
| | Restlessness | 0 | 0.0 | 0 | 0 | 0.0 | 0 | 0 | 0.0 | 0 | 0 | 0.0 | 0 |
| | Sleeping disorder | 0 | 0.0 | 0 | 0 | 0.0 | 0 | 0 | 0.0 | 0 | 0 | 0.0 | 0 |

(continued)

| | | 100 mg (N=6) | | | | | | Placebo (N=6) | | | | | |
|---|---|---|---|---|---|---|---|---|---|---|---|---|---|
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Nenal and urinary tract disorders | | 0 | 0.0 | 0 | 0 | 0.0 | 0 | 0 | 0.0 | 0 | 0 | 0.0 | 0 |
| | Kidney failure | 0 | 0.0 | 0 | 0 | 0.0 | 0 | 0 | 0.0 | 0 | 0 | 0.0 | 0 |
| Reproductive system and breast disorders | | 0 | 0.0 | 0 | 0 | 0.0 | 0 | 0 | 0.0 | 0 | 0 | 0.0 | 0 |
| | Gynecomastia | 0 | 0.0 | 0 | 0 | 0.0 | 0 | 0 | 0.0 | 0 | 0 | 0.0 | 0 |
| breast discordes Respiratory, thoracic and mediastinal disorders | | 0 | 0.0 | 0 | 0 | 0.0 | 0 | 0 | 0.0 | 0 | 0 | 0.0 | 0 |
| | Atelectasis | 0 | 0.0 | 0 | 0 | 0.0 | 0 | 0 | 0.0 | 0 | 0 | 0.0 | 0 |
| | Difficulty breathing | 0 | 0.0 | 0 | 0 | 0.0 | 0 | 0 | 0.0 | 0 | 0 | 0.0 | 0 |
| | Pleural effusion | 0 | 0.0 | 0 | 0 | 0.0 | 0 | 0 | 0.0 | 0 | 0 | 0.0 | 0 |
| | Aspiration pneumonia | 0 | 0.0 | 0 | 0 | 0.0 | 0 | 0 | 0.0 | 0 | 0 | 0.0 | 0 |
| | Pulmonary congestion | 0 | 0.0 | 0 | 0 | 0.0 | 0 | 0 | 0.0 | 0 | 0 | 0.0 | 0 |
| | Respiratory failure | 0 | 0.0 | 0 | 0 | 0.0 | 0 | 0 | 0.0 | 0 | 0 | 0.0 | 0 |
| Skin and subcutaneous tissue disorders | | 0 | 0.0 | 0 | 0 | 0.0 | 0 | 0 | 0.0 | 0 | 0 | 0.0 | 0 |
| | Decubitus ulcer | 0 | 0.0 | 0 | 0 | 0.0 | 0 | 0 | 0.0 | 0 | 0 | 0.0 | 0 |
| | Drug eruption | 0 | 0.0 | 0 | 0 | 0.0 | 0 | 0 | 0.0 | 0 | 0 | 0.0 | 0 |
| | Rash | 0 | 0.0 | 0 | 0 | 0.0 | 0 | 0 | 0.0 | 0 | 0 | 0.0 | 0 |
| | Hives | 0 | 0.0 | 0 | 0 | 0.0 | 0 | 0 | 0.0 | 0 | 0 | 0.0 | 0 |
| MedDRA/J Version 18.1 | | | | | | | | | | | | | |

(continued)

| | | Whole period | | | | | | | | | | | |
|---|---|---|---|---|---|---|---|---|---|---|---|---|---|
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 | 0 | 0.0 | 0 |
| MedDRA/J Version 18.1 | | | | | | | | | | | | | |
| | | Whole period | | | | | | | | | | | |
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 1 | 16.7 | 1 | 0 | 0.0 | 0 | 0 | 0.0 | 0 | 0 | 0.0 | 0 |
| | Thrombosis | 1 | 16.7 | 1 | 0 | 0.0 | 0 | 0 | 0.0 | 0 | 0 | 0.0 | 0 |
| | | Safety evaluation period (within 6 weeks after administration) | | | | | | | | | | | |
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 | 0 | 0.0 | 0 |

(continued)

| | | Safety evaluation period (within 6 weeks after administration) | | | | | | | | | | | |
|---|---|---|---|---|---|---|---|---|---|---|---|---|---|
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 1 | 16.7 | 1 | 0 | 0.0 | 0 | 0 | 0.0 | 0 | 0 | 0.0 | 0 |
| | Thrombosis | 1 | 16.7 | 1 | 0 | 0.0 | 0 | 0 | 0.0 | 0 | 0 | 0.0 | 0 |
| | | Safety and efficacy evaluation period (more than 6 weeks after administration to 26 weeks after administration) | | | | | | | | | | | |
| | | 10 mg (N=6) | | | | | | 30 mg (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 | 0 | 0.0 | 0 |
| | | 100 mg (N=6) | | | | | | Placebo (N=6) | | | | | |
| Causal relationship | | Unrelated | | | Related | | | Unrelated | | | Related | | |
| System organ classification | Preferred term | Number of examples | Incidence [%] | Number of occurrences | Number of examples | Incidence [%] | Number of occurrences | Number of examples | Incidence [%] | Number of occurrences | Number of examples | Incidence [%] | Number of occurrences |
| Vascular disorder | | 0 | 0.0 | 0 | 0 | 0.0 | 0 | 0 | 0.0 | 0 | 0 | 0.0 | 0 |
| | Thrombosis | 0 | 0.0 | 0 | 0 | 0.0 | 0 | 0 | 0.0 | 0 | 0 | 0.0 | 0 |
| MedDRA/J Version 18.1 | | | | | | | | | | | | | |

Table 56

| | | Whole period | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 1 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

MedDRA/J Version 18.1

| | | Whole period | | | | | | | | |
|---|---|---|---|---|---|---|---|---|---|---|
| | | 10 mg (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 1 | 4 | 66.7 | 123 | 0 | 0.0 | 0 |
| Heart failure | | 0 | 0.0 | 0 | 2 | 33.3 | 2 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 10 mg (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Tachycardia | 0 | 0.0 | 0 | 2 | 33.3 | 2 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 3 | 50.0 | 5 | 0 | 0.0 | 0 |
| | Constipation | 0 | 0.0 | 0 | 1 | 16.7 | 1 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 1 | 16.7 | 1 | 0 | 0.0 | 0 |
| | Lip ulcer | 0 | 0.0 | 0 | 1 | 16.7 | 1 | 0 | 0.0 | 0 |
| | Vomiting | 0 | 0.0 | 0 | 1 | 16.7 | 1 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 1 | 16.7 | 1 | 0 | 0.0 | 0 |
| General/systemic disorders and administration site conditions | | 0 | 0.0 | 0 | 3 | 50.0 | 4 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 1 | 16.7 | 1 | 0 | 0.0 | 0 |
| | Fever | 0 | 0.0 | 0 | 2 | 33.3 | 3 | 0 | 0.0 | 0 |
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 2 | 33.3 | 4 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 3 | 50.0 | 5 | 1 | 16.7 | 110 | 0 | 0.0 | 0 |
| Heart failure | | 0 | 0.0 | 0 | 1 | 16.7 | 2 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 2 | 33.3 | 2 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 5 | 83.3 | 8 | 0 | 0.0 | 0 |
| | Chills | 0 | 0.0 | 0 | 0 | 0.0 | 0 | 0 | 0.0 | 0 |
| | Poor healing | 0 | 0.0 | 0 | 1 | 16.7 | 1 | 0 | 0.0 | 0 |
| | Edema | 0 | 0.0 | 0 | 0 | 0.0 | 0 | 0 | 0.0 | 0 |
| | Fever | 0 | 0.0 | 0 | 5 | 83.3 | 7 | 0 | 0.0 | 0 |
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 2 | 33.3 | 13 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 2 | 33.3 | 3 | 2 | 33.3 | 91 | 0 | 0.0 | 0 |
| Heart failure | | 1 | 16.7 | 1 | 4 | 66.7 | 7 | 0 | 0.0 | 0 |
| | Atrial fibrillation | 0 | 0.0 | 0 | 3 | 50.0 | 3 | 0 | 0.0 | 0 |
| | First-degree atrioventricular block | 0 | 0.0 | 0 | 1 | 16.7 | 1 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 1 | 16.7 | 1 | 0 | 0.0 | 0 |
| | Mitral regurgitation | 1 | 16.7 | 1 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Pericardial effusion | 0 | 0.0 | 0 | 1 | 16.7 | 1 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 1 | 16.7 | 1 | 0 | 0.0 | 0 |
| Gastrointestinal disorders | | 0 | 0.0 | 0 | 1 | 16.7 | 3 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 1 | 16.7 | 2 | 0 | 0.0 | 0 |
| | Anal bleeding | 0 | 0.0 | 0 | 1 | 16.7 | 1 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 5 | 83.3 | 6 | 0 | 0.0 | 0 |
| | Chills | 0 | 0.0 | 0 | 1 | 16.7 | 1 | 0 | 0.0 | 0 |
| | Poor healing | 0 | 0.0 | 0 | 0 | 0.0 | 0 | 0 | 0.0 | 0 |
| | Edema | 0 | 0.0 | 0 | 2 | 33.3 | 2 | 0 | 0.0 | 0 |
| | Fever | 0 | 0.0 | 0 | 3 | 50.0 | 3 | 0 | 0.0 | 0 |

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 1 | 16.7 | 1 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 1 | 5 | 83.3 | 108 | 0 | 0.0 | 0 |
| Heart failure | | 0 | 0.0 | 0 | 3 | 50.0 | 4 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 1 | 16.7 | 1 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 1 | 16.7 | 1 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 3 | 50.0 | 3 | 0 | 0.0 | 0 |
| | Constipation | 0 | 0.0 | 0 | 1 | 16.7 | 1 | 0 | 0.0 | 0 |
| | Diarrhea | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 4 | 66.7 | 4 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 4 | 66.7 | 4 | 0 | 0.0 | 0 |

| | | Safety evaluation period (within 6 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 1 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 1 | 4 | 66.7 | 123 | 0 | 0.0 | 0 |
| Heart failure | | 0 | 0.0 | 0 | 2 | 33.3 | 2 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 2 | 33.3 | 2 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 3 | 50.0 | 5 | 0 | 0.0 | 0 |
| | Constipation | 0 | 0.0 | 0 | 1 | 16.7 | 1 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 1 | 16.7 | 1 | 0 | 0.0 | 0 |
| | Lip ulcer | 0 | 0.0 | 0 | 1 | 16.7 | 1 | 0 | 0.0 | 0 |
| | Vomiting | 0 | 0.0 | 0 | 1 | 16.7 | 1 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 1 | 16.7 | 1 | 0 | 0.0 | 0 |
| General/systemic disorders and administration site conditions | | 0 | 0.0 | 0 | 3 | 50.0 | 4 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 1 | 16.7 | 1 | 0 | 0.0 | 0 |
| | Fever | 0 | 0.0 | 0 | 2 | 33.3 | 3 | 0 | 0.0 | 0 |

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 2 | 33.3 | 2 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 3 | 50.0 | 4 | 1 | 16.7 | 103 | 0 | 0.0 | 0 |
| Heart failure | | 0 | 0.0 | 0 | 1 | 16.7 | 2 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 2 | 33.3 | 2 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 5 | 83.3 | 7 | 0 | 0.0 | 0 |
| | Chills | 0 | 0.0 | 0 | 0 | 0.0 | 0 | 0 | 0.0 | 0 |
| | Poor healing | 0 | 0.0 | 0 | 1 | 16.7 | 1 | 0 | 0.0 | 0 |
| | Edema | 0 | 0.0 | 0 | 0 | 0.0 | 0 | 0 | 0.0 | 0 |
| | Fever | 0 | 0.0 | 0 | 5 | 83.3 | 6 | 0 | 0.0 | 0 |
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 12 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 2 | 33.3 | 2 | 3 | 50.0 | 88 | 0 | 0.0 | 0 |
| Heart failure | | 0 | 0.0 | 0 | 5 | 83.3 | 7 | 0 | 0.0 | 0 |
| | Atrial fibrillation | 0 | 0.0 | 0 | 3 | 50.0 | 3 | 0 | 0.0 | 0 |
| | First-degree atrioventricular block | 0 | 0.0 | 0 | 1 | 16.7 | 1 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 1 | 16.7 | 1 | 0 | 0.0 | 0 |
| | Mitral regurgitation | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Pericardial effusion | 0 | 0.0 | 0 | 1 | 16.7 | 1 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 1 | 16.7 | 1 | 0 | 0.0 | 0 |
| Gastrointestinal disorders | | 0 | 0.0 | 0 | 1 | 16.7 | 1 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 1 | 16.7 | 1 | 0 | 0.0 | 0 |
| | Loose stool Loose stool | 0 | 0.0 | 0 | 0 | 0.0 | 0 | 0 | 0.0 | 0 |
| General/systemic disorders and administration site conditions | | 0 | 0.0 | 0 | 5 | 83.3 | 6 | 0 | 0.0 | 0 |
| | Chills | 0 | 0.0 | 0 | 1 | 16.7 | 1 | 0 | 0.0 | 0 |
| | Poor healing | 0 | 0.0 | 0 | 0 | 0.0 | 0 | 0 | 0.0 | 0 |
| | Edema | 0 | 0.0 | 0 | 2 | 33.3 | 2 | 0 | 0.0 | 0 |
| | Fever | 0 | 0.0 | 0 | 3 | 50.0 | 3 | 0 | 0.0 | 0 |

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 1 | 16.7 | 1 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| | | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 1 | 5 | 83.3 | 108 | 0 | 0.0 | 0 |
| Heart failure | | 0 | 0.0 | 0 | 3 | 50.0 | 4 | 0 | 0.0 | 0 |
| | Atrial fibrillation | 0 | 0.0 | 0 | 1 | 16.7 | 1 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 1 | 16.7 | 1 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 1 | 16.7 | 1 | 0 | 0.0 | 0 |
| | Tachycardia | 0 | 0.0 | 0 | 1 | 16.7 | 1 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 3 | 50.0 | 3 | 0 | 0.0 | 0 |
| | Constipation | 0 | 0.0 | 0 | 1 | 16.7 | 1 | 0 | 0.0 | 0 |
| | Diarrhea | 0 | 0.0 | 0 | 2 | 33.3 | 2 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 4 | 66.7 | 4 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 4 | 66.7 | 4 | 0 | 0.0 | 0 |

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 2 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 1 | 3 | 50.0 | 7 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 1 | 16.7 | 1 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 1 | 16.7 | 1 | 0 | 0.0 | 0 |
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 1 | 16.7 | 1 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 1 | 16.7 | 1 | 1 | 16.7 | 3 | 0 | 0.0 | 0 |
| Heart failure | | 1 | 16.7 | 1 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 1 | 16.7 | 1 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 1 | 16.7 | 2 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 1 | 16.7 | 2 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Whole | | 0 | 0.0 | 0 | 0 | 0.0 | 0 | 0 | 0.0 | 0 |
| Heart failure | | 0 | 0.0 | 0 | 0 | 0.0 | 0 | 0 | 0.0 | 0 |
| | Atrial fibrillation | 0 | 0.0 | 0 | 0 | 0.0 | 0 | 0 | 0.0 | 0 |
| | First-degree atrioventricular block | 0 | 0.0 | 0 | 0 | 0.0 | 0 | 0 | 0.0 | 0 |
| | Right bundle branch block | 0 | 0.0 | 0 | 0 | 0.0 | 0 | 0 | 0.0 | 0 |
| | Cardiac tamponade | 0 | 0.0 | 0 | 0 | 0.0 | 0 | 0 | 0.0 | 0 |
| | Mitral regurgitation | 0 | 0.0 | 0 | 0 | 0.0 | 0 | 0 | 0.0 | 0 |
| | Pericardial effusion | 0 | 0.0 | 0 | 0 | 0.0 | 0 | 0 | 0.0 | 0 |
| | Sinus tachycardia | 0 | 0.0 | 0 | 0 | 0.0 | 0 | 0 | 0.0 | 0 |
| | Tachycardia | 0 | 0.0 | 0 | 0 | 0.0 | 0 | 0 | 0.0 | 0 |
| | Ventricular fibrillation | 0 | 0.0 | 0 | 0 | 0.0 | 0 | 0 | 0.0 | 0 |
| Gastrointestinal disorders | | 0 | 0.0 | 0 | 0 | 0.0 | 0 | 0 | 0.0 | 0 |
| | Constipation | 0 | 0.0 | 0 | 0 | 0.0 | 0 | 0 | 0.0 | 0 |
| | Diarrhea | 0 | 0.0 | 0 | 0 | 0.0 | 0 | 0 | 0.0 | 0 |
| | Dry lips | 0 | 0.0 | 0 | 0 | 0.0 | 0 | 0 | 0.0 | 0 |
| | Lip ulcer | 0 | 0.0 | 0 | 0 | 0.0 | 0 | 0 | 0.0 | 0 |
| | Vomiting | 0 | 0.0 | 0 | 0 | 0.0 | 0 | 0 | 0.0 | 0 |
| | Anal bleeding | 0 | 0.0 | 0 | 0 | 0.0 | 0 | 0 | 0.0 | 0 |
| | Loose stool | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| General/systemic disorders and administration site conditions | | 0 | 0.0 | 0 | 0 | 0.0 | 0 | 0 | 0.0 | 0 |
| | Chills | 0 | 0.0 | 0 | 0 | 0.0 | 0 | 0 | 0.0 | 0 |
| | Poor healing | 0 | 0.0 | 0 | 0 | 0.0 | 0 | 0 | 0.0 | 0 |
| | Edema | 0 | 0.0 | 0 | 0 | 0.0 | 0 | 0 | 0.0 | 0 |
| | Fever | 0 | 0.0 | 0 | 0 | 0.0 | 0 | 0 | 0.0 | 0 |

| | | Whole period | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 1 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |

(continued)

| Outcome | | Whole period | | | | | | | | |
|---|---|---|---|---|---|---|---|---|---|---|
| | | 10 mg (N=6) | | | | | | | | |
| | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 2 | 33.3 | 2 | 0 | 0.0 | 0 |
| | Bronchitis | 0 | 0.0 | 0 | 1 | 16.7 | 1 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 1 | 16.7 | 1 | 4 | 66.7 | 95 | 0 | 0.0 | 0 |
| | Increased alanine aminotransferase | 0 | 0.0 | 0 | 5 | 83.3 | 5 | 0 | 0.0 | 0 |
| | Increased amylase | 0 | 0.0 | 0 | 1 | 16.7 | 1 | 0 | 0.0 | 0 |
| | Increased aspartate aminotransferase | 0 | 0.0 | 0 | 6 | 100.0 | 6 | 0 | 0.0 | 0 |
| | Decreased blood albumin | 0 | 0.0 | 0 | 6 | 100.0 | 6 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 6 | 100.0 | 6 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 4 | 66.7 | 4 | 0 | 0.0 | 0 |
| | Increased blood lactate dehydrogenase | 0 | 0.0 | 0 | 6 | 100.0 | 6 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 1 | 16.7 | 1 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |

MedDRA/J Version 18.1

| | | Whole period | | | | | | | | |
|---|---|---|---|---|---|---|---|---|---|---|
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 2 | 33.3 | 4 | 0 | 0.0 | 0 |
| | Increased alanine aminotransferase | 0 | 0.0 | 0 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 2 | 33.3 | 2 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 1 | 16.7 | 1 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Clinical test | | 3 | 50.0 | 5 | 1 | 16.7 | 85 | 0 | 0.0 | 0 |
| | Increased alanine aminotransferase | 0 | 0.0 | 0 | 1 | 16.7 | 2 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 2 | 33.3 | 2 | 0 | 0.0 | 0 |
| | Decreased blood albumin | 0 | 0.0 | 0 | 4 | 66.7 | 5 | 0 | 0.0 | 0 |
| | Decreased blood chloride | 0 | 0.0 | 0 | 1 | 16.7 | 1 | 0 | 0.0 | 0 |
| | Decreased blood cholesterol | 0 | 0.0 | 0 | 3 | 50.0 | 3 | 0 | 0.0 | 0 |
| | Increased blood creatine phosphokinase | 0 | 0.0 | 0 | 4 | 66.7 | 4 | 0 | 0.0 | 0 |
| | Increased blood creatinine | 0 | 0.0 | 0 | 2 | 33.3 | 2 | 0 | 0.0 | 0 |
| | Increased blood glucose | 0 | 0.0 | 0 | 2 | 33.3 | 2 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 1 | 16.7 | 1 | 3 | 50.0 | 3 | 0 | 0.0 | 0 |
| | Increased blood potassium | 0 | 0.0 | 0 | 1 | 16.7 | 1 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 1 | 16.7 | 1 | 0 | 0.0 | 0 |
| | Decreased blood sodium | 0 | 0.0 | 0 | 1 | 16.7 | 1 | 0 | 0.0 | 0 |
| | Increased blood triglyceride | 1 | 16.7 | 1 | 0 | 0.0 | 0 | 0 | 0.0 | 0 |
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 1 | 16.7 | 1 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 0 | 0.0 | 0 | 2 | 33.3 | 11 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Whole period | | | | | | | | |
|---|---|---|---|---|---|---|---|---|---|---|
| | | 100 mg (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 2 | 33.3 | 2 | 2 | 33.3 | 64 | 0 | 0.0 | 0 |
| | Increased alanine aminotransferase | 0 | 0.0 | 0 | 1 | 16.7 | 2 | 0 | 0.0 | 0 |

(continued)

| Outcome | | Whole period | | | | | | | | |
|---|---|---|---|---|---|---|---|---|---|---|
| | | 100 mg (N=6) | | | | | | | | |
| | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 0 | 0.0 | 0 | 1 | 16.7 | 1 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 4 | 66.7 | 4 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 2 | 33.3 | 2 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |

(continued)

| | | Whole period | | | | | | | | |
|---|---|---|---|---|---|---|---|---|---|---|
| | | 100 mg (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood triglyceride | 0 | 0.0 | 0 | 0 | 0.0 | 0 | 0 | 0.0 | 0 |
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 1 | 16.7 | 1 | 0 | 0.0 | 0 |
| | Acute cholangitis | 0 | 0.0 | 0 | 1 | 16.7 | 1 | 0 | 0.0 | 0 |
| Infectious and parasitic diseases | | 0 | 0.0 | 0 | 1 | 16.7 | 2 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 1 | 16.7 | 1 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomysite infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Clinical test | | 1 | 16.7 | 1 | 5 | 83.3 | 85 | 0 | 0.0 | 0 |
| | Increased alanine aminotransferase | 0 | 0.0 | 0 | 2 | 33.3 | 2 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 3 | 50.0 | 3 | 0 | 0.0 | 0 |
| | Decreased blood albumin | 0 | 0.0 | 0 | 2 | 33.3 | 2 | 0 | 0.0 | 0 |
| | Decreased blood chloride | 0 | 0.0 | 0 | 1 | 16.7 | 1 | 0 | 0.0 | 0 |
| | Decreased blood cholesterol | 0 | 0.0 | 0 | 1 | 16.7 | 1 | 0 | 0.0 | 0 |
| | Increased blood creatine phosphokinase | 0 | 0.0 | 0 | 5 | 83.3 | 5 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 1 | 16.7 | 1 | 0 | 0.0 | 0 |
| | Increased blood glucose | 0 | 0.0 | 0 | 3 | 50.0 | 3 | 0 | 0.0 | 0 |
| | Increased blood lactate dehydrogenase | 0 | 0.0 | 0 | 6 | 100.0 | 6 | 0 | 0.0 | 0 |
| | Increased blood potassium Decreased blood pressure | 0 | 0.0 | 0 | 2 0 | 33.3 0.0 | 2 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 1 | 16.7 | 1 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Safety evaluation period (within 6 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 1 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 2 | 33.3 | 2 | 0 | 0.0 | 0 |
| | Bronchitis | 0 | 0.0 | 0 | 1 | 16.7 | 1 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 1 | 16.7 | 1 | 4 | 66.7 | 95 | 0 | 0.0 | 0 |
| | Increased alanine aminotransferase | 0 | 0.0 | 0 | 5 | 83.3 | 5 | 0 | 0.0 | 0 |
| | Increased amylase | 0 | 0.0 | 0 | 1 | 16.7 | 1 | 0 | 0.0 | 0 |
| | Increased aspartate aminotransferase | 0 | 0.0 | 0 | 6 | 100.0 | 6 | 0 | 0.0 | 0 |
| | Decreased blood albumin | 0 | 0.0 | 0 | 6 | 100.0 | 6 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 6 | 100.0 | 6 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 4 | 66.7 | 4 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 0 | 0.0 | 0 | 6 | 100.0 | 6 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 1 | 16.7 | 1 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 1 | 16.7 | 1 | 0 | 0.0 | 0 |

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 0 | 0.0 | 0 | 2 | 33.3 | 2 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Safety evaluation period (within 6 weeks after administration) | | | | | | | | |
|---|---|---|---|---|---|---|---|---|---|---|
| | | 30·mg (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

| | | 30 mg (N=6) | | | | | | | | |
|---|---|---|---|---|---|---|---|---|---|---|
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 1 | 16.7 | 1 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |

(continued)

| | | 30 mg (N=6) | | | | | | | | |
|---|---|---|---|---|---|---|---|---|---|---|
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 3 | 50.0 | 4 | 1 | 16.7 | 82 | 0 | 0.0 | 0 |
| | Increased alanine aminotransferase | 0 | 0.0 | 0 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 2 | 33.3 | 2 | 0 | 0.0 | 0 |
| | Decreased blood albumin | 0 | 0.0 | 0 | 4 | 66.7 | 5 | 0 | 0.0 | 0 |
| | Decreased blood chloride | 0 | 0.0 | 0 | 1 | 16.7 | 1 | 0 | 0.0 | 0 |
| | Decreased blood cholesterol | 0 | 0.0 | 0 | 3 | 50.0 | 3 | 0 | 0.0 | 0 |
| | Increased blood creatine phosphokinase | 0 | 0.0 | 0 | 4 | 66.7 | 4 | 0 | 0.0 | 0 |
| | Increased blood creatinine | 0 | 0.0 | 0 | 2 | 33.3 | 2 | 0 | 0.0 | 0 |
| | Increased blood glucose | 0 | 0.0 | 0 | 2 | 33.3 | 2 | 0 | 0.0 | 0 |
| | Increased blood lactate dehydrogenase | 1 | 16.7 | 1 | 3 | 50.0 | 3 | 0 | 0.0 | 0 |
| | Increased blood potassium | 0 | 0.0 | 0 | 1 | 16.7 | 1 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 1 | 16.7 | 1 | 0 | 0.0 | 0 |
| | Increased blood triglyceride | 0 | 0.0 | 0 | 0 | 0.0 | 0 | 0 | 0.0 | 0 |

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 1 | 16.7 | 1 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 10 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |

(continued)

| | | 100 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 1 | 16.7 | 1 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 2 | 33.3 | 2 | 3 | 50.0 | 63 | 0 | 0.0 | 0 |
| | Increased alanine aminotransferase | 0 | 0.0 | 0 | 1 | 16.7 | 2 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 0 | 0.0 | 0 | 1 | 16.7 | 1 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 4 | 66.7 | 4 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 2 | 33.3 | 2 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Safety evaluation period (within 6 weeks after administration) | | | | | | | | |
|---|---|---|---|---|---|---|---|---|---|---|
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |

(continued)

| | | Safety evaluation period (within 6 weeks after administration) | | | | | | | | |
|---|---|---|---|---|---|---|---|---|---|---|
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 1 | 16.7 | 1 | 0 | 0.0 | 0 |
| | Acute cholangitis | 0 | 0.0 | 0 | 1 | 16.7 | 1 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 1 | 16.7 | 2 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 1 | 16.7 | 1 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 1 | 16.7 | 1 | 0 | 0.0 | 0 |
| | Wound dehiscence | 0 | 0.0 | 0 | 1 | 16.7 | 1 | 0 | 0.0 | 0 |
| Clinical test | | 1 | 16.7 | 1 | 5 | 83.3 | 85 | 0 | 0.0 | 0 |
| | Increased alanine aminotransferase | 0 | 0.0 | 0 | 2 | 33.3 | 2 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 3 | 50.0 | 3 | 0 | 0.0 | 0 |
| | Decreased blood albumin | 0 | 0.0 | 0 | 2 | 33.3 | 2 | 0 | 0.0 | 0 |
| | Decreased blood chloride | 0 | 0.0 | 0 | 1 | 16.7 | 1 | 0 | 0.0 | 0 |
| | Decreased blood cholesterol | 0 | 0.0 | 0 | 1 | 16.7 | 1 | 0 | 0.0 | 0 |
| | Increased blood creatine phosphokinase | 0 | 0.0 | 0 | 5 | 83.3 | 5 | 0 | 0.0 | 0 |
| | Increased blood creatinine | 0 | 0.0 | 0 | 1 | 16.7 | 1 | 0 | 0.0 | 0 |
| | Increased blood glucose | 0 | 0.0 | 0 | 3 | 50.0 | 3 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 0 | 0.0 | 0 | 6 | 100.0 | 6 | 0 | 0.0 | 0 |
| | Increased blood potassium | 0 | 0.0 | 0 | 2 | 33.3 | 2 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 1 | 16.7 | 1 | 0 | 0.0 | 0 |
| | Increased blood triglyceride | 0 | 0.0 | 0 | 0 | 0.0 | 0 | 0 | 0.0 | 0 |

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |

(continued)

| Outcome | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | Preferred term | Mild | | | Recovered | | | Not clear | | |
| System organ classification | | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |

(continued)

| Outcome | Preferred term | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| | | Mild | | | Recovered | | | Not clear | | |
| System organ classification | | Number of examples | Incidence [%] | Number of occurrences | Number of examples | Incidence [%] | Number of occurrences | Number of examples | Incidence [%] | Number of occurrences |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 10 mg (N=6) | | | | | | | | |
| Outcome | Preferred term | Mild | | | Recovered | | | Not clear | | |
| System organ classification | | Number of examples | Incidence [%] | Number of occurrences | Number of examples | Incidence [%] | Number of occurrences | Number of examples | Incidence [%] | Number of occurrences |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | | 30 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 2 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased alanine aminotransferase | 0 | 0.0 | 0 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 1 | 16.7 | 1 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |

(continued)

| | | 30 mg (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood triglyceride | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 1 | 16.7 | 1 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 1 | 16.7 | 1 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 1 | 16.7 | 1 | 2 | 33.3 | 3 | 0 | 0.0 | 0 |
| | Increased alanine aminotransferase | 0 | 0.0 | 0 | 1 | 16.7 | 1 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 1 | 16.7 | 1 | 0 | 0.0 | 0 |
| | Decreased blood sodium | 0 | 0.0 | 0 | 0 | 0.0 | 0 | 0 | 0.0 | 0 |
| | Increased blood triglyceride | 1 | 16.7 | 1 | 0 | 0.0 | 0 | 0 | 0.0 | 0 |

MedDRA/J Version 18.1

(continued)

| | | Safety and efficacy evaluation period (more than 6 weeks after administration to 26 weeks after administration) | | | | | | | | |
|---|---|---|---|---|---|---|---|---|---|---|
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 1 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | 100 mg (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 1 | 16.7 | 1 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |

(continued)

| Outcome | | Mild | | | Recovered | | | Not clear | | |
|---|---|---|---|---|---|---|---|---|---|---|
| System organ classification | Preferred term | Number of examples | Incidence [%] | Number of occurrences | Number of examples | Incidence [%] | Number of occurrences | Number of examples | Incidence [%] | Number of occurrences |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |
| | | Placebo (N=6) | | | | | | | | |
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |

(continued)

| | | Placebo (N=6) | | | | | | | | |
|---|---|---|---|---|---|---|---|---|---|---|
| Outcome | | Death | | | Unrecovered | | | With sequelae | | |
| System organ classification | Preferred term | Number of examples | Incidence [%] | Number of occurrences | Number of examples | Incidence [%] | Number of occurrences | Number of examples | Incidence [%] | Number of occurrences |
| | Decreased blood sodium | 0 | 0.0 | 0 | 0 | 0.0 | 0 | 0 | 0.0 | 0 |
| | Increased blood triglyceride | 0 | 0.0 | 0 | 0 | 0.0 | 0 | 0 | 0.0 | 0 |

MedDRA/J Version 18.1

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| Hepatobiliary disorders | | 0 | 0.0 | 0 | 0 | 0.0 | 0 | 0 | 0.0 | 0 |
| | Acute cholangitis | 0 | 0.0 | 0 | 0 | 0.0 | 0 | 0 | 0.0 | 0 |
| Infectious and parasitic diseases | | 0 | 0.0 | 0 | 0 | 0.0 | 0 | 0 | 0.0 | 0 |
| | Bronchitis | 0 | 0.0 | 0 | 0 | 0.0 | 0 | 0 | 0.0 | 0 |
| | Lung abscess | 0 | 0.0 | 0 | 0 | 0.0 | 0 | 0 | 0.0 | 0 |
| | Mediastinitis | 0 | 0.0 | 0 | 0 | 0.0 | 0 | 0 | 0.0 | 0 |
| | Pneumonia | 0 | 0.0 | 0 | 0 | 0.0 | 0 | 0 | 0.0 | 0 |
| | Tracheostomy site infection | 0 | 0.0 | 0 | 0 | 0.0 | 0 | 0 | 0.0 | 0 |
| Injury, poisoning and procedural complications | | 0 | 0.0 | 0 | 0 | 0.0 | 0 | 0 | 0.0 | 0 |
| | Wound dehiscence | 0 | 0.0 | 0 | 0 | 0.0 | 0 | 0 | 0.0 | 0 |
| Clinical test | | 0 | 0.0 | 0 | 0 | 0.0 | 0 | 0 | 0.0 | 0 |
| | Increased alanine aminotransferase | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Increased amylase | 0 | 0.0 | 0 | 0 | 0.0 | 0 | 0 | 0.0 | 0 |
| | Increased aspartate aminotransferase | 0 | 0.0 | 0 | 0 | 0.0 | 0 | 0 | 0.0 | 0 |
| | Decreased blood albumin | 0 | 0.0 | 0 | 0 | 0.0 | 0 | 0 | 0.0 | 0 |
| | Decreased blood chloride | 0 | 0.0 | 0 | 0 | 0.0 | 0 | 0 | 0.0 | 0 |
| | Decreased blood cholesterol | 0 | 0.0 | 0 | 0 | 0.0 | 0 | 0 | 0.0 | 0 |
| | Increased blood creatine phosphokinase | 0 | 0.0 | 0 | 0 | 0.0 | 0 | 0 | 0.0 | 0 |
| | Increased blood creatinine | 0 | 0.0 | 0 | 0 | 0.0 | 0 | 0 | 0.0 | 0 |
| | Increased blood glucose | 0 | 0.0 | 0 | 0 | 0.0 | 0 | 0 | 0.0 | 0 |
| | Increased blood lactate dehydrogenase | 0 | 0.0 | 0 | 0 | 0.0 | 0 | 0 | 0.0 | 0 |
| | Increased blood potassium | 0 | 0.0 | 0 | 0 | 0.0 | 0 | 0 | 0.0 | 0 |
| | Decreased blood pressure | 0 | 0.0 | 0 | 0 | 0.0 | 0 | 0 | 0.0 | 0 |
| | Increased blood pressure | 0 | 0.0 | 0 | 0 | 0.0 | 0 | 0 | 0.0 | 0 |
| | Decreased blood sodium | 0 | 0.0 | 0 | 0 | 0.0 | 0 | 0 | 0.0 | 0 |

(continued)

| | | Safety and efficacy evaluation period (more than 6 weeks after administration to 26 weeks after administration) | | | | | | | | |
|---|---|---|---|---|---|---|---|---|---|---|
| | | Placebo (N=6) | | | | | | | | |
| Outcome | | Mild | | | Recovered | | | Not clear | | |
| System organ classification | Preferred term | Number of examples | Incidence [%] | Number of occurrences | Number of examples | Incidence [%] | Number of occurrences | Number of examples | Incidence [%] | Number of occurrences |
| | Increased blood triglyceride | 0 | 0.0 | 0 | 0 | 0.0 | 0 | 0 | 0.0 | 0 |
| MedDRA/J Version 18.1 | | | | | | | | | | |

## Claims

1. A pharmaceutical composition for improving cardiac function, comprising:

   (A) a release formulation comprising at least poly(lactic-co-glycolic acid) (PLGA) and a prostaglandin I2 receptor agonist, the PLGA having an average molecular weight of 10000 to 30000; and
   (B) a release formulation comprising at least poly(lactic-co-glycolic acid) (PLGA) and a prostaglandin I2 receptor agonist, the PLGA having an average molecular weight of 40000 to 60000.

2. The pharmaceutical composition for improving cardiac function according to claim 1, wherein the ratio of the release formulation (B) to the release formulation (A) (A:B) is 1:1 to 100:1 or 1:1 to 1:100.

3. The pharmaceutical composition for improving cardiac function according to claim 1 or 2, wherein the release formulation (A) comprises 0.5 to 50 mg of PGI2 receptor agonist in one vial, and/or the release formulation (B) comprises 0.5 to 50 mg of PGI2 receptor agonist in one vial.

4. The pharmaceutical composition according to any one of claims 1 to 3, comprising a patch liquid.

5. The pharmaceutical composition according to claim 4, wherein the patch liquid is a 5 w/v% mannitol aqueous solution comprising 0.2 w/v% of polysorbate.

6. The pharmaceutical composition according to any one of claims 1 to 5, comprising a gelatin patch.

7. The pharmaceutical composition according to claim 6, wherein the gelatin patch is a porous sterile formulation comprising 10 g of gelatin per 1000 $cm^3$.

8. The pharmaceutical composition according to any one of claims 1 to 7, comprising a plasma fraction formulation.

9. The pharmaceutical composition according to claim 8, wherein the plasma fraction formulation comprises a fibrinogen powder, an aprotinin solution, a thrombin powder, and a calcium chloride solution.

10. The pharmaceutical composition according to any one of claims 1 to 9, comprising, as the prostaglandin I2 receptor agonist, at least a compound of the following formula (I) or a salt thereof:

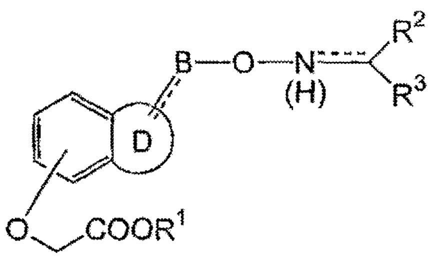

(I)

wherein

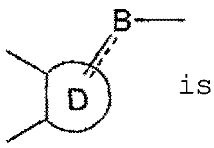

is

(i) 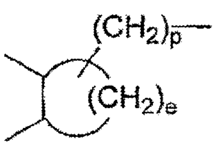 , (ii) 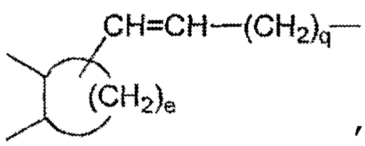 ,

(iii) 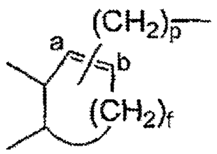 , or (iv) 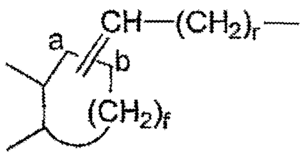 ,

wherein

$R^1$ represents a hydrogen atom or a C1-4 alkyl group,
$R^2$ represents (i) a hydrogen atom, (ii) a C1-8 alkyl group, (iii) a phenyl group or a C4-7 cycloalkyl group, (iv) a 4-to 7-membered monocyclic ring containing one nitrogen atom, (v) a C1-4 alkyl group substituted with a benzene ring or a C4-7 cycloalkyl group, or (vi) a C1-4 alkyl group substituted with a 4- to 7-membered monocyclic ring containing one nitrogen atom,
$R^3$ represents (i) a C1-8 alkyl group, (ii) a phenyl group or a C4-7 cycloalkyl group, (iii) a 4- to 7-membered monocyclic ring containing one nitrogen atom, (iv) a C1-4 alkyl group substituted with a benzene ring or a C4-7 cycloalkyl group, or (v) a C1-4 alkyl group substituted with a 4- to 7-membered monocyclic ring containing one nitrogen atom,
e represents an integer of 3 to 5,
f represents an integer of 1 to 3,
p represents an integer of 1 to 4,
q represents 1 or 2, and
r represents an integer of 1 to 3;

provided that when

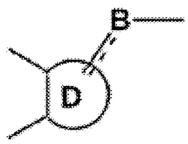

is a group represented by (iii) or (iv) above,

$-(CH_2)p-$ and $=CH-(CH_2)s-$ bind to the position of a or b on the ring, and
ring structures in $R^2$ and $R^3$ are optionally substituted with 1 to 3 C1-4 alkyl groups, C1-4 alkoxy groups, halogen atoms, nitro groups, or trihalomethyl groups.

**11.** The pharmaceutical composition according to any one of claims 1 to 10, comprising, as the prostaglandin I2 receptor agonist, at least the following compound (A) or a salt thereof:

(A) ({5-[2-({[(1E)-phenyl(pyridin-3-yl)methylene]amino}oxy)ethyl]-7,8-dihydronaphthalen-1-yl}oxy)acetic acid (ONO-1301) represented by the following formula (II):

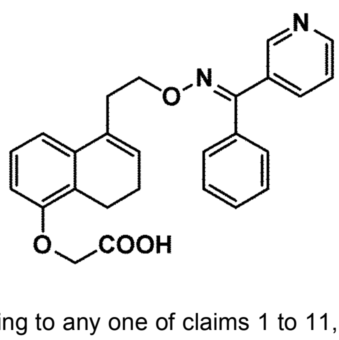 (II)

**12.** The pharmaceutical composition according to any one of claims 1 to 11, wherein the pharmaceutical composition is a sheet patch.

**13.** The pharmaceutical composition according to any one of claims 1 to 12, wherein the pharmaceutical composition is administered to a patient with ischemic cardiomyopathy who undergoes coronary artery bypass surgery.

**14.** The pharmaceutical composition according to any one of claims 1 to 13, wherein the prostaglandin I2 receptor agonist is released over 4 weeks after administration.

**15.** The pharmaceutical composition according to any one of claims 1 to 14, which is a sustained-release formulation of microspheres (MS).

**16.** The pharmaceutical composition according to claim 15, wherein the sustained-release formulation has an average particle size of 3 to 300 um.

Fig. 1

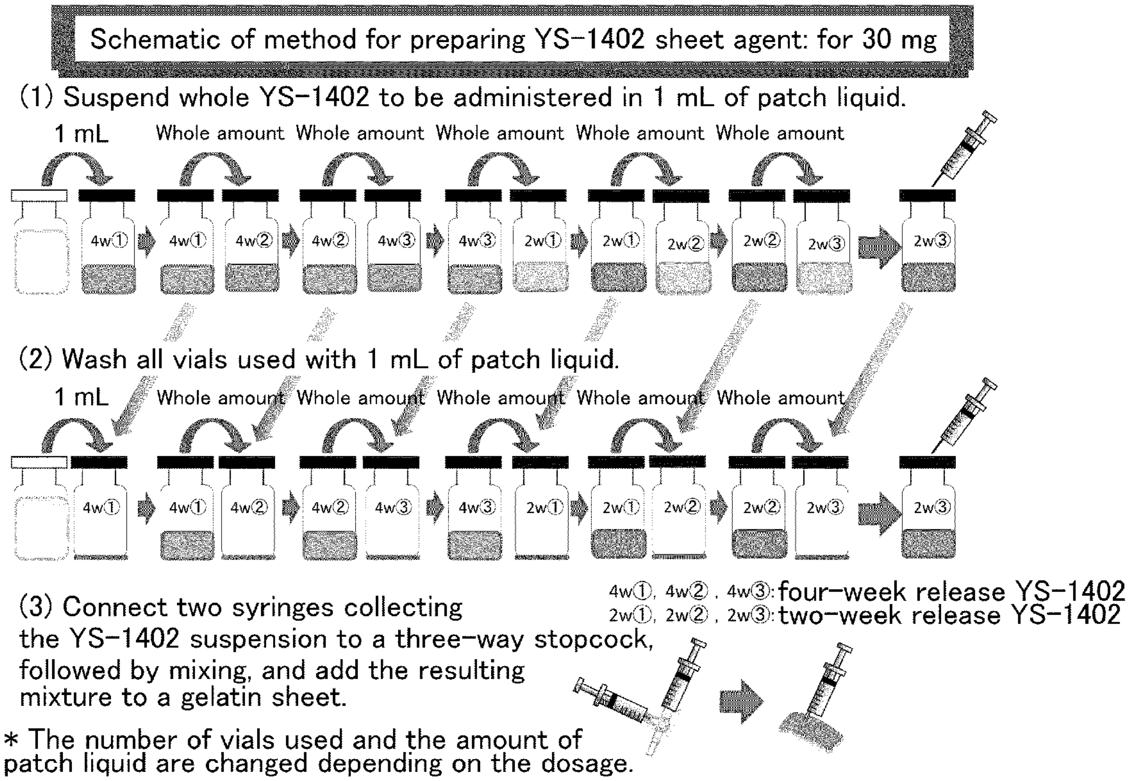
Schematic of method for preparing YS-1402 sheet agent: for 30 mg
(1) Suspend whole YS-1402 to be administered in 1 mL of patch liquid.
1 mL
Whole amount Whole amount Whole amount Whole amount Whole amount
4w① 4w① 4w② 4w② 4w③ 4w③ 2w① 2w① 2w② 2w② 2w③ 2w③
(2) Wash all vials used with 1 mL of patch liquid.
1 mL
Whole amount Whole amount Whole amount Whole amount Whole amount
4w① 4w① 4w② 4w② 4w③ 4w③ 2w① 2w① 2w② 2w② 2w③ 2w③
(3) Connect two syringes collecting the YS-1402 suspension to a three-way stopcock, followed by mixing, and add the resulting mixture to a gelatin sheet.
4w①, 4w②, 4w③: four-week release YS-1402
2w①, 2w②, 2w③: two-week release YS-1402
* The number of vials used and the amount of patch liquid are changed depending on the dosage.

Fig. 2

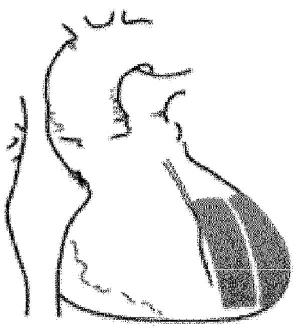

Clinical administration method

1) Two YS-1402 dosing sheets (Gelfoam®) are impregnated with the therapeutic agent.

2) The two YS-1402 dosing sheets are attached to a site of the cardiac ischemic area where graft running is not affected.

3) A physiological tissue adhesive (Beriplast® P Combi-Set Tissue adhesion: a plasma fraction formulation) is dropped around the attachment area to enclose the YS-1402 dosing sheets.

YS-1402 dosing sheets impregnated with YS-1402 therapeutic agent

Fig. 3

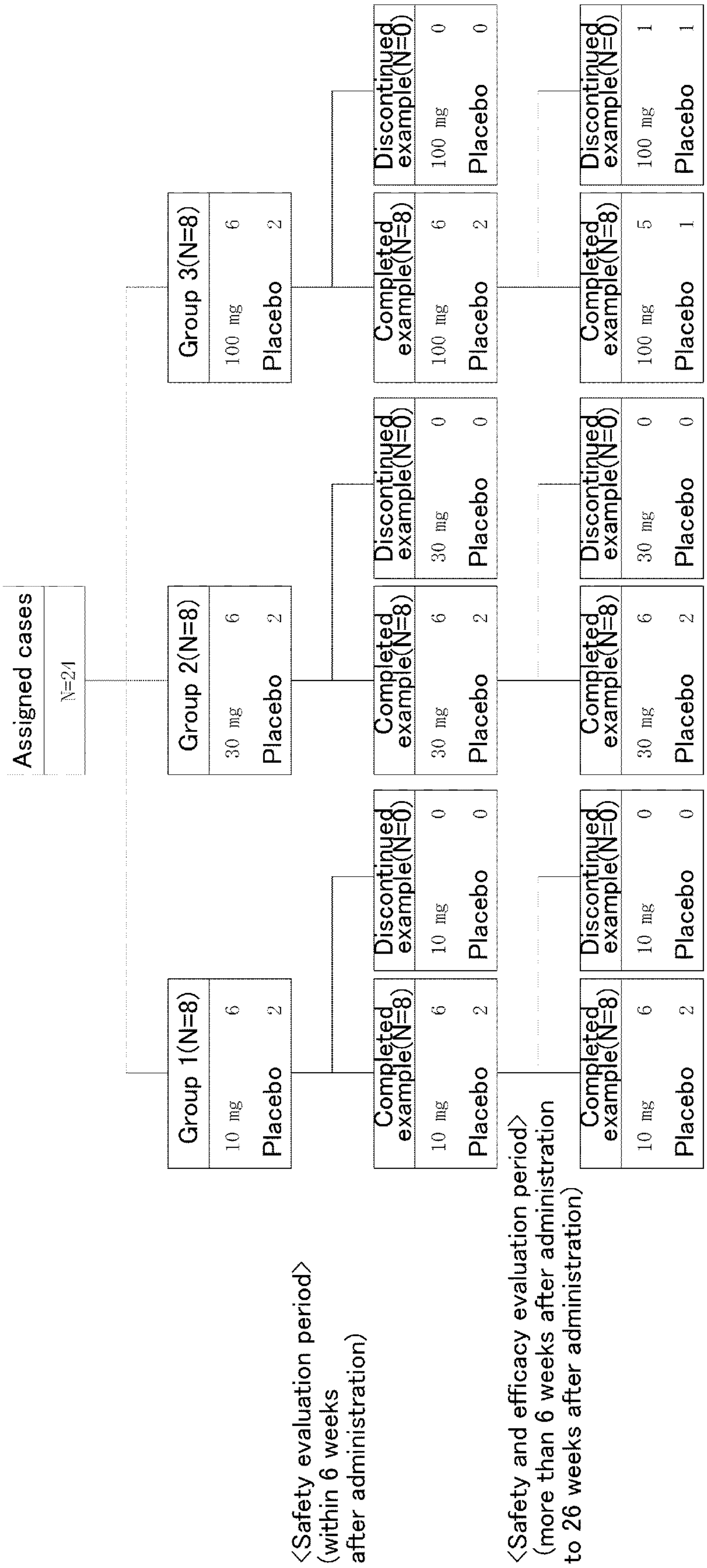
Assigned cases
N=24
Group 1(N=8)
10 mg 6
Placebo 2
Group 2(N=8)
30 mg 6
Placebo 2
Group 3(N=8)
100 mg 6
Placebo 2
<Safety evaluation period>
(within 6 weeks
after administration)
Completed example(N=8)
10 mg 6
Placebo 2
Discontinued example(N=0)
10 mg 0
Placebo 0
Completed example(N=8)
30 mg 6
Placebo 2
Discontinued example(N=0)
30 mg 0
Placebo 0
Completed example(N=8)
100 mg 6
Placebo 2
Discontinued example(N=0)
100 mg 0
Placebo 0
<Safety and efficacy evaluation period>
(more than 6 weeks after administration
to 26 weeks after administration)
Completed example(N=8)
10 mg 6
Placebo 2
Discontinued example(N=0)
10 mg 0
Placebo 0
Completed example(N=8)
30 mg 6
Placebo 2
Discontinued example(N=0)
30 mg 0
Placebo 0
Completed example(N=8)
100 mg 5
Placebo 1
Discontinued example(N=0)
100 mg 1
Placebo 1

Fig. 4

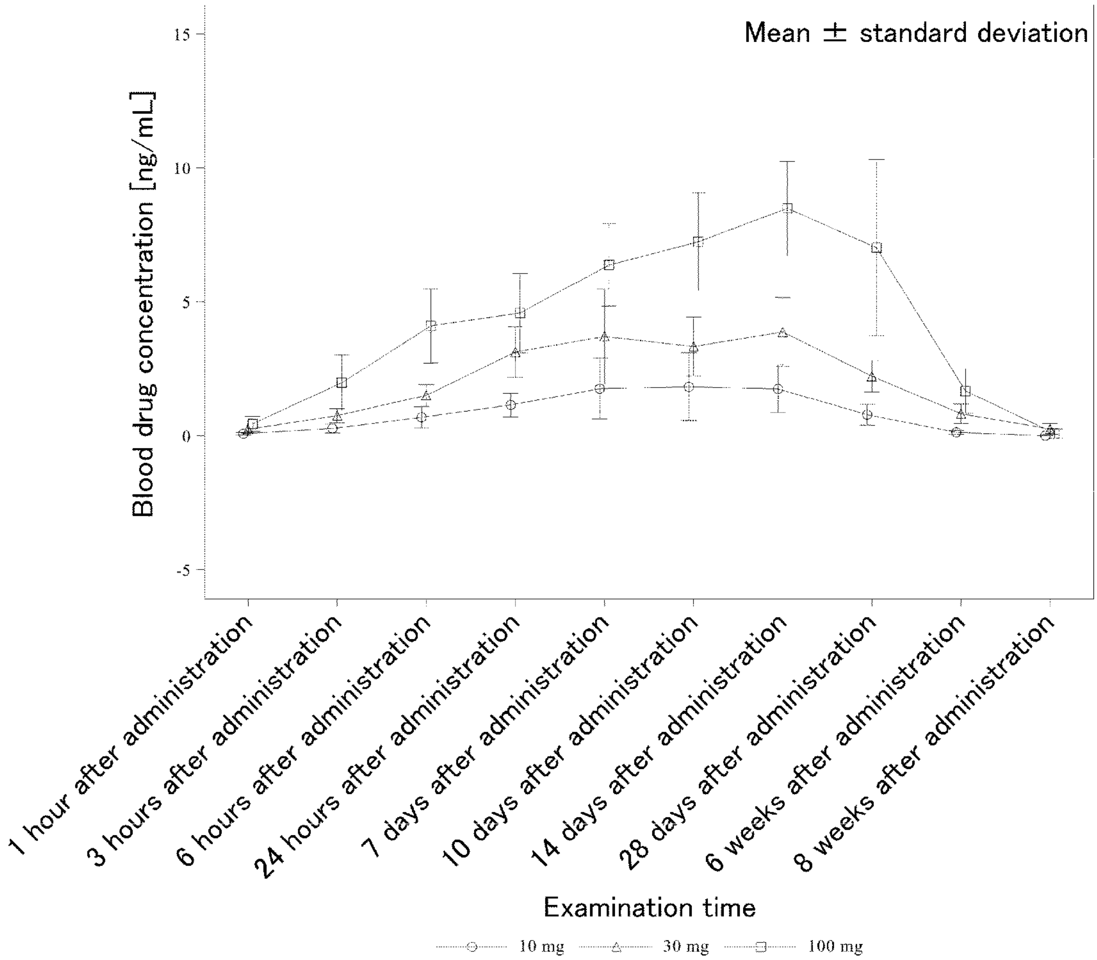
Mean ± standard deviation
15
10
5
0
-5
Blood drug concentration [ng/mL]
1 hour after administration
3 hours after administration
6 hours after administration
24 hours after administration
7 days after administration
10 days after administration
14 days after administration
28 days after administration
6 weeks after administration
8 weeks after administration
Examination time
10 mg
30 mg
100 mg

Fig. 5

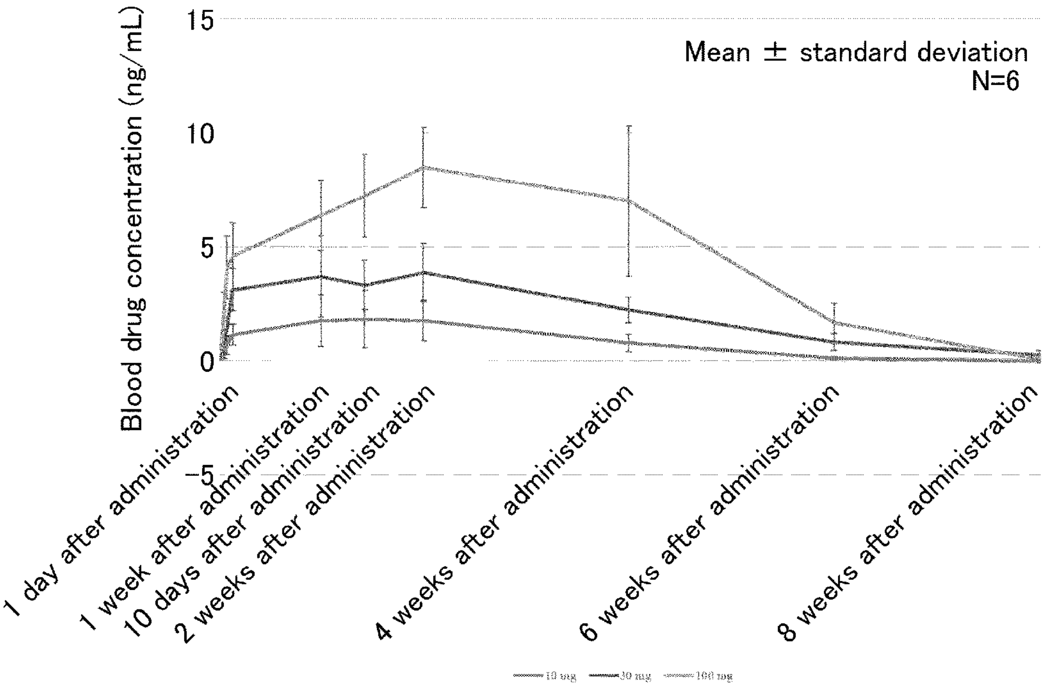
Blood drug concentration (ng/mL)
15
10
5
0
-5
Mean ± standard deviation
N=6
1 day after administration
1 week after administration
10 days after administration
2 weeks after administration
4 weeks after administration
6 weeks after administration
8 weeks after administration
10 mg
30 mg
100 mg

Fig. 6

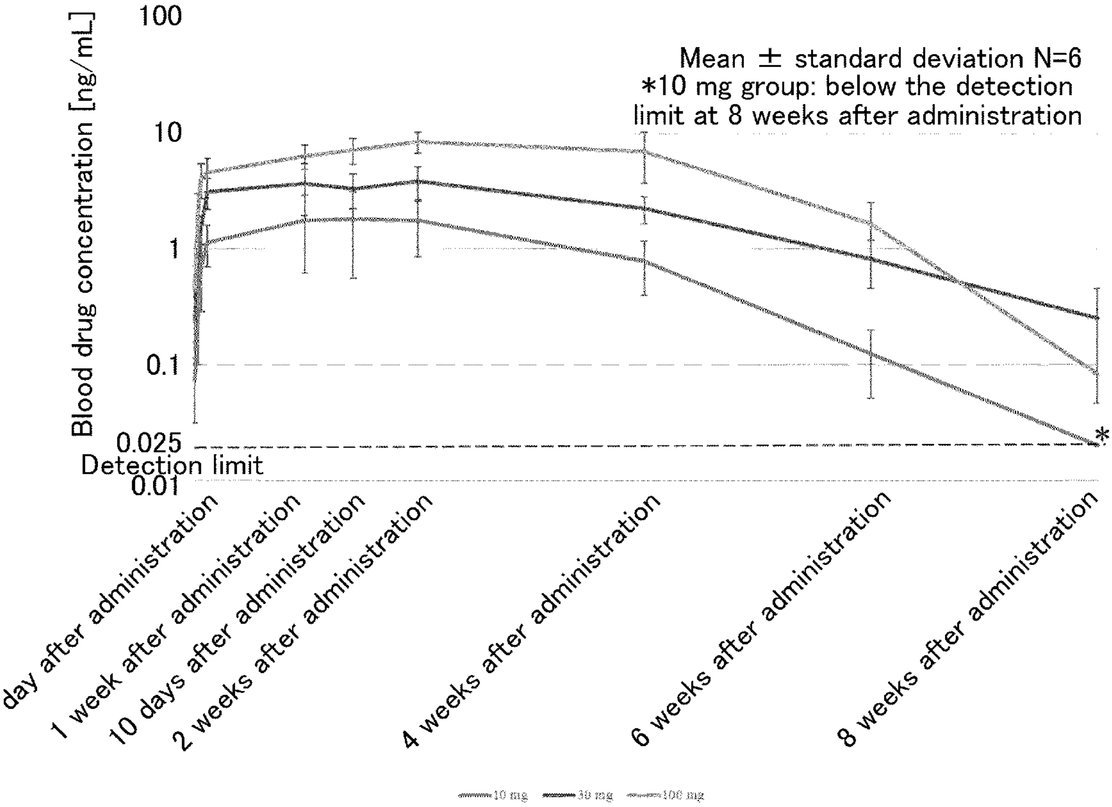
Blood drug concentration [ng/mL]
100
10
1
0.1
0.025
Detection limit
0.01
Mean ± standard deviation N=6
*10 mg group: below the detection limit at 8 weeks after administration
*
1 day after administration
1 week after administration
10 days after administration
2 weeks after administration
4 weeks after administration
6 weeks after administration
8 weeks after administration
10 mg
30 mg
100 mg

Fig. 7

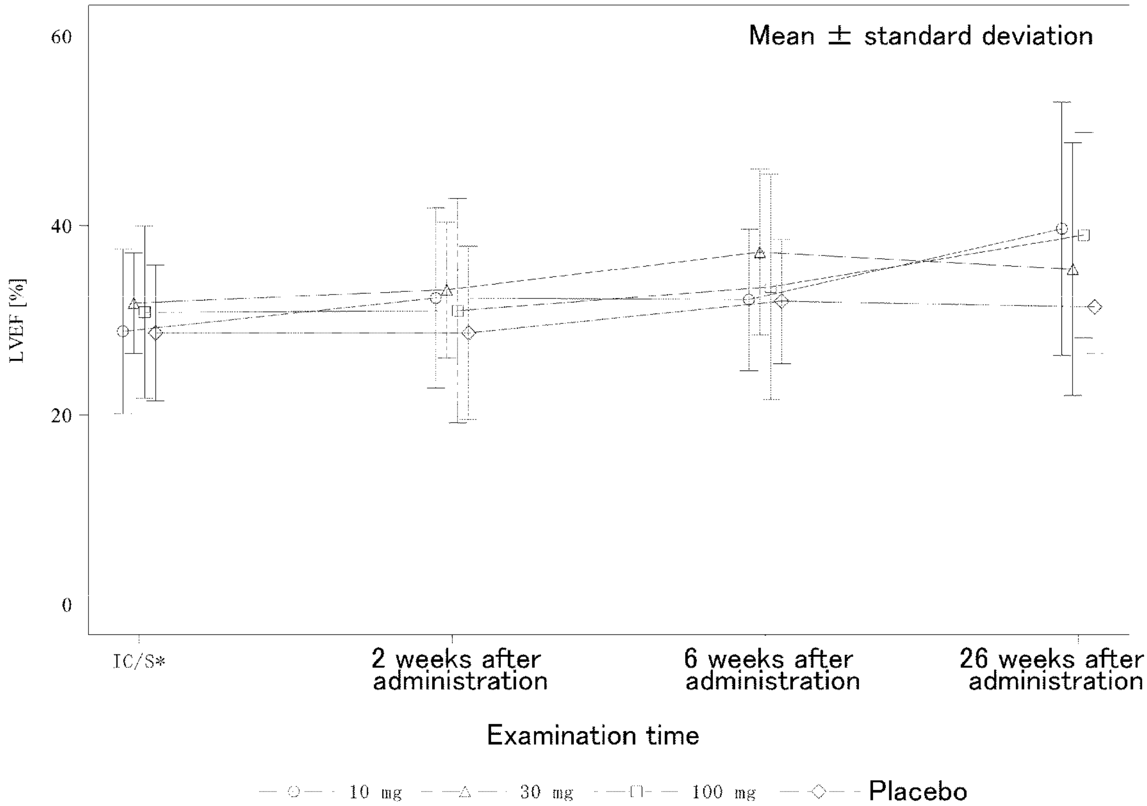


LVEF: left ventricular ejection fraction
*: Consent acquisition/screening

Fig. 8

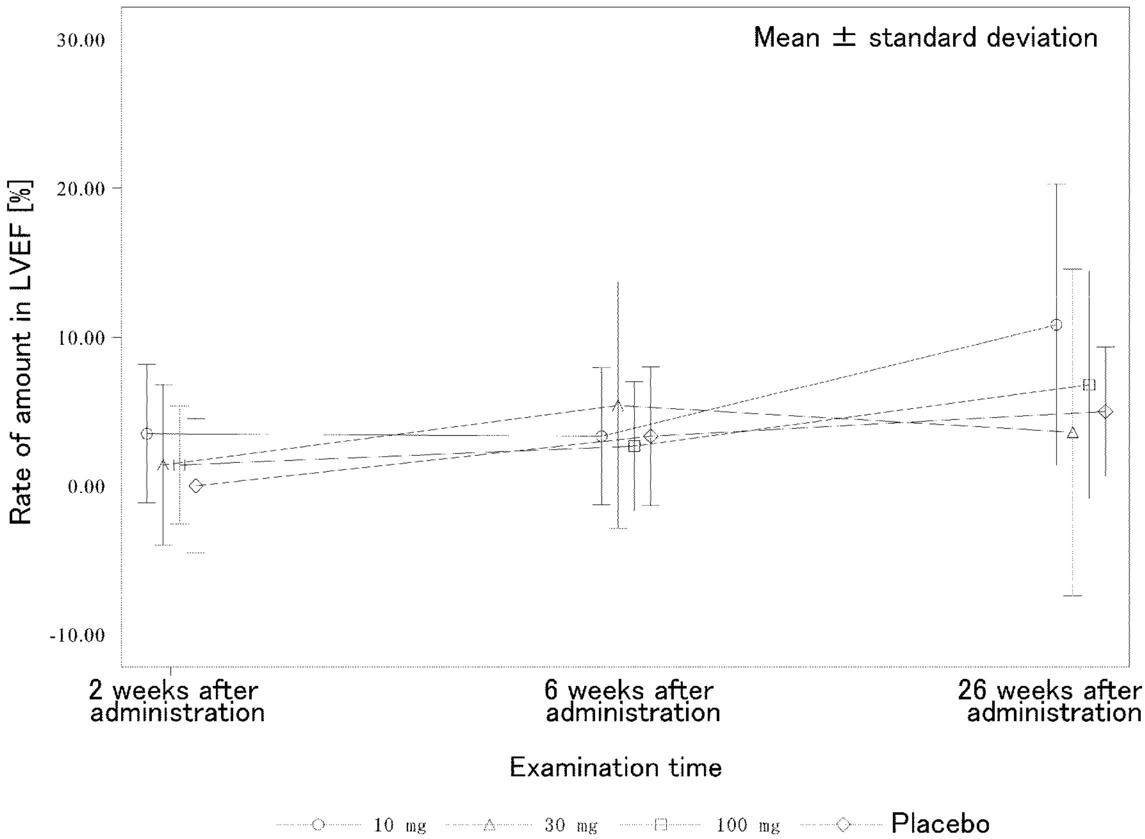


LVEF: left ventricular ejection fraction

Fig. 9

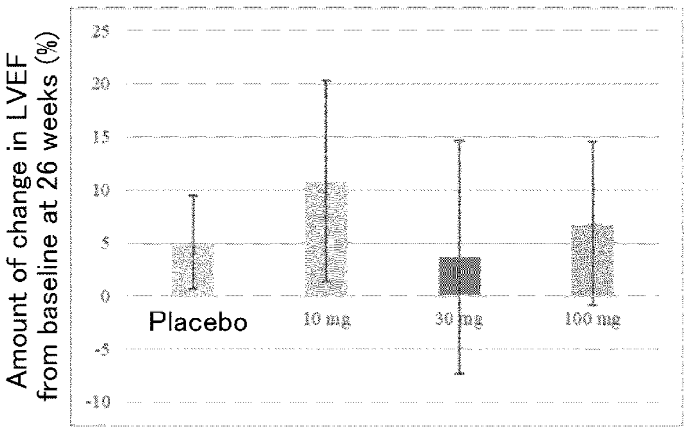


| Dose | 26 weeks after administration |
|---|---|
| Placebo (N=5) | 5.0±4.4% |
| 10mg (N=6) | 10.8±9.5% |
| 30mg (N=5) | 3.6±11.0% |
| 100mg (N=5) | 6.8±7.7% |

One case in each of the placebo and 100 mg groups was excluded from analysis at 26 weeks after administration due to discontinuation. One case in the 30 mg group was excluded from analysis because LVEF was calculated by the Teichholz formula.

Fig. 10

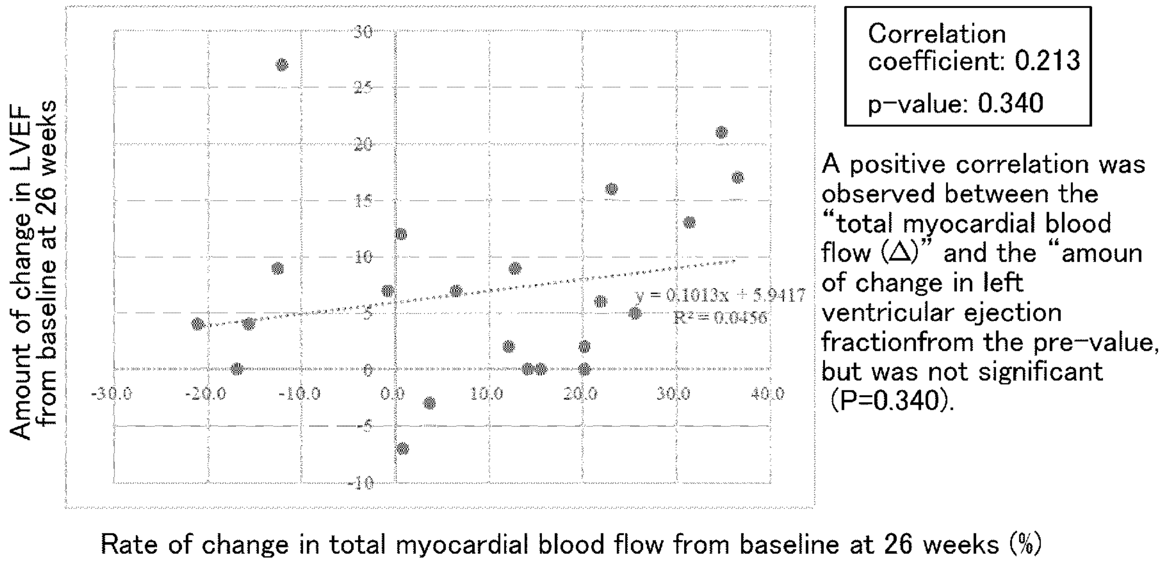
Amount of change in LVEF from baseline at 26 weeks
y = 0.1013x + 5.9417
R² = 0.0456
Correlation coefficient: 0.213
p-value: 0.340
A positive correlation was observed between the "total myocardial blood flow (Δ)" and the "amoun of change in left ventricular ejection fractionfrom the pre-value, but was not significant (P=0.340).
Rate of change in total myocardial blood flow from baseline at 26 weeks (%)

Fig. 11

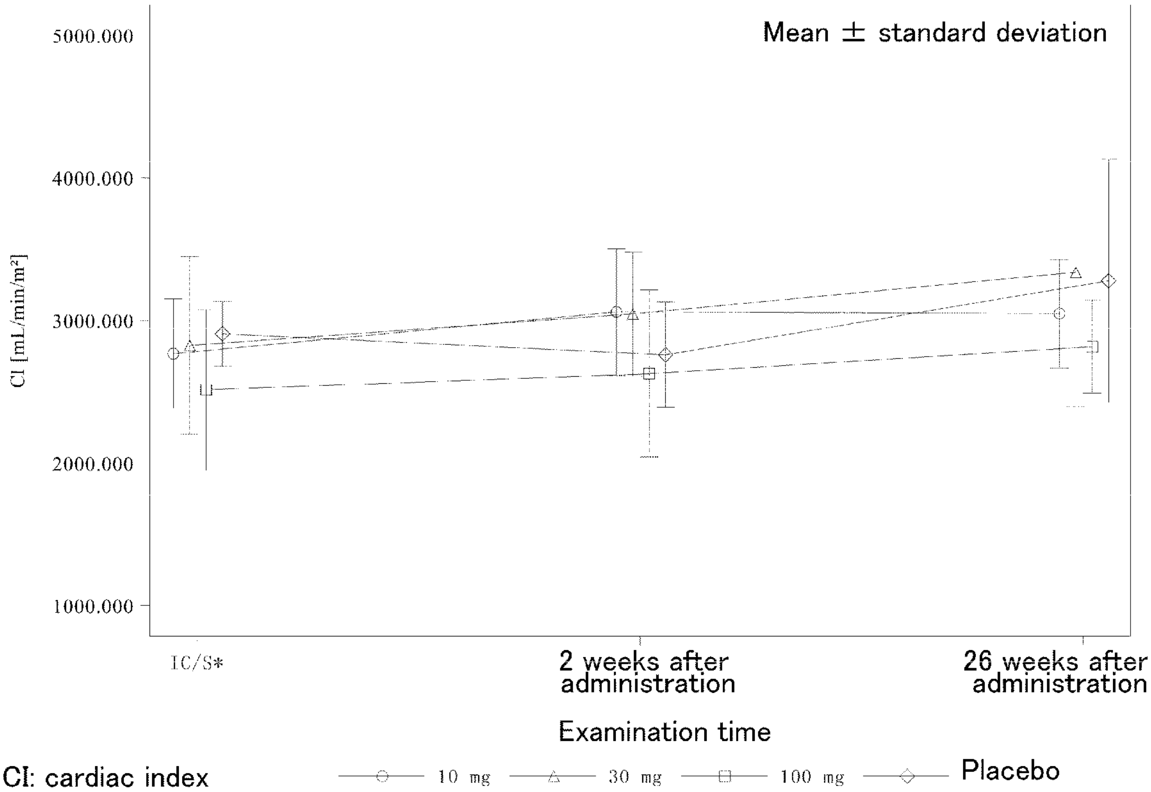


CI: cardiac index
*: Consent acquisition/screening

Fig. 12

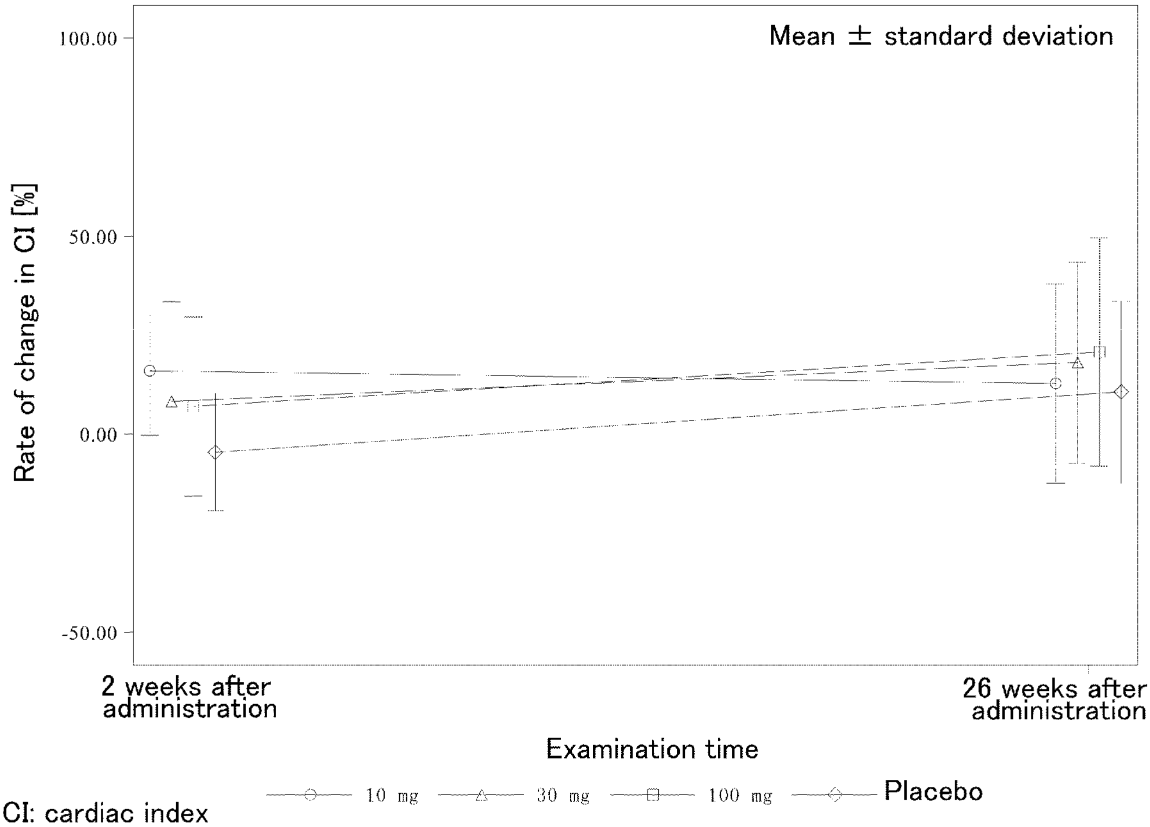


CI: cardiac index

Fig. 13

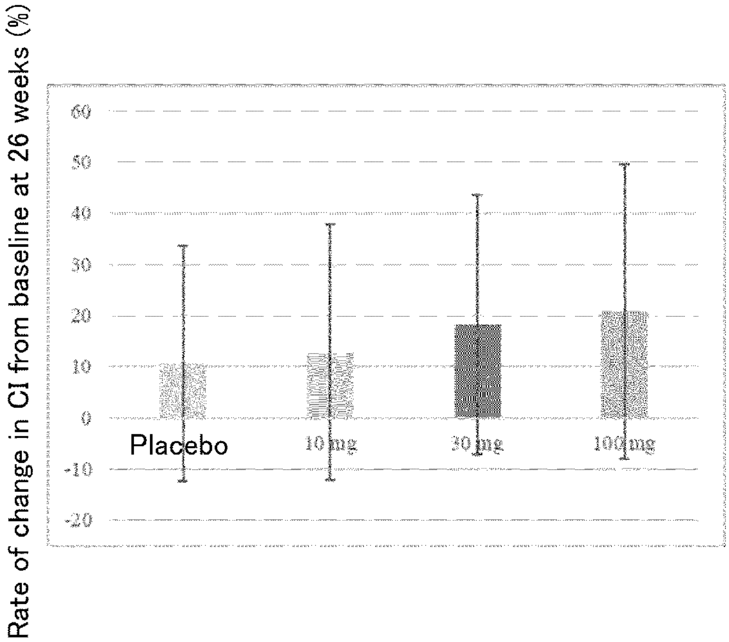


| Dose | 26 weeks after administration |
|---|---|
| Placebo (N=5) | 10.62±23.01% |
| 10mg (N=6) | 12.82±25.10% |
| 30mg (N=5) | 18.14±25.39% |
| 100mg (N=5) | 20.78±28.83% |

One case in each of the placebo and 100 mg groups was excluded from analysis at 26 weeks after administration due to discontinuation.

Fig. 14

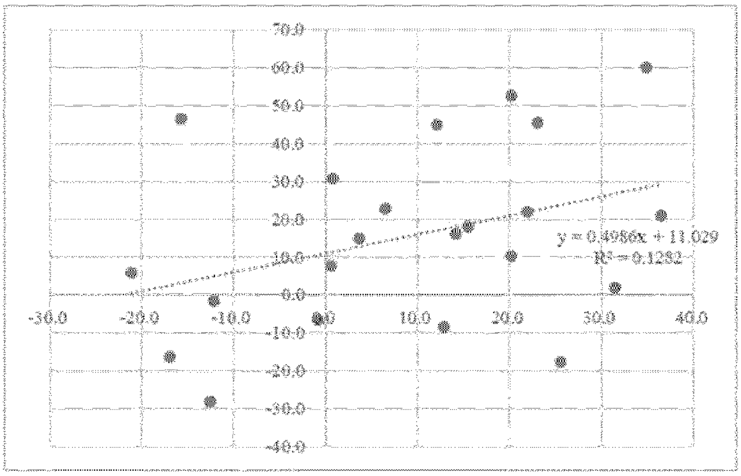


Correlation coefficient: 0.358

p-value: 0.102

A positive correlation was observed between the "total myocardial blood flow (Δ)" and the "rate of change in cardiac index from the pre-value, but was not significant (P=0.102).

Fig. 15

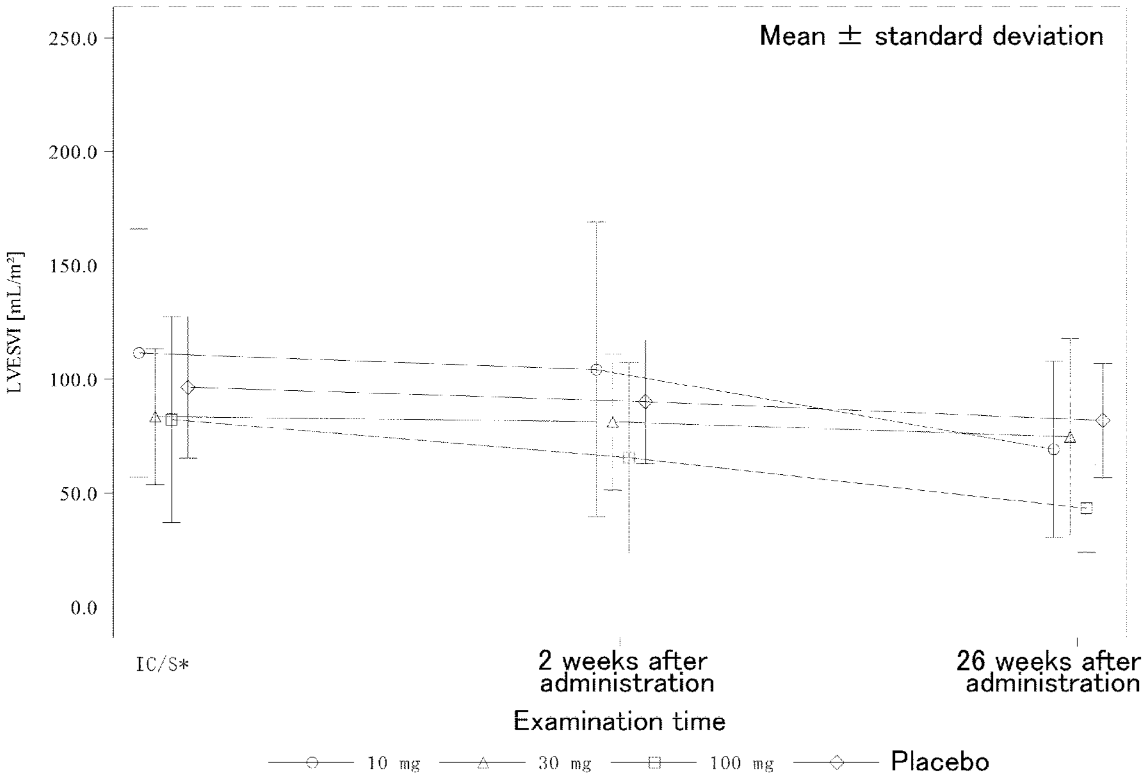


LVESVI: left ventricular end-systolic volume index

*: Consent acquisition/screening

Fig. 16

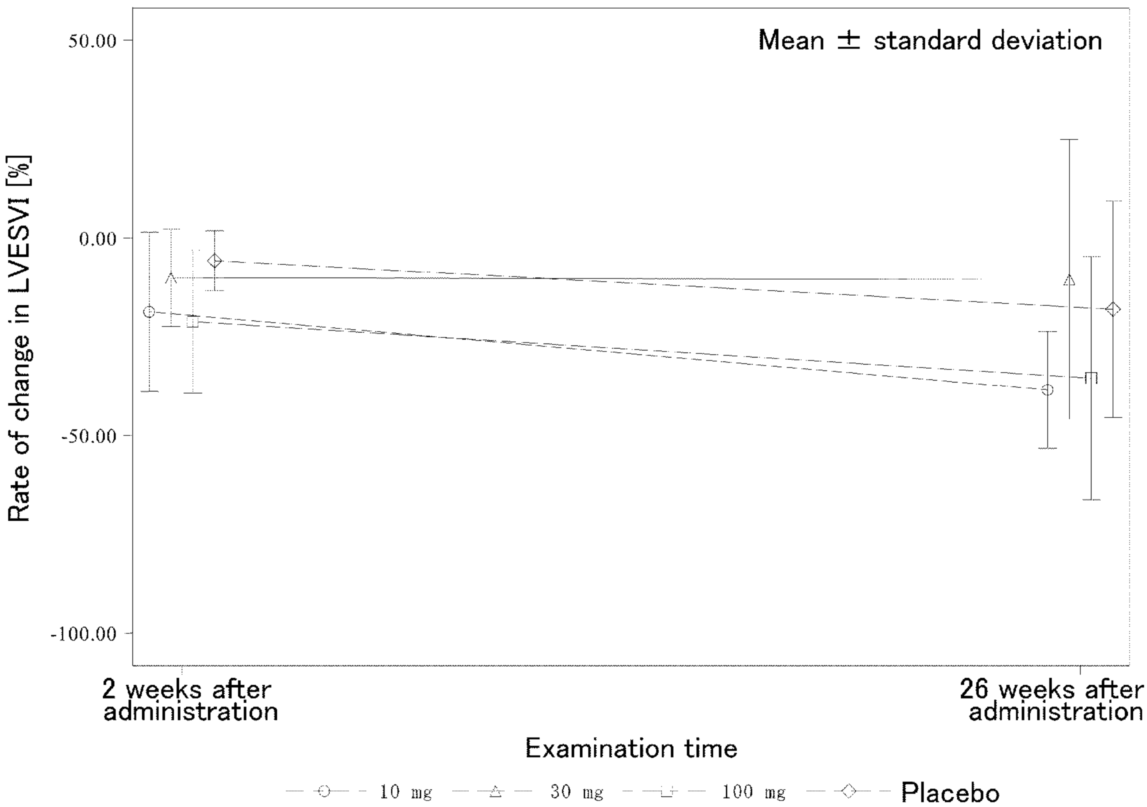


LVESVI: left ventricular end-systolic volume index

Fig. 17

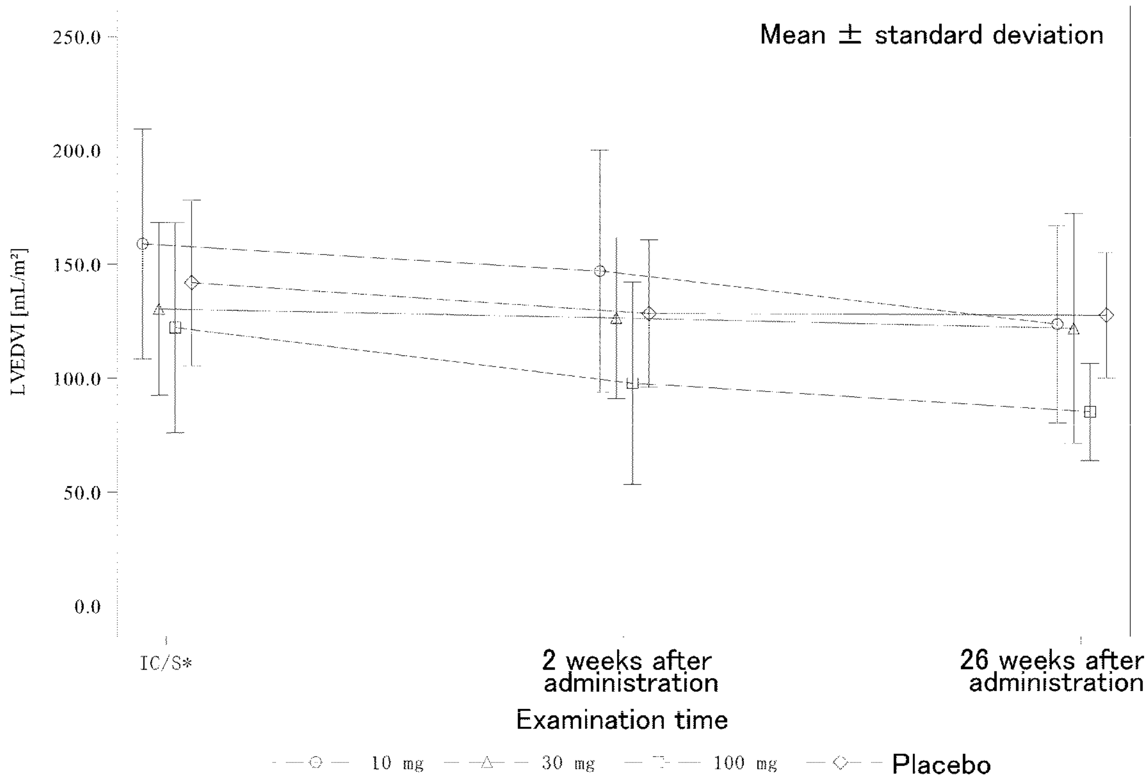


LVEDVI: left ventricular end-diastolic volume index

*: Consent acquisition/screening

Fig. 18

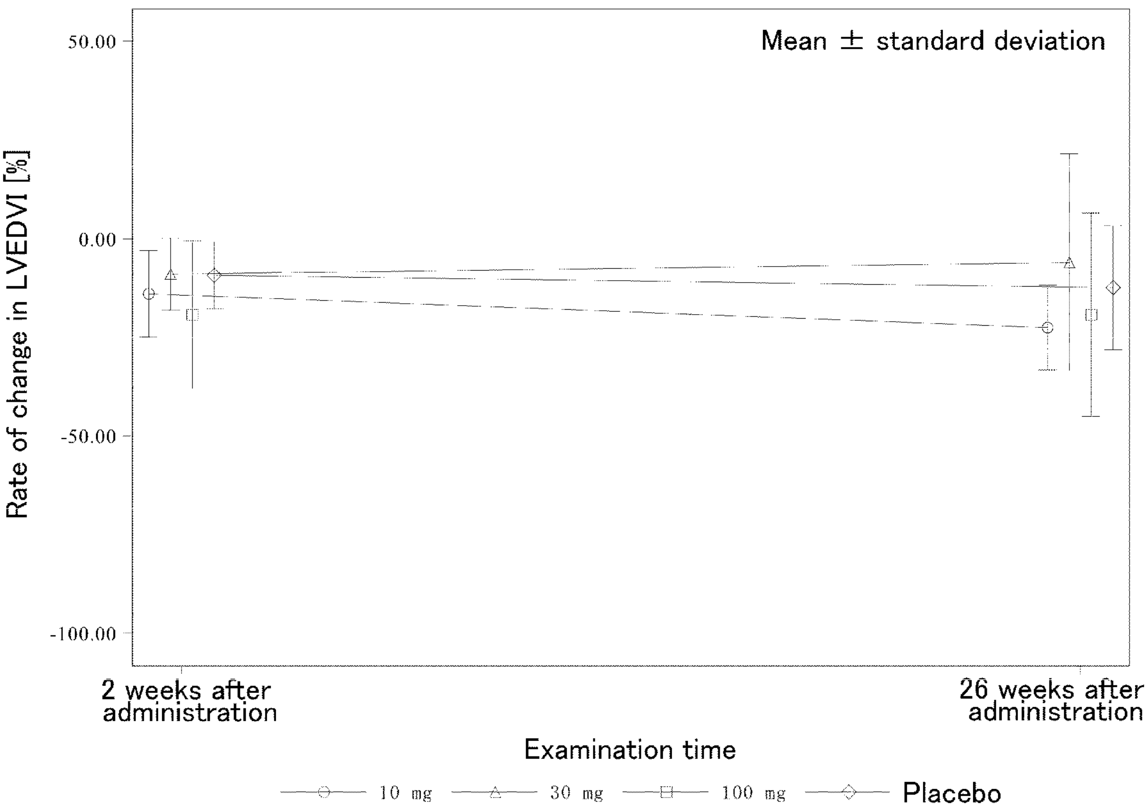


LVEDVI: left ventricular end-diastolic volume index

Fig. 19

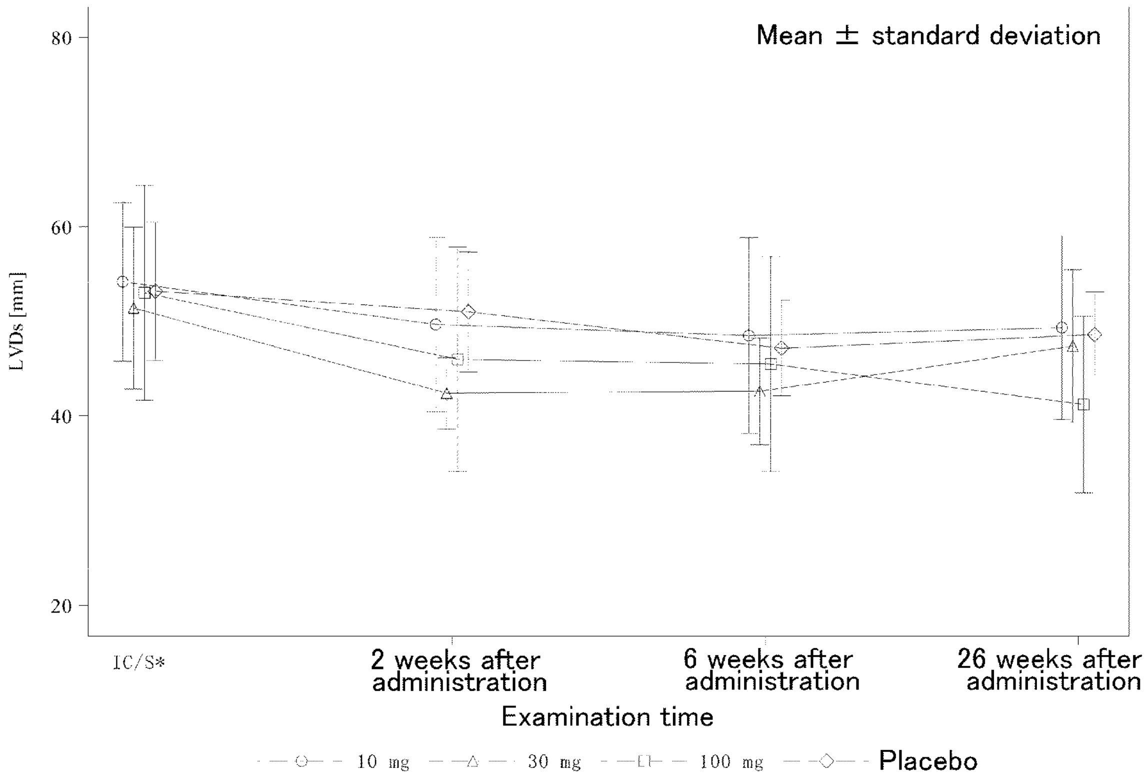


LVDs: left ventricular end-systolic internal diameter
*: Consent acquisition/screening

Fig. 20

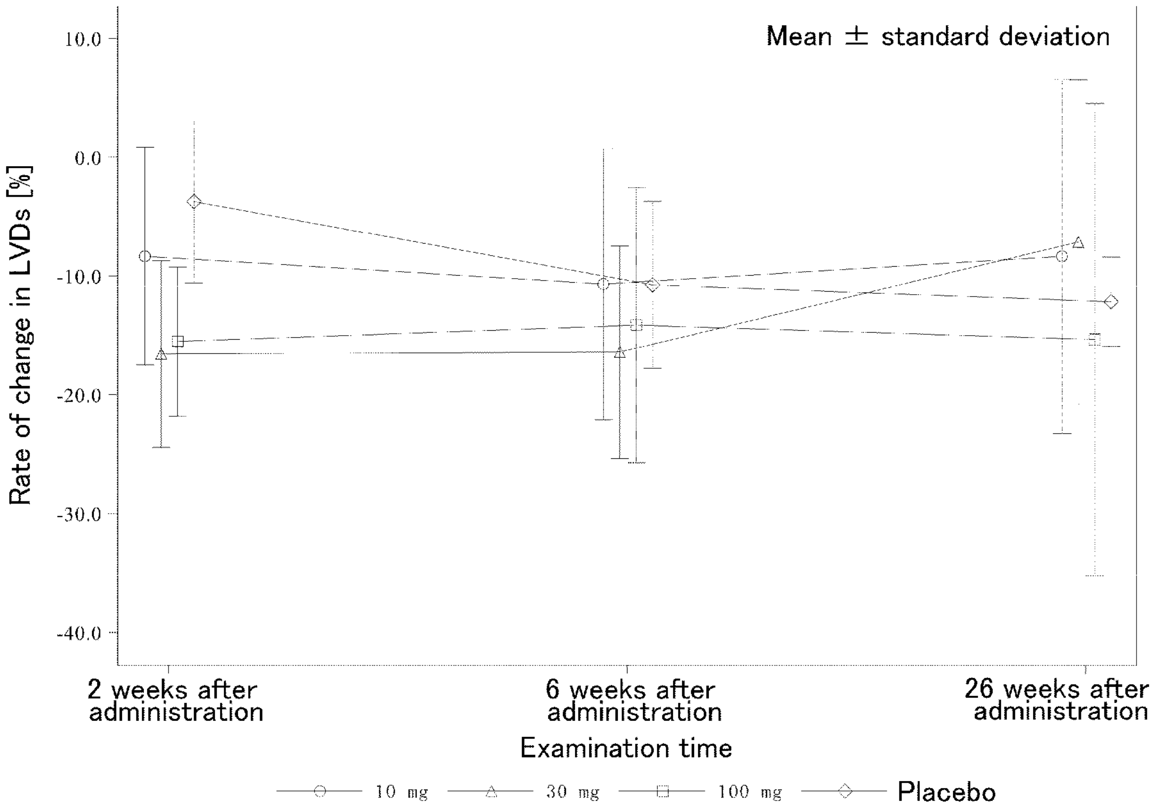


LVDs: left ventricular end-systolic internal diameter

Fig. 21

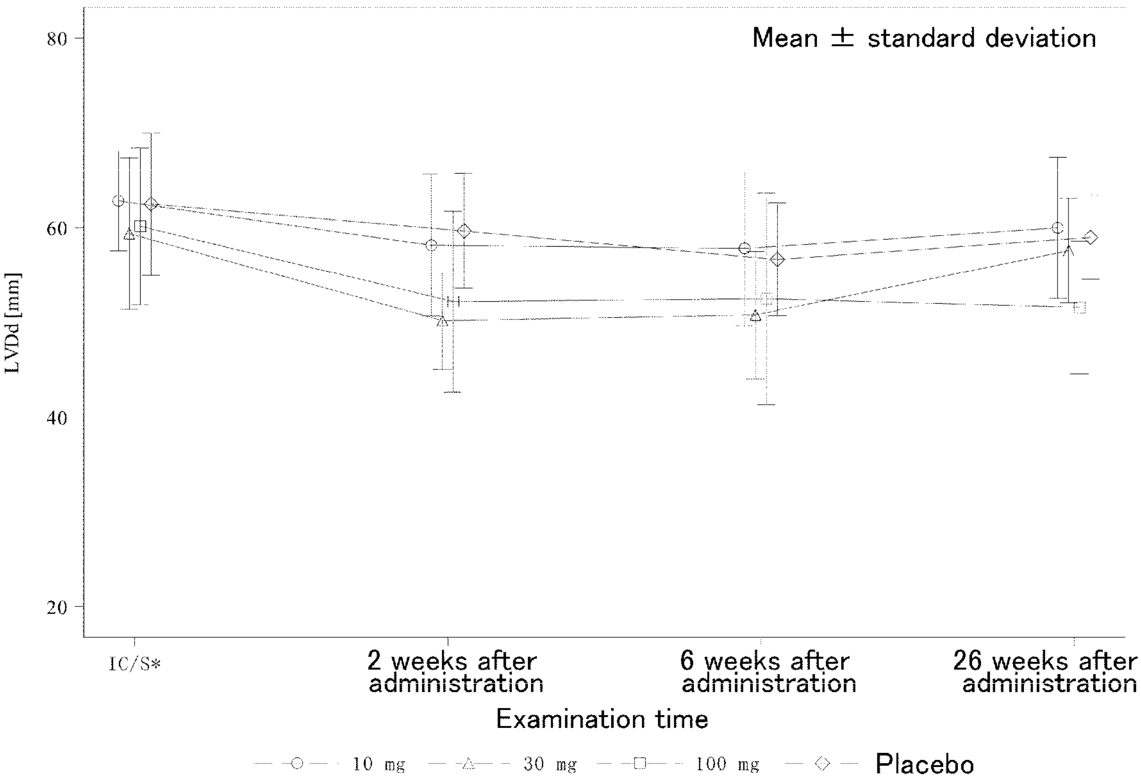


LVDd: left ventricular end-diastolic internal diameter
*: Consent acquisition/screening

Fig. 22

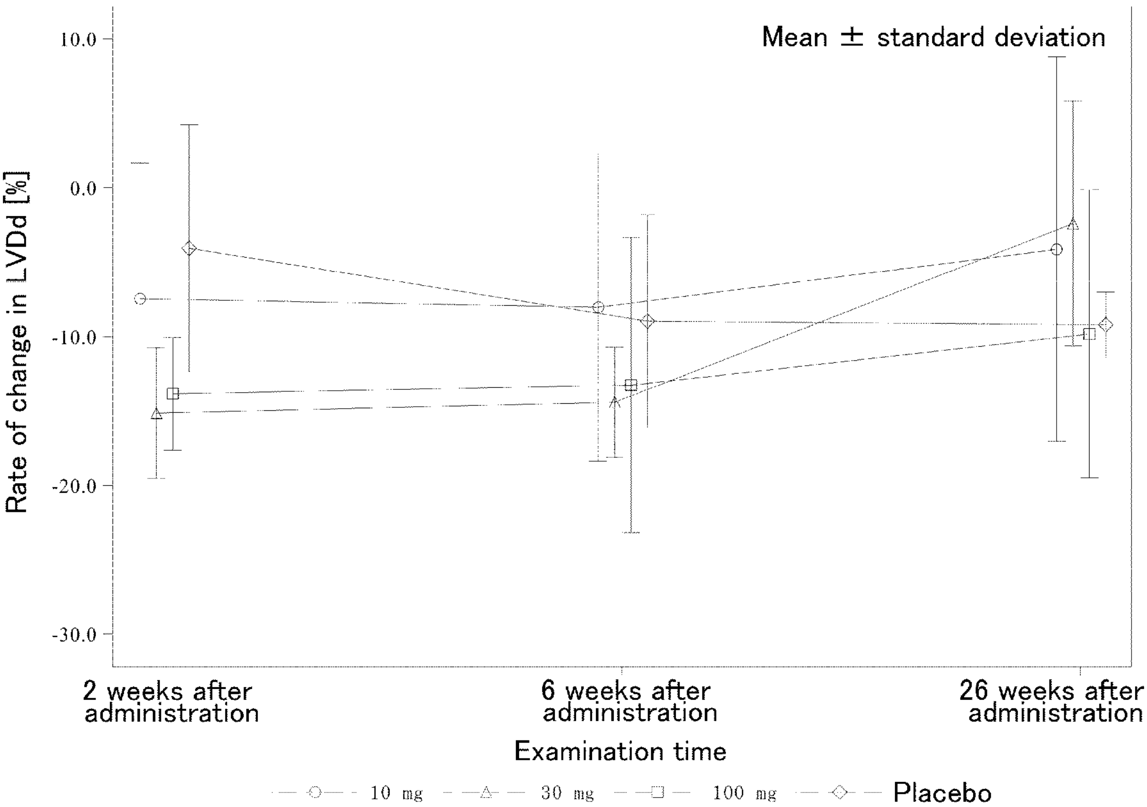


LVDd: left ventricular end-diastolic internal diameter

Fig. 23

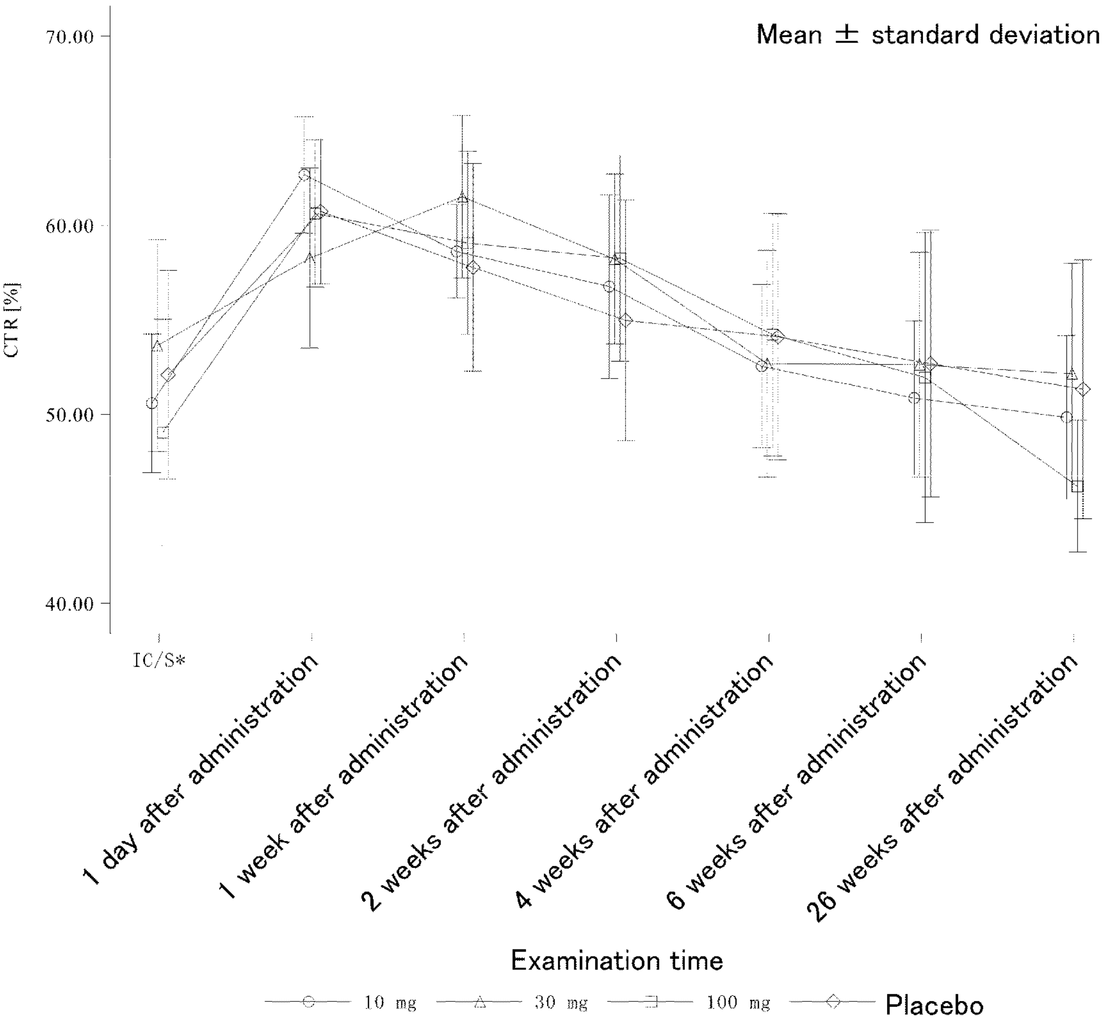


CTR: cardiothoracic ratio
*: Consent acquisition/screening

Fig. 24

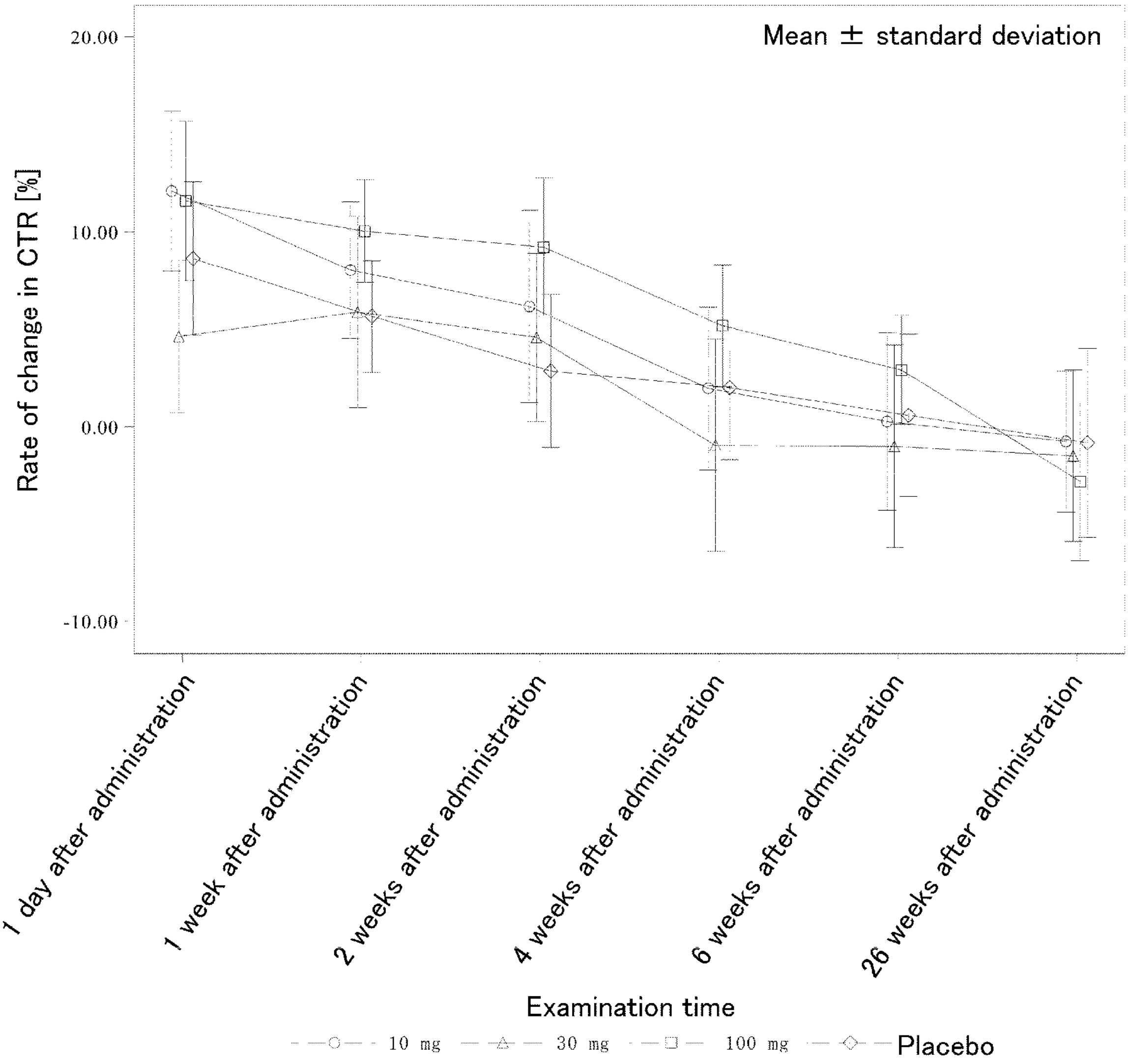


TR: cardiothoracic ratio

Fig. 25

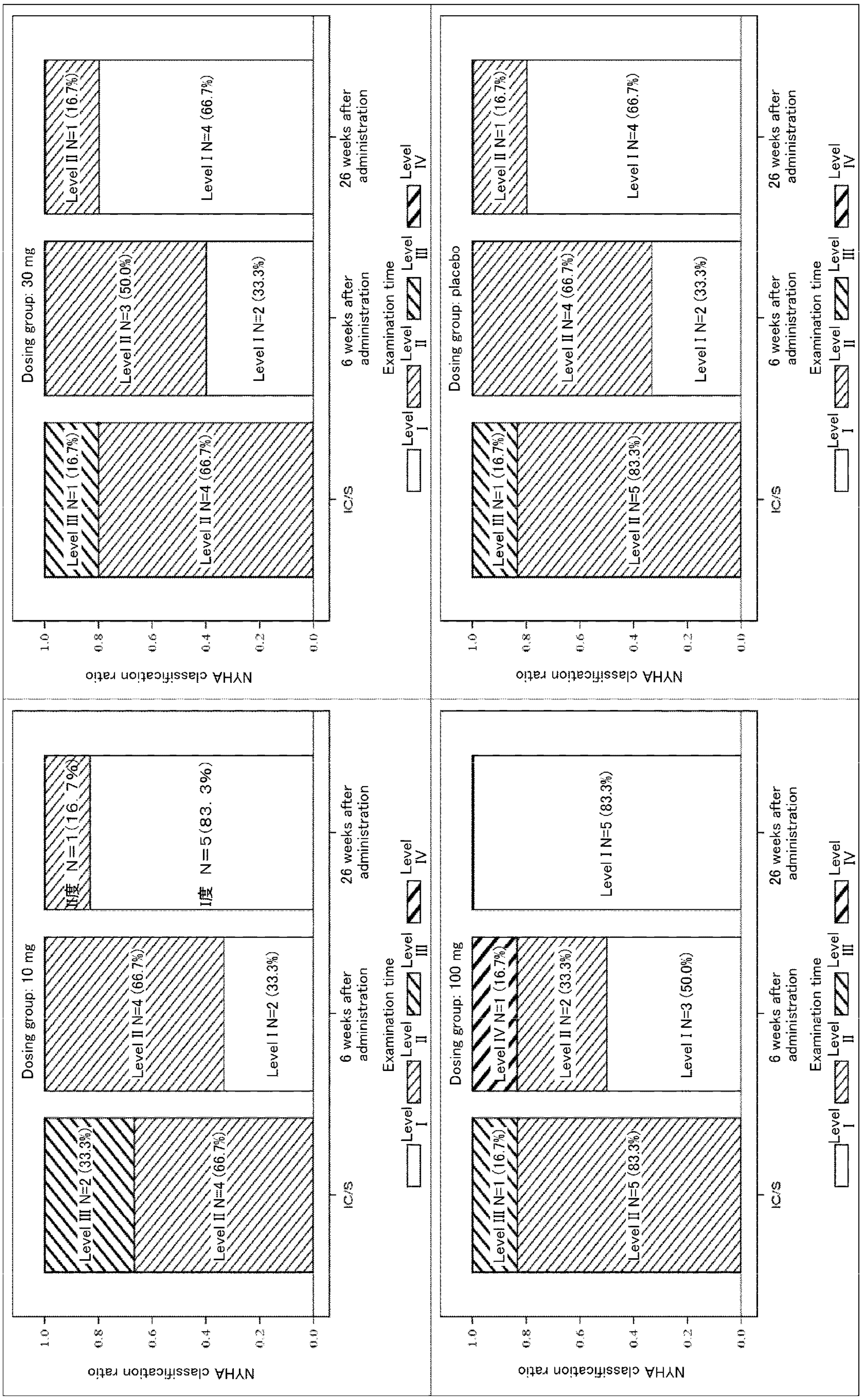
Dosing group: 10 mg
Dosing group: 30 mg
Dosing group: 100 mg
Dosing group: placebo
NYHA classification ratio
1.0
0.8
0.6
0.4
0.2
0.0
IC/S
6 weeks after administration
26 weeks after administration
Examination time
Level I
Level II
Level III
Level IV
Level III N=2 (33.3%)
Level II N=4 (66.7%)
Level II N=4 (66.7%)
Level I N=2 (33.3%)
II度 N=1 (16. 7%)
I度 N=5 (83. 3%)
Level III N=1 (16.7%)
Level II N=4 (66.7%)
Level II N=3 (50.0%)
Level I N=2 (33.3%)
Level II N=1 (16.7%)
Level I N=4 (66.7%)
Level III N=1 (16.7%)
Level II N=5 (83.3%)
Level IV N=1 (16.7%)
Level II N=2 (33.3%)
Level I N=3 (50.0%)
Level I N=5 (83.3%)
Level III N=1 (16.7%)
Level II N=5 (83.3%)
Level II N=4 (66.7%)
Level I N=2 (33.3%)
Level II N=1 (16.7%)
Level I N=4 (66.7%)

Fig. 26

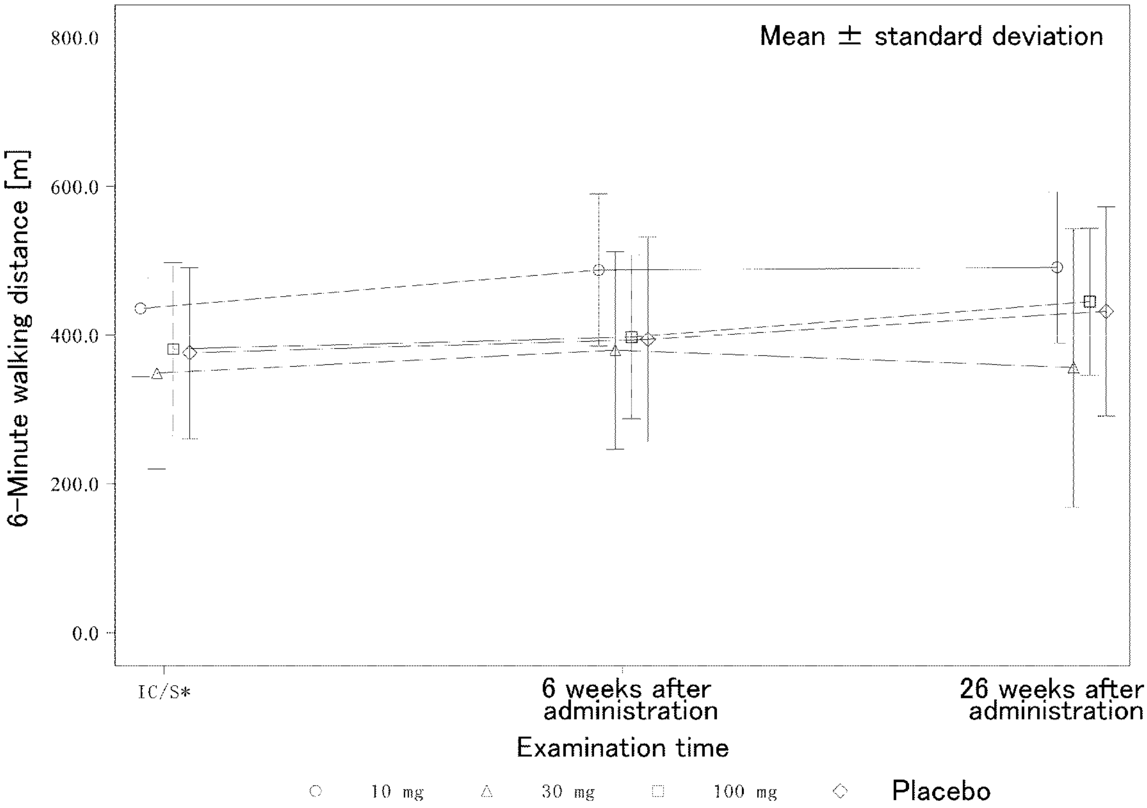


*: Consent acquisition/screening

Fig. 27

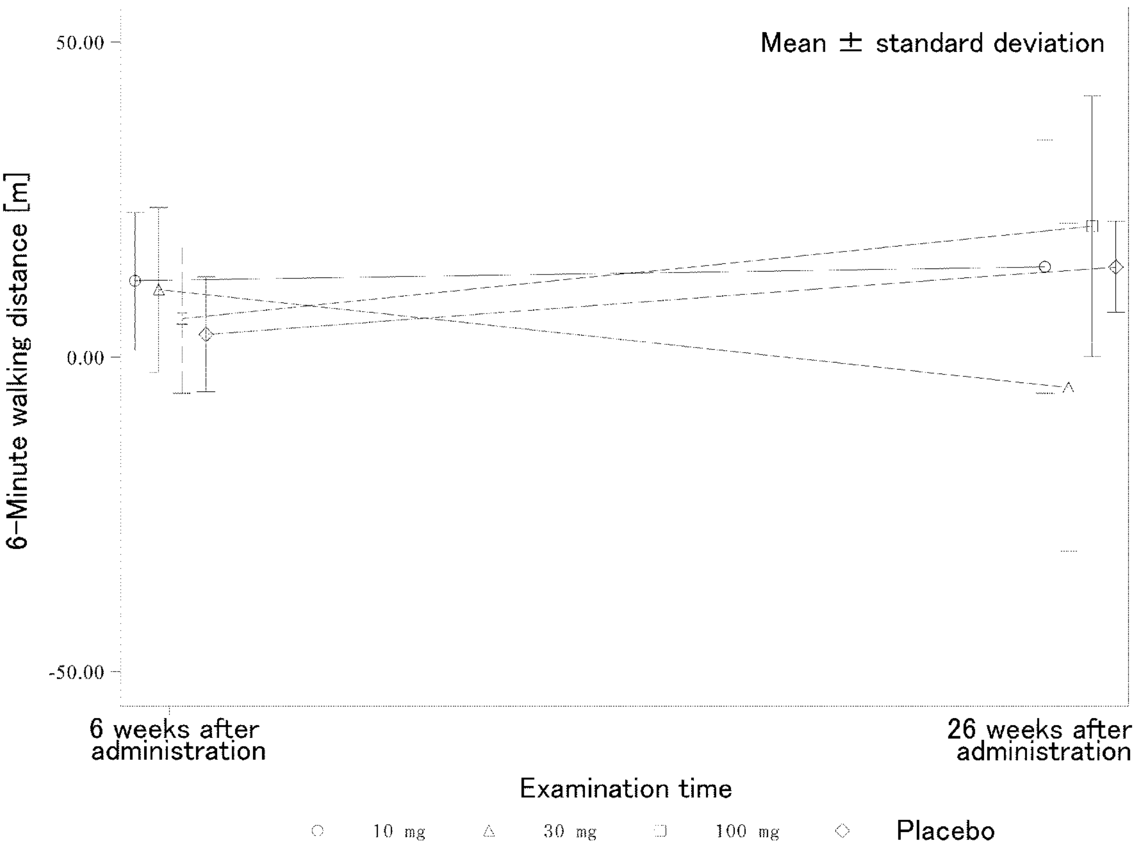
Mean ± standard deviation
50.00
0.00
-50.00
6-Minute walking distance [m]
6 weeks after administration
26 weeks after administration
Examination time
10 mg
30 mg
100 mg
Placebo

Fig. 28

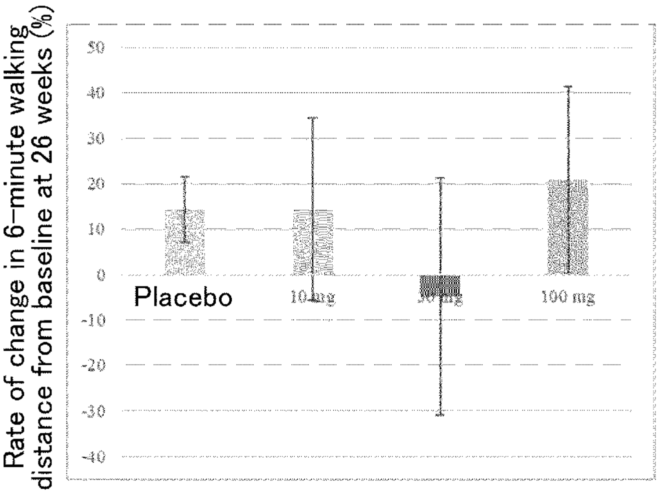


| Dose | 26 weeks after administration |
|---|---|
| Placebo (N=5) | 14.28±7.24% |
| 10mg (N=6) | 14.33±20.14% |
| 30mg (N=5) | −4.83±26.07% |
| 100mg (N=5) | 20.77±20.69% |

One case in each of the placebo and 100 mg groups was excluded from analysis at 26 weeks after administration due to discontinuation. One case in the 30 mg group was excluded from analysis because of impossibility of measurement due to complications of right hemiparesis.

Fig. 29

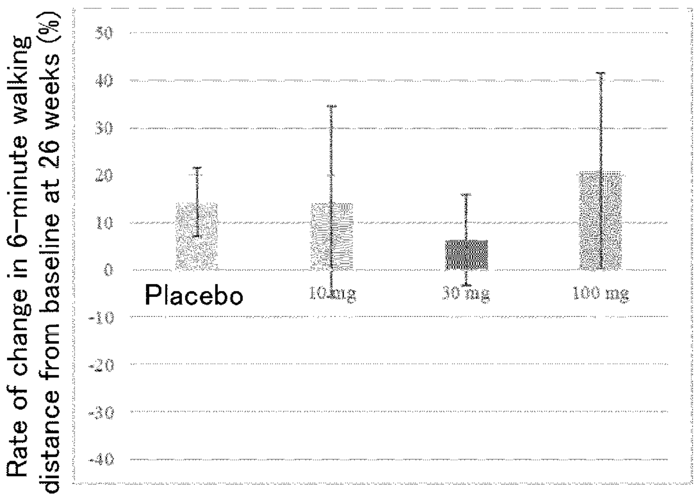


Reference: the amount of change in 6-minute walking distance at 26 weeks after administration when excluding a case of poor medication compliance in the 30 mg group

| Dose | 26 weeks after administration |
|---|---|
| Placebo (N=5) | 14.28±7.24% |
| 10mg (N=6) | 14.33±20.14% |
| 30mg (N=5) | 6.23±9.54% |
| 100mg (N=5) | 20.77±20.69% |

Fig. 30A

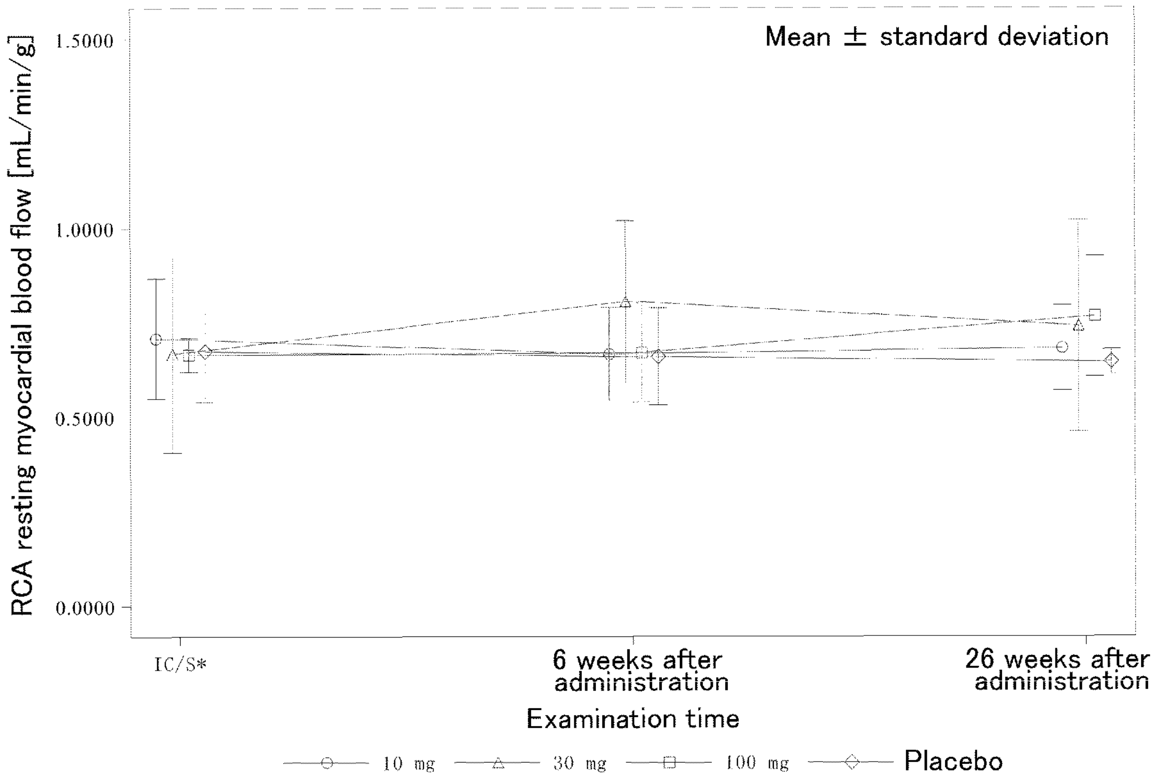


RCA: right coronary artery

*: Consent acquisition/screening

Fig. 30B

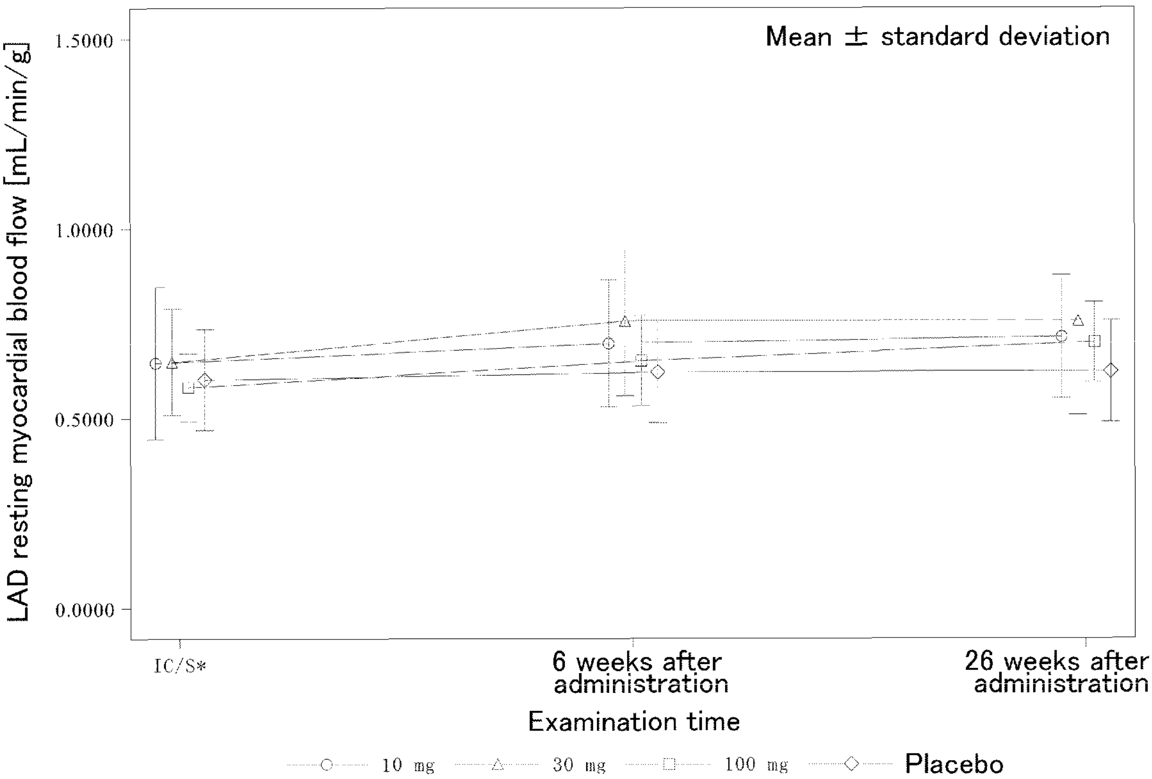


LAD: left anterior descending coronary artery
*: Consent acquisition/screening

Fig. 30C

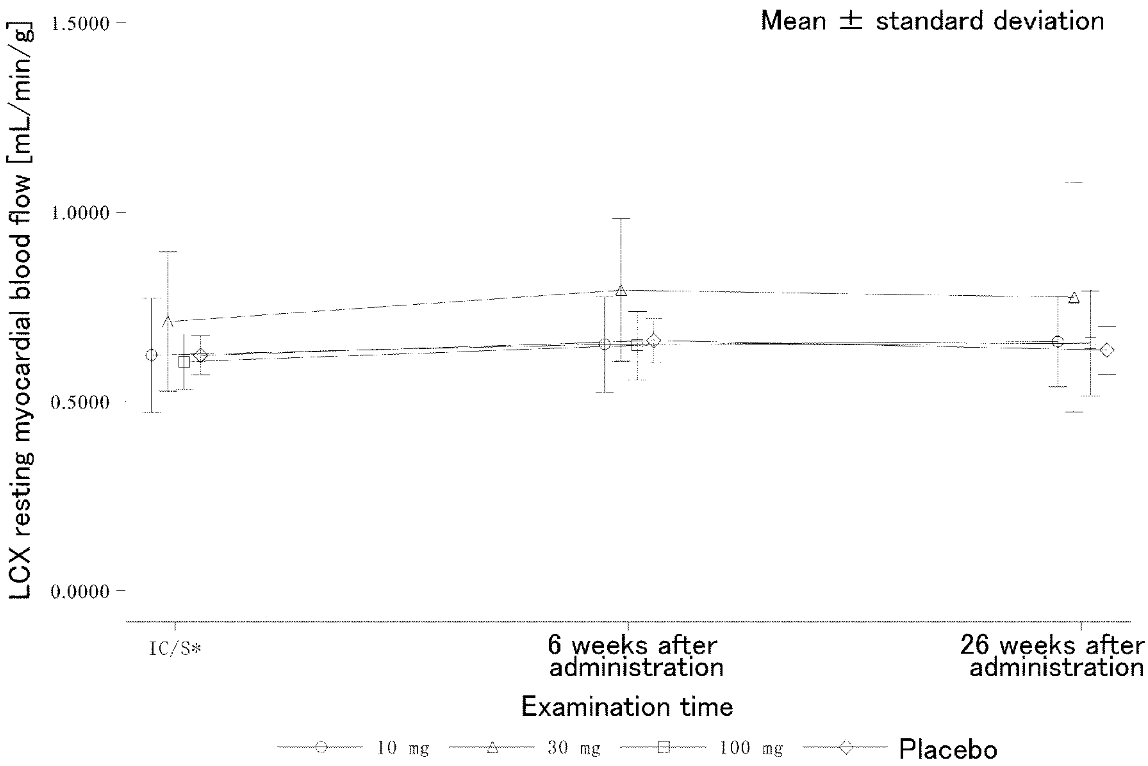


LCX: left circumflex coronary artery
*: Consent acquisition/screening

Fig. 30D

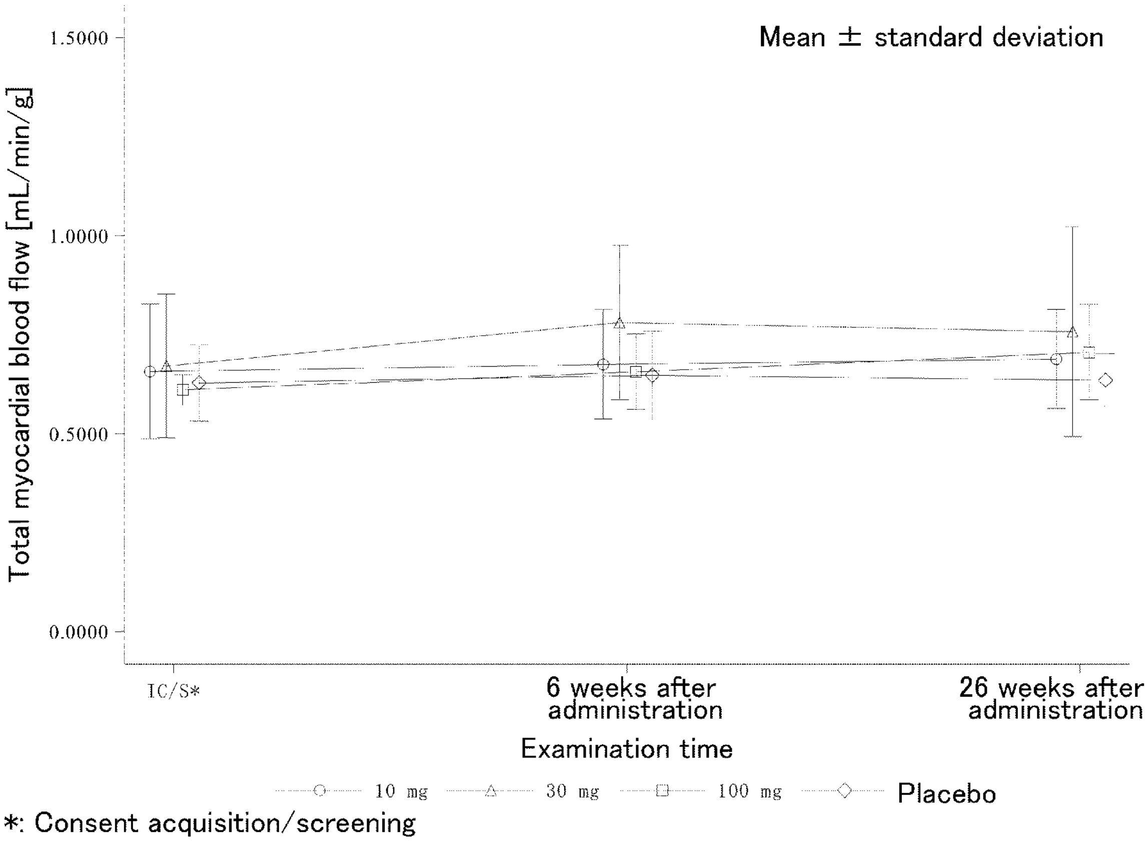


*: Consent acquisition/screening

Fig. 31A

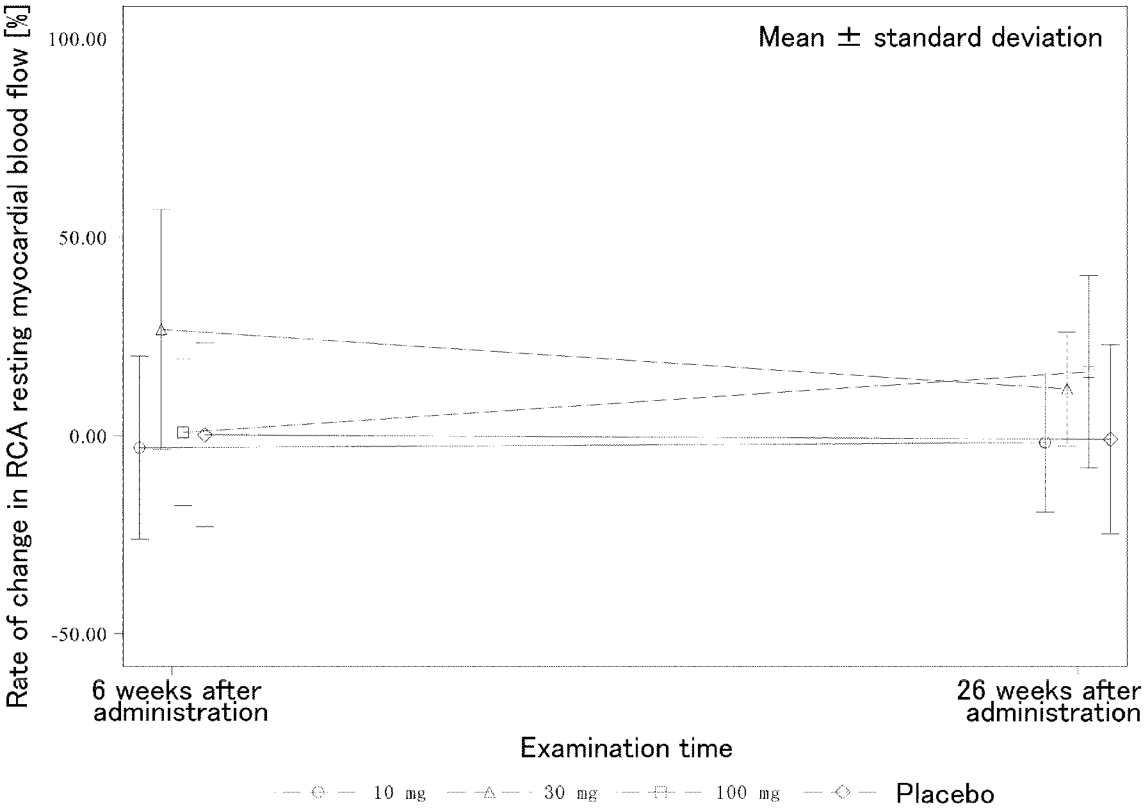


RCA: right coronary artery

Fig. 31B

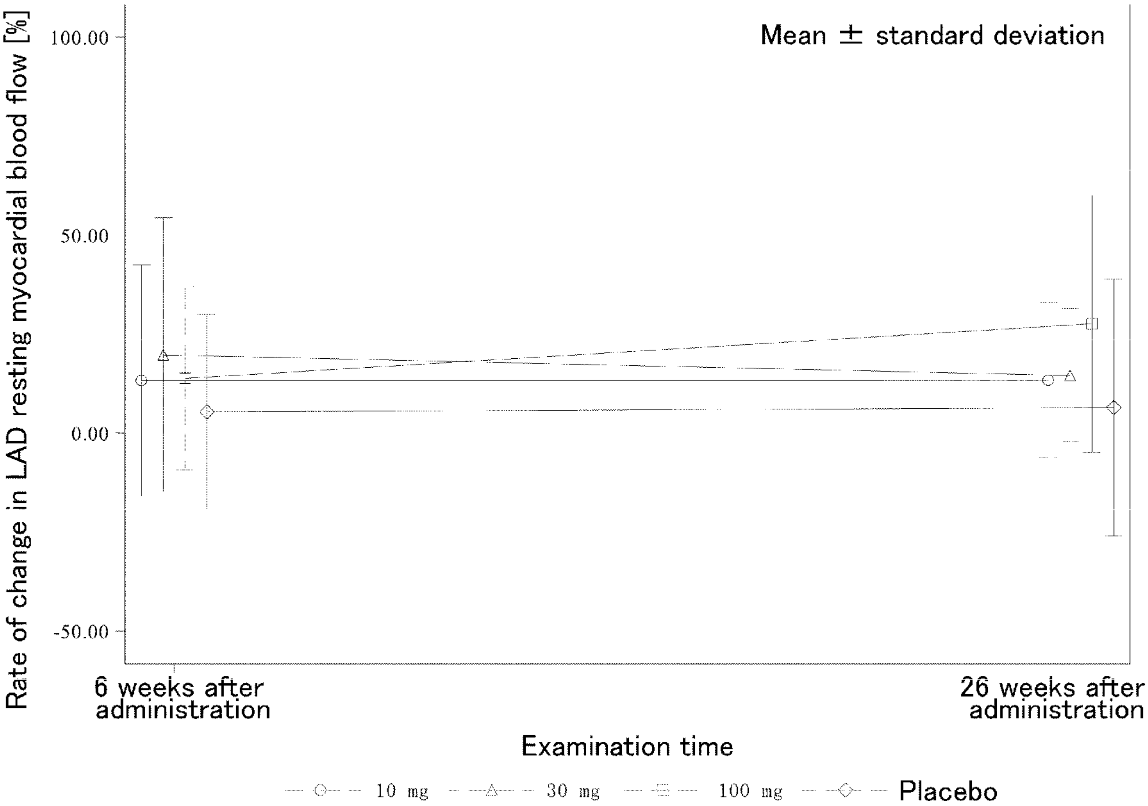


LAD: left anterior descending coronary artery

Fig. 31C

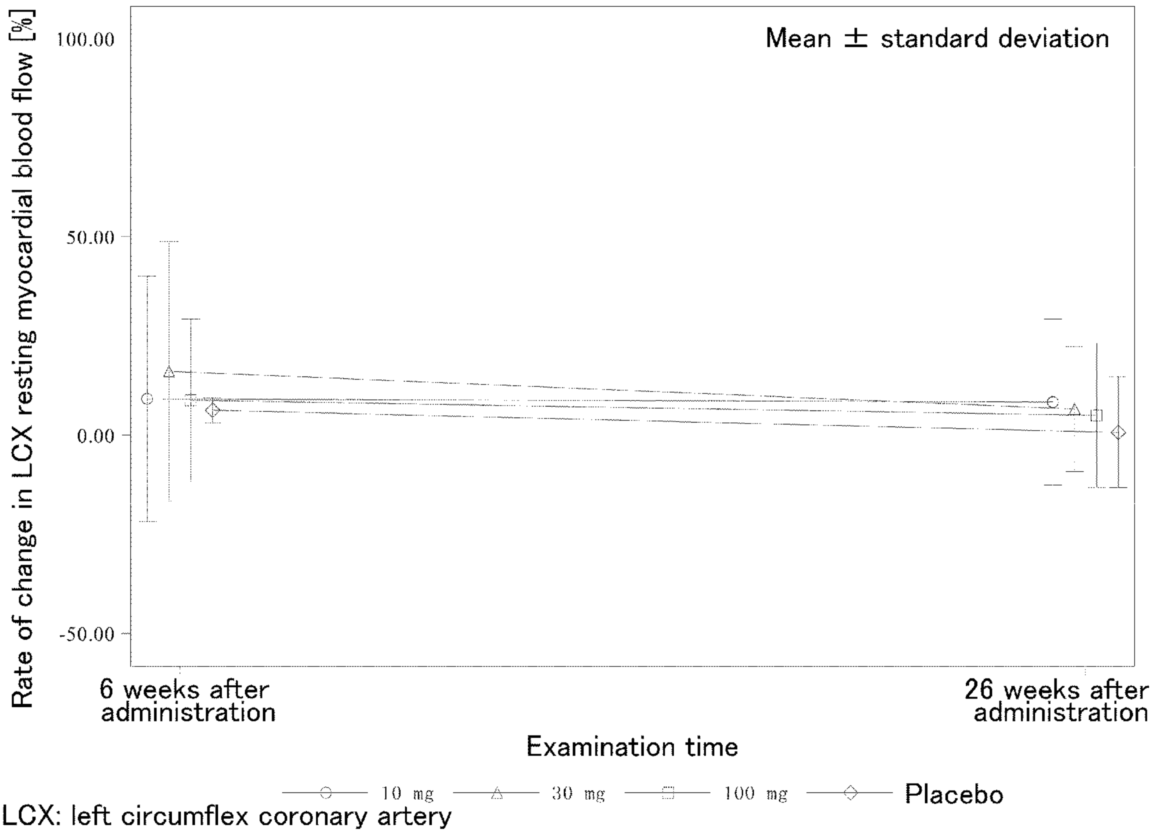


LCX: left circumflex coronary artery

Fig. 31D

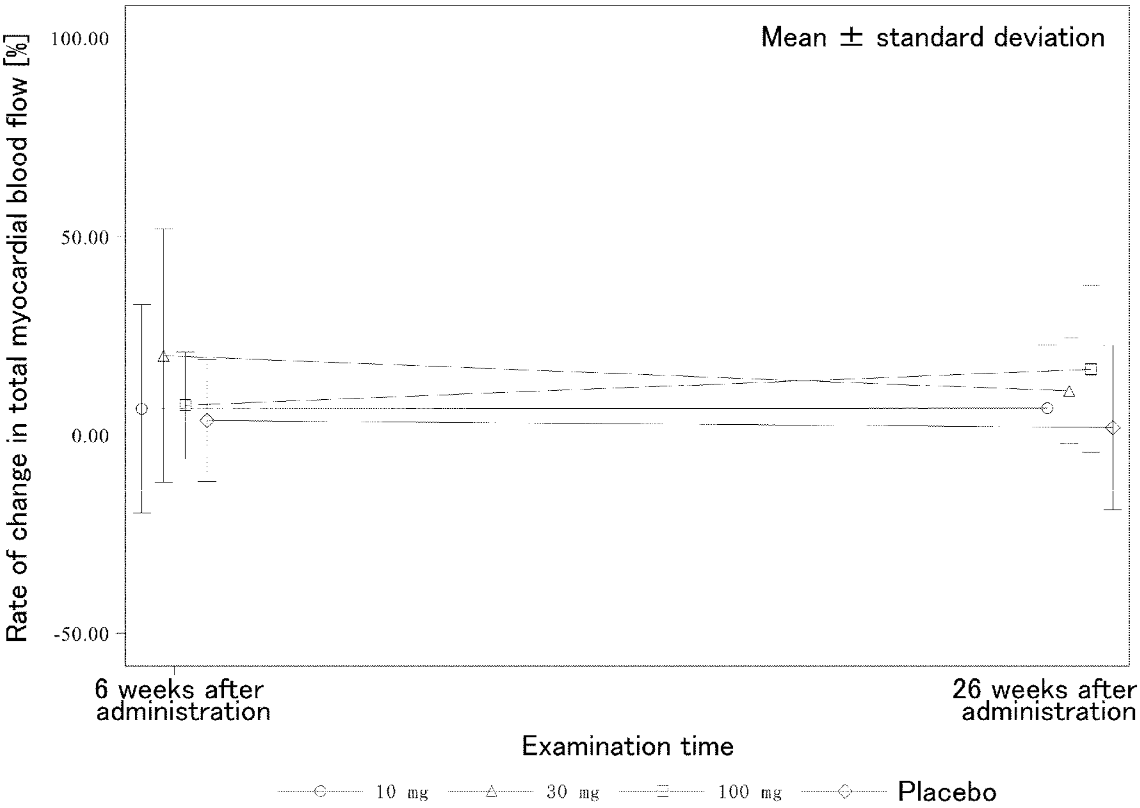
Mean ± standard deviation
Rate of change in total myocardial blood flow [%]
100.00
50.00
0.00
-50.00
6 weeks after administration
26 weeks after administration
Examination time
10 mg
30 mg
100 mg
Placebo

Fig. 32

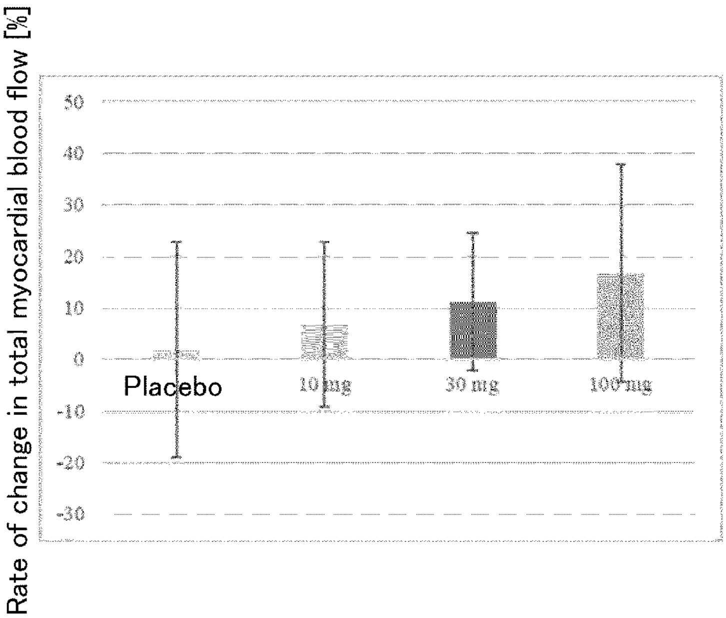


| Dose | 26 weeks after administration |
|---|---|
| Placebo (N=5) | 1.89±20.80% |
| 10mg (N=6) | 6.78±15.98% |
| 30mg (N=5) | 11.15±13.34% |
| 100mg (N=5) | 16.66±21.04% |

One case in each of the placebo and 100 mg groups was excluded from analysis at 26 weeks after administration due to discontinuation.

Fig. 33

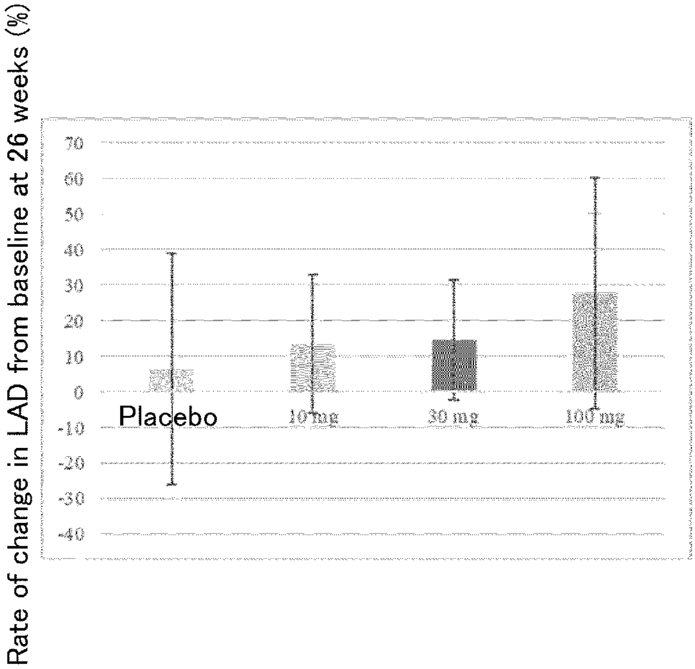


| Dose | 26 weeks after administration |
|---|---|
| Placebo (N=5) | 6.41±32.39% |
| 10mg (N=6) | 13.38±19.48% |
| 30mg (N=5) | 14.59±16.85% |
| 100mg (N=5) | 27.59±32.47% |

One case in each of the placebo and 100 mg groups was excluded from analysis at 26 weeks after administration due to discontinuation.

Fig. 34

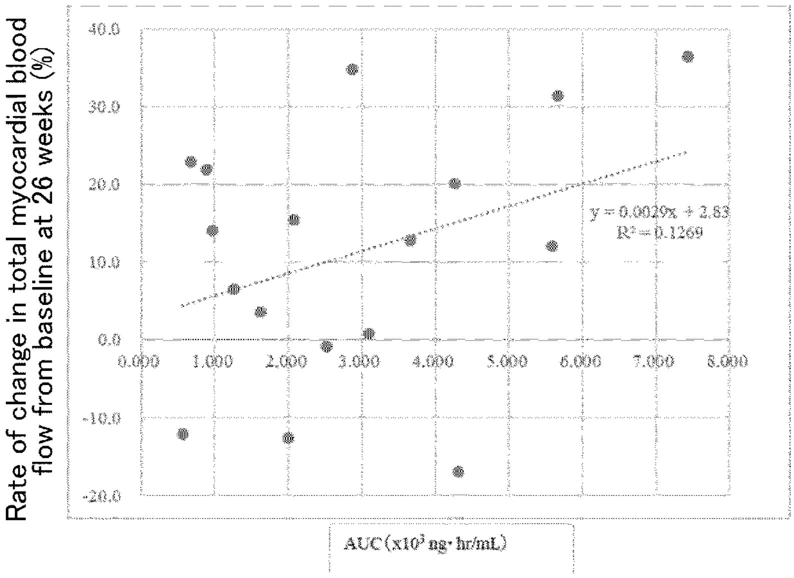


Correlation coefficient: 0.356

p-value: 0.160

A positive correlation was observed between the "area under drug concentration-time curve (AUC)" and the "rate of change in total myocardial blood flow from the pre-value (Δ)," but was not significant (P=0.160).

Fig. 35

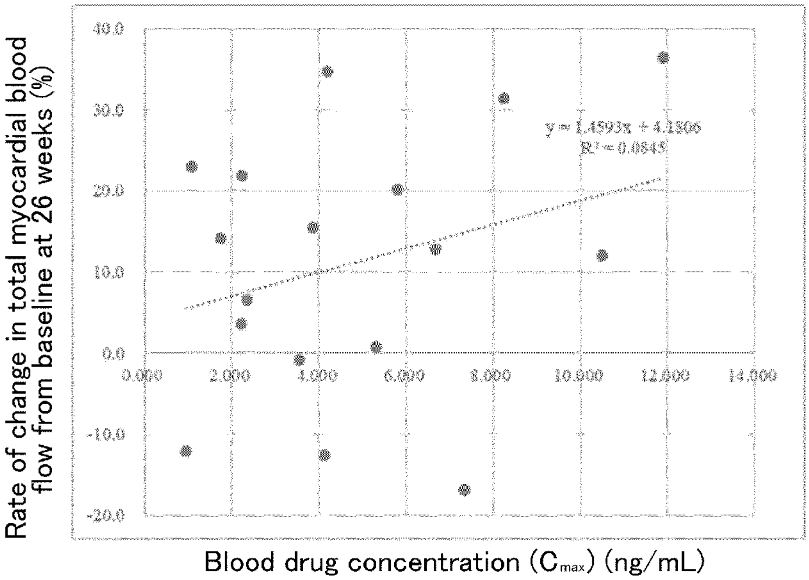


Correlation coefficient: 0.291

p-value: 0.258

A positive correlation was observed between the "blood drug concentration ($C_{max}$)" and the "rate of change in total myocardial blood flow from the pre-value (Δ)," but was not significant (P=0.258).

Fig. 36

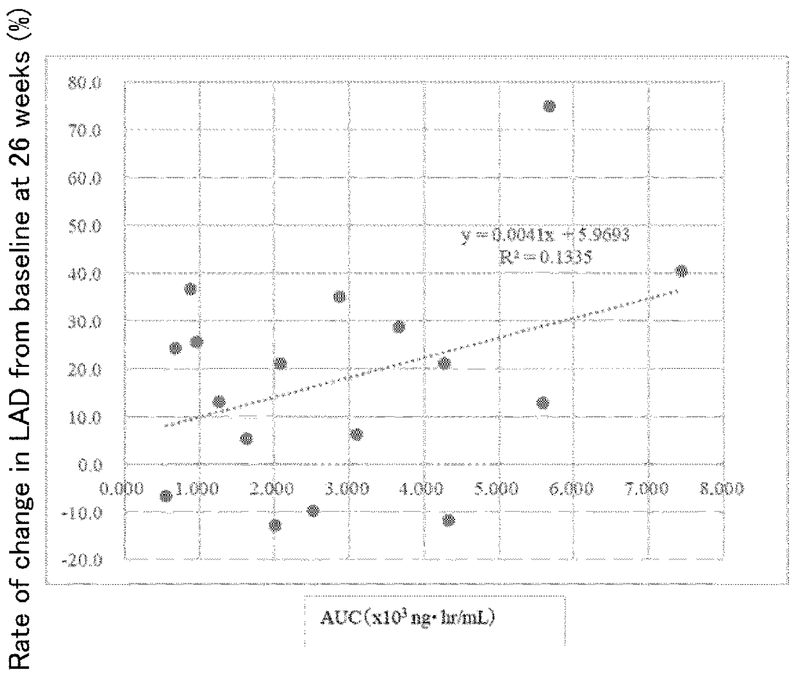


Correlation coefficient: 0.365

p-value: 0.149

A positive correlation was observed between the "area under drug concentration-time curve (AUC)" and the "rate of change in LAD blood flow from the pre-value (Δ)," but was not significant (P=0.149).

Fig. 37

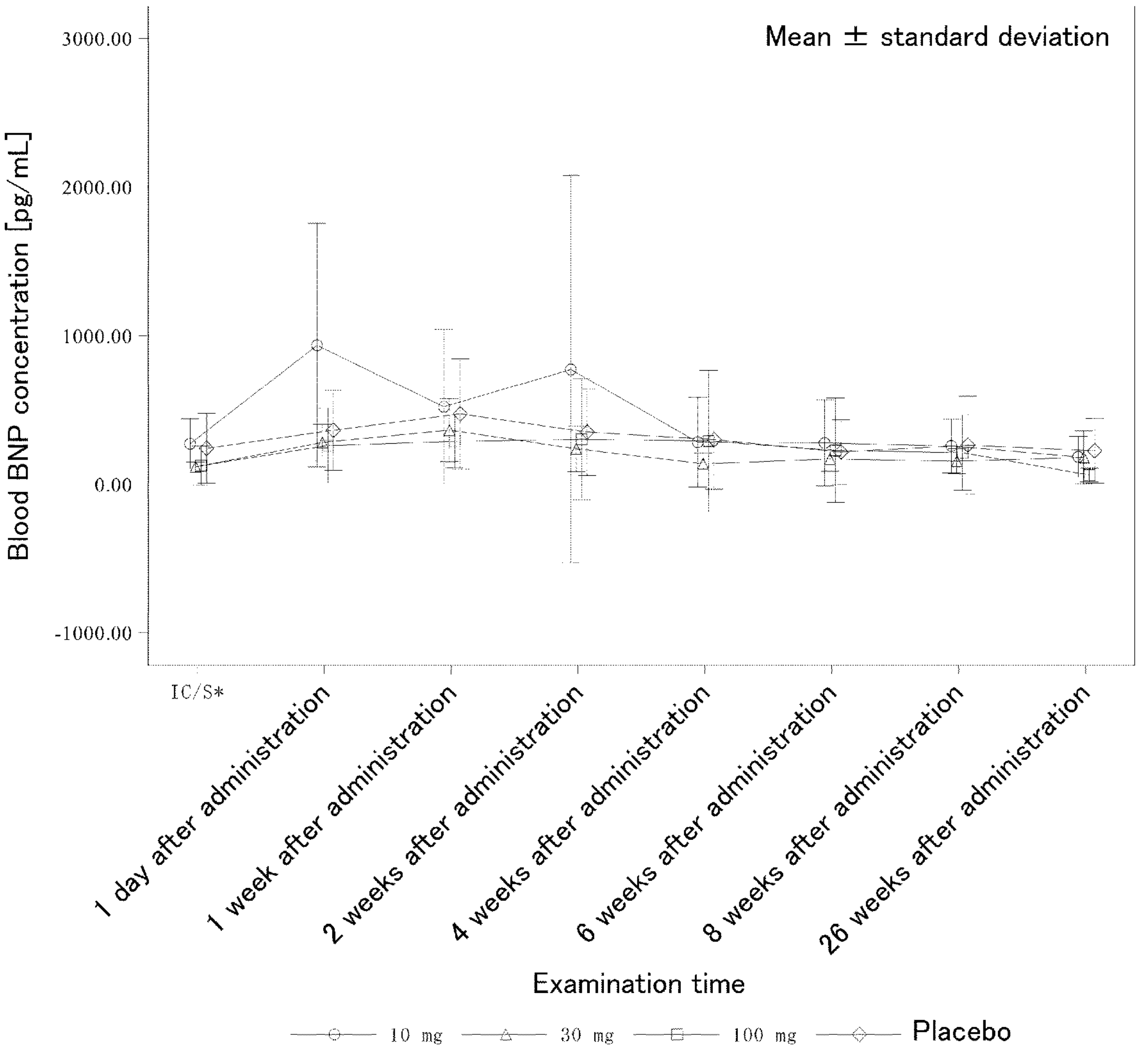


*: Consent acquisition/screening

Fig. 38

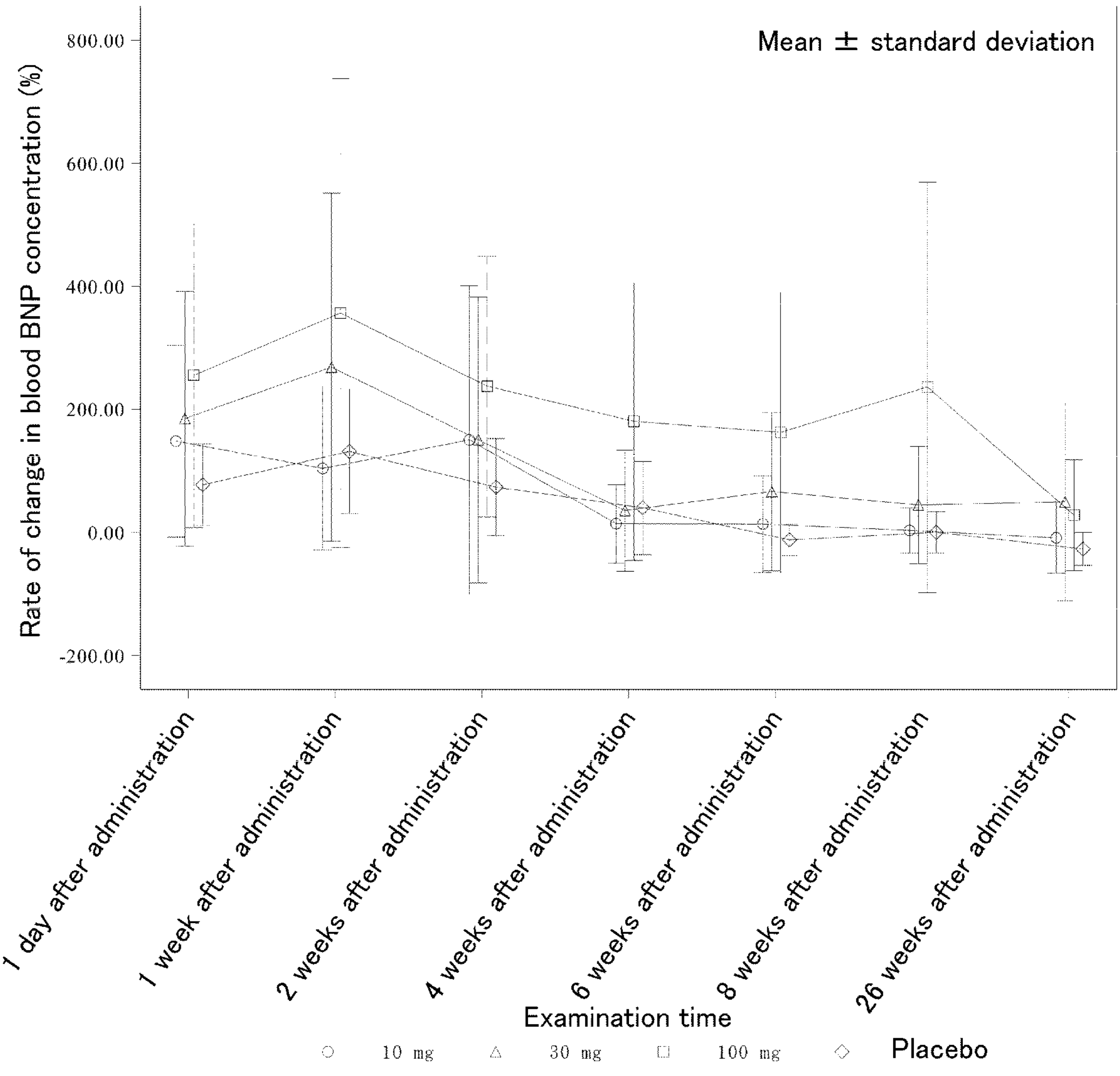
Mean ± standard deviation
Rate of change in blood BNP concentration (%)
800.00
600.00
400.00
200.00
0.00
-200.00
1 day after administration
1 week after administration
2 weeks after administration
4 weeks after administration
6 weeks after administration
8 weeks after administration
26 weeks after administration
Examination time
10 mg
30 mg
100 mg
Placebo

Fig. 39A

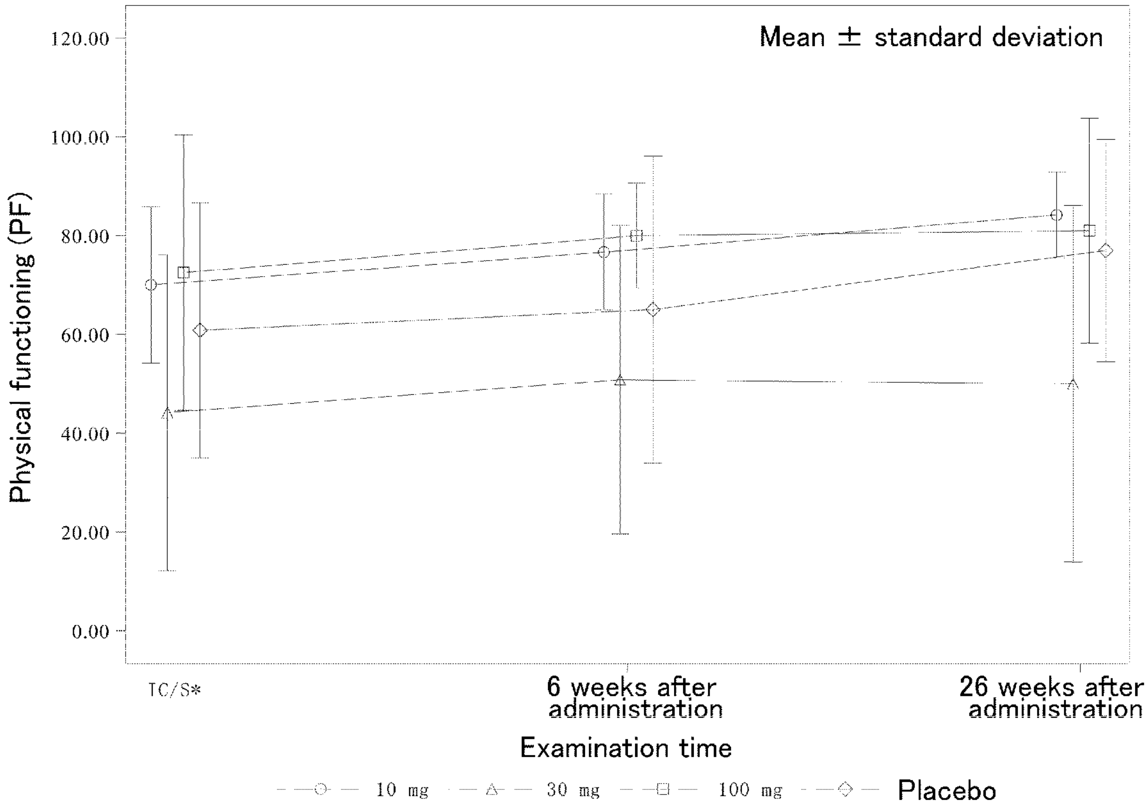


*: Consent acquisition/screening

Fig. 39B

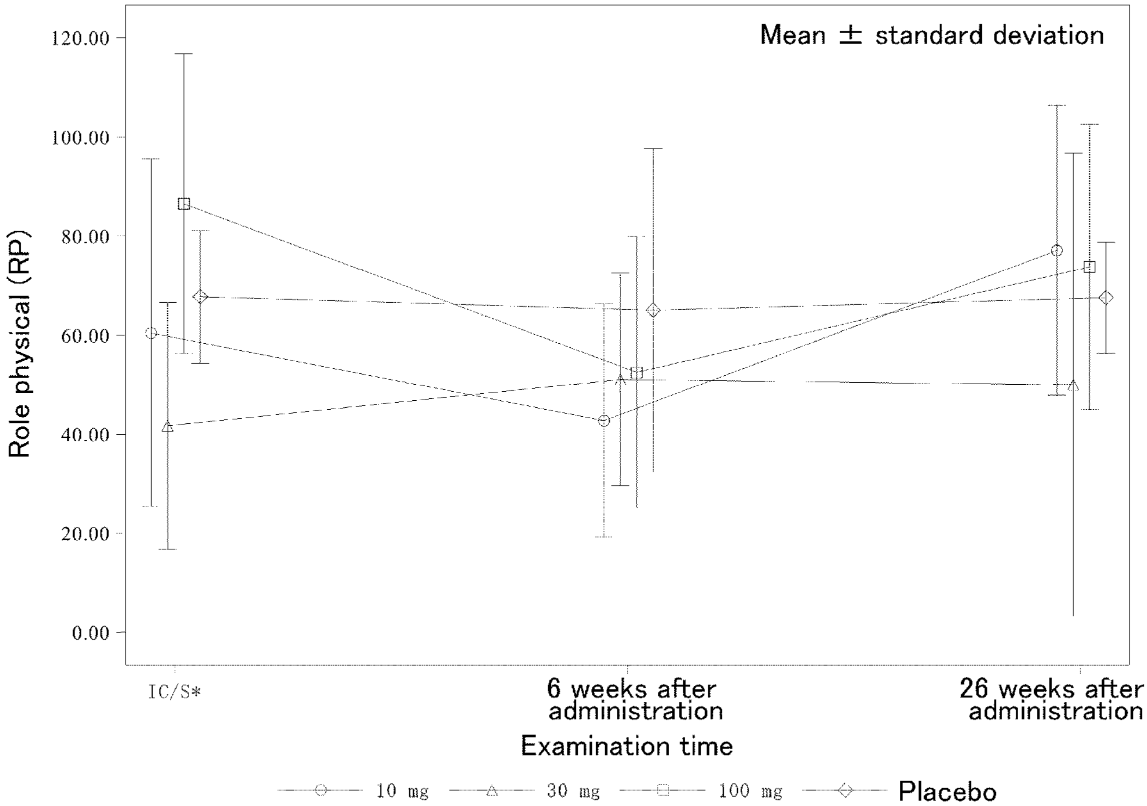


*: Consent acquisition/screening

Fig. 39C

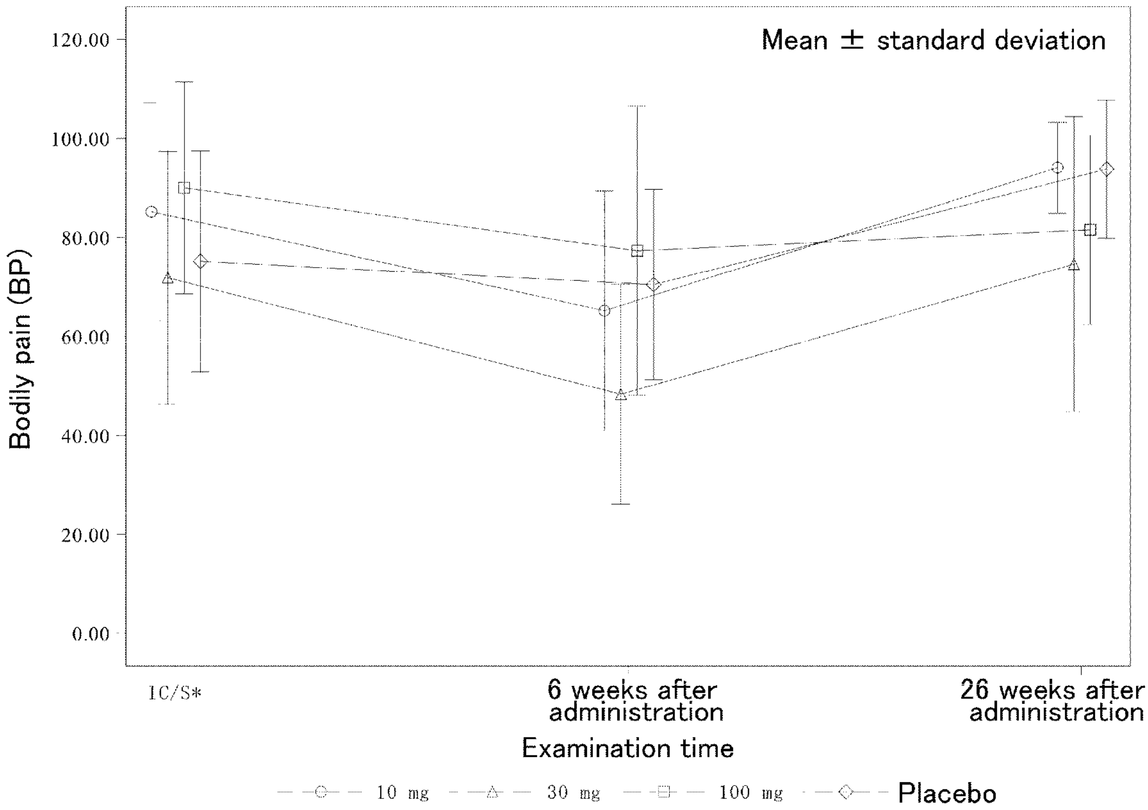


*: Consent acquisition/screening

Fig. 39D

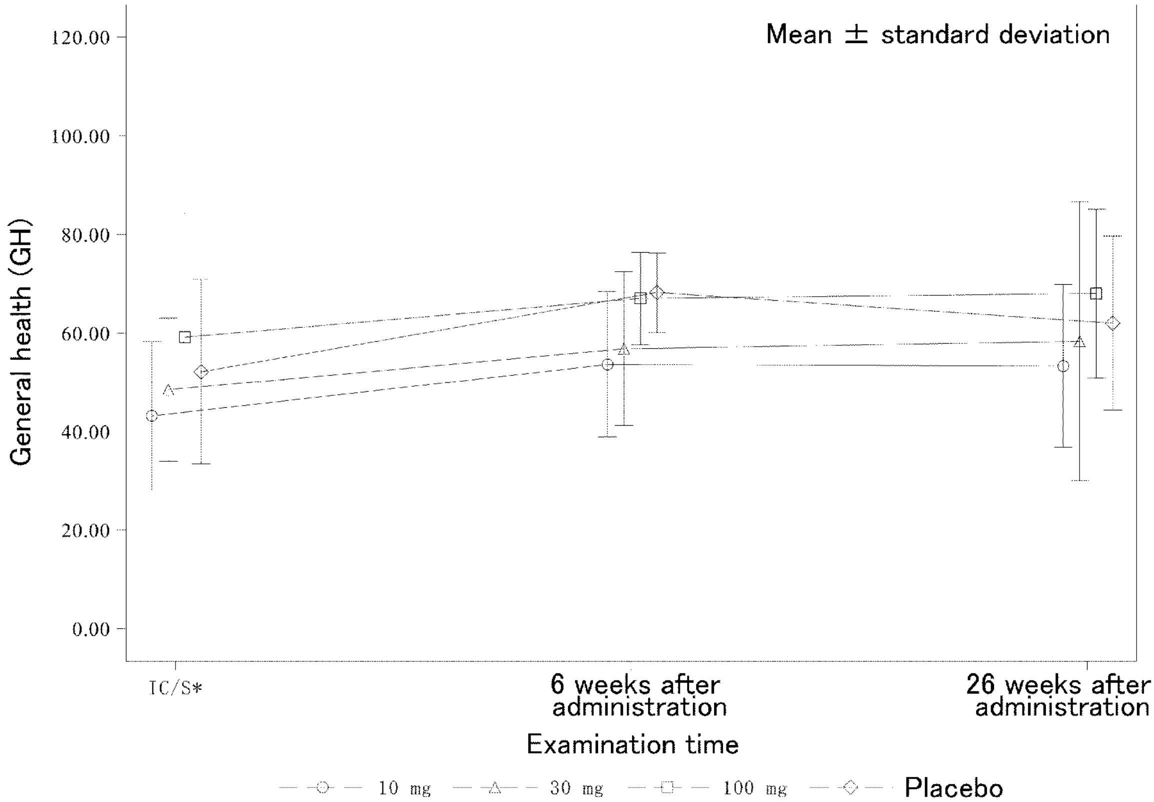


*: Consent acquisition/screening

Fig. 39E

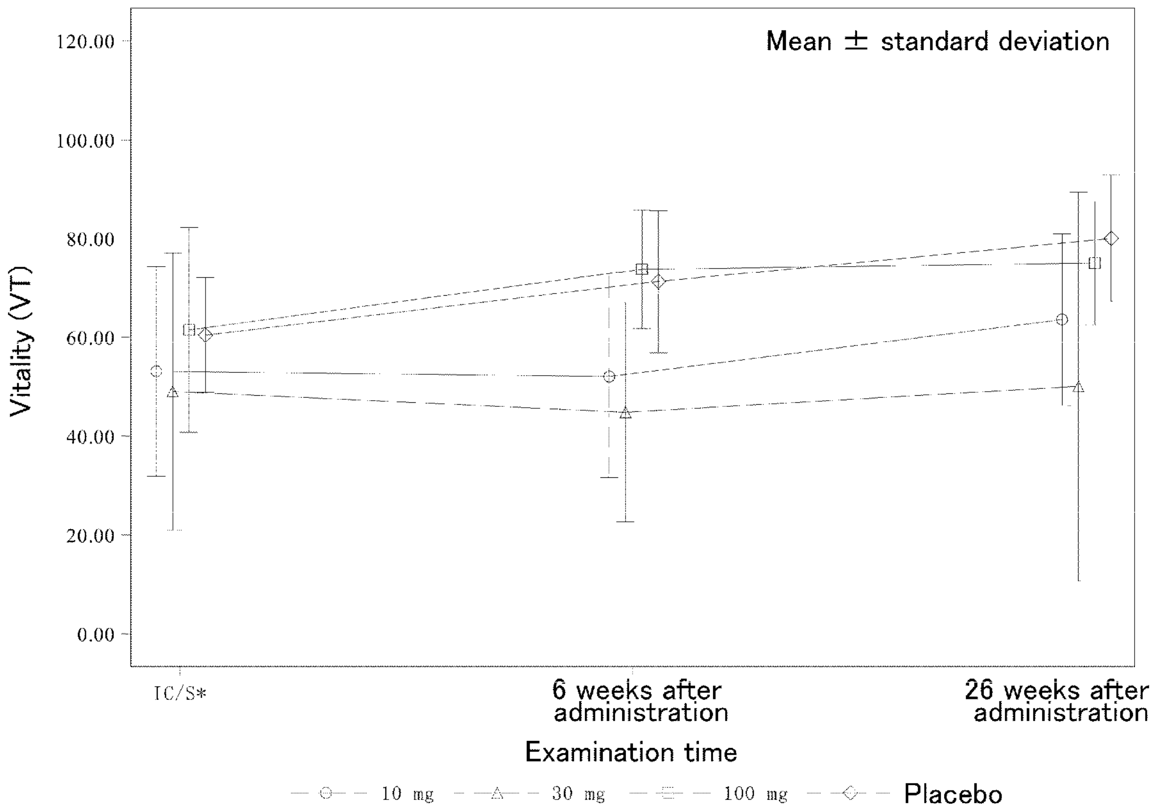


*: Consent acquisition/screening

Fig. 39F

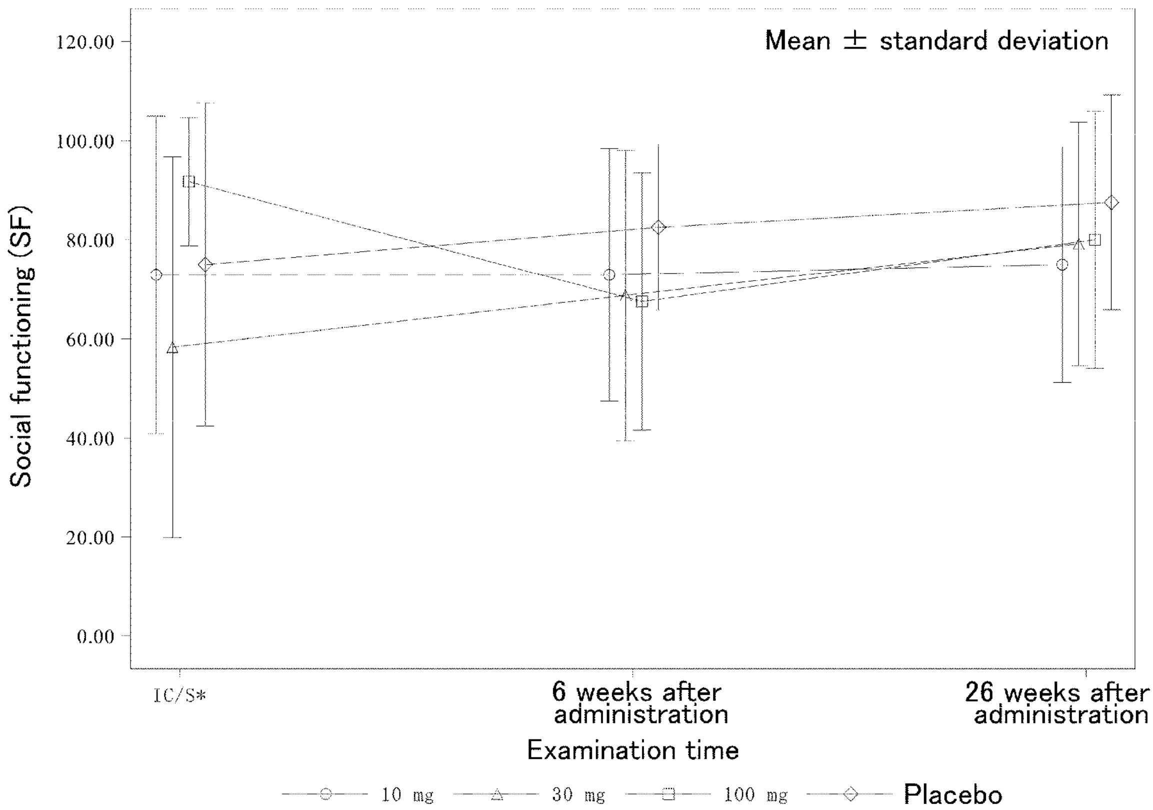


*: Consent acquisition/screening

Fig. 39G

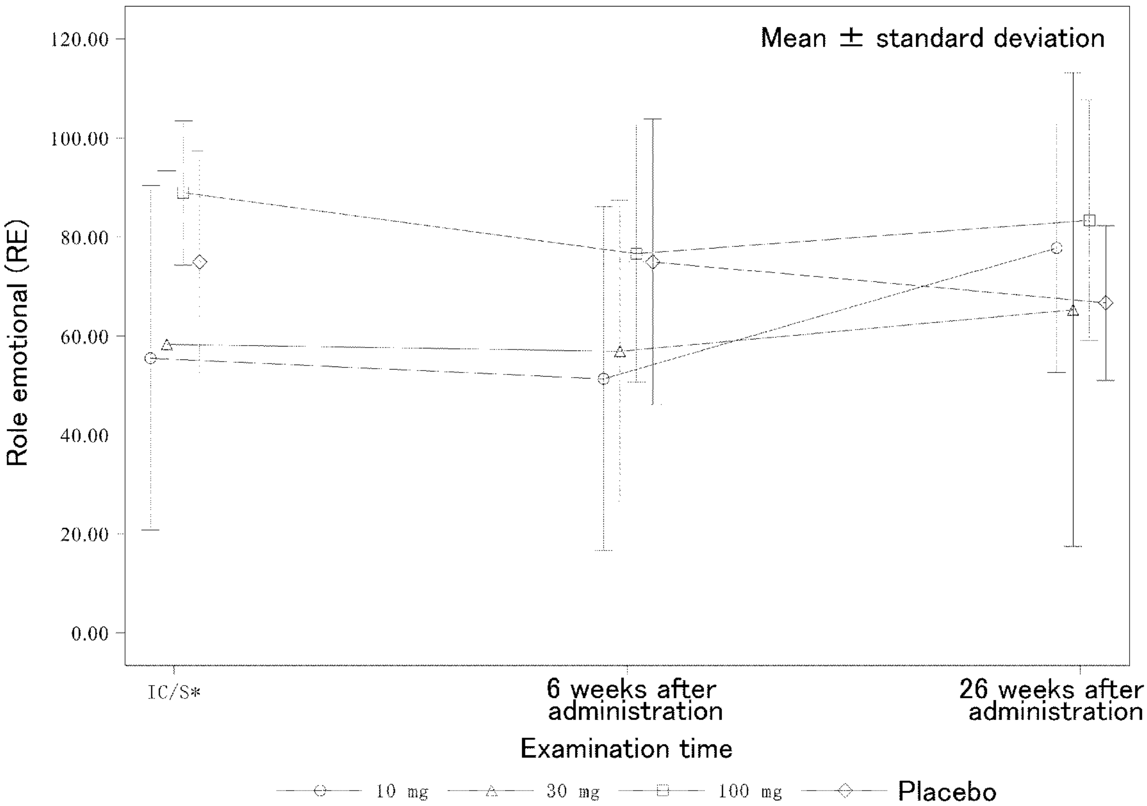


*: Consent acquisition/screening

Fig. 39H

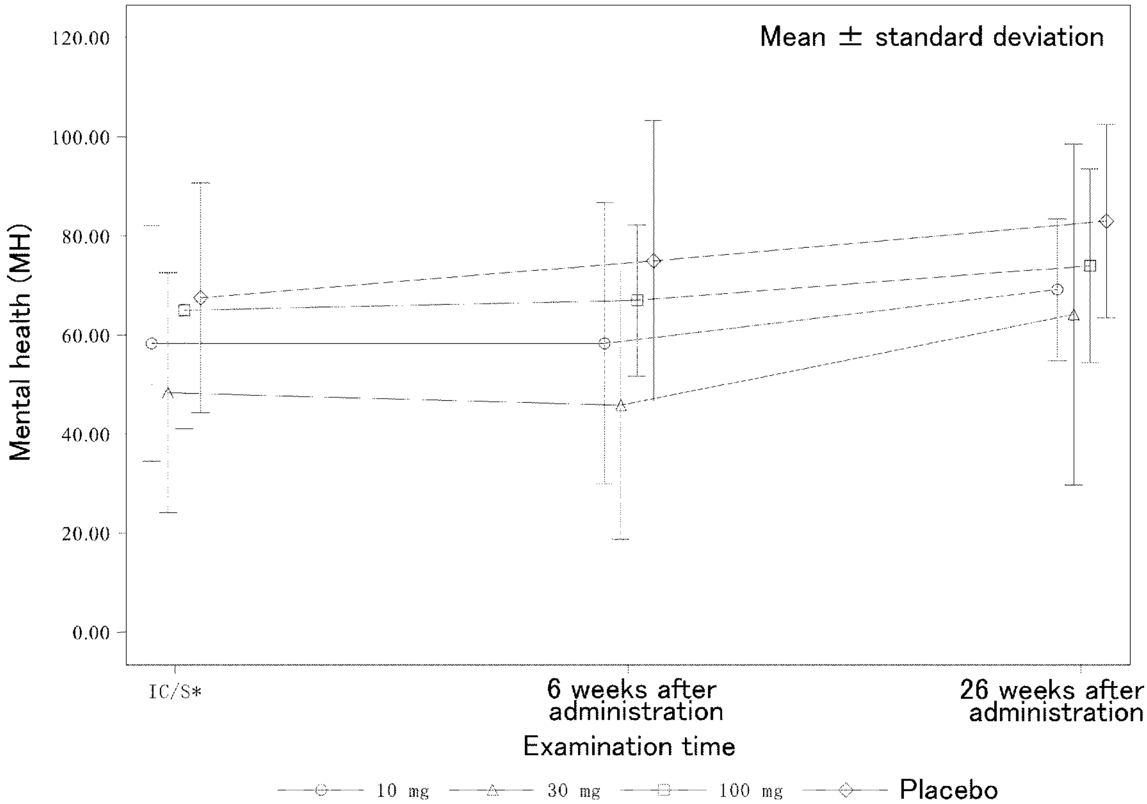


*: Consent acquisition/screening

Fig. 40A

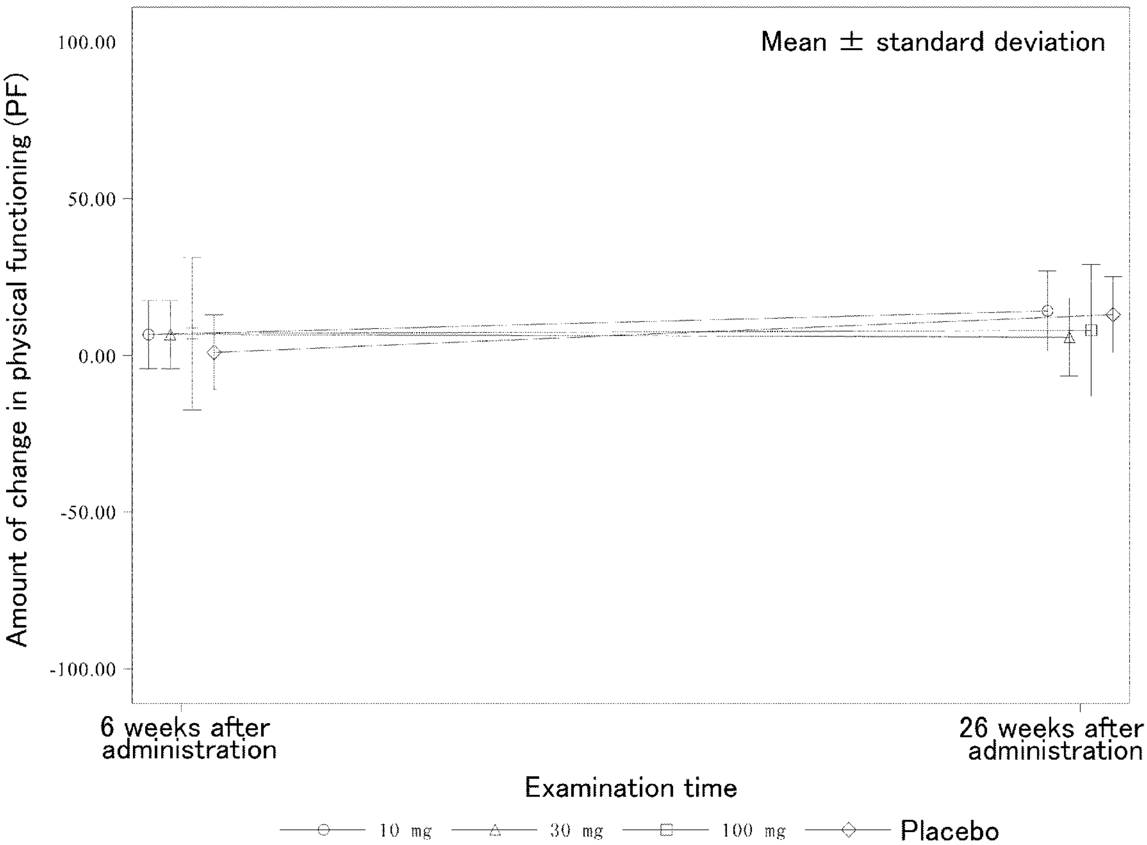
Mean ± standard deviation
100.00
50.00
0.00
-50.00
-100.00
Amount of change in physical functioning (PF)
6 weeks after administration
26 weeks after administration
Examination time
10 mg
30 mg
100 mg
Placebo

Fig. 40B

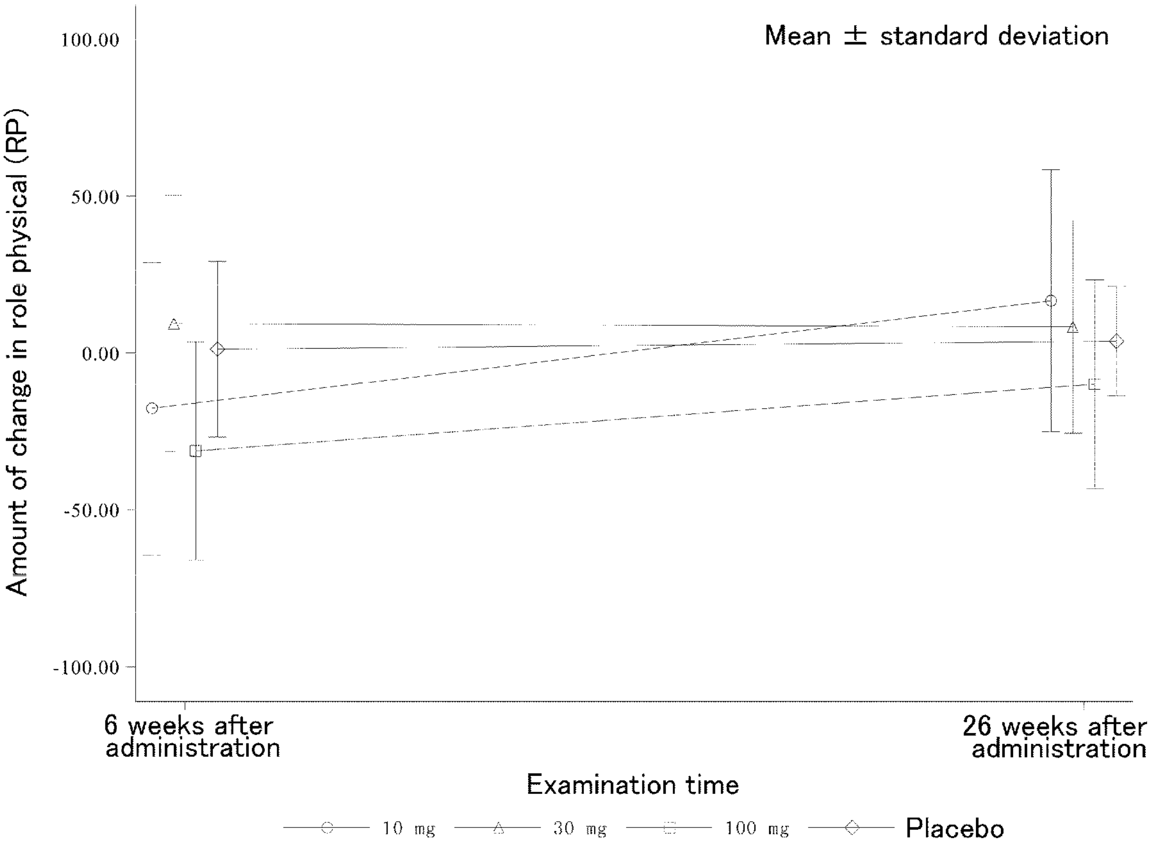
Mean ± standard deviation
100.00
50.00
0.00
-50.00
-100.00
Amount of change in role physical (RP)
6 weeks after administration
26 weeks after administration
Examination time
10 mg
30 mg
100 mg
Placebo

Fig. 40C

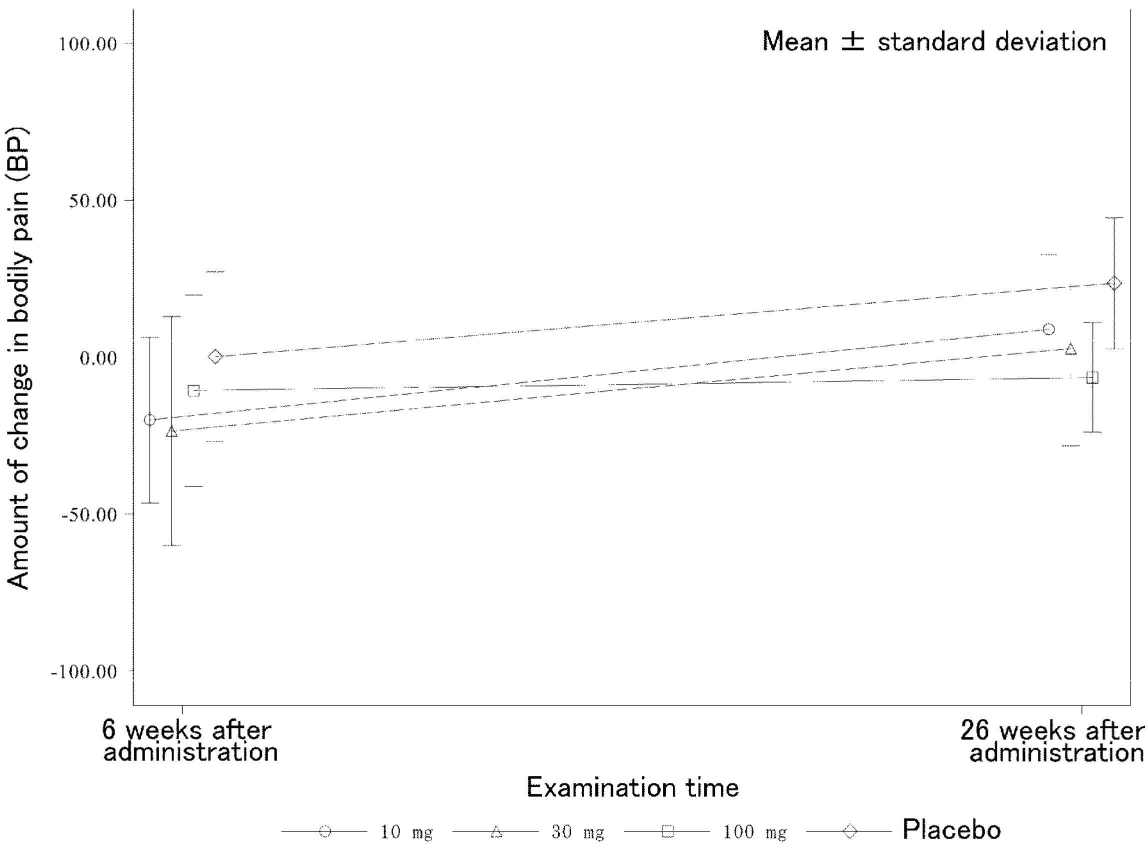
Mean ± standard deviation
100.00
50.00
0.00
-50.00
-100.00
Amount of change in bodily pain (BP)
6 weeks after administration
26 weeks after administration
Examination time
10 mg
30 mg
100 mg
Placebo

Fig. 40D

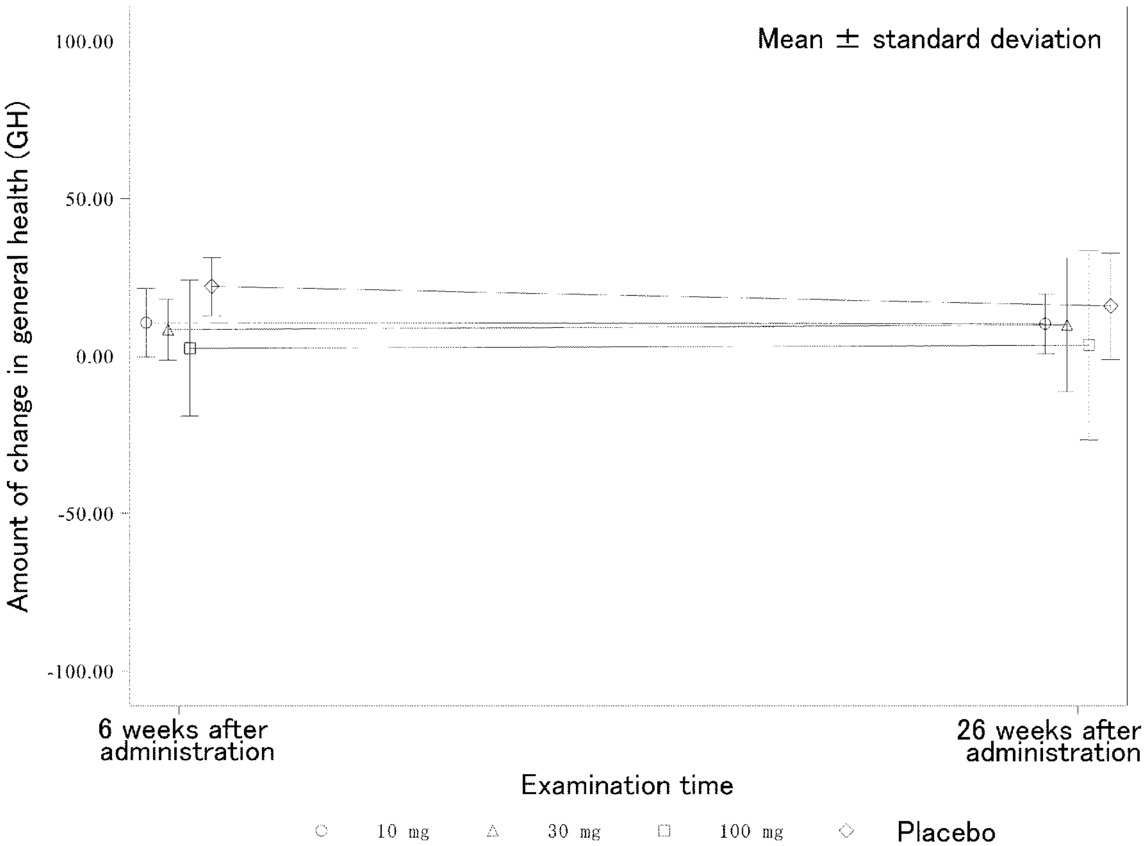
Mean ± standard deviation
100.00
50.00
0.00
-50.00
-100.00
Amount of change in general health (GH)
6 weeks after administration
26 weeks after administration
Examination time
10 mg
30 mg
100 mg
Placebo

Fig. 40E

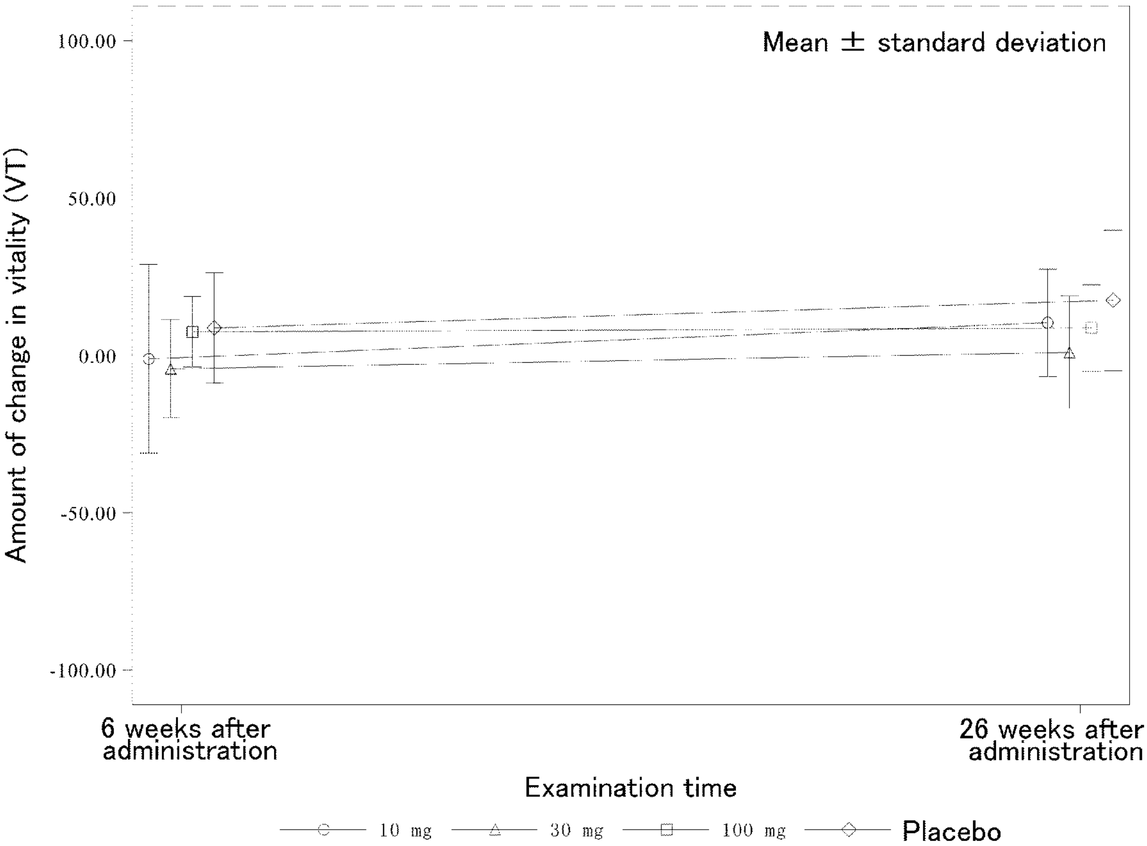
Mean ± standard deviation
100.00
50.00
0.00
-50.00
-100.00
Amount of change in vitality (VT)
6 weeks after administration
26 weeks after administration
Examination time
10 mg
30 mg
100 mg
Placebo

Fig. 40F

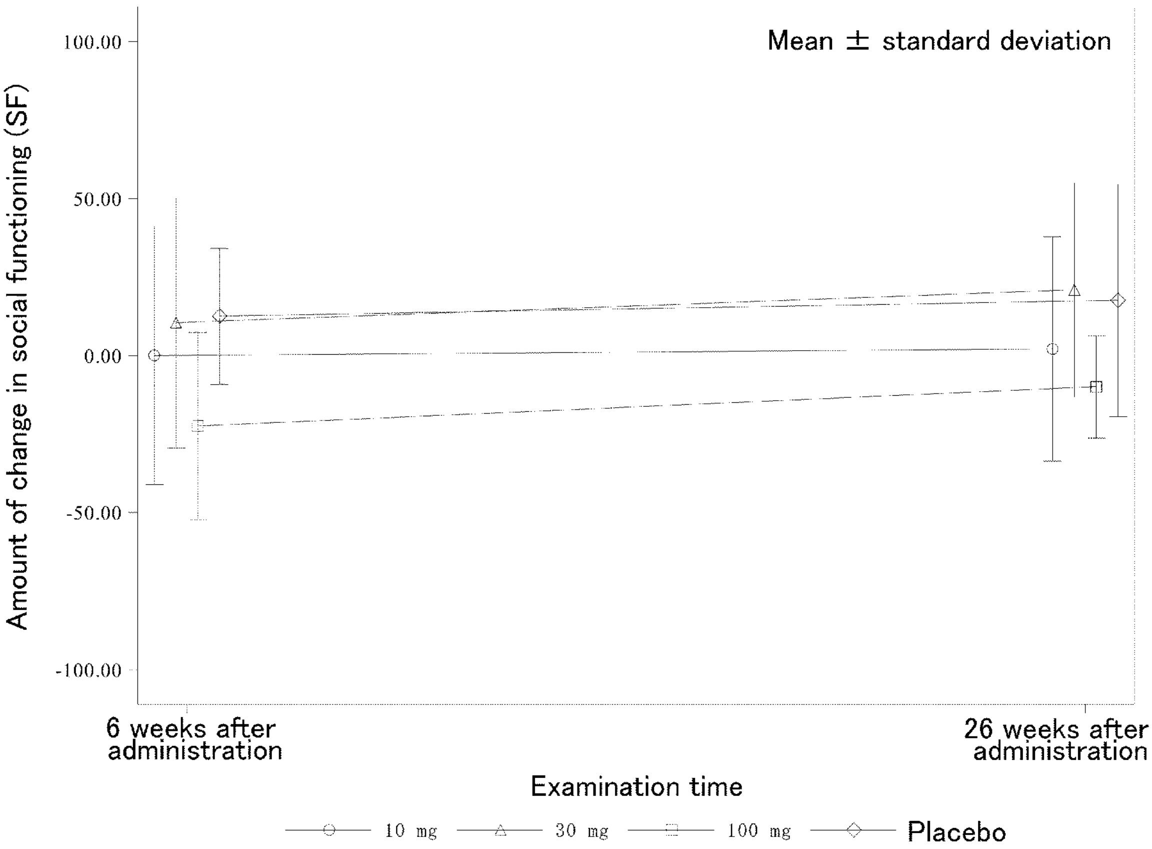
Mean ± standard deviation
Amount of change in social functioning (SF)
100.00
50.00
0.00
-50.00
-100.00
6 weeks after administration
26 weeks after administration
Examination time
10 mg
30 mg
100 mg
Placebo

Fig. 40G

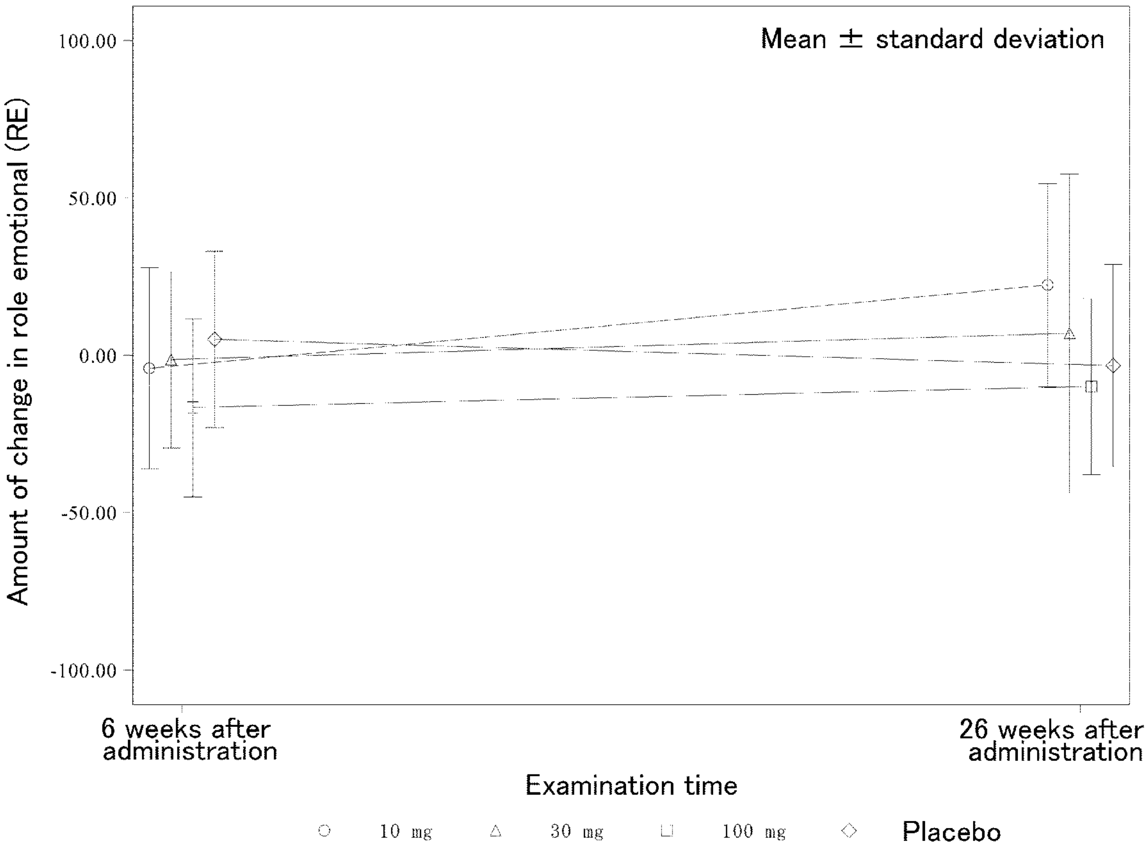
Mean ± standard deviation
100.00
50.00
0.00
-50.00
-100.00
Amount of change in role emotional (RE)
6 weeks after administration
26 weeks after administration
Examination time
10 mg
30 mg
100 mg
Placebo

Fig. 40H

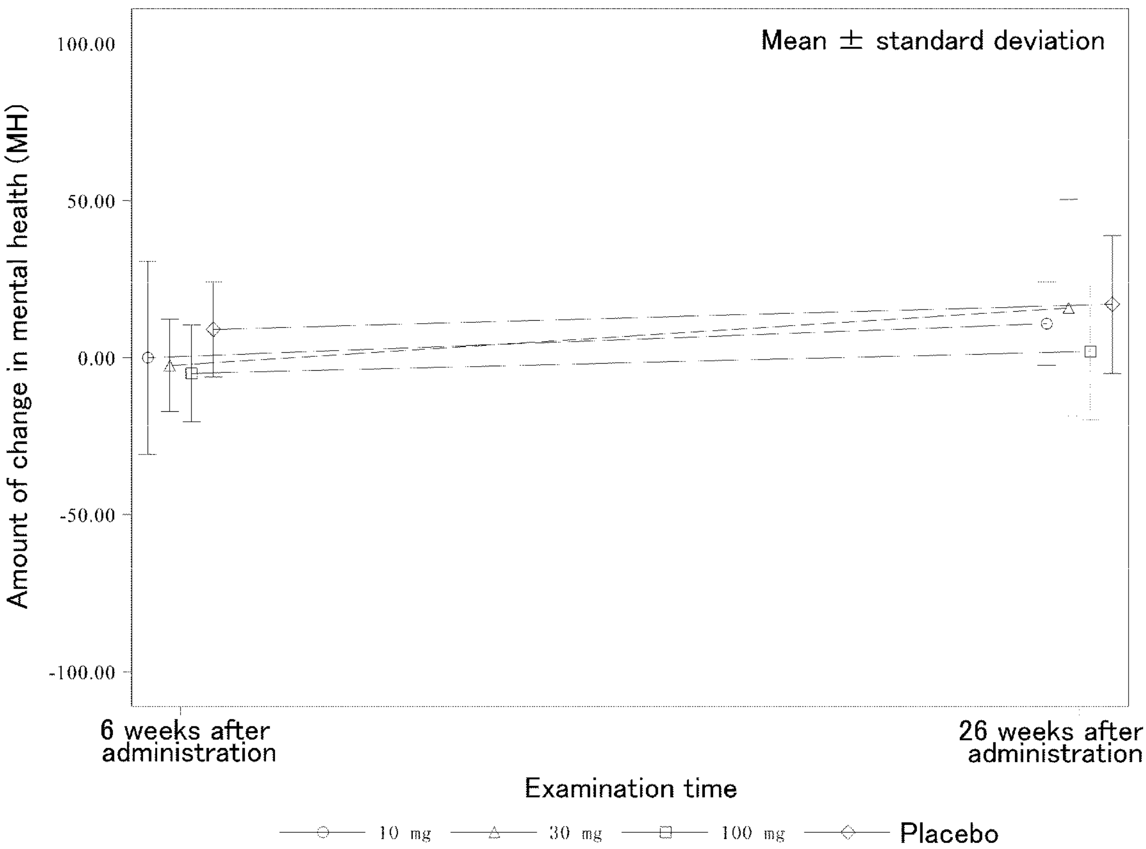
Mean ± standard deviation
100.00
50.00
0.00
-50.00
-100.00
Amount of change in mental health (MH)
6 weeks after administration
26 weeks after administration
Examination time
10 mg
30 mg
100 mg
Placebo

Fig. 41

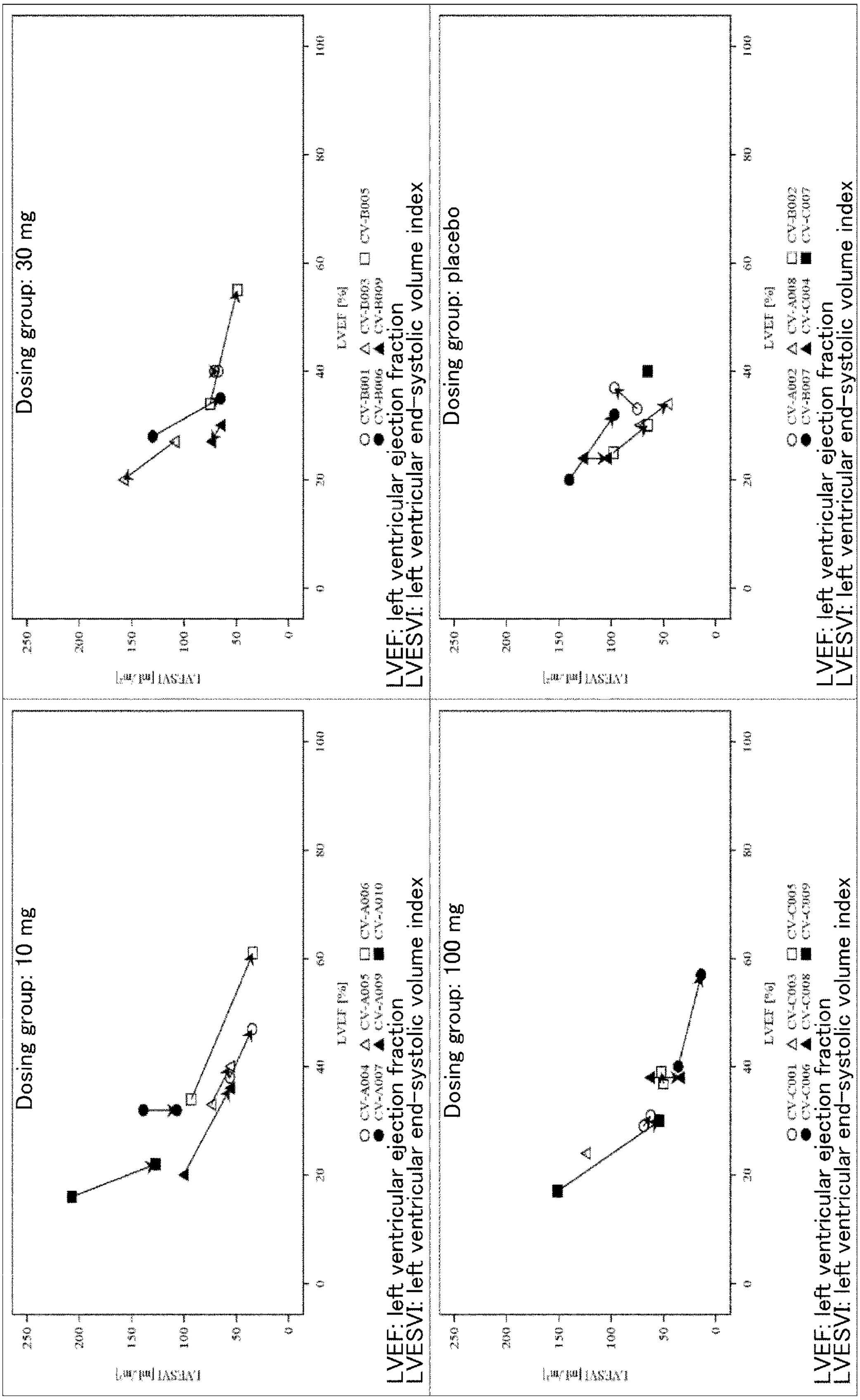
Dosing group: 10 mg
Dosing group: 30 mg
Dosing group: 100 mg
Dosing group: placebo
LVEF [%]
LVESVI [ml/m²]
CV-A004 CV-A005 CV-A006 CV-A007 CV-A009 CV-A010
CV-B001 CV-B003 CV-B005 CV-B006 CV-B009
CV-C001 CV-C003 CV-C005 CV-C006 CV-C008 CV-C009
CV-A002 CV-A008 CV-B002 CV-B007 CV-C004 CV-C007
LVEF: left ventricular ejection fraction
LVESVI: left ventricular end-systolic volume index

## INTERNATIONAL SEARCH REPORT

International application No.
**PCT/JP2022/014632**

**A. CLASSIFICATION OF SUBJECT MATTER**

***A61K 45/00***(2006.01)i; ***A61P 9/00***(2006.01)i; ***A61K 9/70***(2006.01)i; ***A61K 47/10***(2006.01)i; ***A61K 47/34***(2017.01)i; ***A61K 47/42***(2017.01)i; ***A61K 38/46***(2006.01)i; ***A61K 38/48***(2006.01)i; ***A61K 31/4406***(2006.01)i
FI: A61K45/00; A61P9/00; A61K47/34; A61K47/42; A61K31/4406; A61K9/70 401; A61K47/10; A61K38/46; A61K38/48 100

According to International Patent Classification (IPC) or to both national classification and IPC

**B. FIELDS SEARCHED**

Minimum documentation searched (classification system followed by classification symbols)

A61K45/00; A61P9/00; A61K9/70; A61K47/10; A61K47/34; A61K47/42; A61K38/46; A61K38/48; A61K31/4406

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2014/046065 A1 (OSAKA UNIVERSITY) 27 March 2014 (2014-03-27) claims, paragraphs [0048]-[0050], [0054], [0158], [0159], examples, preparation example 4, fig. 5 | 1-4, 6, 8-12, 14-16 |
| Y | | 1-16 |
| X | FUKUSHIMA, S. et al. A sustained-release drug-delivery system of synthetic prostacyclin agonist, ONO-1301SR: a new reagent to enhance cardiac tissue salvage and/or regeneration in the damaged heart, Heart Fail. Rev., 2015, vol. 20, pp. 401-413, DOI 10.1007/s10741-015-9477-8 abstract, page 402, right column, 2nd paragraph, page 406, left column to page 407, left column, 1st paragraph | 1, 2, 10-12, 14-16 |
| Y | | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 May 2022** | **07 June 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/014632**

**C. DOCUMENTS CONSIDERED TO BE RELEVANT**

| Category* | Citation of document, with indication, where appropriate, of the relevant passages | Relevant to claim No. |
|---|---|---|
| Y | UCHIDA, T. et al. Effect of antioxidants on the stability of ONO-1301, a novel long-acting prostacyclin agonist, loaded in PLGA microspheres. Journal of Microencapsulation, 2013, vol. 30, no. 3, pp. 245-256, DOI: 10.3109/02652048.2012.720721<br>abstract, page 245, right column to page 246, left column, page 254, right column, tables 1, 2, fig. 2, 8 | 1-16 |
| Y | 福嶌　五月,　他, 徐放性プロスタサイクリン作動薬を用いたCell-Free型再生医療の重症心不全への応用, Organ Biology, 2016, vol. 23, no. 2, pp.180-185 non-official translation (FUKUSHIMA, Satsuki et al. Applying cell-free cardiac regeneration therapy with sustained release prostacyclin to advanced cardiac failure.)<br>pp. 181-184, left column | 1-16 |
| A | HAZEKAWA, M. et al. The angiogenic effect of ONO-1301, a novel long-acting prostacyclin agonist loaded in PLGA microspheres prepared using different molecular weights of PLGA, in a murine sponge model, Drug Development and Industrial Pharmacy, 2014, vol. 40, no.11, pp. 1435-1442<br>abstract, page 1437, left column (i), fig. 2, 3 | 1-16 |
| A | SHIRASAKA, T. et al. A slow-releasing form of prostacyclin agonist (ONO1301SR) enhances endogenous secretion of multiple cardiotherapeutic cytokines and improves cardiac function in a rapid-pacing-induced model of canine heart failure, J. Thorac. Cardiovasc. Surg., 2013, vol. 146, pp. 413-421<br>abstract | 1-16 |
| A | ISHIMARU, K. et al. Synthetic prostacyclin agonist, ONO1301, enhances endogenous myocardial repair in a hamster model of dilated cardiomyopathy: A promising regenerative therapy for the failing heart, J. Thorac. Cardiovasc. Surg., 2013, vol. 146, pp. 1516-1525<br>abstract | 1-16 |

Form PCT/ISA/210 (second sheet) (January 2015)

**INTERNATIONAL SEARCH REPORT**
**Information on patent family members**

International application No.
**PCT/JP2022/014632**

| Patent document cited in search report | Publication date (day/month/year) | Patent family member(s) | Publication date (day/month/year) |
|---|---|---|---|
| WO 2014/046065 A1 | 27 March 2014 | US 2015/0231312 A1<br>claims, paragraphs [0051]-[0054], [0058], [0173], examples, fig. 5<br>EP 2898902 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS,* 176391-41-6
- *CHEMICAL ABSTRACTS,* 88475-69-8
- *CHEMICAL ABSTRACTS,* 475085-57-5
- *CHEMICAL ABSTRACTS,* 74397-12-9
- *CHEMICAL ABSTRACTS,* 475086-01-2